# EUROPEAN PATENT SPECIFICATION

(11) **EP 1 385 934 B1**
(45) Date of publication and mention of the grant of the patent: **09.12.2015**
(21) Application number: 02731415.2
(22) Date of filing: 16.04.2002
(51) Int. Cl.: C12N 5/10, C12N 15/52, C12N 15/63, C12N 1/20, C12N 9/00, C12N 15/00, C12N 15/09, C12N 15/70, C12N 15/74, C12N 15/82, C07H 21/02, C07H 21/04, C12P 7/62, C12P 7/64, C12P 19/62

(54) **PUFA POLYKETIDE SYNTHASE SYSTEMS AND USES THEREOF**
PUFA-POLYKETIDSYNTHASE-SYSTEME UND DEREN VERWENDUNGEN
SYSTEMES A POLYKETIDE SYNTHASE AGPI ET LEURS APPLICATIONS

(30) Priority: 16.04.2001 US 284066 P; 15.06.2001 US 298796 P; 18.09.2001 US 323269 P
(43) Date of publication of application: 04.02.2004
(60) Divisional application: 10178938.6
(73) Proprietor: DSM IP Assets B.V., 6411 TE Heerlen (NL)
(72) Inventor: METZ, James, G., Longmont, CO 80501 (US); BARCLAY, William, R., Boulder, CO 80303 (US); FLATT, James, H., Longmont, CO 80503 (US); KUNER, Jerry, M., Longmont, CO 80503 (US)
(74) Representative: D Young & Co LLP
(86) International application number: PCT/US2002/012254
(87) International publication number: WO 2002/083870

(56) References cited:
- WO-A1-98/55625
- WO-A2-00/42195
- US-A- 5 130 242
- DATABASE Geneseq [Online] 11 December 2000 (2000-12-11), "S. aggregatum PKS cluster ORF6 homolog DNA." XP002368912 retrieved from EBI accession no. GSN:AAA71567 Database accession no. AAA71567 -& DATABASE Geneseq [Online] 11 December 2000 (2000-12-11), "S. aggregatum PKS cluster ORF6 homolog protein." XP002368914 retrieved from EBI accession no. GSP:AAB10482 Database accession no. AAB10482 & WO 00/42195 A (CALGENE, LLC) 20 July 2000 (2000-07-20)
- NICHOLSON THOMAS P ET AL: "Design and utility of oligonucleotide gene probes for fungal polyketide synthases" CHEMISTRY AND BIOLOGY (LONDON), vol. 8, no. 2, February 2001 (2001-02), pages 157-178, XP002338562 ISSN: 1074-5521
- KEALEY JAMES T ET AL: "Production of a polyketide natural product in nonpolyketide-producing prokaryotic and eukaryotic hosts" PROCEEDINGS OF THE NATIONAL ACADEMY OF SCIENCES OF THE UNITED STATES OF AMERICA, vol. 95, no. 2, 20 January 1998 (1998-01-20), pages 505-509, XP002338563 ISSN: 0027-8424
- JEZ JOSEPH M ET AL: "Structural control of polyketide formation in plant-specific polyketide synthases" CHEMISTRY AND BIOLOGY (LONDON), vol. 7, no. 12, December 2000 (2000-12), pages 919-930, XP002338564 ISSN: 1074-5521
- CHUCK J-A ET AL: "MOLECULAR RECOGNITION OF DIKETIDE SUBSTRATES BY A BETA-KETOACYL- ACYL CARRIER PROTEIN SYNTHASE DOMAIN WITHIN A BIMODULAR POLYKETIDE SYNTHASE" CHEMISTRY AND BIOLOGY, CURRENT BIOLOGY, LONDON, GB, vol. 4, no. 10, 1997, pages 757-766, XP000884721 ISSN: 1074-5521
- WALLIS J G ET AL: "Polyunsaturated fatty acid synthesis: what will they think of next?" TIBS TRENDS IN BIOCHEMICAL SCIENCES, ELSEVIER PUBLICATION, CAMBRIDGE, EN, vol. 27, no. 9, 1 September 2002 (2002-09-01), pages 467-473, XP004378766 ISSN: 0968-0004
- METZ ET AL.: 'Production of polyunsaturated fatty acids by polyketide synthases in both prokaryotes and eukaryotes' SCIENCE vol. 293, July 2001, pages 290 - 293, XP002956819
- NAPIER J.A.: 'Plumbing the depths of PUFA biosynthesis: a novel polyketide synthase-like pathway from marine organisms' TRENDS IN PLANT SCIENCE vol. 7, no. 2, February 2002, pages 51 - 54, XP002956820
- CANE ET AL.: 'Harnessing the biosynthetic code: combinations, permutations and mutations' SCIENCE vol. 282, 1998, pages 63 - 68, XP000910223
- YAZAWA K.: 'Production of eicosapentaenoic acid from marine bacteria' LIPIDS vol. 31, no. SUPPLEMENT, 1996, pages S297 - S300, XP000996341

### SEQUENCE LISTING

<110> Metz, James Barclay, William Flatt, James Kuner, Jerry
<120> PUFA POLYKETIDE SYNTHASE SYSTEMS AND USES THEREOF
<130> 2997-29-PCT
<150> 60/284,066
   <151> 2001-04-16
<150> 60/298,796
   <151> 2001-06-15
<150> 60/323,269
   <151> 2001-09-18
<160> 37
<170> PatentIn version 3.1
<210> 1
   <211> 8730
   <212> DNA
   <213> Schizochytrium sp.
<220>
   <221> CDS
   <222> (1)..(8730)
   <223>
<400> 1
   atg gcg gcc cgt ctg cag gag caa aag gga ggc gag atg gat acc cgc        48
   Met Ala Ala Arg Leu Gln Glu Gln Lys Gly Gly Glu Met Asp Thr Arg
   1               5                   10                  15
   att gcc atc atc ggc atg tcg gcc atc ctc ccc tgc ggc acg acc gtg        96
   Ile Ala Ile Ile Gly Met Ser Ala Ile Leu Pro Cys Gly Thr Thr Val
               20                  25                  30
   cgc gag tcg tgg gag acc atc cgc gcc ggc atc gac tgc ctg tcg gat       144
   Arg Glu Ser Trp Glu Thr Ile Arg Ala Gly Ile Asp Cys Leu Ser Asp
           35                  40                  45
   ctc ccc gag gac cgc gtc gac gtg acg gcg tac ttt gac ccc gtc aag       192
   Leu Pro Glu Asp Arg Val Asp Val Thr Ala Tyr Phe Asp Pro Val Lys
       50                  55                  60
   acc acc aag gac aag atc tac tgc aag cgc ggt ggc ttc att ccc gag       240
   Thr Thr Lys Asp Lys Ile Tyr Cys Lys Arg Gly Gly Phe Ile Pro Glu
   65                  70                  75                  80
   tac gac ttt gac gcc cgc gag ttc gga ctc aac atg ttc cag atg gag       288
   Tyr Asp Phe Asp Ala Arg Glu Phe Gly Leu Asn Met Phe Gln Met Glu
                   85                  90                  95
   gac tcg gac gca aac cag acc atc tcg ctt ctc aag gtc aag gag gcc       336
   Asp Ser Asp Ala Asn Gln Thr Ile Ser Leu Leu Lys Val Lys Glu Ala
               100                 105                 110
   ctc cag gac gcc ggc atc gac gcc ctc ggc aag gaa aag aag aac atc       384
   Leu Gln Asp Ala Gly Ile Asp Ala Leu Gly Lys Glu Lys Lys Asn Ile
           115                 120                 125
   ggc tgc gtg ctc ggc att ggc ggc ggc caa aag tcc agc cac gag ttc       432
   Gly Cys Val Leu Gly Ile Gly Gly Gly Gln Lys Ser Ser His Glu Phe
       130                 135                 140
   tac tcg cgc ctt aat tat gtt gtc gtg gag aag gtc ctc cgc aag atg       480
   Tyr Ser Arg Leu Asn Tyr Val Val Val Glu Lys Val Leu Arg Lys Met
   145                 150                 155                 160
   ggc atg ccc gag gag gac gtc aag gtc gcc gtc gaa aag tac aag gcc       528
   Gly Met Pro Glu Glu Asp Val Lys Val Ala Val Glu Lys Tyr Lys Ala
                   165                 170                 175
   aac ttc ccc gag tgg cgc ctc gac tcc ttc cct ggc ttc ctc ggc aac      576
   Asn Phe Pro Glu Trp Arg Leu Asp Ser Phe Pro Gly Phe Leu Gly Asn
               180                 185                 190
   gtc acc gcc ggt cgc tgc acc aac acc ttc aac ctc gac ggc atg aac      624
   Val Thr Ala Gly Arg Cys Thr Asn Thr Phe Asn Leu Asp Gly Met Asn
           195                 200                 205
   tgc gtt gtc gac gcc gca tgc gcc tcg tcc ctc atc gcc gtc aag gtc      672
   Cys Val Val Asp Ala Ala Cys Ala Ser Ser Leu Ile Ala Val Lys Val
       210                 215                 220
   gcc atc gac gag ctg ctc tac ggt gac tgc gac atg atg gtc acc ggt      720
   Ala Ile Asp Glu Leu Leu Tyr Gly Asp Cys Asp Met Met Val Thr Gly
   225                 230                 235                 240
   gcc acc tgc acg gat aac tcc atc ggc atg tac atg gcc ttc tcc aag      768
   Ala Thr Cys Thr Asp Asn Ser Ile Gly Met Tyr Met Ala Phe Ser Lys
                   245                 250                 255
   acc ccc gtg ttc tcc acg gac ccc agc gtg cgc gcc tac gac gaa aag      816
   Thr Pro Val Phe Ser Thr Asp Pro Ser Val Arg Ala Tyr Asp Glu Lys
               260                 265                 270
   aca aag ggc atg ctc atc ggc gag ggc tcc gcc atg ctc gtc ctc aag      864
   Thr Lys Gly Met Leu Ile Gly Glu Gly Ser Ala Met Leu Val Leu Lys
           275                 280                 285
   cgc tac gcc gac gcc gtc cgc gac ggc gat gag atc cac gct gtt att      912
   Arg Tyr Ala Asp Ala Val Arg Asp Gly Asp Glu Ile His Ala Val Ile
       290                 295                 300
   cgc ggc tgc gcc tcc tcc agt gat ggc aag gcc gcc ggc atc tac acg      960
   Arg Gly Cys Ala Ser Ser Ser Asp Gly Lys Ala Ala Gly Ile Tyr Thr
   305                 310                 315                 320
   ccc acc att tcg ggc cag gag gag gcc ctc cgc cgc gcc tac aac cgc     1008
   Pro Thr Ile Ser Gly Gln Glu Glu Ala Leu Arg Arg Ala Tyr Asn Arg
                   325                 330                 335
   gcc tgt gtc gac ccg gcc acc gtc act ctc gtc gag ggt cac ggc acc     1056
   Ala Cys Val Asp Pro Ala Thr Val Thr Leu Val Glu Gly His Gly Thr
               340                 345                 350
   ggt act ccc gtt ggc gac cgc atc gag ctc acc gcc ttg cgc aac ctc     1104
   Gly Thr Pro Val Gly Asp Arg Ile Glu Leu Thr Ala Leu Arg Asn Leu
           355                 360                 365
   ttt gac aag gcc tac ggc gag ggc aac acc gaa aag gtc gct gtg ggc     1152
   Phe Asp Lys Ala Tyr Gly Glu Gly Asn Thr Glu Lys Val Ala Val Gly
       370                 375                 380
   agc atc aag tcc agc atc ggc cat ctc aag gcc gtc gcc ggt ctc gcc     1200
   Ser Ile Lys Ser Ser Ile Gly His Leu Lys Ala Val Ala Gly Leu Ala
   385                 390                 395                 400
   ggt atg atc aag gtc atc atg gcg ctc aag cac aag act ctc ccg ggc     1248
   Gly Met Ile Lys Val Ile Met Ala Leu Lys His Lys Thr Leu Pro Gly
                   405                 410                 415
   acc atc aac gtc gac aac cca ccc aac ctc tac gac aac acg ccc atc     1296
   Thr Ile Asn Val Asp Asn Pro Pro Asn Leu Tyr Asp Asn Thr Pro Ile
               420                 425                 430
   aac gag tcc tcg ctc tac att aac acc atg aac cgc ccc tgg ttc ccg     1344
   Asn Glu Ser Ser Leu Tyr Ile Asn Thr Met Asn Arg Pro Trp Phe Pro
           435                 440                 445
   ccc cct ggt gtg ccc cgc cgc gcc ggc att tcg agc ttt ggc ttt ggt     1392
   Pro Pro Gly Val Pro Arg Arg Ala Gly Ile Ser Ser Phe Gly Phe Gly
       450                 455                 460
   ggc gcc aac tac cac gcc gtc ctc gag gag gcc gag ccc gag cac acg     1440
   Gly Ala Asn Tyr His Ala Val Leu Glu Glu Ala Glu Pro Glu His Thr
   465                 470                 475                 480
   acc gcg tac cgc ctc aac aag cgc ccg cag ccc gtg ctc atg atg gcc     1488
   Thr Ala Tyr Arg Leu Asn Lys Arg Pro Gln Pro Val Leu Met Met Ala
                   485                 490                 495
   gcc acg ccc gcg gcc ctc cag tcg ctc tgc gag gcc cag ctc aag gag     1536
   Ala Thr Pro Ala Ala Leu Gln Ser Leu Cys Glu Ala Gln Leu Lys Glu
               500                 505                 510
   ttc gag gcc gcc atc aag gag aac gag acc gtc aag aac acc gcc tac      1584
   Phe Glu Ala Ala Ile Lys Glu Asn Glu Thr Val Lys Asn Thr Ala Tyr
           515                 520                 525
   atc aag tgc gtc aag ttc ggc gag cag ttc aaa ttc cct ggc tcc atc      1632
   Ile Lys Cys Val Lys Phe Gly Glu Gln Phe Lys Phe Pro Gly Ser Ile
       530                 535                 540
   ccg gcc aca aac gcg cgc ctc ggc ttc ctc gtc aag gat gct gag gat      1680
   Pro Ala Thr Asn Ala Arg Leu Gly Phe Leu Val Lys Asp Ala Glu Asp
   545                 550                 555                 560
   gcc tgc tcc acc ctc cgt gcc atc tgc gcc caa ttc gcc aag gat gtc      1728
   Ala Cys Ser Thr Leu Arg Ala Ile Cys Ala Gln Phe Ala Lys Asp Val
                   565                 570                 575
   acc aag gag gcc tgg cgc ctc ccc cgc gag ggc gtc agc ttc cgc gcc      1776
   Thr Lys Glu Ala Trp Arg Leu Pro Arg Glu Gly Val Ser Phe Arg Ala
               580                 585                 590
   aag ggc atc gcc acc aac ggc gct gtc gcc gcg ctc ttc tcc ggc cag      1824
   Lys Gly Ile Ala Thr Asn Gly Ala Val Ala Ala Leu Phe Ser Gly Gln
           595                 600                 605
   ggc gcg cag tac acg cac atg ttt agc gag gtg gcc atg aac tgg ccc      1872
   Gly Ala Gln Tyr Thr His Met Phe Ser Glu Val Ala Met Asn Trp Pro
       610                 615                 620
   cag ttc cgc cag agc att gcc gcc atg gac gcc gcc cag tcc aag gtc      1920
   Gln Phe Arg Gln Ser Ile Ala Ala Met Asp Ala Ala Gln Ser Lys Val
   625                 630                 635                 640
   gct gga agc gac aag gac ttt gag cgc gtc tcc cag gtc ctc tac ccg      1968
   Ala Gly Ser Asp Lys Asp Phe Glu Arg Val Ser Gln Val Leu Tyr Pro
                   645                 650                 655
   cgc aag ccg tac gag cgt gag ccc gag cag aac ccc aag aag atc tcc      2016
   Arg Lys Pro Tyr Glu Arg Glu Pro Glu Gln Asn Pro Lys Lys Ile Ser
               660                 665                 670
   ctc acc gcc tac tcg cag ccc tcg acc ctg gcc tgc gct ctc ggt gcc      2064
   Leu Thr Ala Tyr Ser Gln Pro Ser Thr Leu Ala Cys Ala Leu Gly Ala
           675                 680                 685
   ttt gag atc ttc aag gag gcc ggc ttc acc ccg gac ttt gcc gcc ggc      2112
   Phe Glu Ile Phe Lys Glu Ala Gly Phe Thr Pro Asp Phe Ala Ala Gly
       690                 695                 700
   cat tcg ctc ggt gag ttc gcc gcc ctc tac gcc gcg ggc tgc gtc gac      2160
   His Ser Leu Gly Glu Phe Ala Ala Leu Tyr Ala Ala Gly Cys Val Asp
   705                 710                 715                 720
   cgc gac gag ctc ttt gag ctt gtc tgc cgc cgc gcc cgc atc atg ggc      2208
   Arg Asp Glu Leu Phe Glu Leu Val Cys Arg Arg Ala Arg Ile Met Gly
                   725                 730                 735
   ggc aag gac gca ccg gcc acc ccc aag gga tgc atg gcc gcc gtc att      2256
   Gly Lys Asp Ala Pro Ala Thr Pro Lys Gly Cys Met Ala Ala Val Ile
               740                 745                 750
   ggc ccc aac gcc gag aac atc aag gtc cag gcc gcc aac gtc tgg ctc      2304
   Gly Pro Asn Ala Glu Asn Ile Lys Val Gln Ala Ala Asn Val Trp Leu
           755                 760                 765
   ggc aac tcc aac tcg cct tcg cag acc gtc atc acc ggc tcc gtc gaa      2352
   Gly Asn Ser Asn Ser Pro Ser Gln Thr Val Ile Thr Gly Ser Val Glu
       770                 775                 780
   ggt atc cag gcc gag agc gcc cgc ctc cag aag gag ggc ttc cgc gtc      2400
   Gly Ile Gln Ala Glu Ser Ala Arg Leu Gln Lys Glu Gly Phe Arg Val
   785                 790                 795                 800
   gtg cct ctt gcc tgc gag agc gcc ttc cac tcg ccc cag atg gag aac      2448
   Val Pro Leu Ala Cys Glu Ser Ala Phe His Ser Pro Gln Met Glu Asn
                   805                 810                 815
   gcc tcg tcg gcc ttc aag gac gtc atc tcc aag gtc tcc ttc cgc acc      2496
   Ala Ser Ser Ala Phe Lys Asp Val Ile Ser Lys Val Ser Phe Arg Thr
               820                 825                 830
   ccc aag gcc gag acc aag ctc ttc agc aac gtc tct ggc gag acc tac      2544
   Pro Lys Ala Glu Thr Lys Leu Phe Ser Asn Val Ser Gly Glu Thr Tyr
           835                 840                 845
   ccc acg gac gcc cgc gag atg ctt acg cag cac atg acc agc agc gtc      2592
   Pro Thr Asp Ala Arg Glu Met Leu Thr Gln His Met Thr Ser Ser Val
       850                 855                 860
   aag ttc ctc acc cag gtc cgc aac atg cac cag gcc ggt gcg cgc atc      2640
   Lys Phe Leu Thr Gln Val Arg Asn Met His Gln Ala Gly Ala Arg Ile
   865                 870                 875                 880
   ttt gtc gag ttc gga ccc aag cag gtg ctc tcc aag ctt gtc tcc gag      2688
   Phe Val Glu Phe Gly Pro Lys Gln Val Leu Ser Lys Leu Val Ser Glu
                   885                 890                 895
   acc ctc aag gat gac ccc tcg gtt gtc acc gtc tct gtc aac ccg gcc      2736
   Thr Leu Lys Asp Asp Pro Ser Val Val Thr Val Ser Val Asn Pro Ala
               900                 905                 910
   tcg ggc acg gat tcg gac atc cag ctc cgc gac gcg gcc gtc cag ctc      2784
   Ser Gly Thr Asp Ser Asp Ile Gln Leu Arg Asp Ala Ala Val Gln Leu
           915                 920                 925
   gtt gtc gct ggc gtc aac ctt cag ggc ttt gac aag tgg gac gcc ccc      2832
   Val Val Ala Gly Val Asn Leu Gln Gly Phe Asp Lys Trp Asp Ala Pro
       930                 935                 940
   gat gcc acc cgc atg cag gcc atc aag aag aag cgc act acc ctc cgc      2880
   Asp Ala Thr Arg Met Gln Ala Ile Lys Lys Lys Arg Thr Thr Leu Arg
   945                 950                 955                 960
   ctt tcg gcc gcc acc tac gtc tcg gac aag acc aag aag gtc cgc gac      2928
   Leu Ser Ala Ala Thr Tyr Val Ser Asp Lys Thr Lys Lys Val Arg Asp
                   965                 970                 975
   gcc gcc atg aac gat ggc cgc tgc gtc acc tac ctc aag ggc gcc gca      2976
   Ala Ala Met Asn Asp Gly Arg Cys Val Thr Tyr Leu Lys Gly Ala Ala
               980                 985                 990
   ccg ctc atc aag gcc ccg gag ccc  gtt gtc gac gag gcc  gcc aag cgc    3024
   Pro Leu Ile Lys Ala Pro Glu Pro  Val Val Asp Glu Ala  Ala Lys Arg
           995                 1000                 1005
   gag gcc  gag cgt ctc cag aag  gag ctt cag gat gcc  cag cgc cag      3069
   Glu Ala  Glu Arg Leu Gln Lys  Glu Leu Gln Asp Ala  Gln Arg Gln
       1010                 1015                 1020
   ctc gac  gac gcc aag cgc gcc  gcc gcc gag gcc aac  tcc aag ctc      3114
   Leu Asp  Asp Ala Lys Arg Ala  Ala Ala Glu Ala Asn  Ser Lys Leu
       1025                 1030                 1035
   gcc gct  gcc aag gag gag gcc  aag acc gcc gct gct  tcg gcc aag      3159
   Ala Ala  Ala Lys Glu Glu Ala  Lys Thr Ala Ala Ala  Ser Ala Lys
       1040                 1045                 1050
   ccc gca  gtt gac act gct gtt  gtc gaa aag cat cgt  gcc atc ctc      3204
   Pro Ala  Val Asp Thr Ala Val  Val Glu Lys His Arg  Ala Ile Leu
       1055                 1060                 1065
   aag tcc  atg ctc gcg gag ctc  gat ggc tac gga tcg  gtc gac gct      3249
   Lys Ser  Met Leu Ala Glu Leu  Asp Gly Tyr Gly Ser  Val Asp Ala
       1070                 1075                 1080
   tct tcc  ctc cag cag cag cag  cag cag cag acg gcc  ccc gcc ccg      3294
   Ser Ser  Leu Gln Gln Gln Gln  Gln Gln Gln Thr Ala  Pro Ala Pro
       1085                 1090                 1095
   gtc aag  gct gct gcg cct gcc  gcc ccc gtt gcc tcg  gcc cct gcc      3339
   Val Lys  Ala Ala Ala Pro Ala  Ala Pro Val Ala Ser  Ala Pro Ala
       1100                 1105                 1110
   ccg gct  gtc tcg aac gag ctt  ctt gag aag gcc gag  act gtc gtc      3384
   Pro Ala  Val Ser Asn Glu Leu  Leu Glu Lys Ala Glu  Thr Val Val
       1115                 1120                 1125
   atg gag  gtc ctc gcc gcc aag  acc ggc tac gag acc  gac atg atc      3429
   Met Glu  Val Leu Ala Ala Lys  Thr Gly Tyr Glu Thr  Asp Met Ile
       1130                 1135                 1140
   gag gct  gac atg gag ctc gag  acc gag ctc ggc att  gac tcc atc      3474
   Glu Ala  Asp Met Glu Leu Glu  Thr Glu Leu Gly Ile  Asp Ser Ile
       1145                 1150                 1155
   aag cgt  gtc gag atc ctc tcc  gag gtc cag gcc atg  ctc aat gtc      3519
   Lys Arg  Val Glu Ile Leu Ser  Glu Val Gln Ala Met  Leu Asn Val
       1160                 1165                 1170
   gag gcc  aag gat gtc gat gcc  ctc agc cgc act cgc  act gtt ggt      3564
   Glu Ala  Lys Asp Val Asp Ala  Leu Ser Arg Thr Arg  Thr Val Gly
       1175                 1180                 1185
   gag gtt  gtc aac gcc atg aag  gcc gag atc gct ggc  agc tct gcc      3609
   Glu Val  Val Asn Ala Met Lys  Ala Glu Ile Ala Gly  Ser Ser Ala
       1190                 1195                 1200
   ccg gcg  cct gct gcc gct gct  ccg gct ccg gcc aag  gct gcc cct      3654
   Pro Ala  Pro Ala Ala Ala Ala  Pro Ala Pro Ala Lys  Ala Ala Pro
       1205                 1210                 1215
   gcc gcc  gct gcg cct gct gtc  tcg aac gag ctt ctc  gag aag gcc      3699
   Ala Ala  Ala Ala Pro Ala Val  Ser Asn Glu Leu Leu  Glu Lys Ala
       1220                 1225                 1230
   gag acc  gtc gtc atg gag gtc  ctc gcc gcc aag act  ggc tac gag      3744
   Glu Thr  Val Val Met Glu Val  Leu Ala Ala Lys Thr  Gly Tyr Glu
       1235                 1240                 1245
   act gac  atg atc gag tcc gac  atg gag ctc gag act  gag ctc ggc      3789
   Thr Asp  Met Ile Glu Ser Asp  Met Glu Leu Glu Thr  Glu Leu Gly
       1250                 1255                 1260
   att gac  tcc atc aag cgt gtc  gag atc ctc tcc gag  gtt cag gcc      3834
   Ile Asp  Ser Ile Lys Arg Val  Glu Ile Leu Ser Glu  Val Gln Ala
       1265                 1270                 1275
   atg ctc  aac gtc gag gcc aag  gac gtc gac gct ctc  agc cgc act      3879
   Met Leu  Asn Val Glu Ala Lys  Asp Val Asp Ala Leu  Ser Arg Thr
       1280                 1285                 1290
   cgc act  gtg ggt gag gtc gtc  aac gcc atg aag gct  gag atc gct      3924
   Arg Thr  Val Gly Glu Val Val  Asn Ala Met Lys Ala  Glu Ile Ala
       1295                 1300                 1305
   ggt ggc  tct gcc ccg gcg cct  gcc gcc gct gcc cca  ggt ccg gct      3969
   Gly Gly  Ser Ala Pro Ala Pro  Ala Ala Ala Ala Pro  Gly Pro Ala
       1310                 1315                 1320
   gct gcc  gcc cct gcg cct gcc  gcc gcc gcc cct gct  gtc tcg aac      4014
   Ala Ala  Ala Pro Ala Pro Ala  Ala Ala Ala Pro Ala  Val Ser Asn
       1325                 1330                 1335
   gag ctt  ctt gag aag gcc gag  acc gtc gtc atg gag  gtc ctc gcc      4059
   Glu Leu  Leu Glu Lys Ala Glu  Thr Val Val Met Glu  Val Leu Ala
       1340                 1345                 1350
   gcc aag  act ggc tac gag act  gac atg atc gag tcc  gac atg gag      4104
   Ala Lys  Thr Gly Tyr Glu Thr  Asp Met Ile Glu Ser  Asp Met Glu
       1355                 1360                 1365
   ctc gag  acc gag ctc ggc att  gac tcc atc aag cgt  gtc gag att      4149
   Leu Glu  Thr Glu Leu Gly Ile  Asp Ser Ile Lys Arg  Val Glu Ile
       1370                 1375                 1380
   ctc tcc  gag gtc cag gcc atg  ctc aac gtc gag gcc  aag gac gtc      4194
   Leu Ser  Glu Val Gln Ala Met  Leu Asn Val Glu Ala  Lys Asp Val
       1385                 1390                 1395
   gac gct  ctc agc cgc acc cgc  act gtt ggc gag gtc  gtc gat gcc      4239
   Asp Ala  Leu Ser Arg Thr Arg  Thr Val Gly Glu Val  Val Asp Ala
       1400                 1405                 1410
   atg aag  gcc gag atc gct ggt  ggc tct gcc ccg gcg  cct gcc gcc      4284
   Met Lys  Ala Glu Ile Ala Gly  Gly Ser Ala Pro Ala  Pro Ala Ala
       1415                 1420                 1425
   gct gct  cct gct ccg gct gct  gcc gcc cct gcg cct  gcc gcc cct      4329
   Ala Ala  Pro Ala Pro Ala Ala  Ala Ala Pro Ala Pro  Ala Ala Pro
       1430                 1435                 1440
   gcg cct  gct gtc tcg agc gag  ctt ctc gag aag gcc  gag act gtc      4374
   Ala Pro  Ala Val Ser Ser Glu  Leu Leu Glu Lys Ala  Glu Thr Val
       1445                 1450                 1455
   gtc atg  gag gtc ctc gcc gcc  aag act ggc tac gag  act gac atg      4419
   Val Met  Glu Val Leu Ala Ala  Lys Thr Gly Tyr Glu  Thr Asp Met
       1460                 1465                 1470
   atc gag  tcc gac atg gag ctc  gag acc gag ctc ggc  att gac tcc      4464
   Ile Glu  Ser Asp Met Glu Leu  Glu Thr Glu Leu Gly  Ile Asp Ser
       1475                 1480                 1485
   atc aag  cgt gtc gag att ctc  tcc gag gtc cag gcc  atg ctc aac       4509
   Ile Lys  Arg Val Glu Ile Leu  Ser Glu Val Gln Ala  Met Leu Asn
       1490                 1495                 1500
   gtc gag  gcc aag gac gtc gac  gct ctc agc cgc acc  cgc act gtt       4554
   Val Glu  Ala Lys Asp Val Asp  Ala Leu Ser Arg Thr  Arg Thr Val
       1505                 1510                 1515
   ggc gag  gtc gtc gat gcc atg  aag gcc gag atc gct  ggt ggc tct       4599
   Gly Glu  Val Val Asp Ala Met  Lys Ala Glu Ile Ala  Gly Gly Ser
       1520                 1525                 1530
   gcc ccg  gcg cct gcc gcc gct  gct cct gct ccg gct  gct gcc gcc       4644
   Ala Pro  Ala Pro Ala Ala Ala  Ala Pro Ala Pro Ala  Ala Ala Ala
       1535                 1540                 1545
   cct gcg  cct gcc gcc cct gcg  cct gcc gcc cct gcg  cct gct gtc       4689
   Pro Ala  Pro Ala Ala Pro Ala  Pro Ala Ala Pro Ala  Pro Ala Val
       1550                 1555                 1560
   tcg agc  gag ctt ctc gag aag  gcc gag act gtc gtc  atg gag gtc       4734
   Ser Ser  Glu Leu Leu Glu Lys  Ala Glu Thr Val Val  Met Glu Val
       1565                 1570                 1575
   ctc gcc  gcc aag act ggc tac  gag act gac atg att  gag tcc gac       4779
   Leu Ala  Ala Lys Thr Gly Tyr  Glu Thr Asp Met Ile  Glu Ser Asp
       1580                 1585                 1590
   atg gag  ctc gag acc gag ctc  ggc att gac tcc atc  aag cgt gtc       4824
   Met Glu  Leu Glu Thr Glu Leu  Gly Ile Asp Ser Ile  Lys Arg Val
       1595                 1600                 1605
   gag att  ctc tcc gag gtt cag  gcc atg ctc aac gtc  gag gcc aag       4869
   Glu Ile  Leu Ser Glu Val Gln  Ala Met Leu Asn Val  Glu Ala Lys
       1610                 1615                 1620
   gac gtc  gac gct ctc agc cgc  act cgc act gtt ggt  gag gtc gtc       4914
   Asp Val  Asp Ala Leu Ser Arg  Thr Arg Thr Val Gly  Glu Val Val
       1625                 1630                 1635
   gat gcc  atg aag gct gag atc  gct ggc agc tcc gcc  tcg gcg cct       4959
   Asp Ala  Met Lys Ala Glu Ile  Ala Gly Ser Ser Ala  Ser Ala Pro
       1640                 1645                 1650
   gcc gcc  gct gct cct gct ccg  gct gct gcc gct cct  gcg ccc gct       5004
   Ala Ala  Ala Ala Pro Ala Pro  Ala Ala Ala Ala Pro  Ala Pro Ala
       1655                 1660                 1665
   gcc gcc  gcc cct gct gtc tcg  aac gag ctt ctc gag  aaa gcc gag       5049
   Ala Ala  Ala Pro Ala Val Ser  Asn Glu Leu Leu Glu  Lys Ala Glu
       1670                 1675                 1680
   act gtc  gtc atg gag gtc ctc  gcc gcc aag act ggc  tac gag act       5094
   Thr Val  Val Met Glu Val Leu  Ala Ala Lys Thr Gly  Tyr Glu Thr
       1685                 1690                 1695
   gac atg  atc gag tcc gac atg  gag ctc gag act gag  ctc ggc att       5139
   Asp Met  Ile Glu Ser Asp Met  Glu Leu Glu Thr Glu  Leu Gly Ile
       1700                 1705                 1710
   gac tcc  atc aag cgt gtc gag  atc ctc tcc gag gtt  cag gcc atg       5184
   Asp Ser  Ile Lys Arg Val Glu  Ile Leu Ser Glu Val  Gln Ala Met
       1715                 1720                 1725
   ctc aac  gtc gag gcc aag gac  gtc gat gcc ctc agc  cgc acc cgc       5229
   Leu Asn  Val Glu Ala Lys Asp  Val Asp Ala Leu Ser  Arg Thr Arg
       1730                 1735                 1740
   act gtt  ggc gag gtt gtc gat  gcc atg aag gcc gag  atc gct ggt       5274
   Thr Val  Gly Glu Val Val Asp  Ala Met Lys Ala Glu  Ile Ala Gly
       1745                 1750                 1755
   ggc tct  gcc ccg gcg cct gcc  gcc gct gcc cct gct  ccg gct gcc       5319
   Gly Ser  Ala Pro Ala Pro Ala  Ala Ala Ala Pro Ala  Pro Ala Ala
       1760                 1765                 1770
   gcc gcc  cct gct gtc tcg aac  gag ctt ctc gag aag  gcc gag act       5364
   Ala Ala  Pro Ala Val Ser Asn  Glu Leu Leu Glu Lys  Ala Glu Thr
       1775                 1780                 1785
   gtc gtc  atg gag gtc ctc gcc  gcc aag act ggc tac  gag acc gac       5409
   Val Val  Met Glu Val Leu Ala  Ala Lys Thr Gly Tyr  Glu Thr Asp
       1790                 1795                 1800
   atg atc  gag tcc gac atg gag  ctc gag acc gag ctc  ggc att gac      5454
   Met Ile  Glu Ser Asp Met Glu  Leu Glu Thr Glu Leu  Gly Ile Asp
       1805                 1810                 1815
   tcc atc  aag cgt gtc gag att  ctc tcc gag gtt cag  gcc atg ctc      5499
   Ser Ile  Lys Arg Val Glu Ile  Leu Ser Glu Val Gln  Ala Met Leu
       1820                 1825                 1830
   aac gtc  gag gcc aag gac gtc  gat gct ctc agc cgc  act cgc act      5544
   Asn Val  Glu Ala Lys Asp Val  Asp Ala Leu Ser Arg  Thr Arg Thr
       1835                 1840                 1845
   gtt ggc  gag gtc gtc gat gcc  atg aag gct gag atc  gcc ggc agc      5589
   Val Gly  Glu Val Val Asp Ala  Met Lys Ala Glu Ile  Ala Gly Ser
       1850                 1855                 1860
   tcc gcc  ccg gcg cct gcc gcc  gct gct cct gct ccg  gct gct gcc      5634
   Ser Ala  Pro Ala Pro Ala Ala  Ala Ala Pro Ala Pro  Ala Ala Ala
       1865                 1870                 1875
   gct cct  gcg ccc gct gcc gct  gcc cct gct gtc tcg  agc gag ctt      5679
   Ala Pro  Ala Pro Ala Ala Ala  Ala Pro Ala Val Ser  Ser Glu Leu
       1880                 1885                 1890
   ctc gag  aag gcc gag acc gtc  gtc atg gag gtc ctc  gcc gcc aag      5724
   Leu Glu  Lys Ala Glu Thr Val  Val Met Glu Val Leu  Ala Ala Lys
       1895                 1900                 1905
   act ggc  tac gag act gac atg  att gag tcc gac atg  gag ctc gag      5769
   Thr Gly  Tyr Glu Thr Asp Met  Ile Glu Ser Asp Met  Glu Leu Glu
       1910                 1915                 1920
   act gag  ctc ggc att gac tcc  atc aag cgt gtc gag  atc ctc tcc      5814
   Thr Glu  Leu Gly Ile Asp Ser  Ile Lys Arg Val Glu  Ile Leu Ser
       1925                 1930                 1935
   gag gtt  cag gcc atg ctc aac  gtc gag gcc aag gac  gtc gat gcc      5859
   Glu Val  Gln Ala Met Leu Asn  Val Glu Ala Lys Asp  Val Asp Ala
       1940                 1945                 1950
   ctc agc  cgc acc cgc act gtt  ggc gag gtt gtc gat  gcc atg aag      5904
   Leu Ser  Arg Thr Arg Thr Val  Gly Glu Val Val Asp  Ala Met Lys
       1955                 1960                 1965
   gcc gag  atc gct ggt ggc tct  gcc ccg gcg cct gcc  gcc gct gcc      5949
   Ala Glu  Ile Ala Gly Gly Ser  Ala Pro Ala Pro Ala  Ala Ala Ala
       1970                 1975                 1980
   cct gct  ccg gct gcc gcc gcc  cct gct gtc tcg aac  gag ctt ctt      5994
   Pro Ala  Pro Ala Ala Ala Ala  Pro Ala Val Ser Asn  Glu Leu Leu
       1985                 1990                 1995
   gag aag  gcc gag acc gtc gtc  atg gag gtc ctc gcc  gcc aag act      6039
   Glu Lys  Ala Glu Thr Val Val  Met Glu Val Leu Ala  Ala Lys Thr
       2000                 2005                 2010
   ggc tac  gag acc gac atg atc  gag tcc gac atg gag  ctc gag acc      6084
   Gly Tyr  Glu Thr Asp Met Ile  Glu Ser Asp Met Glu  Leu Glu Thr
       2015                 2020                 2025
   gag ctc  ggc att gac tcc atc  aag cgt gtc gag att  ctc tcc gag      6129
   Glu Leu  Gly Ile Asp Ser Ile  Lys Arg Val Glu Ile  Leu Ser Glu
       2030                 2035                 2040
   gtt cag  gcc atg ctc aac gtc  gag gcc aag gac gtc  gac gct ctc      6174
   Val Gln  Ala Met Leu Asn Val  Glu Ala Lys Asp Val  Asp Ala Leu
       2045                 2050                 2055
   agc cgc  act cgc act gtt ggc  gag gtc gtc gat gcc  atg aag gct      6219
   Ser Arg  Thr Arg Thr Val Gly  Glu Val Val Asp Ala  Met Lys Ala
       2060                 2065                 2070
   gag atc  gct ggt ggc tct gcc  ccg gcg cct gcc gcc  gct gct cct      6264
   Glu Ile  Ala Gly Gly Ser Ala  Pro Ala Pro Ala Ala  Ala Ala Pro
       2075                 2080                 2085
   gcc tcg  gct ggc gcc gcg cct  gcg gtc aag att gac  tcg gtc cac      6309
   Ala Ser  Ala Gly Ala Ala Pro  Ala Val Lys Ile Asp  Ser Val His
       2090                 2095                 2100
   ggc gct  gac tgt gat gat ctt  tcc ctg atg cac gcc  aag gtg gtt      6354
   Gly Ala  Asp Cys Asp Asp Leu  Ser Leu Met His Ala  Lys Val Val
       2105                 2110                 2115
   gac atc  cgc cgc ccg gac gag  ctc atc ctg gag cgc  ccc gag aac      6399
   Asp Ile  Arg Arg Pro Asp Glu  Leu Ile Leu Glu Arg  Pro Glu Asn
       2120                 2125                 2130
   cgc ccc  gtt ctc gtt gtc gat  gac ggc agc gag ctc  acc ctc gcc      6444
   Arg Pro  Val Leu Val Val Asp  Asp Gly Ser Glu Leu  Thr Leu Ala
       2135                 2140                 2145
   ctg gtc  cgc gtc ctc ggc gcc  tgc gcc gtt gtc ctg  acc ttt gag      6489
   Leu Val  Arg Val Leu Gly Ala  Cys Ala Val Val Leu  Thr Phe Glu
       2150                 2155                 2160
   ggt ctc  cag ctc gct cag cgc  gct ggt gcc gct gcc  atc cgc cac      6534
   Gly Leu  Gln Leu Ala Gln Arg  Ala Gly Ala Ala Ala  Ile Arg His
       2165                 2170                 2175
   gtg ctc  gcc aag gat ctt tcc  gcg gag agc gcc gag  aag gcc atc      6579
   Val Leu  Ala Lys Asp Leu Ser  Ala Glu Ser Ala Glu  Lys Ala Ile
       2180                 2185                 2190
   aag gag  gcc gag cag cgc ttt  ggc gct ctc ggc ggc  ttc atc tcg      6624
   Lys Glu  Ala Glu Gln Arg Phe  Gly Ala Leu Gly Gly  Phe Ile Ser
       2195                 2200                 2205
   cag cag  gcg gag cgc ttc gag  ccc gcc gaa atc ctc  ggc ttc acg      6669
   Gln Gln  Ala Glu Arg Phe Glu  Pro Ala Glu Ile Leu  Gly Phe Thr
       2210                 2215                 2220
   ctc atg  tgc gcc aag ttc gcc  aag gct tcc ctc tgc  acg gct gtg      6714
   Leu Met  Cys Ala Lys Phe Ala  Lys Ala Ser Leu Cys  Thr Ala Val
       2225                 2230                 2235
   gct ggc  ggc cgc ccg gcc ttt  atc ggt gtg gcg cgc  ctt gac ggc      6759
   Ala Gly  Gly Arg Pro Ala Phe  Ile Gly Val Ala Arg  Leu Asp Gly
       2240                 2245                 2250
   cgc ctc  gga ttc act tcg cag  ggc act tct gac gcg  ctc aag cgt      6804
   Arg Leu  Gly Phe Thr Ser Gln  Gly Thr Ser Asp Ala  Leu Lys Arg
       2255                 2260                 2265
   gcc cag  cgt ggt gcc atc ttt  ggc ctc tgc aag acc  atc ggc ctc      6849
   Ala Gln  Arg Gly Ala Ile Phe  Gly Leu Cys Lys Thr  Ile Gly Leu
       2270                 2275                 2280
   gag tgg  tcc gag tct gac gtc  ttt tcc cgc ggc gtg  gac att gct      6894
   Glu Trp  Ser Glu Ser Asp Val  Phe Ser Arg Gly Val  Asp Ile Ala
       2285                 2290                 2295
   cag ggc  atg cac ccc gag gat  gcc gcc gtg gcg att  gtg cgc gag      6939
   Gln Gly  Met His Pro Glu Asp  Ala Ala Val Ala Ile  Val Arg Glu
       2300                 2305                 2310
   atg gcg  tgc gct gac att cgc  att cgc gag gtc ggc  att ggc gca      6984
   Met Ala  Cys Ala Asp Ile Arg  Ile Arg Glu Val Gly  Ile Gly Ala
       2315                 2320                 2325
   aac cag  cag cgc tgc acg atc  cgt gcc gcc aag ctc  gag acc ggc      7029
   Asn Gln  Gln Arg Cys Thr Ile  Arg Ala Ala Lys Leu  Glu Thr Gly
       2330                 2335                 2340
   aac ccg  cag cgc cag atc gcc  aag gac gac gtg ctg  ctc gtt tct      7074
   Asn Pro  Gln Arg Gln Ile Ala  Lys Asp Asp Val Leu  Leu Val Ser
       2345                 2350                 2355
   ggc ggc  gct cgc ggc atc acg  cct ctt tgc atc cgg  gag atc acg      7119
   Gly Gly  Ala Arg Gly Ile Thr  Pro Leu Cys Ile Arg  Glu Ile Thr
       2360                 2365                 2370
   cgc cag  atc gcg ggc ggc aag  tac att ctg ctt ggc  cgc agc aag      7164
   Arg Gln  Ile Ala Gly Gly Lys  Tyr Ile Leu Leu Gly  Arg Ser Lys
       2375                 2380                 2385
   gtc tct  gcg agc gaa ccg gca  tgg tgc gct ggc atc  act gac gag      7209
   Val Ser  Ala Ser Glu Pro Ala  Trp Cys Ala Gly Ile  Thr Asp Glu
       2390                 2395                 2400
   aag gct  gtg caa aag gct gct  acc cag gag ctc aag  cgc gcc ttt      7254
   Lys Ala  Val Gln Lys Ala Ala  Thr Gln Glu Leu Lys  Arg Ala Phe
       2405                 2410                 2415
   agc gct  ggc gag ggc ccc aag  ccc acg ccc cgc gct  gtc act aag      7299
   Ser Ala  Gly Glu Gly Pro Lys  Pro Thr Pro Arg Ala  Val Thr Lys
       2420                 2425                 2430
   ctt gtg  ggc tct gtt ctt ggc  gct cgc gag gtg cgc  agc tct att      7344
   Leu Val  Gly Ser Val Leu Gly  Ala Arg Glu Val Arg  Ser Ser Ile
       2435                 2440                 2445
   gct gcg  att gaa gcg ctc ggc  ggc aag gcc atc tac  tcg tcg tgc      7389
   Ala Ala  Ile Glu Ala Leu Gly  Gly Lys Ala Ile Tyr  Ser Ser Cys
       2450                 2455                 2460
   gac gtg  aac tct gcc gcc gac  gtg gcc aag gcc gtg  cgc gat gcc      7434
   Asp Val  Asn Ser Ala Ala Asp  Val Ala Lys Ala Val  Arg Asp Ala
       2465                 2470                 2475
   gag tcc  cag ctc ggt gcc cgc  gtc tcg ggc atc gtt  cat gcc tcg      7479
   Glu Ser  Gln Leu Gly Ala Arg  Val Ser Gly Ile Val  His Ala Ser
       2480                 2485                 2490
   ggc gtg  ctc cgc gac cgt ctc  atc gag aag aag ctc  ccc gac gag      7524
   Gly Val  Leu Arg Asp Arg Leu  Ile Glu Lys Lys Leu  Pro Asp Glu
       2495                 2500                 2505
   ttc gac  gcc gtc ttt ggc acc  aag gtc acc ggt ctc  gag aac ctc      7569
   Phe Asp  Ala Val Phe Gly Thr  Lys Val Thr Gly Leu  Glu Asn Leu
       2510                 2515                 2520
   ctc gcc  gcc gtc gac cgc gcc  aac ctc aag cac atg  gtc ctc ttc      7614
   Leu Ala  Ala Val Asp Arg Ala  Asn Leu Lys His Met  Val Leu Phe
       2525                 2530                 2535
   agc tcg  ctc gcc ggc ttc cac  ggc aac gtc ggc cag  tct gac tac      7659
   Ser Ser  Leu Ala Gly Phe His  Gly Asn Val Gly Gln  Ser Asp Tyr
       2540                 2545                 2550
   gcc atg  gcc aac gag gcc ctt  aac aag atg ggc ctc  gag ctc gcc      7704
   Ala Met  Ala Asn Glu Ala Leu  Asn Lys Met Gly Leu  Glu Leu Ala
       2555                 2560                 2565
   aag gac  gtc tcg gtc aag tcg  atc tgc ttc ggt ccc  tgg gac ggt      7749
   Lys Asp  Val Ser Val Lys Ser  Ile Cys Phe Gly Pro  Trp Asp Gly
       2570                 2575                 2580
   ggc atg  gtg acg ccg cag ctc  aag aag cag ttc cag  gag atg ggc      7794
   Gly Met  Val Thr Pro Gln Leu  Lys Lys Gln Phe Gln  Glu Met Gly
       2585                 2590                 2595
   gtg cag  atc atc ccc cgc gag  ggc ggc gct gat acc  gtg gcg cgc      7839
   Val Gln  Ile Ile Pro Arg Glu  Gly Gly Ala Asp Thr  Val Ala Arg
       2600                 2605                 2610
   atc gtg  ctc ggc tcc tcg ccg  gct gag atc ctt gtc  ggc aac tgg      7884
   Ile Val  Leu Gly Ser Ser Pro  Ala Glu Ile Leu Val  Gly Asn Trp
       2615                 2620                 2625
   cgc acc  ccg tcc aag aag gtc  ggc tcg gac acc atc  acc ctg cac      7929
   Arg Thr  Pro Ser Lys Lys Val  Gly Ser Asp Thr Ile  Thr Leu His
       2630                 2635                 2640
   cgc aag  att tcc gcc aag tcc  aac ccc ttc ctc gag  gac cac gtc      7974
   Arg Lys  Ile Ser Ala Lys Ser  Asn Pro Phe Leu Glu  Asp His Val
       2645                 2650                 2655
   atc cag  ggc cgc cgc gtg ctg  ccc atg acg ctg gcc  att ggc tcg      8019
   Ile Gln  Gly Arg Arg Val Leu  Pro Met Thr Leu Ala  Ile Gly Ser
       2660                 2665                 2670
   ctc gcg  gag acc tgc ctc ggc  ctc ttc ccc ggc tac  tcg ctc tgg      8064
   Leu Ala  Glu Thr Cys Leu Gly  Leu Phe Pro Gly Tyr  Ser Leu Trp
       2675                 2680                 2685
   gcc att  gac gac gcc cag ctc  ttc aag ggt gtc act  gtc gac ggc      8109
   Ala Ile  Asp Asp Ala Gln Leu  Phe Lys Gly Val Thr  Val Asp Gly
       2690                 2695                 2700
   gac gtc  aac tgc gag gtg acc  ctc acc ccg tcg acg  gcg ccc tcg      8154
   Asp Val  Asn Cys Glu Val Thr  Leu Thr Pro Ser Thr  Ala Pro Ser
       2705                 2710                 2715
   ggc cgc  gtc aac gtc cag gcc  acg ctc aag acc ttt  tcc agc ggc      8199
   Gly Arg  Val Asn Val Gln Ala  Thr Leu Lys Thr Phe  Ser Ser Gly
       2720                 2725                 2730
   aag ctg  gtc ccg gcc tac cgc  gcc gtc atc gtg ctc  tcc aac cag      8244
   Lys Leu  Val Pro Ala Tyr Arg  Ala Val Ile Val Leu  Ser Asn Gln
       2735                 2740                 2745
   ggc gcg  ccc ccg gcc aac gcc  acc atg cag ccg ccc  tcg ctc gat       8289
   Gly Ala  Pro Pro Ala Asn Ala  Thr Met Gln Pro Pro  Ser Leu Asp
       2750                 2755                 2760
   gcc gat  ccg gcg ctc cag ggc  tcc gtc tac gac ggc  aag acc ctc       8334
   Ala Asp  Pro Ala Leu Gln Gly  Ser Val Tyr Asp Gly  Lys Thr Leu
       2765                 2770                 2775
   ttc cac  ggc ccg gcc ttc cgc  ggc atc gat gac gtg  ctc tcg tgc       8379
   Phe His  Gly Pro Ala Phe Arg  Gly Ile Asp Asp Val  Leu Ser Cys
       2780                 2785                 2790
   acc aag  agc cag ctt gtg gcc  aag tgc agc gct gtc  ccc ggc tcc       8424
   Thr Lys  Ser Gln Leu Val Ala  Lys Cys Ser Ala Val  Pro Gly Ser
       2795                 2800                 2805
   gac gcc  gct cgc ggc gag ttt  gcc acg gac act gac  gcc cat gac       8469
   Asp Ala  Ala Arg Gly Glu Phe  Ala Thr Asp Thr Asp  Ala His Asp
       2810                 2815                 2820
   ccc ttc  gtg aac gac ctg gcc  ttt cag gcc atg ctc  gtc tgg gtg       8514
   Pro Phe  Val Asn Asp Leu Ala  Phe Gln Ala Met Leu  Val Trp Val
       2825                 2830                 2835
   cgc cgc  acg ctc ggc cag gct  gcg ctc ccc aac tcg  atc cag cgc       8559
   Arg Arg  Thr Leu Gly Gln Ala  Ala Leu Pro Asn Ser  Ile Gln Arg
       2840                 2845                 2850
   atc gtc  cag cac cgc ccg gtc  ccg cag gac aag ccc  ttc tac att       8604
   Ile Val  Gln His Arg Pro Val  Pro Gln Asp Lys Pro  Phe Tyr Ile
       2855                 2860                 2865
   acc ctc  cgc tcc aac cag tcg  ggc ggt cac tcc cag  cac aag cac       8649
   Thr Leu  Arg Ser Asn Gln Ser  Gly Gly His Ser Gln  His Lys His
       2870                 2875                 2880
   gcc ctt  cag ttc cac aac gag  cag ggc gat ctc ttc  att gat gtc       8694
   Ala Leu  Gln Phe His Asn Glu  Gln Gly Asp Leu Phe  Ile Asp Val
       2885                 2890                 2895
   cag gct  tcg gtc atc gcc acg  gac agc ctt gcc ttc                    8730
   Gln Ala  Ser Val Ile Ala Thr  Asp Ser Leu Ala Phe
       2900                 2905                 2910
<210> 2
   <211> 2910
   <212> PRT
   <213> Schizochytrium sp.
<400> 2
   Met Ala Ala Arg Leu Gln Glu Gln Lys Gly Gly Glu Met Asp Thr Arg
   1               5                   10                  15
   Ile Ala Ile Ile Gly Met Ser Ala Ile Leu Pro Cys Gly Thr Thr Val
               20                  25                  30
   Arg Glu Ser Trp Glu Thr Ile Arg Ala Gly Ile Asp Cys Leu Ser Asp
           35                  40                  45
   Leu Pro Glu Asp Arg Val Asp Val Thr Ala Tyr Phe Asp Pro Val Lys
       50                  55                  60
   Thr Thr Lys Asp Lys Ile Tyr Cys Lys Arg Gly Gly Phe Ile Pro Glu
   65                  70                  75                  80
   Tyr Asp Phe Asp Ala Arg Glu Phe Gly Leu Asn Met Phe Gln Met Glu
                   85                  90                  95
   Asp Ser Asp Ala Asn Gln Thr Ile Ser Leu Leu Lys Val Lys Glu Ala
               100                 105                 110
   Leu Gln Asp Ala Gly Ile Asp Ala Leu Gly Lys Glu Lys Lys Asn Ile
   115 120 125
   Gly Cys Val Leu Gly Ile Gly Gly Gly Gln Lys Ser Ser His Glu Phe
   130 135 140
   Tyr Ser Arg Leu Asn Tyr Val Val Val Glu Lys Val Leu Arg Lys Met
   145 150 155 160
   Gly Met Pro Glu Glu Asp Val Lys Val Ala Val Glu Lys Tyr Lys Ala
   165 170 175
   Asn Phe Pro Glu Trp Arg Leu Asp Ser Phe Pro Gly Phe Leu Gly Asn
   180 185 190
   Val Thr Ala Gly Arg Cys Thr Asn Thr Phe Asn Leu Asp Gly Met Asn
   195 200 205
   Cys Val Val Asp Ala Ala Cys Ala Ser Ser Leu Ile Ala Val Lys Val
   210 215 220
   Ala Ile Asp Glu Leu Leu Tyr Gly Asp Cys Asp Met Met Val Thr Gly
   225 230 235 240
   Ala Thr Cys Thr Asp Asn Ser Ile Gly Met Tyr Met Ala Phe Ser Lys
   245 250 255
   Thr Pro Val Phe Ser Thr Asp Pro Ser Val Arg Ala Tyr Asp Glu Lys
   260 265 270
   Thr Lys Gly Met Leu Ile Gly Glu Gly Ser Ala Met Leu Val Leu Lys
   275 280 285
   Arg Tyr Ala Asp Ala Val Arg Asp Gly Asp Glu Ile His Ala Val Ile
   290 295 300
   Arg Gly Cys Ala Ser Ser Ser Asp Gly Lys Ala Ala Gly Ile Tyr Thr
   305 310 315 320
   Pro Thr Ile Ser Gly Gln Glu Glu Ala Leu Arg Arg Ala Tyr Asn Arg
   325 330 335
   Ala Cys Val Asp Pro Ala Thr Val Thr Leu Val Glu Gly His Gly Thr
   340 345 350
   Gly Thr Pro Val Gly Asp Arg Ile Glu Leu Thr Ala Leu Arg Asn Leu
   355 360 365
   Phe Asp Lys Ala Tyr Gly Glu Gly Asn Thr Glu Lys Val Ala Val Gly
   370 375 380
   Ser Ile Lys Ser Ser Ile Gly His Leu Lys Ala Val Ala Gly Leu Ala
   385 390 395 400
   Gly Met Ile Lys Val Ile Met Ala Leu Lys His Lys Thr Leu Pro Gly
   405 410 415
   Thr Ile Asn Val Asp Asn Pro Pro Asn Leu Tyr Asp Asn Thr Pro Ile
   420 425 430
   Asn Glu Ser Ser Leu Tyr Ile Asn Thr Met Asn Arg Pro Trp Phe Pro
   435 440 445
   Pro Pro Gly Val Pro Arg Arg Ala Gly Ile Ser Ser Phe Gly Phe Gly
   450 455 460
   Gly Ala Asn Tyr His Ala Val Leu Glu Glu Ala Glu Pro Glu His Thr
   465 470 475 480
   Thr Ala Tyr Arg Leu Asn Lys Arg Pro Gln Pro Val Leu Met Met Ala
   485 490 495
   Ala Thr Pro Ala Ala Leu Gln Ser Leu Cys Glu Ala Gln Leu Lys Glu
   500 505 510
   Phe Glu Ala Ala Ile Lys Glu Asn Glu Thr Val Lys Asn Thr Ala Tyr
   515 520 525
   Ile Lys Cys Val Lys Phe Gly Glu Gln Phe Lys Phe Pro Gly Ser Ile
   530 535 540
   Pro Ala Thr Asn Ala Arg Leu Gly Phe Leu Val Lys Asp Ala Glu Asp
   545 550 555 560
   Ala Cys Ser Thr Leu Arg Ala Ile Cys Ala Gln Phe Ala Lys Asp Val
   565 570 575
   Thr Lys Glu Ala Trp Arg Leu Pro Arg Glu Gly Val Ser Phe Arg Ala
   580 585 590
   Lys Gly Ile Ala Thr Asn Gly Ala Val Ala Ala Leu Phe Ser Gly Gln
   595 600 605
   Gly Ala Gln Tyr Thr His Met Phe Ser Glu Val Ala Met Asn Trp Pro
   610 615 620
   Gln Phe Arg Gln Ser Ile Ala Ala Met Asp Ala Ala Gln Ser Lys Val
   625 630 635 640
   Ala Gly Ser Asp Lys Asp Phe Glu Arg Val Ser Gln Val Leu Tyr Pro
   645 650 655
   Arg Lys Pro Tyr Glu Arg Glu Pro Glu Gln Asn Pro Lys Lys Ile Ser
   660 665 670
   Leu Thr Ala Tyr Ser Gln Pro Ser Thr Leu Ala Cys Ala Leu Gly Ala
   675 680 685
   Phe Glu Ile Phe Lys Glu Ala Gly Phe Thr Pro Asp Phe Ala Ala Gly
   690 695 700
   His Ser Leu Gly Glu Phe Ala Ala Leu Tyr Ala Ala Gly Cys Val Asp
   705 710 715 720
   Arg Asp Glu Leu Phe Glu Leu Val Cys Arg Arg Ala Arg Ile Met Gly
   725 730 735
   Gly Lys Asp Ala Pro Ala Thr Pro Lys Gly Cys Met Ala Ala Val Ile
   740 745 750
   Gly Pro Asn Ala Glu Asn Ile Lys Val Gln Ala Ala Asn Val Trp Leu
   755 760 765
   Gly Asn Ser Asn Ser Pro Ser Gln Thr Val Ile Thr Gly Ser Val Glu
   770 775 780
   Gly Ile Gln Ala Glu Ser Ala Arg Leu Gln Lys Glu Gly Phe Arg Val
   785 790 795 800
   Val Pro Leu Ala Cys Glu Ser Ala Phe His Ser Pro Gln Met Glu Asn
   805 810 815
   Ala Ser Ser Ala Phe Lys Asp Val Ile Ser Lys Val Ser Phe Arg Thr
   820 825 830
   Pro Lys Ala Glu Thr Lys Leu Phe Ser Asn Val Ser Gly Glu Thr Tyr
   835 840 845
   Pro Thr Asp Ala Arg Glu Met Leu Thr Gln His Met Thr Ser Ser Val
   850 855 860
   Lys Phe Leu Thr Gln Val Arg Asn Met His Gln Ala Gly Ala Arg Ile
   865 870 875 880
   Phe Val Glu Phe Gly Pro Lys Gln Val Leu Ser Lys Leu Val Ser Glu
   885 890 895
   Thr Leu Lys Asp Asp Pro Ser Val Val Thr Val Ser Val Asn Pro Ala
   900 905 910
   Ser Gly Thr Asp Ser Asp Ile Gln Leu Arg Asp Ala Ala Val Gln Leu
   915 920 925
   Val Val Ala Gly Val Asn Leu Gln Gly Phe Asp Lys Trp Asp Ala Pro
   930 935 940
   Asp Ala Thr Arg Met Gln Ala Ile Lys Lys Lys Arg Thr Thr Leu Arg
   945 950 955 960
   Leu Ser Ala Ala Thr Tyr Val Ser Asp Lys Thr Lys Lys Val Arg Asp
   965 970 975
   Ala Ala Met Asn Asp Gly Arg Cys Val Thr Tyr Leu Lys Gly Ala Ala
   980 985 990
   Pro Leu Ile Lys Ala Pro Glu Pro Val Val Asp Glu Ala Ala Lys Arg
   995 1000 1005
   Glu Ala Glu Arg Leu Gln Lys Glu Leu Gln Asp Ala Gln Arg Gln
   1010 1015 1020
   Leu Asp Asp Ala Lys Arg Ala Ala Ala Glu Ala Asn Ser Lys Leu
   1025 1030 1035
   Ala Ala Ala Lys Glu Glu Ala Lys Thr Ala Ala Ala Ser Ala Lys
   1040 1045 1050
   Pro Ala Val Asp Thr Ala Val Val Glu Lys His Arg Ala Ile Leu
   1055 1060 1065
   Lys Ser Met Leu Ala Glu Leu Asp Gly Tyr Gly Ser Val Asp Ala
   1070 1075 1080
   Ser Ser Leu Gln Gln Gln Gln Gln Gln Gln Thr Ala Pro Ala Pro
   1085 1090 1095
   Val Lys Ala Ala Ala Pro Ala Ala Pro Val Ala Ser Ala Pro Ala
   1100 1105 1110
   Pro Ala Val Ser Asn Glu Leu Leu Glu Lys Ala Glu Thr Val Val
   1115 1120 1125
   Met Glu Val Leu Ala Ala Lys Thr Gly Tyr Glu Thr Asp Met Ile
   1130 1135 1140
   Glu Ala Asp Met Glu Leu Glu Thr Glu Leu Gly Ile Asp Ser Ile
   1145 1150 1155
   Lys Arg Val Glu Ile Leu Ser Glu Val Gln Ala Met Leu Asn Val
   1160 1165 1170
   Glu Ala Lys Asp Val Asp Ala Leu Ser Arg Thr Arg Thr Val Gly
   1175 1180 1185
   Glu Val Val Asn Ala Met Lys Ala Glu Ile Ala Gly Ser Ser Ala
   1190 1195 1200
   Pro Ala Pro Ala Ala Ala Ala Pro Ala Pro Ala Lys Ala Ala Pro
   1205 1210 1215
   Ala Ala Ala Ala Pro Ala Val Ser Asn Glu Leu Leu Glu Lys Ala
   1220 1225 1230
   Glu Thr Val Val Met Glu Val Leu Ala Ala Lys Thr Gly Tyr Glu
   1235 1240 1245
   Thr Asp Met Ile Glu Ser Asp Met Glu Leu Glu Thr Glu Leu Gly
   1250 1255 1260
   Ile Asp Ser Ile Lys Arg Val Glu Ile Leu Ser Glu Val Gln Ala
   1265 1270 1275
   Met Leu Asn Val Glu Ala Lys Asp Val Asp Ala Leu Ser Arg Thr
   1280 1285 1290
   Arg Thr Val Gly Glu Val Val Asn Ala Met Lys Ala Glu Ile Ala
   1295 1300 1305
   Gly Gly Ser Ala Pro Ala Pro Ala Ala Ala Ala Pro Gly Pro Ala
   1310 1315 1320
   Ala Ala Ala Pro Ala Pro Ala Ala Ala Ala Pro Ala Val Ser Asn
   1325 1330 1335
   Glu Leu Leu Glu Lys Ala Glu Thr Val Val Met Glu Val Leu Ala
   1340 1345 1350
   Ala Lys Thr Gly Tyr Glu Thr Asp Met Ile Glu Ser Asp Met Glu
   1355 1360 1365
   Leu Glu Thr Glu Leu Gly Ile Asp Ser Ile Lys Arg Val Glu Ile
   1370 1375 1380
   Leu Ser Glu Val Gln Ala Met Leu Asn Val Glu Ala Lys Asp Val
   1385 1390 1395
   Asp Ala Leu Ser Arg Thr Arg Thr Val Gly Glu Val Val Asp Ala
   1400 1405 1410
   Met Lys Ala Glu Ile Ala Gly Gly Ser Ala Pro Ala Pro Ala Ala
   1415 1420 1425
   Ala Ala  Pro Ala Pro Ala Ala  Ala Ala Pro Ala Pro  Ala Ala Pro
       1430                 1435                 1440
   Ala Pro  Ala Val Ser Ser Glu  Leu Leu Glu Lys Ala  Glu Thr Val
       1445                 1450                 1455
   Val Met  Glu Val Leu Ala Ala  Lys Thr Gly Tyr Glu  Thr Asp Met
       1460                 1465                 1470
   Ile Glu  Ser Asp Met Glu Leu  Glu Thr Glu Leu Gly  Ile Asp Ser
       1475                 1480                 1485
   Ile Lys  Arg Val Glu Ile Leu  Ser Glu Val Gln Ala  Met Leu Asn
       1490                 1495                 1500
   Val Glu  Ala Lys Asp Val Asp  Ala Leu Ser Arg Thr  Arg Thr Val
       1505                 1510                 1515
   Gly Glu  Val Val Asp Ala Met  Lys Ala Glu Ile Ala  Gly Gly Ser
       1520                 1525                 1530
   Ala Pro  Ala Pro Ala Ala Ala  Ala Pro Ala Pro Ala  Ala Ala Ala
       1535                 1540                 1545
   Pro Ala  Pro Ala Ala Pro Ala  Pro Ala Ala Pro Ala  Pro Ala Val
       1550                 1555                 1560
   Ser Ser  Glu Leu Leu Glu Lys  Ala Glu Thr Val Val  Met Glu Val
       1565                 1570                 1575
   Leu Ala  Ala Lys Thr Gly Tyr  Glu Thr Asp Met Ile  Glu Ser Asp
       1580                 1585                 1590
   Met Glu  Leu Glu Thr Glu Leu  Gly Ile Asp Ser Ile  Lys Arg Val
       1595                 1600                 1605
   Glu Ile  Leu Ser Glu Val Gln  Ala Met Leu Asn Val  Glu Ala Lys
       1610                 1615                 1620
   Asp Val  Asp Ala Leu Ser Arg  Thr Arg Thr Val Gly  Glu Val Val
       1625                 1630                 1635
   Asp Ala  Met Lys Ala Glu Ile  Ala Gly Ser Ser Ala  Ser Ala Pro
       1640                 1645                 1650
   Ala Ala  Ala Ala Pro Ala Pro  Ala Ala Ala Ala Pro  Ala Pro Ala
       1655                 1660                 1665
   Ala Ala  Ala Pro Ala Val Ser  Asn Glu Leu Leu Glu  Lys Ala Glu
       1670                 1675                 1680
   Thr Val  Val Met Glu Val Leu  Ala Ala Lys Thr Gly  Tyr Glu Thr
       1685                 1690                 1695
   Asp Met  Ile Glu Ser Asp Met  Glu Leu Glu Thr Glu  Leu Gly Ile
       1700                 1705                 1710
   Asp Ser  Ile Lys Arg Val Glu  Ile Leu Ser Glu Val  Gln Ala Met
       1715                 1720                 1725
   Leu Asn  Val Glu Ala Lys Asp  Val Asp Ala Leu Ser  Arg Thr Arg
       1730                 1735                 1740
   Thr Val  Gly Glu Val Val Asp  Ala Met Lys Ala Glu  Ile Ala Gly
       1745                 1750                 1755
   Gly Ser  Ala Pro Ala Pro Ala  Ala Ala Ala Pro Ala  Pro Ala Ala
       1760                 1765                 1770
   Ala Ala  Pro Ala Val Ser Asn  Glu Leu Leu Glu Lys  Ala Glu Thr
       1775                 1780                 1785
   Val Val  Met Glu Val Leu Ala  Ala Lys Thr Gly Tyr  Glu Thr Asp
       1790                 1795                 1800
   Met Ile  Glu Ser Asp Met Glu  Leu Glu Thr Glu Leu  Gly Ile Asp
       1805                 1810                 1815
   Ser Ile  Lys Arg Val Glu Ile  Leu Ser Glu Val Gln  Ala Met Leu
       1820                 1825                 1830
   Asn Val  Glu Ala Lys Asp Val  Asp Ala Leu Ser Arg  Thr Arg Thr
       1835                 1840                 1845
   Val Gly  Glu Val Val Asp Ala  Met Lys Ala Glu Ile  Ala Gly Ser
       1850                 1855                 1860
   Ser Ala  Pro Ala Pro Ala Ala  Ala Ala Pro Ala Pro  Ala Ala Ala
       1865                 1870                 1875
   Ala Pro  Ala Pro Ala Ala Ala  Ala Pro Ala Val Ser  Ser Glu Leu
       1880                 1885                 1890
   Leu Glu  Lys Ala Glu Thr Val  Val Met Glu Val Leu  Ala Ala Lys
       1895                 1900                 1905
   Thr Gly  Tyr Glu Thr Asp Met  Ile Glu Ser Asp Met  Glu Leu Glu
       1910                 1915                 1920
   Thr Glu  Leu Gly Ile Asp Ser  Ile Lys Arg Val Glu  Ile Leu Ser
       1925                 1930                 1935
   Glu Val  Gln Ala Met Leu Asn  Val Glu Ala Lys Asp  Val Asp Ala
       1940                 1945                 1950
   Leu Ser  Arg Thr Arg Thr Val  Gly Glu Val Val Asp  Ala Met Lys
       1955                 1960                 1965
   Ala Glu  Ile Ala Gly Gly Ser  Ala Pro Ala Pro Ala  Ala Ala Ala
       1970                 1975                 1980
   Pro Ala  Pro Ala Ala Ala Ala  Pro Ala Val Ser Asn  Glu Leu Leu
       1985                 1990                 1995
   Glu Lys  Ala Glu Thr Val Val  Met Glu Val Leu Ala  Ala Lys Thr
       2000                 2005                 2010
   Gly Tyr  Glu Thr Asp Met Ile  Glu Ser Asp Met Glu  Leu Glu Thr
       2015                 2020                 2025
   Glu Leu  Gly Ile Asp Ser Ile  Lys Arg Val Glu Ile  Leu Ser Glu
       2030                 2035                 2040
   Val Gln  Ala Met Leu Asn Val  Glu Ala Lys Asp Val  Asp Ala Leu
       2045                 2050                 2055
   Ser Arg Thr Arg Thr Val Gly Glu Val Val Asp Ala Met Lys Ala
   2060 2065 2070
   Glu Ile Ala Gly Gly Ser Ala Pro Ala Pro Ala Ala Ala Ala Pro
   2075 2080 2085
   Ala Ser Ala Gly Ala Ala Pro Ala Val Lys Ile Asp Ser Val His
   2090 2095 2100
   Gly Ala Asp Cys Asp Asp Leu Ser Leu Met His Ala Lys Val Val
   2105 2110 2115
   Asp Ile Arg Arg Pro Asp Glu Leu Ile Leu Glu Arg Pro Glu Asn
   2120 2125 2130
   Arg Pro Val Leu Val Val Asp Asp Gly Ser Glu Leu Thr Leu Ala
   2135 2140 2145
   Leu Val Arg Val Leu Gly Ala Cys Ala Val Val Leu Thr Phe Glu
   2150 2155 2160
   Gly Leu Gln Leu Ala Gln Arg Ala Gly Ala Ala Ala Ile Arg His
   2165 2170 2175
   Val Leu Ala Lys Asp Leu Ser Ala Glu Ser Ala Glu Lys Ala Ile
   2180 2185 2190
   Lys Glu Ala Glu Gln Arg Phe Gly Ala Leu Gly Gly Phe Ile Ser
   2195 2200 2205
   Gln Gln Ala Glu Arg Phe Glu Pro Ala Glu Ile Leu Gly Phe Thr
   2210 2215 2220
   Leu Met Cys Ala Lys Phe Ala Lys Ala Ser Leu Cys Thr Ala Val
   2225 2230 2235
   Ala Gly Gly Arg Pro Ala Phe Ile Gly Val Ala Arg Leu Asp Gly
   2240 2245 2250
   Arg Leu Gly Phe Thr Ser Gln Gly Thr Ser Asp Ala Leu Lys Arg
   2255 2260 2265
   Ala Gln Arg Gly Ala Ile Phe Gly Leu Cys Lys Thr Ile Gly Leu
   2270 2275 2280
   Glu Trp Ser Glu Ser Asp Val Phe Ser Arg Gly Val Asp Ile Ala
   2285 2290 2295
   Gln Gly Met His Pro Glu Asp Ala Ala Val Ala Ile Val Arg Glu
   2300 2305 2310
   Met Ala Cys Ala Asp Ile Arg Ile Arg Glu Val Gly Ile Gly Ala
   2315 2320 2325
   Asn Gln Gln Arg Cys Thr Ile Arg Ala Ala Lys Leu Glu Thr Gly
   2330 2335 2340
   Asn Pro Gln Arg Gln Ile Ala Lys Asp Asp Val Leu Leu Val Ser
   2345 2350 2355
   Gly Gly Ala Arg Gly Ile Thr Pro Leu Cys Ile Arg Glu Ile Thr
   2360 2365 2370
   Arg Gln Ile Ala Gly Gly Lys Tyr Ile Leu Leu Gly Arg Ser Lys
       2375                2380                2385
   Val Ser Ala Ser Glu Pro Ala Trp Cys Ala Gly Ile Thr Asp Glu
       2390                2395                2400
   Lys Ala Val Gln Lys Ala Ala Thr Gln Glu Leu Lys Arg Ala Phe
       2405                2410                2415
   Ser Ala Gly Glu Gly Pro Lys Pro Thr Pro Arg Ala Val Thr Lys
       2420                2425                2430
   Leu Val Gly Ser Val Leu Gly Ala Arg Glu Val Arg Ser Ser Ile
       2435                2440                2445
   Ala Ala Ile Glu Ala Leu Gly Gly Lys Ala Ile Tyr Ser Ser Cys
       2450                2455                2460
   Asp Val Asn Ser Ala Ala Asp Val Ala Lys Ala Val Arg Asp Ala
       2465                2470                2475
   Glu Ser Gln Leu Gly Ala Arg Val Ser Gly Ile Val His Ala Ser
       2480                2485                2490
   Gly Val Leu Arg Asp Arg Leu Ile Glu Lys Lys Leu Pro Asp Glu
       2495                2500                2505
   Phe Asp Ala Val Phe Gly Thr Lys Val Thr Gly Leu Glu Asn Leu
       2510                2515                2520
   Leu Ala Ala Val Asp Arg Ala Asn Leu Lys His Met Val Leu Phe
       2525                2530                2535
   Ser Ser Leu Ala Gly Phe His Gly Asn Val Gly Gln Ser Asp Tyr
       2540                2545                2550
   Ala Met Ala Asn Glu Ala Leu Asn Lys Met Gly Leu Glu Leu Ala
       2555                2560                2565
   Lys Asp Val Ser Val Lys Ser Ile Cys Phe Gly Pro Trp Asp Gly
       2570                2575                2580
   Gly Met Val Thr Pro Gln Leu Lys Lys Gln Phe Gln Glu Met Gly
       2585                2590                2595
   Val Gln Ile Ile Pro Arg Glu Gly Gly Ala Asp Thr Val Ala Arg
       2600                2605                2610
   Ile Val Leu Gly Ser Ser Pro Ala Glu Ile Leu Val Gly Asn Trp
       2615                2620                2625
   Arg Thr Pro Ser Lys Lys Val Gly Ser Asp Thr Ile Thr Leu His
       2630                2635                2640
   Arg Lys Ile Ser Ala Lys Ser Asn Pro Phe Leu Glu Asp His Val
       2645                2650                2655
   Ile Gln Gly Arg Arg Val Leu Pro Met Thr Leu Ala Ile Gly Ser
       2660                2665                2670
   Leu Ala Glu Thr Cys Leu Gly Leu Phe Pro Gly Tyr Ser Leu Trp
       2675                2680                2685
   Ala Ile  Asp Asp Ala Gln Leu  Phe Lys Gly Val Thr  Val Asp Gly
       2690                 2695                 2700
   Asp Val  Asn Cys Glu Val Thr  Leu Thr Pro Ser Thr  Ala Pro Ser
       2705                 2710                 2715
   Gly Arg  Val Asn Val Gln Ala  Thr Leu Lys Thr Phe  Ser Ser Gly
       2720                 2725                 2730
   Lys Leu  Val Pro Ala Tyr Arg  Ala Val Ile Val Leu  Ser Asn Gln
       2735                 2740                 2745
   Gly Ala  Pro Pro Ala Asn Ala  Thr Met Gln Pro Pro  Ser Leu Asp
       2750                 2755                 2760
   Ala Asp  Pro Ala Leu Gln Gly  Ser Val Tyr Asp Gly  Lys Thr Leu
       2765                 2770                 2775
   Phe His  Gly Pro Ala Phe Arg  Gly Ile Asp Asp Val  Leu Ser Cys
       2780                 2785                 2790
   Thr Lys  Ser Gln Leu Val Ala  Lys Cys Ser Ala Val  Pro Gly Ser
       2795                 2800                 2805
   Asp Ala  Ala Arg Gly Glu Phe  Ala Thr Asp Thr Asp  Ala His Asp
       2810                 2815                 2820
   Pro Phe  Val Asn Asp Leu Ala  Phe Gln Ala Met Leu  Val Trp Val
       2825                 2830                 2835
   Arg Arg  Thr Leu Gly Gln Ala  Ala Leu Pro Asn Ser  Ile Gln Arg
       2840                 2845                 2850
   Ile Val  Gln His Arg Pro Val  Pro Gln Asp Lys Pro  Phe Tyr Ile
       2855                 2860                 2865
   Thr Leu  Arg Ser Asn Gln Ser  Gly Gly His Ser Gln  His Lys His
       2870                 2875                 2880
   Ala Leu  Gln Phe His Asn Glu  Gln Gly Asp Leu Phe  Ile Asp Val
       2885                 2890                 2895
   Gln Ala  Ser Val Ile Ala Thr  Asp Ser Leu Ala Phe
       2900                 2905                 2910
<210> 3
   <211> 6177
   <212> DNA
   <213> Schizochytrium sp.
<220>
   <221> CDS
   <222> (1)..(6177)
   <223>
<400> 3
   atg gcc gct cgg aat gtg agc gcc gcg cat gag atg cac gat gaa aag      48
   Met Ala Ala Arg Asn Val Ser Ala Ala His Glu Met His Asp Glu Lys
   1               5                   10                  15
   cgc atc gcc gtc gtc ggc atg gcc gtc cag tac gcc gga tgc aaa acc      96
   Arg Ile Ala Val Val Gly Met Ala Val Gln Tyr Ala Gly Cys Lys Thr
               20                  25                  30
   aag gac gag ttc tgg gag gtg ctc atg aac ggc aag gtc gag tcc aag     144
   Lys Asp Glu Phe Trp Glu Val Leu Met Asn Gly Lys Val Glu Ser Lys
           35                  40                  45
   gtg atc agc gac aaa cga ctc ggc tcc aac tac cgc gcc gag cac tac      192
   Val Ile Ser Asp Lys Arg Leu Gly Ser Asn Tyr Arg Ala Glu His Tyr
       50                  55                  60
   aaa gca gag cgc agc aag tat gcc gac acc ttt tgc aac gaa acg tac      240
   Lys Ala Glu Arg Ser Lys Tyr Ala Asp Thr Phe Cys Asn Glu Thr Tyr
   65                  70                  75                  80
   ggc acc ctt gac gag aac gag atc gac aac gag cac gaa ctc ctc ctc      288
   Gly Thr Leu Asp Glu Asn Glu Ile Asp Asn Glu His Glu Leu Leu Leu
                   85                  90                  95
   aac ctc gcc aag cag gca ctc gca gag aca tcc gtc aaa gac tcg aca      336
   Asn Leu Ala Lys Gln Ala Leu Ala Glu Thr Ser Val Lys Asp Ser Thr
               100                 105                 110
   cgc tgc ggc atc gtc agc ggc tgc ctc tcg ttc ccc atg gac aac ctc      384
   Arg Cys Gly Ile Val Ser Gly Cys Leu Ser Phe Pro Met Asp Asn Leu
           115                 120                 125
   cag ggt gaa ctc ctc aac gtg tac caa aac cat gtc gag aaa aag ctc      432
   Gln Gly Glu Leu Leu Asn Val Tyr Gln Asn His Val Glu Lys Lys Leu
       130                 135                 140
   ggg gcc cgc gtc ttc aag gac gcc tcc cat tgg tcc gaa cgc gag cag      480
   Gly Ala Arg Val Phe Lys Asp Ala Ser His Trp Ser Glu Arg Glu Gln
   145                 150                 155                 160
   tcc aac aaa ccc gag gcc ggt gac cgc cgc atc ttc atg gac ccg gcc      528
   Ser Asn Lys Pro Glu Ala Gly Asp Arg Arg Ile Phe Met Asp Pro Ala
                   165                 170                 175
   tcc ttc gtc gcc gaa gaa ctc aac ctc ggc gcc ctt cac tac tcc gtc      576
   Ser Phe Val Ala Glu Glu Leu Asn Leu Gly Ala Leu His Tyr Ser Val
               180                 185                 190
   gac gca gca tgc gcc acg gcg ctc tac gtg ctc cgc ctc gcg cag gat      624
   Asp Ala Ala Cys Ala Thr Ala Leu Tyr Val Leu Arg Leu Ala Gln Asp
           195                 200                 205
   cat ctc gtc tcc ggc gcc gcc gac gtc atg ctc tgc ggt gcc acc tgc      672
   His Leu Val Ser Gly Ala Ala Asp Val Met Leu Cys Gly Ala Thr Cys
       210                 215                 220
   ctg ccg gag ccc ttt ttc atc ctt tcg ggc ttt tcc acc ttc cag gcc      720
   Leu Pro Glu Pro Phe Phe Ile Leu Ser Gly Phe Ser Thr Phe Gln Ala
   225                 230                 235                 240
   atg ccc gtc ggc acg ggc cag aac gtg tcc atg ccg ctg cac aag gac      768
   Met Pro Val Gly Thr Gly Gln Asn Val Ser Met Pro Leu His Lys Asp
                   245                 250                 255
   agc cag ggc ctc acc ccg ggt gag ggc ggc tcc atc atg gtc ctc aag      816
   Ser Gln Gly Leu Thr Pro Gly Glu Gly Gly Ser Ile Met Val Leu Lys
               260                 265                 270
   cgt ctc gat gat gcc atc cgc gac ggc gac cac att tac ggc acc ctt      864
   Arg Leu Asp Asp Ala Ile Arg Asp Gly Asp His Ile Tyr Gly Thr Leu
           275                 280                 285
   ctc ggc gcc aat gtc agc aac tcc ggc aca ggt ctg ccc ctc aag ccc      912
   Leu Gly Ala Asn Val Ser Asn Ser Gly Thr Gly Leu Pro Leu Lys Pro
       290                 295                 300
   ctt ctc ccc agc gag aaa aag tgc ctc atg gac acc tac acg cgc att      960
   Leu Leu Pro Ser Glu Lys Lys Cys Leu Met Asp Thr Tyr Thr Arg Ile
   305                 310                 315                 320
   aac gtg cac ccg cac aag att cag tac gtc gag tgc cac gcc acc ggc     1008
   Asn Val His Pro His Lys Ile Gln Tyr Val Glu Cys His Ala Thr Gly
                   325                 330                 335
   acg ccc cag ggt gat cgt gtg gaa atc gac gcc gtc aag gcc tgc ttt     1056
   Thr Pro Gln Gly Asp Arg Val Glu Ile Asp Ala Val Lys Ala Cys Phe
               340                 345                 350
   gaa ggc aag gtc ccc cgt ttc ggt acc aca aag ggc aac ttt gga cac     1104
   Glu Gly Lys Val Pro Arg Phe Gly Thr Thr Lys Gly Asn Phe Gly His
           355                 360                 365
   acc cts gyc gca gcc ggc ttt gcc ggt atg tgc aag gtc ctc ctc tcc     1152
   Thr Xaa Xaa Ala Ala Gly Phe Ala Gly Met Cys Lys Val Leu Leu Ser
       370                 375                 380
   atg aag cat ggc atc atc ccg ccc acc ccg ggt atc gat gac gag acc    1200
   Met Lys His Gly Ile Ile Pro Pro Thr Pro Gly Ile Asp Asp Glu Thr
   385                 390                 395                 400
   aag atg gac cct ctc gtc gtc tcc ggt gag gcc atc cca tgg cca gag    1248
   Lys Met Asp Pro Leu Val Val Ser Gly Glu Ala Ile Pro Trp Pro Glu
                   405                 410                 415
   acc aac ggc gag ccc aag cgc gcc ggt ctc tcg gcc ttt ggc ttt ggt    1296
   Thr Asn Gly Glu Pro Lys Arg Ala Gly Leu Ser Ala Phe Gly Phe Gly
               420                 425                 430
   ggc acc aac gcc cat gcc gtc ttt gag gag cat gac ccc tcc aac gcc    1344
   Gly Thr Asn Ala His Ala Val Phe Glu Glu His Asp Pro Ser Asn Ala
           435                 440                 445
   gcc tgc acg ggc cac gac tcc att tct gcg ctc tcg gcc cgc tgc ggc    1392
   Ala Cys Thr Gly His Asp Ser Ile Ser Ala Leu Ser Ala Arg Cys Gly
       450                 455                 460
   ggt gaa agc aac atg cgc atc gcc atc act ggt atg gac gcc acc ttt    1440
   Gly Glu Ser Asn Met Arg Ile Ala Ile Thr Gly Met Asp Ala Thr Phe
   465                 470                 475                 480
   ggc gct ctc aag gga ctc gac gcc ttc gag cgc gcc att tac acc ggc    1488
   Gly Ala Leu Lys Gly Leu Asp Ala Phe Glu Arg Ala Ile Tyr Thr Gly
                   485                 490                 495
   gct cac ggt gcc atc cca ctc cca gaa aag cgc tgg cgc ttt ctc ggc    1536
   Ala His Gly Ala Ile Pro Leu Pro Glu Lys Arg Trp Arg Phe Leu Gly
               500                 505                 510
   aag gac aag gac ttt ctt gac ctc tgc ggc gtc aag gcc acc ccg cac    1584
   Lys Asp Lys Asp Phe Leu Asp Leu Cys Gly Val Lys Ala Thr Pro His
           515                 520                 525
   ggc tgc tac att gaa gat gtt gag gtc gac ttc cag cgc ctc cgc acg    1632
   Gly Cys Tyr Ile Glu Asp Val Glu Val Asp Phe Gln Arg Leu Arg Thr
       530                 535                 540
   ccc atg acc cct gaa gac atg ctc ctc cct cag cag ctt ctg gcc gtc    1680
   Pro Met Thr Pro Glu Asp Met Leu Leu Pro Gln Gln Leu Leu Ala Val
   545                 550                 555                 560
   acc acc att gac cgc gcc atc ctc gac tcg gga atg aaa aag ggt ggc    1728
   Thr Thr Ile Asp Arg Ala Ile Leu Asp Ser Gly Met Lys Lys Gly Gly
                   565                 570                 575
   aat gtc gcc gtc ttt gtc ggc ctc ggc acc gac ctc gag ctc tac cgt    1776
   Asn Val Ala Val Phe Val Gly Leu Gly Thr Asp Leu Glu Leu Tyr Arg
               580                 585                 590
   cac cgt gct cgc gtc gct ctc aag gag cgc gtc cgc cct gaa gcc tcc    1824
   His Arg Ala Arg Val Ala Leu Lys Glu Arg Val Arg Pro Glu Ala Ser
           595                 600                 605
   aag aag ctc aat gac atg atg cag tac att aac gac tgc ggc aca tcc    1872
   Lys Lys Leu Asn Asp Met Met Gln Tyr Ile Asn Asp Cys Gly Thr Ser
       610                 615                 620
   aca tcg tac acc tcg tac att ggc aac ctc gtc gcc acg cgc gtc tcg    1920
   Thr Ser Tyr Thr Ser Tyr Ile Gly Asn Leu Val Ala Thr Arg Val Ser
   625                 630                 635                 640
   tcg cag tgg ggc ttc acg ggc ccc tcc ttt acg atc acc gag ggc aac    1968
   Ser Gln Trp Gly Phe Thr Gly Pro Ser Phe Thr Ile Thr Glu Gly Asn
                   645                 650                 655
   aac tcc gtc tac cgc tgc gcc gag ctc ggc aag tac ctc ctc gag acc    2016
   Asn Ser Val Tyr Arg Cys Ala Glu Leu Gly Lys Tyr Leu Leu Glu Thr
               660                 665                 670
   ggc gag gtc gat ggc gtc gtc gtt gcg ggt gtc gat ctc tgc ggc agt    2064
   Gly Glu Val Asp Gly Val Val Val Ala Gly Val Asp Leu Cys Gly Ser
           675                 680                 685
   gcc gaa aac ctt tac gtc aag tct cgc cgc ttc aag gtg tcc acc tcc    2112
   Ala Glu Asn Leu Tyr Val Lys Ser Arg Arg Phe Lys Val Ser Thr Ser
       690                 695                 700
   gat acc ccg cgc gcc agc ttt gac gcc gcc gcc gat ggc tac ttt gtc    2160
   Asp Thr Pro Arg Ala Ser Phe Asp Ala Ala Ala Asp Gly Tyr Phe Val
   705                 710                 715                 720
   ggc gag ggc tgc ggt gcc ttt gtg ctc aag cgt gag act agc tgc acc     2208
   Gly Glu Gly Cys Gly Ala Phe Val Leu Lys Arg Glu Thr Ser Cys Thr
                   725                 730                 735
   aag gac gac cgt atc tac gct tgc atg gat gcc atc gtc cct ggc aac     2256
   Lys Asp Asp Arg Ile Tyr Ala Cys Met Asp Ala Ile Val Pro Gly Asn
               740                 745                 750
   gtc cct agc gcc tgc ttg cgc gag gcc ctc gac cag gcg cgc gtc aag     2304
   Val Pro Ser Ala Cys Leu Arg Glu Ala Leu Asp Gln Ala Arg Val Lys
           755                 760                 765
   ccg ggc gat atc gag atg ctc gag ctc agc gcc gac tcc gcc cgc cac     2352
   Pro Gly Asp Ile Glu Met Leu Glu Leu Ser Ala Asp Ser Ala Arg His
       770                 775                 780
   ctc aag gac ccg tcc gtc ctg ccc aag gag ctc act gcc gag gag gaa     2400
   Leu Lys Asp Pro Ser Val Leu Pro Lys Glu Leu Thr Ala Glu Glu Glu
   785                 790                 795                 800
   atc ggc ggc ctt cag acg atc ctt cgt gac gat gac aag ctc ccg cgc     2448
   Ile Gly Gly Leu Gln Thr Ile Leu Arg Asp Asp Asp Lys Leu Pro Arg
                   805                 810                 815
   aac gtc gca acg ggc agt gtc aag gcc acc gtc ggt gac acc ggt tat     2496
   Asn Val Ala Thr Gly Ser Val Lys Ala Thr Val Gly Asp Thr Gly Tyr
               820                 825                 830
   gcc tct ggt gct gcc agc ctc atc aag gct gcg ctt tgc atc tac aac     2544
   Ala Ser Gly Ala Ala Ser Leu Ile Lys Ala Ala Leu Cys Ile Tyr Asn
           835                 840                 845
   cgc tac ctg ccc agc aac ggc gac gac tgg gat gaa ccc gcc cct gag     2592
   Arg Tyr Leu Pro Ser Asn Gly Asp Asp Trp Asp Glu Pro Ala Pro Glu
       850                 855                 860
   gcg ccc tgg gac agc acc ctc ttt gcg tgc cag acc tcg cgc gct tgg     2640
   Ala Pro Trp Asp Ser Thr Leu Phe Ala Cys Gln Thr Ser Arg Ala Trp
   865                 870                 875                 880
   ctc aag aac cct ggc gag cgt cgc tat gcg gcc gtc tcg ggc gtc tcc     2688
   Leu Lys Asn Pro Gly Glu Arg Arg Tyr Ala Ala Val Ser Gly Val Ser
                   885                 890                 895
   gag acg cgc tcg tgc tat tcc gtg ctc ctc tcc gaa gcc gag ggc cac     2736
   Glu Thr Arg Ser Cys Tyr Ser Val Leu Leu Ser Glu Ala Glu Gly His
               900                 905                 910
   tac gag cgc gag aac cgc atc tcg ctc gac gag gag gcg ccc aag ctc     2784
   Tyr Glu Arg Glu Asn Arg Ile Ser Leu Asp Glu Glu Ala Pro Lys Leu
           915                 920                 925
   att gtg ctt cgc gcc gac tcc cac gag gag atc ctt ggt cgc ctc gac     2832
   Ile Val Leu Arg Ala Asp Ser His Glu Glu Ile Leu Gly Arg Leu Asp
       930                 935                 940
   aag atc cgc gag cgc ttc ttg cag ccc acg ggc gcc gcc ccg cgc gag     2880
   Lys Ile Arg Glu Arg Phe Leu Gln Pro Thr Gly Ala Ala Pro Arg Glu
   945                 950                 955                 960
   tcc gag ctc aag gcg cag gcc cgc cgc atc ttc ctc gag ctc ctc ggc     2928
   Ser Glu Leu Lys Ala Gln Ala Arg Arg Ile Phe Leu Glu Leu Leu Gly
                   965                 970                 975
   gag acc ctt gcc cag gat gcc gct tct tca ggc tcg caa aag ccc ctc     2976
   Glu Thr Leu Ala Gln Asp Ala Ala Ser Ser Gly Ser Gln Lys Pro Leu
               980                 985                 990
   gct ctc agc ctc gtc tcc acg ccc  tcc aag ctc cag cgc  gag gtc gag   3024
   Ala Leu Ser Leu Val Ser Thr Pro  Ser Lys Leu Gln Arg  Glu Val Glu
           995                 1000                 1005
   ctc gcg  gcc aag ggt atc ccg  cgc tgc ctc aag atg  cgc cgc gat      3069
   Leu Ala  Ala Lys Gly Ile Pro  Arg Cys Leu Lys Met  Arg Arg Asp
       1010                 1015                 1020
   tgg agc  tcc cct gct ggc agc  cgc tac gcg cct gag  ccg ctc gcc      3114
   Trp Ser  Ser Pro Ala Gly Ser  Arg Tyr Ala Pro Glu  Pro Leu Ala
       1025                 1030                 1035
   agc gac  cgc gtc gcc ttc atg  tac ggc gaa ggt cgc  agc cct tac      3159
   Ser Asp  Arg Val Ala Phe Met  Tyr Gly Glu Gly Arg  Ser Pro Tyr
       1040                 1045                 1050
   tac ggc  atc acc caa gac att  cac cgc att tgg ccc  gaa ctc cac      3204
   Tyr Gly  Ile Thr Gln Asp Ile  His Arg Ile Trp Pro  Glu Leu His
       1055                 1060                 1065
   gag gtc  atc aac gaa aag acg  aac cgt ctc tgg gcc  gaa ggc gac      3249
   Glu Val  Ile Asn Glu Lys Thr  Asn Arg Leu Trp Ala  Glu Gly Asp
       1070                 1075                 1080
   cgc tgg  gtc atg ccg cgc gcc  agc ttc aag tcg gag  ctc gag agc      3294
   Arg Trp  Val Met Pro Arg Ala  Ser Phe Lys Ser Glu  Leu Glu Ser
       1085                 1090                 1095
   cag cag  caa gag ttt gat cgc  aac atg att gaa atg  ttc cgt ctt      3339
   Gln Gln  Gln Glu Phe Asp Arg  Asn Met Ile Glu Met  Phe Arg Leu
       1100                 1105                 1110
   gga atc  ctc acc tca att gcc  ttc acc aat ctg gcg  cgc gac gtt      3384
   Gly Ile  Leu Thr Ser Ile Ala  Phe Thr Asn Leu Ala  Arg Asp Val
       1115                 1120                 1125
   ctc aac  atc acg ccc aag gcc  gcc ttt ggc ctc agt  ctt ggc gag      3429
   Leu Asn  Ile Thr Pro Lys Ala  Ala Phe Gly Leu Ser  Leu Gly Glu
       1130                 1135                 1140
   att tcc  atg att ttt gcc ttt  tcc aag aag aac ggt  ctc atc tcc      3474
   Ile Ser  Met Ile Phe Ala Phe  Ser Lys Lys Asn Gly  Leu Ile Ser
       1145                 1150                 1155
   gac cag  ctc acc aag gat ctt  cgc gag tcc gac gtg  tgg aac aag      3519
   Asp Gln  Leu Thr Lys Asp Leu  Arg Glu Ser Asp Val  Trp Asn Lys
       1160                 1165                 1170
   gct ctg  gcc gtt gaa ttt aat  gcg ctg cgc gag gcc  tgg ggc att      3564
   Ala Leu  Ala Val Glu Phe Asn  Ala Leu Arg Glu Ala  Trp Gly Ile
       1175                 1180                 1185
   cca cag  agt gtc ccc aag gac  gag ttc tgg caa ggc  tac att gtg      3609
   Pro Gln  Ser Val Pro Lys Asp  Glu Phe Trp Gln Gly  Tyr Ile Val
       1190                 1195                 1200
   cgc ggc  acc aag cag gat atc  gag gcg gcc atc gcc  ccg gac agc      3654
   Arg Gly  Thr Lys Gln Asp Ile  Glu Ala Ala Ile Ala  Pro Asp Ser
       1205                 1210                 1215
   aag tac  gtg cgc ctc acc atc  atc aat gat gcc aac  acc gcc ctc      3699
   Lys Tyr  Val Arg Leu Thr Ile  Ile Asn Asp Ala Asn  Thr Ala Leu
       1220                 1225                 1230
   att agc  ggc aag ccc gac gcc  tgc aag gct gcg atc  gcg cgt ctc      3744
   Ile Ser  Gly Lys Pro Asp Ala  Cys Lys Ala Ala Ile  Ala Arg Leu
       1235                 1240                 1245
   ggt ggc  aac att cct gcg ctt  ccc gtg acc cag ggc  atg tgc ggc      3789
   Gly Gly  Asn Ile Pro Ala Leu  Pro Val Thr Gln Gly  Met Cys Gly
       1250                 1255                 1260
   cac tgc  ccc gag gtg gga cct  tat acc aag gat atc  gcc aag atc      3834
   His Cys  Pro Glu Val Gly Pro  Tyr Thr Lys Asp Ile  Ala Lys Ile
       1265                 1270                 1275
   cat gcc  aac ctt gag ttc ccc  gtt gtc gac ggc ctt  gac ctc tgg      3879
   His Ala  Asn Leu Glu Phe Pro  Val Val Asp Gly Leu  Asp Leu Trp
       1280                 1285                 1290
   acc aca  atc aac cag aag cgc  ctc gtg cca cgc gcc  acg ggc gcc      3924
   Thr Thr  Ile Asn Gln Lys Arg  Leu Val Pro Arg Ala  Thr Gly Ala
       1295                 1300                 1305
   aag gac  gaa tgg gcc cct tct  tcc ttt ggc gag tac  gcc ggc cag      3969
   Lys Asp  Glu Trp Ala Pro Ser  Ser Phe Gly Glu Tyr  Ala Gly Gln
       1310                 1315                 1320
   ctc tac  gag aag cag gct aac  ttc ccc caa atc gtc  gag acc att      4014
   Leu Tyr  Glu Lys Gln Ala Asn  Phe Pro Gln Ile Val  Glu Thr Ile
       1325                 1330                 1335
   tac aag  caa aac tac gac gtc  ttt gtc gag gtt ggg  ccc aac aac      4059
   Tyr Lys  Gln Asn Tyr Asp Val  Phe Val Glu Val Gly  Pro Asn Asn
       1340                 1345                 1350
   cac cgt  agc acc gca gtg cgc  acc acg ctt ggt ccc  cag cgc aac      4104
   His Arg Ser Thr Ala Val Arg Thr Thr Leu Gly Pro Gln Arg Asn
       1355                1360                1365
   cac ctt gct ggc gcc atc gac aag cag aac gag gat gct tgg acg    4149
   His Leu Ala Gly Ala Ile Asp Lys Gln Asn Glu Asp Ala Trp Thr
       1370                1375                1380
   acc atc gtc aag ctt gtg gct tcg ctc aag gcc cac ctt gtt cct    4194
   Thr Ile Val Lys Leu Val Ala Ser Leu Lys Ala His Leu Val Pro
       1385                1390                1395
   ggc gtc acg atc tcg ccg ctg tac cac tcc aag ctt gtg gcg gag    4239
   Gly Val Thr Ile Ser Pro Leu Tyr His Ser Lys Leu Val Ala Glu
       1400                1405                1410
   gct cag gct tgc tac gct gcg ctc tgc aag ggt gaa aag ccc aag    4284
   Ala Gln Ala Cys Tyr Ala Ala Leu Cys Lys Gly Glu Lys Pro Lys
       1415                1420                1425
   aag aac aag ttt gtg cgc aag att cag ctc aac ggt cgc ttc aac    4329
   Lys Asn Lys Phe Val Arg Lys Ile Gln Leu Asn Gly Arg Phe Asn
       1430                1435                1440
   agc aag gcg gac ccc atc tcc tcg gcc gat ctt gcc agc ttt ccg    4374
   Ser Lys Ala Asp Pro Ile Ser Ser Ala Asp Leu Ala Ser Phe Pro
       1445                1450                1455
   cct gcg gac cct gcc att gaa gcc gcc atc tcg agc cgc atc atg    4419
   Pro Ala Asp Pro Ala Ile Glu Ala Ala Ile Ser Ser Arg Ile Met
       1460                1465                1470
   aag cct gtc gct ccc aag ttc tac gcg cgt ctc aac att gac gag    4464
   Lys Pro Val Ala Pro Lys Phe Tyr Ala Arg Leu Asn Ile Asp Glu
       1475                1480                1485
   cag gac gag acc cga gat ccg atc ctc aac aag gac aac gcg ccg    4509
   Gln Asp Glu Thr Arg Asp Pro Ile Leu Asn Lys Asp Asn Ala Pro
       1490                1495                1500
   tct tct tct tct tct tct tct tct tct tct tct tct tct tct tct    4554
   Ser Ser Ser Ser Ser Ser Ser Ser Ser Ser Ser Ser Ser Ser Ser
       1505                1510                1515
   ccg tcg cct gct cct tcg gcc ccc gtg caa aag aag gct gct ccc    4599
   Pro Ser Pro Ala Pro Ser Ala Pro Val Gln Lys Lys Ala Ala Pro
       1520                1525                1530
   gcc gcg gag acc aag gct gtt gct tcg gct gac gca ctt cgc agt    4644
   Ala Ala Glu Thr Lys Ala Val Ala Ser Ala Asp Ala Leu Arg Ser
       1535                1540                1545
   gcc ctg ctc gat ctc gac agt atg ctt gcg ctg agc tct gcc agt    4689
   Ala Leu Leu Asp Leu Asp Ser Met Leu Ala Leu Ser Ser Ala Ser
       1550                1555                1560
   gcc tcc ggc aac ctt gtt gag act gcg cct agc gac gcc tcg gtc    4734
   Ala Ser Gly Asn Leu Val Glu Thr Ala Pro Ser Asp Ala Ser Val
       1565                1570                1575
   att gtg ccg ccc tgc aac att gcg gat ctc ggc agc cgc gcc ttc    4779
   Ile Val Pro Pro Cys Asn Ile Ala Asp Leu Gly Ser Arg Ala Phe
       1580                1585                1590
   atg aaa acg tac ggt gtt tcg gcg cct ctg tac acg ggc gcc atg    4824
   Met Lys Thr Tyr Gly Val Ser Ala Pro Leu Tyr Thr Gly Ala Met
       1595                1600                1605
   gcc aag ggc att gcc tct gcg gac ctc gtc att gcc gcc ggc cgc    4869
   Ala Lys Gly Ile Ala Ser Ala Asp Leu Val Ile Ala Ala Gly Arg
       1610                1615                1620
   cag ggc atc ctt gcg tcc ttt ggc gcc ggc gga ctt ccc atg cag    4914
   Gln Gly Ile Leu Ala Ser Phe Gly Ala Gly Gly Leu Pro Met Gln
       1625                1630                1635
   gtt gtg cgt gag tcc atc gaa aag att cag gcc gcc ctg ccc aat    4959
   Val Val Arg Glu Ser Ile Glu Lys Ile Gln Ala Ala Leu Pro Asn
       1640                1645                1650
   ggc ccg tac gct gtc aac ctt atc cat tct ccc ttt gac agc aac    5004
   Gly Pro Tyr Ala Val Asn Leu Ile His Ser Pro Phe Asp Ser Asn
       1655                1660                1665
   ctc gaa aag ggc aat gtc gat ctc ttc ctc gag aag ggt gtc acc    5049
   Leu Glu Lys Gly Asn Val Asp Leu Phe Leu Glu Lys Gly Val Thr
       1670                1675                1680
   ttt gtc gag gcc tcg gcc ttt atg acg ctc acc ccg cag gtc gtg      5094
   Phe Val Glu Ala Ser Ala Phe Met Thr Leu Thr Pro Gln Val Val
       1685                1690                1695
   cgg tac cgc gcg gct ggc ctc acg cgc aac gcc gac ggc tcg gtc      5139
   Arg Tyr Arg Ala Ala Gly Leu Thr Arg Asn Ala Asp Gly Ser Val
       1700                1705                1710
   aac atc cgc aac cgt atc att ggc aag gtc tcg cgc acc gag ctc      5184
   Asn Ile Arg Asn Arg Ile Ile Gly Lys Val Ser Arg Thr Glu Leu
       1715                1720                1725
   gcc gag atg ttc atg cgt cct gcg ccc gag cac ctt ctt cag aag      5229
   Ala Glu Met Phe Met Arg Pro Ala Pro Glu His Leu Leu Gln Lys
       1730                1735                1740
   ctc att gct tcc ggc gag atc aac cag gag cag gcc gag ctc gcc      5274
   Leu Ile Ala Ser Gly Glu Ile Asn Gln Glu Gln Ala Glu Leu Ala
       1745                1750                1755
   cgc cgt gtt ccc gtc gct gac gac atc gcg gtc gaa gct gac tcg      5319
   Arg Arg Val Pro Val Ala Asp Asp Ile Ala Val Glu Ala Asp Ser
       1760                1765                1770
   ggt ggc cac acc gac aac cgc ccc atc cac gtc att ctg ccc ctc      5364
   Gly Gly His Thr Asp Asn Arg Pro Ile His Val Ile Leu Pro Leu
       1775                1780                1785
   atc atc aac ctt cgc gac cgc ctt cac cgc gag tgc ggc tac ccg      5409
   Ile Ile Asn Leu Arg Asp Arg Leu His Arg Glu Cys Gly Tyr Pro
       1790                1795                1800
   gcc aac ctt cgc gtc cgt gtg ggc gcc ggc ggt ggc att ggg tgc      5454
   Ala Asn Leu Arg Val Arg Val Gly Ala Gly Gly Gly Ile Gly Cys
       1805                1810                1815
   ccc cag gcg gcg ctg gcc acc ttc aac atg ggt gcc tcc ttt att      5499
   Pro Gln Ala Ala Leu Ala Thr Phe Asn Met Gly Ala Ser Phe Ile
       1820                1825                1830
   gtc acc ggc acc gtg aac cag gtc gcc aag cag tcg ggc acg tgc      5544
   Val Thr Gly Thr Val Asn Gln Val Ala Lys Gln Ser Gly Thr Cys
       1835                1840                1845
   gac aat gtg cgc aag cag ctc gcg aag gcc act tac tcg gac gta      5589
   Asp Asn Val Arg Lys Gln Leu Ala Lys Ala Thr Tyr Ser Asp Val
       1850                1855                1860
   tgc atg gcc ccg gct gcc gac atg ttc gag gaa ggc gtc aag ctt      5634
   Cys Met Ala Pro Ala Ala Asp Met Phe Glu Glu Gly Val Lys Leu
       1865                1870                1875
   cag gtc ctc aag aag gga acc atg ttt ccc tcg cgc gcc aac aag      5679
   Gln Val Leu Lys Lys Gly Thr Met Phe Pro Ser Arg Ala Asn Lys
       1880                1885                1890
   ctc tac gag ctc ttt tgc aag tac gac tcg ttc gag tcc atg ccc      5724
   Leu Tyr Glu Leu Phe Cys Lys Tyr Asp Ser Phe Glu Ser Met Pro
       1895                1900                1905
   ccc gca gag ctt gcg cgc gtc gag aag cgc atc ttc agc cgc gcg      5769
   Pro Ala Glu Leu Ala Arg Val Glu Lys Arg Ile Phe Ser Arg Ala
       1910                1915                1920
   ctc gaa gag gtc tgg gac gag acc aaa aac ttt tac att aac cgt      5814
   Leu Glu Glu Val Trp Asp Glu Thr Lys Asn Phe Tyr Ile Asn Arg
       1925                1930                1935
   ctt cac aac ccg gag aag atc cag cgc gcc gag cgc gac ccc aag      5859
   Leu His Asn Pro Glu Lys Ile Gln Arg Ala Glu Arg Asp Pro Lys
       1940                1945                1950
   ctc aag atg tcg ctg tgc ttt cgc tgg tac ctg agc ctg gcg agc      5904
   Leu Lys Met Ser Leu Cys Phe Arg Trp Tyr Leu Ser Leu Ala Ser
       1955                1960                1965
   cgc tgg gcc aac act gga gct tcc gat cgc gtc atg gac tac cag      5949
   Arg Trp Ala Asn Thr Gly Ala Ser Asp Arg Val Met Asp Tyr Gln
       1970                1975                1980
   gtc tgg tgc ggt cct gcc att ggt tcc ttc aac gat ttc atc aag      5994
   Val Trp Cys Gly Pro Ala Ile Gly Ser Phe Asn Asp Phe Ile Lys
       1985                1990                1995
   gga act tac ctt gat ccg gcc gtc gca aac gag tac ccg tgc gtc      6039
   Gly Thr Tyr Leu Asp Pro Ala Val Ala Asn Glu Tyr Pro Cys Val
       2000                2005                2010
   gtt cag att aac aag cag atc ctt cgt gga gcg tgc ttc ttg cgc      6084
   Val Gln Ile Asn Lys Gln Ile Leu Arg Gly Ala Cys Phe Leu Arg
       2015                2020                2025
   cgt ctc gaa att ctg cgc aac gca cgc ctt tcc gat ggc gct gcc      6129
   Arg Leu Glu Ile Leu Arg Asn Ala Arg Leu Ser Asp Gly Ala Ala
       2030                2035                2040
   gct ctt gtg gcc agc atc gat gac aca tac gtc ccg gcc gag aag      6174
   Ala Leu Val Ala Ser Ile Asp Asp Thr Tyr Val Pro Ala Glu Lys
       2045                2050                2055
   ctg                                                              6177
   Leu
<210> 4
   <211> 2059
   <212> PRT
   <213> Schizochytrium sp.
<220>
   <221> misc_feature
   <222> (370)..(370)
   <223> The 'Xaa' at location 370 stands for Leu.
<220>
   <221> misc_feature
   <222> (371)..(371)
   <223> The 'Xaa' at location 371 stands for Ala, or Val.
<400> 4
   Met Ala Ala Arg Asn Val Ser Ala Ala His Glu Met His Asp Glu Lys
   1               5                   10                  15
   Arg Ile Ala Val Val Gly Met Ala Val Gln Tyr Ala Gly Cys Lys Thr
               20                  25                  30
   Lys Asp Glu Phe Trp Glu Val Leu Met Asn Gly Lys Val Glu Ser Lys
           35                  40                  45
   Val Ile Ser Asp Lys Arg Leu Gly Ser Asn Tyr Arg Ala Glu His Tyr
       50                  55                  60
   Lys Ala Glu Arg Ser Lys Tyr Ala Asp Thr Phe Cys Asn Glu Thr Tyr
   65                  70                  75                  80
   Gly Thr Leu Asp Glu Asn Glu Ile Asp Asn Glu His Glu Leu Leu Leu
                   85                  90                  95
   Asn Leu Ala Lys Gln Ala Leu Ala Glu Thr Ser Val Lys Asp Ser Thr
               100                 105                 110
   Arg Cys Gly Ile Val Ser Gly Cys Leu Ser Phe Pro Met Asp Asn Leu
           115                 120                 125
   Gln Gly Glu Leu Leu Asn Val Tyr Gln Asn His Val Glu Lys Lys Leu
       130                 135                 140
   Gly Ala Arg Val Phe Lys Asp Ala Ser His Trp Ser Glu Arg Glu Gln
   145                 150                 155                 160
   Ser Asn Lys Pro Glu Ala Gly Asp Arg Arg Ile Phe Met Asp Pro Ala
   165 170 175
   Ser Phe Val Ala Glu Glu Leu Asn Leu Gly Ala Leu His Tyr Ser Val
   180 185 190
   Asp Ala Ala Cys Ala Thr Ala Leu Tyr Val Leu Arg Leu Ala Gln Asp
   195 200 205
   His Leu Val Ser Gly Ala Ala Asp Val Met Leu Cys Gly Ala Thr Cys
   210 215 220
   Leu Pro Glu Pro Phe Phe Ile Leu Ser Gly Phe Ser Thr Phe Gln Ala
   225 230 235 240
   Met Pro Val Gly Thr Gly Gln Asn Val Ser Met Pro Leu His Lys Asp
   245 250 255
   Ser Gln Gly Leu Thr Pro Gly Glu Gly Gly Ser Ile Met Val Leu Lys
   260 265 270
   Arg Leu Asp Asp Ala Ile Arg Asp Gly Asp His Ile Tyr Gly Thr Leu
   275 280 285
   Leu Gly Ala Asn Val Ser Asn Ser Gly Thr Gly Leu Pro Leu Lys Pro
   290 295 300
   Leu Leu Pro Ser Glu Lys Lys Cys Leu Met Asp Thr Tyr Thr Arg Ile
   305 310 315 320
   Asn Val His Pro His Lys Ile Gln Tyr Val Glu Cys His Ala Thr Gly
   325 330 335
   Thr Pro Gln Gly Asp Arg Val Glu Ile Asp Ala Val Lys Ala Cys Phe
   340 345 350
   Glu Gly Lys Val Pro Arg Phe Gly Thr Thr Lys Gly Asn Phe Gly His
   355 360 365
   Thr Xaa Xaa Ala Ala Gly Phe Ala Gly Met Cys Lys Val Leu Leu Ser
   370 375 380
   Met Lys His Gly Ile Ile Pro Pro Thr Pro Gly Ile Asp Asp Glu Thr
   385 390 395 400
   Lys Met Asp Pro Leu Val Val Ser Gly Glu Ala Ile Pro Trp Pro Glu
   405 410 415
   Thr Asn Gly Glu Pro Lys Arg Ala Gly Leu Ser Ala Phe Gly Phe Gly
   420 425 430
   Gly Thr Asn Ala His Ala Val Phe Glu Glu His Asp Pro Ser Asn Ala
   435 440 445
   Ala Cys Thr Gly His Asp Ser Ile Ser Ala Leu Ser Ala Arg Cys Gly
   450 455 460
   Gly Glu Ser Asn Met Arg Ile Ala Ile Thr Gly Met Asp Ala Thr Phe
   465 470 475 480
   Gly Ala Leu Lys Gly Leu Asp Ala Phe Glu Arg Ala Ile Tyr Thr Gly
   485 490 495
   Ala His Gly Ala Ile Pro Leu Pro Glu Lys Arg Trp Arg Phe Leu Gly
   500 505 510
   Lys Asp Lys Asp Phe Leu Asp Leu Cys Gly Val Lys Ala Thr Pro His
   515 520 525
   Gly Cys Tyr Ile Glu Asp Val Glu Val Asp Phe Gln Arg Leu Arg Thr
   530 535 540
   Pro Met Thr Pro Glu Asp Met Leu Leu Pro Gln Gln Leu Leu Ala Val
   545 550 555 560
   Thr Thr Ile Asp Arg Ala Ile Leu Asp Ser Gly Met Lys Lys Gly Gly
   565 570 575
   Asn Val Ala Val Phe Val Gly Leu Gly Thr Asp Leu Glu Leu Tyr Arg
   580 585 590
   His Arg Ala Arg Val Ala Leu Lys Glu Arg Val Arg Pro Glu Ala Ser
   595 600 605
   Lys Lys Leu Asn Asp Met Met Gln Tyr Ile Asn Asp Cys Gly Thr Ser
   610 615 620
   Thr Ser Tyr Thr Ser Tyr Ile Gly Asn Leu Val Ala Thr Arg Val Ser
   625 630 635 640
   Ser Gln Trp Gly Phe Thr Gly Pro Ser Phe Thr Ile Thr Glu Gly Asn
   645 650 655
   Asn Ser Val Tyr Arg Cys Ala Glu Leu Gly Lys Tyr Leu Leu Glu Thr
   660 665 670
   Gly Glu Val Asp Gly Val Val Val Ala Gly Val Asp Leu Cys Gly Ser
   675 680 685
   Ala Glu Asn Leu Tyr Val Lys Ser Arg Arg Phe Lys Val Ser Thr Ser
   690 695 700
   Asp Thr Pro Arg Ala Ser Phe Asp Ala Ala Ala Asp Gly Tyr Phe Val
   705 710 715 720
   Gly Glu Gly Cys Gly Ala Phe Val Leu Lys Arg Glu Thr Ser Cys Thr
   725 730 735
   Lys Asp Asp Arg Ile Tyr Ala Cys Met Asp Ala Ile Val Pro Gly Asn
   740 745 750
   Val Pro Ser Ala Cys Leu Arg Glu Ala Leu Asp Gln Ala Arg Val Lys
   755 760 765
   Pro Gly Asp Ile Glu Met Leu Glu Leu Ser Ala Asp Ser Ala Arg His
   770 775 780
   Leu Lys Asp Pro Ser Val Leu Pro Lys Glu Leu Thr Ala Glu Glu Glu
   785 790 795 800
   Ile Gly Gly Leu Gln Thr Ile Leu Arg Asp Asp Asp Lys Leu Pro Arg
   805 810 815
   Asn Val Ala Thr Gly Ser Val Lys Ala Thr Val Gly Asp Thr Gly Tyr
   820 825 830
   Ala Ser Gly Ala Ala Ser Leu Ile Lys Ala Ala Leu Cys Ile Tyr Asn
   835 840 845
   Arg Tyr Leu Pro Ser Asn Gly Asp Asp Trp Asp Glu Pro Ala Pro Glu
   850 855 860
   Ala Pro Trp Asp Ser Thr Leu Phe Ala Cys Gln Thr Ser Arg Ala Trp
   865 870 875 880
   Leu Lys Asn Pro Gly Glu Arg Arg Tyr Ala Ala Val Ser Gly Val Ser
   885 890 895
   Glu Thr Arg Ser Cys Tyr Ser Val Leu Leu Ser Glu Ala Glu Gly His
   900 905 910
   Tyr Glu Arg Glu Asn Arg Ile Ser Leu Asp Glu Glu Ala Pro Lys Leu
   915 920 925
   Ile Val Leu Arg Ala Asp Ser His Glu Glu Ile Leu Gly Arg Leu Asp
   930 935 940
   Lys Ile Arg Glu Arg Phe Leu Gln Pro Thr Gly Ala Ala Pro Arg Glu
   945 950 955 960
   Ser Glu Leu Lys Ala Gln Ala Arg Arg Ile Phe Leu Glu Leu Leu Gly
   965 970 975
   Glu Thr Leu Ala Gln Asp Ala Ala Ser Ser Gly Ser Gln Lys Pro Leu
   980 985 990
   Ala Leu Ser Leu Val Ser Thr Pro Ser Lys Leu Gln Arg Glu Val Glu
   995 1000 1005
   Leu Ala Ala Lys Gly Ile Pro Arg Cys Leu Lys Met Arg Arg Asp
   1010 1015 1020
   Trp Ser Ser Pro Ala Gly Ser Arg Tyr Ala Pro Glu Pro Leu Ala
   1025 1030 1035
   Ser Asp Arg Val Ala Phe Met Tyr Gly Glu Gly Arg Ser Pro Tyr
   1040 1045 1050
   Tyr Gly Ile Thr Gln Asp Ile His Arg Ile Trp Pro Glu Leu His
   1055 1060 1065
   Glu Val Ile Asn Glu Lys Thr Asn Arg Leu Trp Ala Glu Gly Asp
   1070 1075 1080
   Arg Trp Val Met Pro Arg Ala Ser Phe Lys Ser Glu Leu Glu Ser
   1085 1090 1095
   Gln Gln Gln Glu Phe Asp Arg Asn Met Ile Glu Met Phe Arg Leu
   1100 1105 1110
   Gly Ile Leu Thr Ser Ile Ala Phe Thr Asn Leu Ala Arg Asp Val
   1115 1120 1125
   Leu Asn Ile Thr Pro Lys Ala Ala Phe Gly Leu Ser Leu Gly Glu
   1130 1135 1140
   Ile Ser Met Ile Phe Ala Phe Ser Lys Lys Asn Gly Leu Ile Ser
   1145 1150 1155
   Asp Gln Leu Thr Lys Asp Leu Arg Glu Ser Asp Val Trp Asn Lys
   1160 1165 1170
   Ala Leu Ala Val Glu Phe Asn Ala Leu Arg Glu Ala Trp Gly Ile
   1175 1180 1185
   Pro Gln Ser Val Pro Lys Asp Glu Phe Trp Gln Gly Tyr Ile Val
   1190 1195 1200
   Arg Gly Thr Lys Gln Asp Ile Glu Ala Ala Ile Ala Pro Asp Ser
   1205 1210 1215
   Lys Tyr Val Arg Leu Thr Ile Ile Asn Asp Ala Asn Thr Ala Leu
   1220 1225 1230
   Ile Ser Gly Lys Pro Asp Ala Cys Lys Ala Ala Ile Ala Arg Leu
   1235 1240 1245
   Gly Gly Asn Ile Pro Ala Leu Pro Val Thr Gln Gly Met Cys Gly
   1250 1255 1260
   His Cys Pro Glu Val Gly Pro Tyr Thr Lys Asp Ile Ala Lys Ile
   1265 1270 1275
   His Ala Asn Leu Glu Phe Pro Val Val Asp Gly Leu Asp Leu Trp
   1280 1285 1290
   Thr Thr Ile Asn Gln Lys Arg Leu Val Pro Arg Ala Thr Gly Ala
   1295 1300 1305
   Lys Asp Glu Trp Ala Pro Ser Ser Phe Gly Glu Tyr Ala Gly Gln
   1310 1315 1320
   Leu Tyr Glu Lys Gln Ala Asn Phe Pro Gln Ile Val Glu Thr Ile
   1325 1330 1335
   Tyr Lys Gln Asn Tyr Asp Val Phe Val Glu Val Gly Pro Asn Asn
   1340 1345 1350
   His Arg Ser Thr Ala Val Arg Thr Thr Leu Gly Pro Gln Arg Asn
   1355 1360 1365
   His Leu Ala Gly Ala Ile Asp Lys Gln Asn Glu Asp Ala Trp Thr
   1370 1375 1380
   Thr Ile Val Lys Leu Val Ala Ser Leu Lys Ala His Leu Val Pro
   1385 1390 1395
   Gly Val Thr Ile Ser Pro Leu Tyr His Ser Lys Leu Val Ala Glu
   1400 1405 1410
   Ala Gln Ala Cys Tyr Ala Ala Leu Cys Lys Gly Glu Lys Pro Lys
   1415 1420 1425
   Lys Asn Lys Phe Val Arg Lys Ile Gln Leu Asn Gly Arg Phe Asn
   1430 1435 1440
   Ser Lys Ala Asp Pro Ile Ser Ser Ala Asp Leu Ala Ser Phe Pro
   1445 1450 1455
   Pro Ala Asp Pro Ala Ile Glu Ala Ala Ile Ser Ser Arg Ile Met
   1460 1465 1470
   Lys Pro Val Ala Pro Lys Phe Tyr Ala Arg Leu Asn Ile Asp Glu
   1475 1480 1485
   Gln Asp Glu Thr Arg Asp Pro Ile Leu Asn Lys Asp Asn Ala Pro
   1490 1495 1500
   Ser Ser Ser Ser Ser Ser Ser Ser Ser Ser Ser Ser Ser Ser Ser
   1505 1510 1515
   Pro Ser Pro Ala Pro Ser Ala Pro Val Gln Lys Lys Ala Ala Pro
   1520 1525 1530
   Ala Ala Glu Thr Lys Ala Val Ala Ser Ala Asp Ala Leu Arg Ser
   1535 1540 1545
   Ala Leu Leu Asp Leu Asp Ser Met Leu Ala Leu Ser Ser Ala Ser
   1550 1555 1560
   Ala Ser Gly Asn Leu Val Glu Thr Ala Pro Ser Asp Ala Ser Val
   1565 1570 1575
   Ile Val Pro Pro Cys Asn Ile Ala Asp Leu Gly Ser Arg Ala Phe
   1580 1585 1590
   Met Lys Thr Tyr Gly Val Ser Ala Pro Leu Tyr Thr Gly Ala Met
   1595 1600 1605
   Ala Lys Gly Ile Ala Ser Ala Asp Leu Val Ile Ala Ala Gly Arg
   1610 1615 1620
   Gln Gly Ile Leu Ala Ser Phe Gly Ala Gly Gly Leu Pro Met Gln
   1625 1630 1635
   Val Val Arg Glu Ser Ile Glu Lys Ile Gln Ala Ala Leu Pro Asn
   1640 1645 1650
   Gly Pro Tyr Ala Val Asn Leu Ile His Ser Pro Phe Asp Ser Asn
   1655 1660 1665
   Leu Glu Lys Gly Asn Val Asp Leu Phe Leu Glu Lys Gly Val Thr
   1670 1675 1680
   Phe Val Glu Ala Ser Ala Phe Met Thr Leu Thr Pro Gln Val Val
   1685 1690 1695
   Arg Tyr Arg Ala Ala Gly Leu Thr Arg Asn Ala Asp Gly Ser Val
   1700 1705 1710
   Asn Ile Arg Asn Arg Ile Ile Gly Lys Val Ser Arg Thr Glu Leu
   1715 1720 1725
   Ala Glu Met Phe Met Arg Pro Ala Pro Glu His Leu Leu Gln Lys
   1730 1735 1740
   Leu Ile Ala Ser Gly Glu Ile Asn Gln Glu Gln Ala Glu Leu Ala
   1745 1750 1755
   Arg Arg Val Pro Val Ala Asp Asp Ile Ala Val Glu Ala Asp Ser
   1760 1765 1770
   Gly Gly His Thr Asp Asn Arg Pro Ile His Val Ile Leu Pro Leu
   1775 1780 1785
   Ile Ile Asn Leu Arg Asp Arg Leu His Arg Glu Cys Gly Tyr Pro
   1790 1795 1800
   Ala Asn Leu Arg Val Arg Val Gly Ala Gly Gly Gly Ile Gly Cys
   1805 1810 1815
   Pro Gln Ala Ala Leu Ala Thr Phe Asn Met Gly Ala Ser Phe Ile
   1820 1825 1830
   Val Thr Gly Thr Val Asn Gln Val Ala Lys Gln Ser Gly Thr Cys
   1835 1840 1845
   Asp Asn Val Arg Lys Gln Leu Ala Lys Ala Thr Tyr Ser Asp Val
   1850 1855 1860
   Cys Met Ala Pro Ala Ala Asp Met Phe Glu Glu Gly Val Lys Leu
   1865 1870 1875
   Gln Val Leu Lys Lys Gly Thr Met Phe Pro Ser Arg Ala Asn Lys
   1880 1885 1890
   Leu Tyr Glu Leu Phe Cys Lys Tyr Asp Ser Phe Glu Ser Met Pro
   1895 1900 1905
   Pro Ala Glu Leu Ala Arg Val Glu Lys Arg Ile Phe Ser Arg Ala
   1910 1915 1920
   Leu Glu Glu Val Trp Asp Glu Thr Lys Asn Phe Tyr Ile Asn Arg
   1925 1930 1935
   Leu His Asn Pro Glu Lys Ile Gln Arg Ala Glu Arg Asp Pro Lys
   1940 1945 1950
   Leu Lys Met Ser Leu Cys Phe Arg Trp Tyr Leu Ser Leu Ala Ser
   1955 1960 1965
   Arg Trp Ala Asn Thr Gly Ala Ser Asp Arg Val Met Asp Tyr Gln
   1970 1975 1980
   Val Trp Cys Gly Pro Ala Ile Gly Ser Phe Asn Asp Phe Ile Lys
   1985 1990 1995
   Gly Thr Tyr Leu Asp Pro Ala Val Ala Asn Glu Tyr Pro Cys Val
   2000 2005 2010
   Val Gln Ile Asn Lys Gln Ile Leu Arg Gly Ala Cys Phe Leu Arg
   2015 2020 2025
   Arg Leu Glu Ile Leu Arg Asn Ala Arg Leu Ser Asp Gly Ala Ala
   2030 2035 2040
   Ala Leu Val Ala Ser Ile Asp Asp Thr Tyr Val Pro Ala Glu Lys
   2045 2050 2055
   Leu
<210> 5
   <211> 4509
   <212> DNA
   <213> Schizochytrium sp.
<220>
   <221> CDS
   <222> (1)..(4509)
   <223>
<400> 5
   atg gcg ctc cgt gtc aag acg aac aag aag cca tgc tgg gag atg acc       48
   Met Ala Leu Arg Val Lys Thr Asn Lys Lys Pro Cys Trp Glu Met Thr
   1               5                   10                  15
   aag gag gag ctg acc agc ggc aag acc gag gtg ttc aac tat gag gaa       96
   Lys Glu Glu Leu Thr Ser Gly Lys Thr Glu Val Phe Asn Tyr Glu Glu
               20                  25                  30
   ctc ctc gag ttc gca gag ggc gac atc gcc aag gtc ttc gga ccc gag      144
   Leu Leu Glu Phe Ala Glu Gly Asp Ile Ala Lys Val Phe Gly Pro Glu
           35                  40                  45
   ttc gcc gtc atc gac aag tac ccg cgc cgc gtg cgc ctg ccc gcc cgc      192
   Phe Ala Val Ile Asp Lys Tyr Pro Arg Arg Val Arg Leu Pro Ala Arg
       50                  55                  60
   gag tac ctg ctc gtg acc cgc gtc acc ctc atg gac gcc gag gtc aac      240
   Glu Tyr Leu Leu Val Thr Arg Val Thr Leu Met Asp Ala Glu Val Asn
   65                  70                  75                  80
   aac tac cgc gtc ggc gcc cgc atg gtc acc gag tac gat ctc ccc gtc      288
   Asn Tyr Arg Val Gly Ala Arg Met Val Thr Glu Tyr Asp Leu Pro Val
                   85                  90                  95
   aac gga gag ctc tcc gag ggc gga gac tgc ccc tgg gcc gtc ctg gtc      336
   Asn Gly Glu Leu Ser Glu Gly Gly Asp Cys Pro Trp Ala Val Leu Val
               100                 105                 110
   gag agt ggc cag tgc gat ctc atg ctc atc tcc tac atg ggc att gac      384
   Glu Ser Gly Gln Cys Asp Leu Met Leu Ile Ser Tyr Met Gly Ile Asp
           115                 120                 125
   ttc cag aac cag ggc gac cgc gtc tac cgc ctg ctc aac acc acg ctc      432
   Phe Gln Asn Gln Gly Asp Arg Val Tyr Arg Leu Leu Asn Thr Thr Leu
       130                 135                 140
   acc ttt tac ggc gtg gcc cac gag ggc gag acc ctc gag tac gac att      480
   Thr Phe Tyr Gly Val Ala His Glu Gly Glu Thr Leu Glu Tyr Asp Ile
   145                 150                 155                 160
   cgc gtc acc ggc ttc gcc aag cgt ctc gac ggc ggc atc tcc atg ttc      528
   Arg Val Thr Gly Phe Ala Lys Arg Leu Asp Gly Gly Ile Ser Met Phe
                   165                 170                 175
   ttc ttc gag tac gac tgc tac gtc aac ggc cgc ctc ctc atc gag atg      576
   Phe Phe Glu Tyr Asp Cys Tyr Val Asn Gly Arg Leu Leu Ile Glu Met
               180                 185                 190
   cgc gat ggc tgc gcc ggc ttc ttc acc aac gag gag ctc gac gcc ggc      624
   Arg Asp Gly Cys Ala Gly Phe Phe Thr Asn Glu Glu Leu Asp Ala Gly
           195                 200                 205
   aag ggc gtc gtc ttc acc cgc ggc gac ctc gcc gcc cgc gcc aag atc      672
   Lys Gly Val Val Phe Thr Arg Gly Asp Leu Ala Ala Arg Ala Lys Ile
       210                 215                 220
   cca aag cag gac gtc tcc ccc tac gcc gtc gcc ccc tgc ctc cac aag      720
   Pro Lys Gln Asp Val Ser Pro Tyr Ala Val Ala Pro Cys Leu His Lys
   225                 230                 235                 240
   acc aag ctc aac gaa aag gag atg cag acc ctc gtc gac aag gac tgg      768
   Thr Lys Leu Asn Glu Lys Glu Met Gln Thr Leu Val Asp Lys Asp Trp
                   245                 250                 255
   gca tcc gtc ttt ggc tcc aag aac ggc atg ccg gaa atc aac tac aaa      816
   Ala Ser Val Phe Gly Ser Lys Asn Gly Met Pro Glu Ile Asn Tyr Lys
               260                 265                 270
   ctc tgc gcg cgt aag atg ctc atg att gac cgc gtc acc agc att gac      864
   Leu Cys Ala Arg Lys Met Leu Met Ile Asp Arg Val Thr Ser Ile Asp
           275                 280                 285
   cac aag ggc ggt gtc tac ggc ctc ggt cag ctc gtc ggt gaa aag atc      912
   His Lys Gly Gly Val Tyr Gly Leu Gly Gln Leu Val Gly Glu Lys Ile
       290                 295                 300
   ctc gag cgc gac cac tgg tac ttt ccc tgc cac ttt gtc aag gat cag      960
   Leu Glu Arg Asp His Trp Tyr Phe Pro Cys His Phe Val Lys Asp Gln
   305                 310                 315                 320
   gtc atg gcc gga tcc ctc gtc tcc gac ggc tgc agc cag atg ctc aag      1008
   Val Met Ala Gly Ser Leu Val Ser Asp Gly Cys Ser Gln Met Leu Lys
                   325                 330                 335
   atg tac atg atc tgg ctc ggc ctc cac ctc acc acc gga ccc ttt gac      1056
   Met Tyr Met Ile Trp Leu Gly Leu His Leu Thr Thr Gly Pro Phe Asp
               340                 345                 350
   ttc cgc ccg gtc aac ggc cac ccc aac aag gtc cgc tgc cgc ggc caa      1104
   Phe Arg Pro Val Asn Gly His Pro Asn Lys Val Arg Cys Arg Gly Gln
           355                 360                 365
   atc tcc ccg cac aag ggc aag ctc gtc tac gtc atg gag atc aag gag      1152
   Ile Ser Pro His Lys Gly Lys Leu Val Tyr Val Met Glu Ile Lys Glu
       370                 375                 380
   atg ggc ttc gac gag gac aac gac ccg tac gcc att gcc gac gtc aac      1200
   Met Gly Phe Asp Glu Asp Asn Asp Pro Tyr Ala Ile Ala Asp Val Asn
   385                 390                 395                 400
   atc att gat gtc gac ttc gaa aag ggc cag gac ttt agc ctc gac cgc      1248
   Ile Ile Asp Val Asp Phe Glu Lys Gly Gln Asp Phe Ser Leu Asp Arg
                   405                 410                 415
   atc agc gac tac ggc aag ggc gac ctc aac aag aag atc gtc gtc gac      1296
   Ile Ser Asp Tyr Gly Lys Gly Asp Leu Asn Lys Lys Ile Val Val Asp
               420                 425                 430
   ttt aag ggc atc gct ctc aag atg cag aag cgc tcc acc aac aag aac      1344
   Phe Lys Gly Ile Ala Leu Lys Met Gln Lys Arg Ser Thr Asn Lys Asn
           435                 440                 445
   ccc tcc aag gtt cag ccc gtc ttt gcc aac ggc gcc gcc act gtc ggc      1392
   Pro Ser Lys Val Gln Pro Val Phe Ala Asn Gly Ala Ala Thr Val Gly
       450                 455                 460
   ccc gag gcc tcc aag gct tcc tcc ggc gcc agc gcc agc gcc agc gcc      1440
   Pro Glu Ala Ser Lys Ala Ser Ser Gly Ala Ser Ala Ser Ala Ser Ala
   465                 470                 475                 480
   gcc ccg gcc aag cct gcc ttc agc gcc gat gtt ctt gcg ccc aag ccc      1488
   Ala Pro Ala Lys Pro Ala Phe Ser Ala Asp Val Leu Ala Pro Lys Pro
                   485                 490                 495
   gtt gcc ctt ccc gag cac atc ctc aag ggc gac gcc ctc gcc ccc aag      1536
   Val Ala Leu Pro Glu His Ile Leu Lys Gly Asp Ala Leu Ala Pro Lys
               500                 505                 510
   gag atg tcc tgg cac ccc atg gcc cgc atc ccg ggc aac ccg acg ccc      1584
   Glu Met Ser Trp His Pro Met Ala Arg Ile Pro Gly Asn Pro Thr Pro
           515                 520                 525
   tct ttt gcg ccc tcg gcc tac aag ccg cgc aac atc gcc ttt acg ccc      1632
   Ser Phe Ala Pro Ser Ala Tyr Lys Pro Arg Asn Ile Ala Phe Thr Pro
       530                 535                 540
   ttc ccc ggc aac ccc aac gat aac gac cac acc ccg ggc aag atg ccg      1680
   Phe Pro Gly Asn Pro Asn Asp Asn Asp His Thr Pro Gly Lys Met Pro
   545                 550                 555                 560
   ctc acc tgg ttc aac atg gcc gag ttc atg gcc ggc aag gtc agc atg      1728
   Leu Thr Trp Phe Asn Met Ala Glu Phe Met Ala Gly Lys Val Ser Met
                   565                 570                 575
   tgc ctc ggc ccc gag ttc gcc aag ttc gac gac tcg aac acc agc cgc      1776
   Cys Leu Gly Pro Glu Phe Ala Lys Phe Asp Asp Ser Asn Thr Ser Arg
               580                 585                 590
   agc ccc gct tgg gac ctc gct ctc gtc acc cgc gcc gtg tct gtg tct      1824
   Ser Pro Ala Trp Asp Leu Ala Leu Val Thr Arg Ala Val Ser Val Ser
           595                 600                 605
   gac ctc aag cac gtc aac tac cgc aac atc gac ctc gac ccc tcc aag      1872
   Asp Leu Lys His Val Asn Tyr Arg Asn Ile Asp Leu Asp Pro Ser Lys
       610                 615                 620
   ggt acc atg gtc ggc gag ttc gac tgc ccc gcg gac gcc tgg ttc tac      1920
   Gly Thr Met Val Gly Glu Phe Asp Cys Pro Ala Asp Ala Trp Phe Tyr
   625                 630                 635                 640
   aag ggc gcc tgc aac gat gcc cac atg ccg tac tcg atc ctc atg gag      1968
   Lys Gly Ala Cys Asn Asp Ala His Met Pro Tyr Ser Ile Leu Met Glu
                       645                     650                     655
   atc gcc ctc cag acc tcg ggt gtg ctc acc tcg gtg ctc aag gcg ccc      2016
   Ile Ala Leu Gln Thr Ser Gly Val Leu Thr Ser Val Leu Lys Ala Pro
                   660                     665                     670
   ctg acc atg gag aag gac gac atc ctc ttc cgc aac ctc gac gcc aac      2064
   Leu Thr Met Glu Lys Asp Asp Ile Leu Phe Arg Asn Leu Asp Ala Asn
               675                     680                     685
   gcc gag ttc gtg cgc gcc gac ctc gac tac cgc ggc aag act atc cgc      2112
   Ala Glu Phe Val Arg Ala Asp Leu Asp Tyr Arg Gly Lys Thr Ile Arg
       690                     695                     700
   aac gtc acc aag tgc act ggc tac agc atg ctc ggc gag atg ggc gtc      2160
   Asn Val Thr Lys Cys Thr Gly Tyr Ser Met Leu Gly Glu Met Gly Val
   705                     710                     715                     720
   cac cgc ttc acc ttt gag ctc tac gtc gat gat gtg ctc ttt tac aag      2208
   His Arg Phe Thr Phe Glu Leu Tyr Val Asp Asp Val Leu Phe Tyr Lys
                       725                     730                     735
   ggc tcg acc tcg ttc ggc tgg ttc gtg ccc gag gtc ttt gcc gcc cag      2256
   Gly Ser Thr Ser Phe Gly Trp Phe Val Pro Glu Val Phe Ala Ala Gln
                   740                     745                     750
   gcc ggc ctc gac aac ggc cgc aag tcg gag ccc tgg ttc att gag aac      2304
   Ala Gly Leu Asp Asn Gly Arg Lys Ser Glu Pro Trp Phe Ile Glu Asn
               755                     760                     765
   aag gtt ccg gcc tcg cag gtc tcc tcc ttt gac gtg cgc ccc aac ggc      2352
   Lys Val Pro Ala Ser Gln Val Ser Ser Phe Asp Val Arg Pro Asn Gly
       770                     775                     780
   agc ggc cgc acc gcc atc ttc gcc aac gcc ccc agc ggc gcc cag ctc      2400
   Ser Gly Arg Thr Ala Ile Phe Ala Asn Ala Pro Ser Gly Ala Gln Leu
   785                     790                     795                     800
   aac cgc cgc acg gac cag ggc cag tac ctc gac gcc gtc gac att gtc      2448
   Asn Arg Arg Thr Asp Gln Gly Gln Tyr Leu Asp Ala Val Asp Ile Val
                       805                     810                     815
   tcc ggc agc ggc aag aag agc ctc ggc tac gcc cac ggt tcc aag acg      2496
   Ser Gly Ser Gly Lys Lys Ser Leu Gly Tyr Ala His Gly Ser Lys Thr
                   820                     825                     830
   gtc aac ccg aac gac tgg ttc ttc tcg tgc cac ttt tgg ttt gac tcg      2544
   Val Asn Pro Asn Asp Trp Phe Phe Ser Cys His Phe Trp Phe Asp Ser
               835                     840                     845
   gtc atg ccc gga agt ctc ggt gtc gag tcc atg ttc cag ctc gtc gag      2592
   Val Met Pro Gly Ser Leu Gly Val Glu Ser Met Phe Gln Leu Val Glu
       850                     855                     860
   gcc atc gcc gcc cac gag gat ctc gct ggc aaa gca cgg cat tgc caa      2640
   Ala Ile Ala Ala His Glu Asp Leu Ala Gly Lys Ala Arg His Cys Gln
   865                     870                     875                     880
   ccc cac ctt tgt gca cgc ccc cgg gca aga tca agc tgg aag tac cgc      2688
   Pro His Leu Cys Ala Arg Pro Arg Ala Arg Ser Ser Trp Lys Tyr Arg
                       885                     890                     895
   ggc cag ctc acg ccc aag agc aag aag atg gac tcg gag gtc cac atc      2736
   Gly Gln Leu Thr Pro Lys Ser Lys Lys Met Asp Ser Glu Val His Ile
                   900                     905                     910
   gtg tcc gtg gac gcc cac gac ggc gtt gtc gac ctc gtc gcc gac ggc      2784
   Val Ser Val Asp Ala His Asp Gly Val Val Asp Leu Val Ala Asp Gly
               915                     920                     925
   ttc ctc tgg gcc gac agc ctc cgc gtc tac tcg gtg agc aac att cgc      2832
   Phe Leu Trp Ala Asp Ser Leu Arg Val Tyr Ser Val Ser Asn Ile Arg
       930                     935                     940
   gtg cgc atc gcc tcc ggt gag gcc cct gcc gcc gcc tcc tcc gcc gcc      2880
   Val Arg Ile Ala Ser Gly Glu Ala Pro Ala Ala Ala Ser Ser Ala Ala
   945                     950                     955                     960
   tct gtg ggc tcc tcg gct tcg tcc gtc gag cgc acg cgc tcg agc ccc      2928
   Ser Val Gly Ser Ser Ala Ser Ser Val Glu Arg Thr Arg Ser Ser Pro
                       965                     970                     975
   gct gtc gcc tcc ggc ccg gcc cag acc atc gac ctc aag cag ctc aag      2976
   Ala Val Ala Ser Gly Pro Ala Gln Thr Ile Asp Leu Lys Gln Leu Lys
                   980                       985                      990
   acc gag ctc ctc gag ctc gat gcc  ccg ctc tac ctc tcg  cag gac ccg     3024
   Thr Glu Leu Leu Glu Leu Asp Ala  Pro Leu Tyr Leu Ser  Gln Asp Pro
               995                      1000                      1005
   acc agc  ggc cag ctc aag aag  cac acc gac gtg gcc  tcc ggc cag       3069
   Thr Ser  Gly Gln Leu Lys Lys  His Thr Asp Val Ala  Ser Gly Gln
       1010                     1015                      1020
   gcc acc  atc gtg cag ccc tgc  acg ctc ggc gac ctc  ggt gac cgc       3114
   Ala Thr  Ile Val Gln Pro Cys  Thr Leu Gly Asp Leu  Gly Asp Arg
       1025                     1030                      1035
   tcc ttc  atg gag acc tac ggc  gtc gtc gcc ccg ctg  tac acg ggc       3159
   Ser Phe  Met Glu Thr Tyr Gly  Val Val Ala Pro Leu  Tyr Thr Gly
       1040                     1045                      1050
   gcc atg  gcc aag ggc att gcc  tcg gcg gac ctc gtc  atc gcc gcc       3204
   Ala Met  Ala Lys Gly Ile Ala  Ser Ala Asp Leu Val  Ile Ala Ala
       1055                     1060                      1065
   ggc aag  cgc aag atc ctc ggc  tcc ttt ggc gcc ggc  ggc ctc ccc       3249
   Gly Lys  Arg Lys Ile Leu Gly  Ser Phe Gly Ala Gly  Gly Leu Pro
       1070                     1075                      1080
   atg cac  cac gtg cgc gcc gcc  ctc gag aag atc cag  gcc gcc ctg       3294
   Met His  His Val Arg Ala Ala  Leu Glu Lys Ile Gln  Ala Ala Leu
       1085                     1090                      1095
   cct cag  ggc ccc tac gcc gtc  aac ctc atc cac tcg  cct ttt gac       3339
   Pro Gln  Gly Pro Tyr Ala Val  Asn Leu Ile His Ser  Pro Phe Asp
       1100                     1105                      1110
   agc aac  ctc gag aag ggc aac  gtc gat ctc ttc ctc  gag aag ggc       3384
   Ser Asn  Leu Glu Lys Gly Asn  Val Asp Leu Phe Leu  Glu Lys Gly
       1115                     1120                      1125
   gtc act  gtg gtg gag gcc tcg  gca ttc atg acc ctc  acc ccg cag       3429
   Val Thr  Val Val Glu Ala Ser  Ala Phe Met Thr Leu  Thr Pro Gln
       1130                     1135                      1140
   gtc gtg  cgc tac cgc gcc gcc  ggc ctc tcg cgc aac  gcc gac ggt       3474
   Val Val  Arg Tyr Arg Ala Ala  Gly Leu Ser Arg Asn  Ala Asp Gly
       1145                     1150                      1155
   tcg gtc  aac atc cgc aac cgc  atc atc ggc aag gtc  tcg cgc acc       3519
   Ser Val  Asn Ile Arg Asn Arg  Ile Ile Gly Lys Val  Ser Arg Thr
       1160                     1165                      1170
   gag ctc  gcc gag atg ttc atc  cgc ccg gcc ccg gag  cac ctc ctc       3564
   Glu Leu  Ala Glu Met Phe Ile  Arg Pro Ala Pro Glu  His Leu Leu
       1175                     1180                      1185
   gag aag  ctc atc gcc tcg ggc  gag atc acc cag gag  cag gcc gag       3609
   Glu Lys  Leu Ile Ala Ser Gly  Glu Ile Thr Gln Glu  Gln Ala Glu
       1190                     1195                      1200
   ctc gcg  cgc cgc gtt ccc gtc  gcc gac gat atc gct  gtc gag gct       3654
   Leu Ala  Arg Arg Val Pro Val  Ala Asp Asp Ile Ala  Val Glu Ala
       1205                     1210                      1215
   gac tcg  ggc ggc cac acc gac  aac cgc ccc atc cac  gtc atc ctc       3699
   Asp Ser  Gly Gly His Thr Asp  Asn Arg Pro Ile His  Val Ile Leu
       1220                     1225                      1230
   ccg ctc  atc atc aac ctc cgc  aac cgc ctg cac cgc  gag tgc ggc       3744
   Pro Leu  Ile Ile Asn Leu Arg  Asn Arg Leu His Arg  Glu Cys Gly
       1235                     1240                      1245
   tac ccc  gcg cac ctc cgc gtc  cgc gtt ggc gcc ggc  ggt ggc gtc       3789
   Tyr Pro  Ala His Leu Arg Val  Arg Val Gly Ala Gly  Gly Gly Val
       1250                     1255                      1260
   ggc tgc  ccg cag gcc gcc gcc  gcc gcg ctc acc atg  ggc gcc gcc       3834
   Gly Cys  Pro Gln Ala Ala Ala  Ala Ala Leu Thr Met  Gly Ala Ala
       1265                     1270                      1275
   ttc atc  gtc acc ggc act gtc  aac cag gtc gcc aag  cag tcc ggc       3879
   Phe Ile  Val Thr Gly Thr Val  Asn Gln Val Ala Lys  Gln Ser Gly
       1280                     1285                      1290
   acc tgc  gac aac gtg cgc aag  cag ctc tcg cag gcc  acc tac tcg       3924
   Thr Cys  Asp Asn Val Arg Lys  Gln Leu Ser Gln Ala  Thr Tyr Ser
       1295                    1300                    1305
   gat atc tgc atg gcc ccg gcc gcc gac atg ttc gag gag ggc gtc      3969
   Asp Ile Cys Met Ala Pro Ala Ala Asp Met Phe Glu Glu Gly Val
       1310                    1315                    1320
   aag ctc cag gtc ctc aag aag gga acc atg ttc ccc tcg cgc gcc      4014
   Lys Leu Gln Val Leu Lys Lys Gly Thr Met Phe Pro Ser Arg Ala
       1325                    1330                    1335
   aac aag ctc tac gag ctc ttt tgc aag tac gac tcc ttc gac tcc      4059
   Asn Lys Leu Tyr Glu Leu Phe Cys Lys Tyr Asp Ser Phe Asp Ser
       1340                    1345                    1350
   atg cct cct gcc gag ctc gag cgc atc gag aag cgt atc ttc aag      4104
   Met Pro Pro Ala Glu Leu Glu Arg Ile Glu Lys Arg Ile Phe Lys
       1355                    1360                    1365
   cgc gca ctc cag gag gtc tgg gag gag acc aag gac ttt tac att      4149
   Arg Ala Leu Gln Glu Val Trp Glu Glu Thr Lys Asp Phe Tyr Ile
       1370                    1375                    1380
   aac ggt ctc aag aac ccg gag aag atc cag cgc gcc gag cac gac      4194
   Asn Gly Leu Lys Asn Pro Glu Lys Ile Gln Arg Ala Glu His Asp
       1385                    1390                    1395
   ccc aag ctc aag atg tcg ctc tgc ttc cgc tgg tac ctt ggt ctt      4239
   Pro Lys Leu Lys Met Ser Leu Cys Phe Arg Trp Tyr Leu Gly Leu
       1400                    1405                    1410
   gcc agc cgc tgg gcc aac atg ggc gcc ccg gac cgc gtc atg gac      4284
   Ala Ser Arg Trp Ala Asn Met Gly Ala Pro Asp Arg Val Met Asp
       1415                    1420                    1425
   tac cag gtc tgg tgt ggc ccg gcc att ggc gcc ttc aac gac ttc      4329
   Tyr Gln Val Trp Cys Gly Pro Ala Ile Gly Ala Phe Asn Asp Phe
       1430                    1435                    1440
   atc aag ggc acc tac ctc gac ccc gct gtc tcc aac gag tac ccc      4374
   Ile Lys Gly Thr Tyr Leu Asp Pro Ala Val Ser Asn Glu Tyr Pro
       1445                    1450                    1455
   tgt gtc gtc cag atc aac ctg caa atc ctc cgt ggt gcc tgc tac      4419
   Cys Val Val Gln Ile Asn Leu Gln Ile Leu Arg Gly Ala Cys Tyr
       1460                    1465                    1470
   ctg cgc cgt ctc aac gcc ctg cgc aac gac ccg cgc att gac ctc      4464
   Leu Arg Arg Leu Asn Ala Leu Arg Asn Asp Pro Arg Ile Asp Leu
       1475                    1480                    1485
   gag acc gag gat gct gcc ttt gtc tac gag ccc acc aac gcg ctc      4509
   Glu Thr Glu Asp Ala Ala Phe Val Tyr Glu Pro Thr Asn Ala Leu
       1490                    1495                    1500
<210> 6
   <211> 1503
   <212> PRT
   <213> Schizochytrium sp.
<400> 6
   Met Ala Leu Arg Val Lys Thr Asn Lys Lys Pro Cys Trp Glu Met Thr
   1               5                   10                  15
   Lys Glu Glu Leu Thr Ser Gly Lys Thr Glu Val Phe Asn Tyr Glu Glu
               20                  25                  30
   Leu Leu Glu Phe Ala Glu Gly Asp Ile Ala Lys Val Phe Gly Pro Glu
           35                  40                  45
   Phe Ala Val Ile Asp Lys Tyr Pro Arg Arg Val Arg Leu Pro Ala Arg
       50                  55                  60
   Glu Tyr Leu Leu Val Thr Arg Val Thr Leu Met Asp Ala Glu Val Asn
   65                  70                  75                  80
   Asn Tyr Arg Val Gly Ala Arg Met Val Thr Glu Tyr Asp Leu Pro Val
   85 90 95
   Asn Gly Glu Leu Ser Glu Gly Gly Asp Cys Pro Trp Ala Val Leu Val
   100 105 110
   Glu Ser Gly Gln Cys Asp Leu Met Leu Ile Ser Tyr Met Gly Ile Asp
   115 120 125
   Phe Gln Asn Gln Gly Asp Arg Val Tyr Arg Leu Leu Asn Thr Thr Leu
   130 135 140
   Thr Phe Tyr Gly Val Ala His Glu Gly Glu Thr Leu Glu Tyr Asp Ile
   145 150 155 160
   Arg Val Thr Gly Phe Ala Lys Arg Leu Asp Gly Gly Ile Ser Met Phe
   165 170 175
   Phe Phe Glu Tyr Asp Cys Tyr Val Asn Gly Arg Leu Leu Ile Glu Met
   180 185 190
   Arg Asp Gly Cys Ala Gly Phe Phe Thr Asn Glu Glu Leu Asp Ala Gly
   195 200 205
   Lys Gly Val Val Phe Thr Arg Gly Asp Leu Ala Ala Arg Ala Lys Ile
   210 215 220
   Pro Lys Gln Asp Val Ser Pro Tyr Ala Val Ala Pro Cys Leu His Lys
   225 230 235 240
   Thr Lys Leu Asn Glu Lys Glu Met Gln Thr Leu Val Asp Lys Asp Trp
   245 250 255
   Ala Ser Val Phe Gly Ser Lys Asn Gly Met Pro Glu Ile Asn Tyr Lys
   260 265 270
   Leu Cys Ala Arg Lys Met Leu Met Ile Asp Arg Val Thr Ser Ile Asp
   275 280 285
   His Lys Gly Gly Val Tyr Gly Leu Gly Gln Leu Val Gly Glu Lys Ile
   290 295 300
   Leu Glu Arg Asp His Trp Tyr Phe Pro Cys His Phe Val Lys Asp Gln
   305 310 315 320
   Val Met Ala Gly Ser Leu Val Ser Asp Gly Cys Ser Gln Met Leu Lys
   325 330 335
   Met Tyr Met Ile Trp Leu Gly Leu His Leu Thr Thr Gly Pro Phe Asp
   340 345 350
   Phe Arg Pro Val Asn Gly His Pro Asn Lys Val Arg Cys Arg Gly Gln
   355 360 365
   Ile Ser Pro His Lys Gly Lys Leu Val Tyr Val Met Glu Ile Lys Glu
   370 375 380
   Met Gly Phe Asp Glu Asp Asn Asp Pro Tyr Ala Ile Ala Asp Val Asn
   385 390 395 400
   Ile Ile Asp Val Asp Phe Glu Lys Gly Gln Asp Phe Ser Leu Asp Arg
   405 410 415
   Ile Ser Asp Tyr Gly Lys Gly Asp Leu Asn Lys Lys Ile Val Val Asp
                   420                     425                     430
   Phe Lys Gly Ile Ala Leu Lys Met Gln Lys Arg Ser Thr Asn Lys Asn
           435                     440                     445
   Pro Ser Lys Val Gln Pro Val Phe Ala Asn Gly Ala Ala Thr Val Gly
       450                     455                     460
   Pro Glu Ala Ser Lys Ala Ser Ser Gly Ala Ser Ala Ser Ala Ser Ala
   465                     470                     475                     480
   Ala Pro Ala Lys Pro Ala Phe Ser Ala Asp Val Leu Ala Pro Lys Pro
                   485                     490                     495
   Val Ala Leu Pro Glu His Ile Leu Lys Gly Asp Ala Leu Ala Pro Lys
               500                     505                     510
   Glu Met Ser Trp His Pro Met Ala Arg Ile Pro Gly Asn Pro Thr Pro
           515                     520                     525
   Ser Phe Ala Pro Ser Ala Tyr Lys Pro Arg Asn Ile Ala Phe Thr Pro
       530                     535                     540
   Phe Pro Gly Asn Pro Asn Asp Asn Asp His Thr Pro Gly Lys Met Pro
   545                     550                     555                     560
   Leu Thr Trp Phe Asn Met Ala Glu Phe Met Ala Gly Lys Val Ser Met
                   565                     570                     575
   Cys Leu Gly Pro Glu Phe Ala Lys Phe Asp Asp Ser Asn Thr Ser Arg
               580                     585                     590
   Ser Pro Ala Trp Asp Leu Ala Leu Val Thr Arg Ala Val Ser Val Ser
           595                     600                     605
   Asp Leu Lys His Val Asn Tyr Arg Asn Ile Asp Leu Asp Pro Ser Lys
       610                     615                     620
   Gly Thr Met Val Gly Glu Phe Asp Cys Pro Ala Asp Ala Trp Phe Tyr
   625                     630                     635                     640
   Lys Gly Ala Cys Asn Asp Ala His Met Pro Tyr Ser Ile Leu Met Glu
                   645                     650                     655
   Ile Ala Leu Gln Thr Ser Gly Val Leu Thr Ser Val Leu Lys Ala Pro
               660                     665                     670
   Leu Thr Met Glu Lys Asp Asp Ile Leu Phe Arg Asn Leu Asp Ala Asn
           675                     680                     685
   Ala Glu Phe Val Arg Ala Asp Leu Asp Tyr Arg Gly Lys Thr Ile Arg
       690                     695                     700
   Asn Val Thr Lys Cys Thr Gly Tyr Ser Met Leu Gly Glu Met Gly Val
   705                     710                     715                     720
   His Arg Phe Thr Phe Glu Leu Tyr Val Asp Asp Val Leu Phe Tyr Lys
                   725                     730                     735
   Gly Ser Thr Ser Phe Gly Trp Phe Val Pro Glu Val Phe Ala Ala Gln
               740                     745                     750
   Ala Gly Leu Asp Asn Gly Arg Lys Ser Glu Pro Trp Phe Ile Glu Asn
           755                 760                 765
   Lys Val Pro Ala Ser Gln Val Ser Ser Phe Asp Val Arg Pro Asn Gly
       770                 775                 780
   Ser Gly Arg Thr Ala Ile Phe Ala Asn Ala Pro Ser Gly Ala Gln Leu
   785                 790                 795                 800
   Asn Arg Arg Thr Asp Gln Gly Gln Tyr Leu Asp Ala Val Asp Ile Val
                   805                 810                 815
   Ser Gly Ser Gly Lys Lys Ser Leu Gly Tyr Ala His Gly Ser Lys Thr
               820                 825                 830
   Val Asn Pro Asn Asp Trp Phe Phe Ser Cys His Phe Trp Phe Asp Ser
           835                 840                 845
   Val Met Pro Gly Ser Leu Gly Val Glu Ser Met Phe Gln Leu Val Glu
       850                 855                 860
   Ala Ile Ala Ala His Glu Asp Leu Ala Gly Lys Ala Arg His Cys Gln
   865                 870                 875                 880
   Pro His Leu Cys Ala Arg Pro Arg Ala Arg Ser Ser Trp Lys Tyr Arg
                   885                 890                 895
   Gly Gln Leu Thr Pro Lys Ser Lys Lys Met Asp Ser Glu Val His Ile
               900                 905                 910
   Val Ser Val Asp Ala His Asp Gly Val Val Asp Leu Val Ala Asp Gly
           915                 920                 925
   Phe Leu Trp Ala Asp Ser Leu Arg Val Tyr Ser Val Ser Asn Ile Arg
       930                 935                 940
   Val Arg Ile Ala Ser Gly Glu Ala Pro Ala Ala Ala Ser Ser Ala Ala
   945                 950                 955                 960
   Ser Val Gly Ser Ser Ala Ser Ser Val Glu Arg Thr Arg Ser Ser Pro
                   965                 970                 975
   Ala Val Ala Ser Gly Pro Ala Gln Thr Ile Asp Leu Lys Gln Leu Lys
               980                 985                 990
   Thr Glu Leu Leu Glu Leu Asp Ala  Pro Leu Tyr Leu Ser  Gln Asp Pro
           995                 1000                 1005
   Thr Ser  Gly Gln Leu Lys Lys  His Thr Asp Val Ala  Ser Gly Gln
       1010                 1015                 1020
   Ala Thr  Ile Val Gln Pro Cys  Thr Leu Gly Asp Leu  Gly Asp Arg
       1025                 1030                 1035
   Ser Phe  Met Glu Thr Tyr Gly  Val Val Ala Pro Leu  Tyr Thr Gly
       1040                 1045                 1050
   Ala Met  Ala Lys Gly Ile Ala  Ser Ala Asp Leu Val  Ile Ala Ala
       1055                 1060                 1065
   Gly Lys  Arg Lys Ile Leu Gly  Ser Phe Gly Ala Gly  Gly Leu Pro
       1070                 1075                 1080
   Met His  His Val Arg Ala Ala  Leu Glu Lys Ile Gln  Ala Ala Leu
   1085 1090 1095
   Pro Gln Gly Pro Tyr Ala Val Asn Leu Ile His Ser Pro Phe Asp
   1100 1105 1110
   Ser Asn Leu Glu Lys Gly Asn Val Asp Leu Phe Leu Glu Lys Gly
   1115 1120 1125
   Val Thr Val Val Glu Ala Ser Ala Phe Met Thr Leu Thr Pro Gln
   1130 1135 1140
   Val Val Arg Tyr Arg Ala Ala Gly Leu Ser Arg Asn Ala Asp Gly
   1145 1150 1155
   Ser Val Asn Ile Arg Asn Arg Ile Ile Gly Lys Val Ser Arg Thr
   1160 1165 1170
   Glu Leu Ala Glu Met Phe Ile Arg Pro Ala Pro Glu His Leu Leu
   1175 1180 1185
   Glu Lys Leu Ile Ala Ser Gly Glu Ile Thr Gln Glu Gln Ala Glu
   1190 1195 1200
   Leu Ala Arg Arg Val Pro Val Ala Asp Asp Ile Ala Val Glu Ala
   1205 1210 1215
   Asp Ser Gly Gly His Thr Asp Asn Arg Pro Ile His Val Ile Leu
   1220 1225 1230
   Pro Leu Ile Ile Asn Leu Arg Asn Arg Leu His Arg Glu Cys Gly
   1235 1240 1245
   Tyr Pro Ala His Leu Arg Val Arg Val Gly Ala Gly Gly Gly Val
   1250 1255 1260
   Gly Cys Pro Gln Ala Ala Ala Ala Ala Leu Thr Met Gly Ala Ala
   1265 1270 1275
   Phe Ile Val Thr Gly Thr Val Asn Gln Val Ala Lys Gln Ser Gly
   1280 1285 1290
   Thr Cys Asp Asn Val Arg Lys Gln Leu Ser Gln Ala Thr Tyr Ser
   1295 1300 1305
   Asp Ile Cys Met Ala Pro Ala Ala Asp Met Phe Glu Glu Gly Val
   1310 1315 1320
   Lys Leu Gln Val Leu Lys Lys Gly Thr Met Phe Pro Ser Arg Ala
   1325 1330 1335
   Asn Lys Leu Tyr Glu Leu Phe Cys Lys Tyr Asp Ser Phe Asp Ser
   1340 1345 1350
   Met Pro Pro Ala Glu Leu Glu Arg Ile Glu Lys Arg Ile Phe Lys
   1355 1360 1365
   Arg Ala Leu Gln Glu Val Trp Glu Glu Thr Lys Asp Phe Tyr Ile
   1370 1375 1380
   Asn Gly Leu Lys Asn Pro Glu Lys Ile Gln Arg Ala Glu His Asp
   1385 1390 1395
   Pro Lys Leu Lys Met Ser Leu Cys Phe Arg Trp Tyr Leu Gly Leu
           1400                1405                1410
   Ala Ser  Arg Trp Ala Asn Met  Gly Ala Pro Asp Arg  Val Met Asp
       1415                1420                1425
   Tyr Gln  Val Trp Cys Gly Pro  Ala Ile Gly Ala Phe  Asn Asp Phe
       1430                1435                1440
   Ile Lys  Gly Thr Tyr Leu Asp  Pro Ala Val Ser Asn  Glu Tyr Pro
       1445                1450                1455
   Cys Val  Val Gln Ile Asn Leu  Gln Ile Leu Arg Gly  Ala Cys Tyr
       1460                1465                1470
   Leu Arg  Arg Leu Asn Ala Leu  Arg Asn Asp Pro Arg  Ile Asp Leu
       1475                1480                1485
   Glu Thr  Glu Asp Ala Ala Phe  Val Tyr Glu Pro Thr  Asn Ala Leu
       1490                1495                1500
<210> 7
   <211> 600
   <212> DNA
   <213> Schizochytrium sp.
<220>
   <221> CDS
   <222> (1)..(600)
   <223>
<400> 7
   atg gcg gcc cgt ctg cag gag caa aag gga ggc gag atg gat acc cgc      48
   Met Ala Ala Arg Leu Gln Glu Gln Lys Gly Gly Glu Met Asp Thr Arg
   1               5                   10                  15
   att gcc atc atc ggc atg tcg gcc atc ctc ccc tgc ggc acg acc gtg      96
   Ile Ala Ile Ile Gly Met Ser Ala Ile Leu Pro Cys Gly Thr Thr Val
               20                  25                  30
   cgc gag tcg tgg gag acc atc cgc gcc ggc atc gac tgc ctg tcg gat     144
   Arg Glu Ser Trp Glu Thr Ile Arg Ala Gly Ile Asp Cys Leu Ser Asp
           35                  40                  45
   ctc ccc gag gac cgc gtc gac gtg acg gcg tac ttt gac ccc gtc aag     192
   Leu Pro Glu Asp Arg Val Asp Val Thr Ala Tyr Phe Asp Pro Val Lys
       50                  55                  60
   acc acc aag gac aag atc tac tgc aag cgc ggt ggc ttc att ccc gag     240
   Thr Thr Lys Asp Lys Ile Tyr Cys Lys Arg Gly Gly Phe Ile Pro Glu
   65                  70                  75                  80
   tac gac ttt gac gcc cgc gag ttc gga ctc aac atg ttc cag atg gag     288
   Tyr Asp Phe Asp Ala Arg Glu Phe Gly Leu Asn Met Phe Gln Met Glu
                   85                  90                  95
   gac tcg gac gca aac cag acc atc tcg ctt ctc aag gtc aag gag gcc     336
   Asp Ser Asp Ala Asn Gln Thr Ile Ser Leu Leu Lys Val Lys Glu Ala
               100                 105                 110
   ctc cag gac gcc ggc atc gac gcc ctc ggc aag gaa aag aag aac atc     384
   Leu Gln Asp Ala Gly Ile Asp Ala Leu Gly Lys Glu Lys Lys Asn Ile
           115                 120                 125
   ggc tgc gtg ctc ggc att ggc ggc ggc caa aag tcc agc cac gag ttc     432
   Gly Cys Val Leu Gly Ile Gly Gly Gly Gln Lys Ser Ser His Glu Phe
       130                 135                 140
   tac tcg cgc ctt aat tat gtt gtc gtg gag aag gtc ctc cgc aag atg     480
   Tyr Ser Arg Leu Asn Tyr Val Val Val Glu Lys Val Leu Arg Lys Met
   145                 150                 155                 160
   ggc atg ccc gag gag gac gtc aag gtc gcc gtc gaa aag tac aag gcc     528
   Gly Met Pro Glu Glu Asp Val Lys Val Ala Val Glu Lys Tyr Lys Ala
                   165                 170                 175
   aac ttc ccc gag tgg cgc ctc gac tcc ttc cct ggc ttc ctc ggc aac     576
   Asn Phe Pro Glu Trp Arg Leu Asp Ser Phe Pro Gly Phe Leu Gly Asn
               180                 185                 190
   gtc acc gcc ggt cgc tgc acc aac                                     600
   Val Thr Ala Gly Arg Cys Thr Asn
           195                 200
<210> 8
   <211> 200
   <212> PRT
   <213> Schizochytrium sp.
<400> 8
   Met Ala Ala Arg Leu Gln Glu Gln Lys Gly Gly Glu Met Asp Thr Arg
   1               5                   10                  15
   Ile Ala Ile Ile Gly Met Ser Ala Ile Leu Pro Cys Gly Thr Thr Val
               20                  25                  30
   Arg Glu Ser Trp Glu Thr Ile Arg Ala Gly Ile Asp Cys Leu Ser Asp
           35                  40                  45
   Leu Pro Glu Asp Arg Val Asp Val Thr Ala Tyr Phe Asp Pro Val Lys
       50                  55                  60
   Thr Thr Lys Asp Lys Ile Tyr Cys Lys Arg Gly Gly Phe Ile Pro Glu
   65                  70                  75                  80
   Tyr Asp Phe Asp Ala Arg Glu Phe Gly Leu Asn Met Phe Gln Met Glu
                   85                  90                  95
   Asp Ser Asp Ala Asn Gln Thr Ile Ser Leu Leu Lys Val Lys Glu Ala
               100                 105                 110
   Leu Gln Asp Ala Gly Ile Asp Ala Leu Gly Lys Glu Lys Lys Asn Ile
           115                 120                 125
   Gly Cys Val Leu Gly Ile Gly Gly Gly Gln Lys Ser Ser His Glu Phe
       130                 135                 140
   Tyr Ser Arg Leu Asn Tyr Val Val Val Glu Lys Val Leu Arg Lys Met
   145                 150                 155                 160
   Gly Met Pro Glu Glu Asp Val Lys Val Ala Val Glu Lys Tyr Lys Ala
                   165                 170                 175
   Asn Phe Pro Glu Trp Arg Leu Asp Ser Phe Pro Gly Phe Leu Gly Asn
               180                 185                 190
   Val Thr Ala Gly Arg Cys Thr Asn
           195                 200
<210> 9
   <211> 1278
   <212> DNA
   <213> Schizochytrium sp.
<220>
   <221> CDS
   <222> (1)..(1278)
   <223>
<400> 9
   gat gtc acc aag gag gcc tgg cgc ctc ccc cgc gag ggc gtc agc ttc       48
   Asp Val Thr Lys Glu Ala Trp Arg Leu Pro Arg Glu Gly Val Ser Phe
   1               5                   10                  15
   cgc gcc aag ggc atc gcc acc aac ggc gct gtc gcc gcg ctc ttc tcc       96
   Arg Ala Lys Gly Ile Ala Thr Asn Gly Ala Val Ala Ala Leu Phe Ser
               20                  25                  30
   ggc cag ggc gcg cag tac acg cac atg ttt agc gag gtg gcc atg aac      144
   Gly Gln Gly Ala Gln Tyr Thr His Met Phe Ser Glu Val Ala Met Asn
           35                  40                  45
   tgg ccc cag ttc cgc cag agc att gcc gcc atg gac gcc gcc cag tcc      192
   Trp Pro Gln Phe Arg Gln Ser Ile Ala Ala Met Asp Ala Ala Gln Ser
       50                  55                  60
   aag gtc gct gga agc gac aag gac ttt gag cgc gtc tcc cag gtc ctc      240
   Lys Val Ala Gly Ser Asp Lys Asp Phe Glu Arg Val Ser Gln Val Leu
   65                  70                  75                  80
   tac ccg cgc aag ccg tac gag cgt gag ccc gag cag aac ccc aag aag      288
   Tyr Pro Arg Lys Pro Tyr Glu Arg Glu Pro Glu Gln Asn Pro Lys Lys
                   85                  90                  95
   atc tcc ctc acc gcc tac tcg cag ccc tcg acc ctg gcc tgc gct ctc      336
   Ile Ser Leu Thr Ala Tyr Ser Gln Pro Ser Thr Leu Ala Cys Ala Leu
               100                 105                 110
   ggt gcc ttt gag atc ttc aag gag gcc ggc ttc acc ccg gac ttt gcc      384
   Gly Ala Phe Glu Ile Phe Lys Glu Ala Gly Phe Thr Pro Asp Phe Ala
           115                 120                 125
   gcc ggc cat tcg ctc ggt gag ttc gcc gcc ctc tac gcc gcg ggc tgc      432
   Ala Gly His Ser Leu Gly Glu Phe Ala Ala Leu Tyr Ala Ala Gly Cys
       130                 135                 140
   gtc gac cgc gac gag ctc ttt gag ctt gtc tgc cgc cgc gcc cgc atc      480
   Val Asp Arg Asp Glu Leu Phe Glu Leu Val Cys Arg Arg Ala Arg Ile
   145                 150                 155                 160
   atg ggc ggc aag gac gca ccg gcc acc ccc aag gga tgc atg gcc gcc      528
   Met Gly Gly Lys Asp Ala Pro Ala Thr Pro Lys Gly Cys Met Ala Ala
                   165                 170                 175
   gtc att ggc ccc aac gcc gag aac atc aag gtc cag gcc gcc aac gtc      576
   Val Ile Gly Pro Asn Ala Glu Asn Ile Lys Val Gln Ala Ala Asn Val
               180                 185                 190
   tgg ctc ggc aac tcc aac tcg cct tcg cag acc gtc atc acc ggc tcc      624
   Trp Leu Gly Asn Ser Asn Ser Pro Ser Gln Thr Val Ile Thr Gly Ser
           195                 200                 205
   gtc gaa ggt atc cag gcc gag agc gcc cgc ctc cag aag gag ggc ttc      672
   Val Glu Gly Ile Gln Ala Glu Ser Ala Arg Leu Gln Lys Glu Gly Phe
       210                 215                 220
   cgc gtc gtg cct ctt gcc tgc gag agc gcc ttc cac tcg ccc cag atg      720
   Arg Val Val Pro Leu Ala Cys Glu Ser Ala Phe His Ser Pro Gln Met
   225                 230                 235                 240
   gag aac gcc tcg tcg gcc ttc aag gac gtc atc tcc aag gtc tcc ttc      768
   Glu Asn Ala Ser Ser Ala Phe Lys Asp Val Ile Ser Lys Val Ser Phe
                   245                 250                 255
   cgc acc ccc aag gcc gag acc aag ctc ttc agc aac gtc tct ggc gag      816
   Arg Thr Pro Lys Ala Glu Thr Lys Leu Phe Ser Asn Val Ser Gly Glu
               260                 265                 270
   acc tac ccc acg gac gcc cgc gag atg ctt acg cag cac atg acc agc      864
   Thr Tyr Pro Thr Asp Ala Arg Glu Met Leu Thr Gln His Met Thr Ser
           275                 280                 285
   agc gtc aag ttc ctc acc cag gtc cgc aac atg cac cag gcc ggt gcg      912
   Ser Val Lys Phe Leu Thr Gln Val Arg Asn Met His Gln Ala Gly Ala
       290                 295                 300
   cgc atc ttt gtc gag ttc gga ccc aag cag gtg ctc tcc aag ctt gtc      960
   Arg Ile Phe Val Glu Phe Gly Pro Lys Gln Val Leu Ser Lys Leu Val
   305                 310                 315                 320
   tcc gag acc ctc aag gat gac ccc tcg gtt gtc acc gtc tct gtc aac     1008
   Ser Glu Thr Leu Lys Asp Asp Pro Ser Val Val Thr Val Ser Val Asn
                       325                     330                     335
   ccg gcc tcg ggc acg gat tcg gac atc cag ctc cgc gac gcg gcc gtc      1056
   Pro Ala Ser Gly Thr Asp Ser Asp Ile Gln Leu Arg Asp Ala Ala Val
                   340                     345                     350
   cag ctc gtt gtc gct ggc gtc aac ctt cag ggc ttt gac aag tgg gac      1104
   Gln Leu Val Val Ala Gly Val Asn Leu Gln Gly Phe Asp Lys Trp Asp
               355                     360                     365
   gcc ccc gat gcc acc cgc atg cag gcc atc aag aag aag cgc act acc      1152
   Ala Pro Asp Ala Thr Arg Met Gln Ala Ile Lys Lys Lys Arg Thr Thr
       370                     375                     380
   ctc cgc ctt tcg gcc gcc acc tac gtc tcg gac aag acc aag aag gtc      1200
   Leu Arg Leu Ser Ala Ala Thr Tyr Val Ser Asp Lys Thr Lys Lys Val
   385                     390                     395                     400
   cgc gac gcc gcc atg aac gat ggc cgc tgc gtc acc tac ctc aag ggc      1248
   Arg Asp Ala Ala Met Asn Asp Gly Arg Cys Val Thr Tyr Leu Lys Gly
                       405                     410                     415
   gcc gca ccg ctc atc aag gcc ccg gag ccc                              1278
   Ala Ala Pro Leu Ile Lys Ala Pro Glu Pro
                   420                     425
<210> 10
   <211> 426
   <212> PRT
   <213> Schizochytrium sp.
<400> 10
   Asp Val Thr Lys Glu Ala Trp Arg Leu Pro Arg Glu Gly Val Ser Phe
   1               5                   10                  15
   Arg Ala Lys Gly Ile Ala Thr Asn Gly Ala Val Ala Ala Leu Phe Ser
               20                  25                  30
   Gly Gln Gly Ala Gln Tyr Thr His Met Phe Ser Glu Val Ala Met Asn
           35                  40                  45
   Trp Pro Gln Phe Arg Gln Ser Ile Ala Ala Met Asp Ala Ala Gln Ser
       50                  55                  60
   Lys Val Ala Gly Ser Asp Lys Asp Phe Glu Arg Val Ser Gln Val Leu
   65                  70                  75                  80
   Tyr Pro Arg Lys Pro Tyr Glu Arg Glu Pro Glu Gln Asn Pro Lys Lys
                   85                  90                  95
   Ile Ser Leu Thr Ala Tyr Ser Gln Pro Ser Thr Leu Ala Cys Ala Leu
               100                 105                 110
   Gly Ala Phe Glu Ile Phe Lys Glu Ala Gly Phe Thr Pro Asp Phe Ala
           115                 120                 125
   Ala Gly His Ser Leu Gly Glu Phe Ala Ala Leu Tyr Ala Ala Gly Cys
       130                 135                 140
   Val Asp Arg Asp Glu Leu Phe Glu Leu Val Cys Arg Arg Ala Arg Ile
   145                 150                 155                 160
   Met Gly Gly Lys Asp Ala Pro Ala Thr Pro Lys Gly Cys Met Ala Ala
                   165                 170                 175
   Val Ile Gly Pro Asn Ala Glu Asn Ile Lys Val Gln Ala Ala Asn Val
               180                 185                 190
   Trp Leu Gly Asn Ser Asn Ser Pro Ser Gln Thr Val Ile Thr Gly Ser
           195                 200                 205
   Val Glu Gly Ile Gln Ala Glu Ser Ala Arg Leu Gln Lys Glu Gly Phe
       210                 215                 220
   Arg Val Val Pro Leu Ala Cys Glu Ser Ala Phe His Ser Pro Gln Met
   225                 230                 235                 240
   Glu Asn Ala Ser Ser Ala Phe Lys Asp Val Ile Ser Lys Val Ser Phe
                   245                 250                 255
   Arg Thr Pro Lys Ala Glu Thr Lys Leu Phe Ser Asn Val Ser Gly Glu
               260                 265                 270
   Thr Tyr Pro Thr Asp Ala Arg Glu Met Leu Thr Gln His Met Thr Ser
           275                 280                 285
   Ser Val Lys Phe Leu Thr Gln Val Arg Asn Met His Gln Ala Gly Ala
       290                 295                 300
   Arg Ile Phe Val Glu Phe Gly Pro Lys Gln Val Leu Ser Lys Leu Val
   305                 310                 315                 320
   Ser Glu Thr Leu Lys Asp Asp Pro Ser Val Val Thr Val Ser Val Asn
                   325                 330                 335
   Pro Ala Ser Gly Thr Asp Ser Asp Ile Gln Leu Arg Asp Ala Ala Val
               340                 345                 350
   Gln Leu Val Val Ala Gly Val Asn Leu Gln Gly Phe Asp Lys Trp Asp
           355                 360                 365
   Ala Pro Asp Ala Thr Arg Met Gln Ala Ile Lys Lys Lys Arg Thr Thr
       370                 375                 380
   Leu Arg Leu Ser Ala Ala Thr Tyr Val Ser Asp Lys Thr Lys Lys Val
   385                 390                 395                 400
   Arg Asp Ala Ala Met Asn Asp Gly Arg Cys Val Thr Tyr Leu Lys Gly
                   405                 410                 415
   Ala Ala Pro Leu Ile Lys Ala Pro Glu Pro
               420                 425
<210> 11
   <211> 5
   <212> PRT
   <213> Schizochytrium sp.
<220>
   <221> MISC_FEATURE
   <222> (4)..(4)
   <223> X = any amino acid
<400> 11
   Gly His Ser Xaa Gly
   1               5
<210> 12
   <211> 258
   <212> DNA
   <213> Schizochytrium sp.
<220>
   <221> CDS
   <222> (1)..(258)
   <223>
<400> 12
   gct gtc tcg aac gag ctt ctt gag aag gcc gag act gtc gtc atg gag       48
   Ala Val Ser Asn Glu Leu Leu Glu Lys Ala Glu Thr Val Val Met Glu
   1               5                   10                  15
   gtc ctc gcc gcc aag acc ggc tac gag acc gac atg atc gag gct gac       96
   Val Leu Ala Ala Lys Thr Gly Tyr Glu Thr Asp Met Ile Glu Ala Asp
               20                  25                  30
   atg gag ctc gag acc gag ctc ggc att gac tcc atc aag cgt gtc gag      144
   Met Glu Leu Glu Thr Glu Leu Gly Ile Asp Ser Ile Lys Arg Val Glu
           35                  40                  45
   atc ctc tcc gag gtc cag gcc atg ctc aat gtc gag gcc aag gat gtc      192
   Ile Leu Ser Glu Val Gln Ala Met Leu Asn Val Glu Ala Lys Asp Val
       50                  55                  60
   gat gcc ctc agc cgc act cgc act gtt ggt gag gtt gtc aac gcc atg      240
   Asp Ala Leu Ser Arg Thr Arg Thr Val Gly Glu Val Val Asn Ala Met
   65                  70                  75                  80
   aag gcc gag atc gct ggc                                              258
   Lys Ala Glu Ile Ala Gly
                   85
<210> 13
   <211> 86
   <212> PRT
   <213> Schizochytrium sp.
<400> 13
   Ala Val Ser Asn Glu Leu Leu Glu Lys Ala Glu Thr Val Val Met Glu
   1               5                   10                  15
   Val Leu Ala Ala Lys Thr Gly Tyr Glu Thr Asp Met Ile Glu Ala Asp
               20                  25                  30
   Met Glu Leu Glu Thr Glu Leu Gly Ile Asp Ser Ile Lys Arg Val Glu
           35                  40                  45
   Ile Leu Ser Glu Val Gln Ala Met Leu Asn Val Glu Ala Lys Asp Val
       50                  55                  60
   Asp Ala Leu Ser Arg Thr Arg Thr Val Gly Glu Val Val Asn Ala Met
   65                  70                  75                  80
   Lys Ala Glu Ile Ala Gly
                   85
<210> 14
   <211> 5
   <212> PRT
   <213> Schizochytrium sp.
<400> 14
   Leu Gly Ile Asp Ser
   1 5
<210> 15
   <211> 21
   <212> PRT
   <213> Schizochytrium sp.
<400> 15
   Ala Pro Ala Pro Val Lys Ala Ala Ala Pro Ala Ala Pro Val Ala Ser
   1 5 10 15
   Ala Pro Ala Pro Ala
   20
<210> 16
   <211> 3006
   <212> DNA
   <213> Schizochytrium sp.
<400> 16
   gcccccgccc cggtcaaggc tgctgcgcct gccgcccccg ttgcctcggc ccctgccccg      60
   gctgtctcga acgagcttct tgagaaggcc gagactgtcg tcatggaggt cctcgccgcc     120
   aagaccggct acgagaccga catgatcgag gctgacatgg agctcgagac cgagctcggc     180
   attgactcca tcaagcgtgt cgagatcctc tccgaggtcc aggccatgct caatgtcgag     240
   gccaaggatg tcgatgccct cagccgcact cgcactgttg gtgaggttgt caacgccatg     300
   aaggccgaga tcgctggcag ctctgccccg gcgcctgctg ccgctgctcc ggctccggcc     360
   aaggctgccc ctgccgccgc tgcgcctgct gtctcgaacg agcttctcga gaaggccgag     420
   accgtcgtca tggaggtcct cgccgccaag actggctacg agactgacat gatcgagtcc     480
   gacatggagc tcgagactga gctcggcatt gactccatca agcgtgtcga gatcctctcc     540
   gaggttcagg ccatgctcaa cgtcgaggcc aaggacgtcg acgctctcag ccgcactcgc     600
   actgtgggtg aggtcgtcaa cgccatgaag gctgagatcg ctggtggctc tgccccggcg     660
   cctgccgccg ctgccccagg tccggctgct gccgcccctg cgcctgccgc cgccgcccct     720
   gctgtctcga acgagcttct tgagaaggcc gagaccgtcg tcatggaggt cctcgccgcc     780
   aagactggct acgagactga catgatcgag tccgacatgg agctcgagac cgagctcggc     840
   attgactcca tcaagcgtgt cgagattctc tccgaggtcc aggccatgct caacgtcgag     900
   gccaaggacg tcgacgctct cagccgcacc cgcactgttg gcgaggtcgt cgatgccatg     960
   aaggccgaga tcgctggtgg ctctgccccg gcgcctgccg ccgctgctcc tgctccggct    1020
   gctgccgccc ctgcgcctgc cgcccctgcg cctgctgtct cgagcgagct tctcgagaag    1080
   gccgagactg tcgtcatgga ggtcctcgcc gccaagactg gctacgagac tgacatgatc    1140
   gagtccgaca tggagctcga gaccgagctc ggcattgact ccatcaagcg tgtcgagatt    1200
   ctctccgagg tccaggccat gctcaacgtc gaggccaagg acgtcgacgc tctcagccgc    1260
   acccgcactg ttggcgaggt cgtcgatgcc atgaaggccg agatcgctgg tggctctgcc    1320
   ccggcgcctg ccgccgctgc tcctgctccg gctgctgccg cccctgcgcc tgccgcccct    1380
   gcgcctgccg cccctgcgcc tgctgtctcg agcgagcttc tcgagaaggc cgagactgtc    1440
   gtcatggagg tcctcgccgc caagactggc tacgagactg acatgattga gtccgacatg    1500
   gagctcgaga ccgagctcgg cattgactcc atcaagcgtg tcgagattct ctccgaggtt    1560
   caggccatgc tcaacgtcga ggccaaggac gtcgacgctc tcagccgcac tcgcactgtt    1620
   ggtgaggtcg tcgatgccat gaaggctgag atcgctggca gctccgcctc ggcgcctgcc    1680
   gccgctgctc ctgctccggc tgctgccgct cctgcgcccg ctgccgccgc ccctgctgtc    1740
   tcgaacgagc ttctcgagaa agccgagact gtcgtcatgg aggtcctcgc cgccaagact    1800
   ggctacgaga ctgacatgat cgagtccgac atggagctcg agactgagct cggcattgac    1860
   tccatcaagc gtgtcgagat cctctccgag gttcaggcca tgctcaacgt cgaggccaag    1920
   gacgtcgatg ccctcagccg cacccgcact gttggcgagg ttgtcgatgc catgaaggcc    1980
   gagatcgctg gtggctctgc cccggcgcct gccgccgctg cccctgctcc ggctgccgcc    2040
   gcccctgctg tctcgaacga gcttctcgag aaggccgaga ctgtcgtcat ggaggtcctc    2100
   gccgccaaga ctggctacga gaccgacatg atcgagtccg acatggagct cgagaccgag    2160
   ctcggcattg actccatcaa gcgtgtcgag attctctccg aggttcaggc catgctcaac    2220
   gtcgaggcca aggacgtcga tgctctcagc cgcactcgca ctgttggcga ggtcgtcgat    2280
   gccatgaagg ctgagatcgc cggcagctcc gccccggcgc ctgccgccgc tgctcctgct    2340
   ccggctgctg ccgctcctgc gcccgctgcc gctgcccctg ctgtctcgag cgagcttctc    2400
   gagaaggccg agaccgtcgt catggaggtc ctcgccgcca agactggcta cgagactgac    2460
   atgattgagt ccgacatgga gctcgagact gagctcggca ttgactccat caagcgtgtc    2520
   gagatcctct ccgaggttca ggccatgctc aacgtcgagg ccaaggacgt cgatgccctc    2580
   agccgcaccc gcactgttgg cgaggttgtc gatgccatga aggccgagat cgctggtggc    2640
   tctgccccgg cgcctgccgc cgctgcccct gctccggctg ccgccgcccc tgctgtctcg    2700
   aacgagcttc ttgagaaggc cgagaccgtc gtcatggagg tcctcgccgc caagactggc    2760
   tacgagaccg acatgatcga gtccgacatg gagctcgaga ccgagctcgg cattgactcc    2820
   atcaagcgtg tcgagattct ctccgaggtt caggccatgc tcaacgtcga ggccaaggac    2880
   gtcgacgctc tcagccgcac tcgcactgtt ggcgaggtcg tcgatgccat gaaggctgag    2940
   atcgctggtg gctctgcccc ggcgcctgcc gccgctgctc ctgcctcggc tggcgccgcg    3000
   cctgcg                                                               3006
<210> 17
   <211> 2133
   <212> DNA
   <213> Schizochytrium sp.
<220>
   <221> CDS
   <222> (1)..(2133)
   <223>
<400> 17
   ttt ggc gct ctc ggc ggc ttc atc tcg cag cag gcg gag cgc ttc gag        48
   Phe Gly Ala Leu Gly Gly Phe Ile Ser Gln Gln Ala Glu Arg Phe Glu
   1               5                   10                  15
   ccc gcc gaa atc ctc ggc ttc acg ctc atg tgc gcc aag ttc gcc aag        96
   Pro Ala Glu Ile Leu Gly Phe Thr Leu Met Cys Ala Lys Phe Ala Lys
               20                  25                  30
   gct tcc ctc tgc acg gct gtg gct ggc ggc cgc ccg gcc ttt atc ggt       144
   Ala Ser Leu Cys Thr Ala Val Ala Gly Gly Arg Pro Ala Phe Ile Gly
           35                  40                  45
   gtg gcg cgc ctt gac ggc cgc ctc gga ttc act tcg cag ggc act tct       192
   Val Ala Arg Leu Asp Gly Arg Leu Gly Phe Thr Ser Gln Gly Thr Ser
       50                  55                  60
   gac gcg ctc aag cgt gcc cag cgt ggt gcc atc ttt ggc ctc tgc aag       240
   Asp Ala Leu Lys Arg Ala Gln Arg Gly Ala Ile Phe Gly Leu Cys Lys
   65                  70                  75                  80
   acc atc ggc ctc gag tgg tcc gag tct gac gtc ttt tcc cgc ggc gtg       288
   Thr Ile Gly Leu Glu Trp Ser Glu Ser Asp Val Phe Ser Arg Gly Val
                   85                  90                  95
   gac att gct cag ggc atg cac ccc gag gat gcc gcc gtg gcg att gtg       336
   Asp Ile Ala Gln Gly Met His Pro Glu Asp Ala Ala Val Ala Ile Val
               100                 105                 110
   cgc gag atg gcg tgc gct gac att cgc att cgc gag gtc ggc att ggc       384
   Arg Glu Met Ala Cys Ala Asp Ile Arg Ile Arg Glu Val Gly Ile Gly
           115                 120                 125
   gca aac cag cag cgc tgc acg atc cgt gcc gcc aag ctc gag acc ggc       432
   Ala Asn Gln Gln Arg Cys Thr Ile Arg Ala Ala Lys Leu Glu Thr Gly
       130                 135                 140
   aac ccg cag cgc cag atc gcc aag gac gac gtg ctg ctc gtt tct ggc       480
   Asn Pro Gln Arg Gln Ile Ala Lys Asp Asp Val Leu Leu Val Ser Gly
   145                 150                 155                 160
   ggc gct cgc ggc atc acg cct ctt tgc atc cgg gag atc acg cgc cag       528
   Gly Ala Arg Gly Ile Thr Pro Leu Cys Ile Arg Glu Ile Thr Arg Gln
                   165                 170                 175
   atc gcg ggc ggc aag tac att ctg ctt ggc cgc agc aag gtc tct gcg       576
   Ile Ala Gly Gly Lys Tyr Ile Leu Leu Gly Arg Ser Lys Val Ser Ala
               180                 185                 190
   agc gaa ccg gca tgg tgc gct ggc atc act gac gag aag gct gtg caa       624
   Ser Glu Pro Ala Trp Cys Ala Gly Ile Thr Asp Glu Lys Ala Val Gln
           195                 200                 205
   aag gct gct acc cag gag ctc aag cgc gcc ttt agc gct ggc gag ggc       672
   Lys Ala Ala Thr Gln Glu Leu Lys Arg Ala Phe Ser Ala Gly Glu Gly
       210                 215                 220
   ccc aag ccc acg ccc cgc gct gtc act aag ctt gtg ggc tct gtt ctt       720
   Pro Lys Pro Thr Pro Arg Ala Val Thr Lys Leu Val Gly Ser Val Leu
   225                 230                 235                 240
   ggc gct cgc gag gtg cgc agc tct att gct gcg att gaa gcg ctc ggc       768
   Gly Ala Arg Glu Val Arg Ser Ser Ile Ala Ala Ile Glu Ala Leu Gly
                   245                 250                 255
   ggc aag gcc atc tac tcg tcg tgc gac gtg aac tct gcc gcc gac gtg       816
   Gly Lys Ala Ile Tyr Ser Ser Cys Asp Val Asn Ser Ala Ala Asp Val
               260                      265                      270
   gcc aag gcc gtg cgc gat gcc gag tcc cag ctc ggt gcc cgc gtc tcg       864
   Ala Lys Ala Val Arg Asp Ala Glu Ser Gln Leu Gly Ala Arg Val Ser
           275                      280                      285
   ggc atc gtt cat gcc tcg ggc gtg ctc cgc gac cgt ctc atc gag aag       912
   Gly Ile Val His Ala Ser Gly Val Leu Arg Asp Arg Leu Ile Glu Lys
       290                      295                      300
   aag ctc ccc gac gag ttc gac gcc gtc ttt ggc acc aag gtc acc ggt       960
   Lys Leu Pro Asp Glu Phe Asp Ala Val Phe Gly Thr Lys Val Thr Gly
   305                      310                      315                 320
   ctc gag aac ctc ctc gcc gcc gtc gac cgc gcc aac ctc aag cac atg      1008
   Leu Glu Asn Leu Leu Ala Ala Val Asp Arg Ala Asn Leu Lys His Met
                   325                      330                      335
   gtc ctc ttc agc tcg ctc gcc ggc ttc cac ggc aac gtc ggc cag tct      1056
   Val Leu Phe Ser Ser Leu Ala Gly Phe His Gly Asn Val Gly Gln Ser
               340                      345                      350
   gac tac gcc atg gcc aac gag gcc ctt aac aag atg ggc ctc gag ctc      1104
   Asp Tyr Ala Met Ala Asn Glu Ala Leu Asn Lys Met Gly Leu Glu Leu
           355                      360                      365
   gcc aag gac gtc tcg gtc aag tcg atc tgc ttc ggt ccc tgg gac ggt      1152
   Ala Lys Asp Val Ser Val Lys Ser Ile Cys Phe Gly Pro Trp Asp Gly
       370                      375                      380
   ggc atg gtg acg ccg cag ctc aag aag cag ttc cag gag atg ggc gtg      1200
   Gly Met Val Thr Pro Gln Leu Lys Lys Gln Phe Gln Glu Met Gly Val
   385                      390                      395                 400
   cag atc atc ccc cgc gag ggc ggc gct gat acc gtg gcg cgc atc gtg      1248
   Gln Ile Ile Pro Arg Glu Gly Gly Ala Asp Thr Val Ala Arg Ile Val
                   405                      410                      415
   ctc ggc tcc tcg ccg gct gag atc ctt gtc ggc aac tgg cgc acc ccg      1296
   Leu Gly Ser Ser Pro Ala Glu Ile Leu Val Gly Asn Trp Arg Thr Pro
               420                      425                      430
   tcc aag aag gtc ggc tcg gac acc atc acc ctg cac cgc aag att tcc      1344
   Ser Lys Lys Val Gly Ser Asp Thr Ile Thr Leu His Arg Lys Ile Ser
           435                      440                      445
   gcc aag tcc aac ccc ttc ctc gag gac cac gtc atc cag ggc cgc cgc      1392
   Ala Lys Ser Asn Pro Phe Leu Glu Asp His Val Ile Gln Gly Arg Arg
       450                      455                      460
   gtg ctg ccc atg acg ctg gcc att ggc tcg ctc gcg gag acc tgc ctc      1440
   Val Leu Pro Met Thr Leu Ala Ile Gly Ser Leu Ala Glu Thr Cys Leu
   465                      470                      475                 480
   ggc ctc ttc ccc ggc tac tcg ctc tgg gcc att gac gac gcc cag ctc      1488
   Gly Leu Phe Pro Gly Tyr Ser Leu Trp Ala Ile Asp Asp Ala Gln Leu
                   485                      490                      495
   ttc aag ggt gtc act gtc gac ggc gac gtc aac tgc gag gtg acc ctc      1536
   Phe Lys Gly Val Thr Val Asp Gly Asp Val Asn Cys Glu Val Thr Leu
               500                      505                      510
   acc ccg tcg acg gcg ccc tcg ggc cgc gtc aac gtc cag gcc acg ctc      1584
   Thr Pro Ser Thr Ala Pro Ser Gly Arg Val Asn Val Gln Ala Thr Leu
           515                      520                      525
   aag acc ttt tcc agc ggc aag ctg gtc ccg gcc tac cgc gcc gtc atc      1632
   Lys Thr Phe Ser Ser Gly Lys Leu Val Pro Ala Tyr Arg Ala Val Ile
       530                      535                      540
   gtg ctc tcc aac cag ggc gcg ccc ccg gcc aac gcc acc atg cag ccg      1680
   Val Leu Ser Asn Gln Gly Ala Pro Pro Ala Asn Ala Thr Met Gln Pro
   545                      550                      555                 560
   ccc tcg ctc gat gcc gat ccg gcg ctc cag ggc tcc gtc tac gac ggc      1728
   Pro Ser Leu Asp Ala Asp Pro Ala Leu Gln Gly Ser Val Tyr Asp Gly
                   565                      570                      575
   aag acc ctc ttc cac ggc ccg gcc ttc cgc ggc atc gat gac gtg ctc      1776
   Lys Thr Leu Phe His Gly Pro Ala Phe Arg Gly Ile Asp Asp Val Leu
               580                      585                      590
   tcg tgc acc aag agc cag ctt gtg gcc aag tgc agc gct gtc ccc ggc      1824
   Ser Cys Thr Lys Ser Gln Leu Val Ala Lys Cys Ser Ala Val Pro Gly
               595                     600                     605
   tcc gac gcc gct cgc ggc gag ttt gcc acg gac act gac gcc cat gac      1872
   Ser Asp Ala Ala Arg Gly Glu Phe Ala Thr Asp Thr Asp Ala His Asp
       610                     615                     620
   ccc ttc gtg aac gac ctg gcc ttt cag gcc atg ctc gtc tgg gtg cgc      1920
   Pro Phe Val Asn Asp Leu Ala Phe Gln Ala Met Leu Val Trp Val Arg
   625                     630                     635                 640
   cgc acg ctc ggc cag gct gcg ctc ccc aac tcg atc cag cgc atc gtc      1968
   Arg Thr Leu Gly Gln Ala Ala Leu Pro Asn Ser Ile Gln Arg Ile Val
                   645                     650                     655
   cag cac cgc ccg gtc ccg cag gac aag ccc ttc tac att acc ctc cgc      2016
   Gln His Arg Pro Val Pro Gln Asp Lys Pro Phe Tyr Ile Thr Leu Arg
               660                     665                     670
   tcc aac cag tcg ggc ggt cac tcc cag cac aag cac gcc ctt cag ttc      2064
   Ser Asn Gln Ser Gly Gly His Ser Gln His Lys His Ala Leu Gln Phe
           675                     680                     685
   cac aac gag cag ggc gat ctc ttc att gat gtc cag gct tcg gtc atc      2112
   His Asn Glu Gln Gly Asp Leu Phe Ile Asp Val Gln Ala Ser Val Ile
       690                     695                     700
   gcc acg gac agc ctt gcc ttc                                          2133
   Ala Thr Asp Ser Leu Ala Phe
   705                     710
<210> 18
   <211> 711
   <212> PRT
   <213> Schizochytrium sp.
<400> 18
   Phe Gly Ala Leu Gly Gly Phe Ile Ser Gln Gln Ala Glu Arg Phe Glu
   1               5                   10                  15
   Pro Ala Glu Ile Leu Gly Phe Thr Leu Met Cys Ala Lys Phe Ala Lys
               20                  25                  30
   Ala Ser Leu Cys Thr Ala Val Ala Gly Gly Arg Pro Ala Phe Ile Gly
           35                  40                  45
   Val Ala Arg Leu Asp Gly Arg Leu Gly Phe Thr Ser Gln Gly Thr Ser
       50                  55                  60
   Asp Ala Leu Lys Arg Ala Gln Arg Gly Ala Ile Phe Gly Leu Cys Lys
   65                  70                  75                  80
   Thr Ile Gly Leu Glu Trp Ser Glu Ser Asp Val Phe Ser Arg Gly Val
                   85                  90                  95
   Asp Ile Ala Gln Gly Met His Pro Glu Asp Ala Ala Val Ala Ile Val
               100                 105                 110
   Arg Glu Met Ala Cys Ala Asp Ile Arg Ile Arg Glu Val Gly Ile Gly
           115                 120                 125
   Ala Asn Gln Gln Arg Cys Thr Ile Arg Ala Ala Lys Leu Glu Thr Gly
       130                 135                 140
   Asn Pro Gln Arg Gln Ile Ala Lys Asp Asp Val Leu Leu Val Ser Gly
   145                 150                 155                 160
   Gly Ala Arg Gly Ile Thr Pro Leu Cys Ile Arg Glu Ile Thr Arg Gln
                   165                 170                 175
   Ile Ala Gly Gly Lys Tyr Ile Leu Leu Gly Arg Ser Lys Val Ser Ala
   180 185 190
   Ser Glu Pro Ala Trp Cys Ala Gly Ile Thr Asp Glu Lys Ala Val Gln
   195 200 205
   Lys Ala Ala Thr Gln Glu Leu Lys Arg Ala Phe Ser Ala Gly Glu Gly
   210 215 220
   Pro Lys Pro Thr Pro Arg Ala Val Thr Lys Leu Val Gly Ser Val Leu
   225 230 235 240
   Gly Ala Arg Glu Val Arg Ser Ser Ile Ala Ala Ile Glu Ala Leu Gly
   245 250 255
   Gly Lys Ala Ile Tyr Ser Ser Cys Asp Val Asn Ser Ala Ala Asp Val
   260 265 270
   Ala Lys Ala Val Arg Asp Ala Glu Ser Gln Leu Gly Ala Arg Val Ser
   275 280 285
   Gly Ile Val His Ala Ser Gly Val Leu Arg Asp Arg Leu Ile Glu Lys
   290 295 300
   Lys Leu Pro Asp Glu Phe Asp Ala Val Phe Gly Thr Lys Val Thr Gly
   305 310 315 320
   Leu Glu Asn Leu Leu Ala Ala Val Asp Arg Ala Asn Leu Lys His Met
   325 330 335
   Val Leu Phe Ser Ser Leu Ala Gly Phe His Gly Asn Val Gly Gln Ser
   340 345 350
   Asp Tyr Ala Met Ala Asn Glu Ala Leu Asn Lys Met Gly Leu Glu Leu
   355 360 365
   Ala Lys Asp Val Ser Val Lys Ser Ile Cys Phe Gly Pro Trp Asp Gly
   370 375 380
   Gly Met Val Thr Pro Gln Leu Lys Lys Gln Phe Gln Glu Met Gly Val
   385 390 395 400
   Gln Ile Ile Pro Arg Glu Gly Gly Ala Asp Thr Val Ala Arg Ile Val
   405 410 415
   Leu Gly Ser Ser Pro Ala Glu Ile Leu Val Gly Asn Trp Arg Thr Pro
   420 425 430
   Ser Lys Lys Val Gly Ser Asp Thr Ile Thr Leu His Arg Lys Ile Ser
   435 440 445
   Ala Lys Ser Asn Pro Phe Leu Glu Asp His Val Ile Gln Gly Arg Arg
   450 455 460
   Val Leu Pro Met Thr Leu Ala Ile Gly Ser Leu Ala Glu Thr Cys Leu
   465 470 475 480
   Gly Leu Phe Pro Gly Tyr Ser Leu Trp Ala Ile Asp Asp Ala Gln Leu
   485 490 495
   Phe Lys Gly Val Thr Val Asp Gly Asp Val Asn Cys Glu Val Thr Leu
   500 505 510
   Thr Pro Ser Thr Ala Pro Ser Gly Arg Val Asn Val Gln Ala Thr Leu
   515 520 525
   Lys Thr Phe Ser Ser Gly Lys Leu Val Pro Ala Tyr Arg Ala Val Ile
   530 535 540
   Val Leu Ser Asn Gln Gly Ala Pro Pro Ala Asn Ala Thr Met Gln Pro
   545 550 555 560
   Pro Ser Leu Asp Ala Asp Pro Ala Leu Gln Gly Ser Val Tyr Asp Gly
   565 570 575
   Lys Thr Leu Phe His Gly Pro Ala Phe Arg Gly Ile Asp Asp Val Leu
   580 585 590
   Ser Cys Thr Lys Ser Gln Leu Val Ala Lys Cys Ser Ala Val Pro Gly
   595 600 605
   Ser Asp Ala Ala Arg Gly Glu Phe Ala Thr Asp Thr Asp Ala His Asp
   610 615 620
   Pro Phe Val Asn Asp Leu Ala Phe Gln Ala Met Leu Val Trp Val Arg
   625 630 635 640
   Arg Thr Leu Gly Gln Ala Ala Leu Pro Asn Ser Ile Gln Arg Ile Val
   645 650 655
   Gln His Arg Pro Val Pro Gln Asp Lys Pro Phe Tyr Ile Thr Leu Arg
   660 665 670
   Ser Asn Gln Ser Gly Gly His Ser Gln His Lys His Ala Leu Gln Phe
   675 680 685
   His Asn Glu Gln Gly Asp Leu Phe Ile Asp Val Gln Ala Ser Val Ile
   690 695 700
   Ala Thr Asp Ser Leu Ala Phe
   705 710
<210> 19
   <211> 1350
   <212> DNA
   <213> Schizochytrium sp.
<220>
   <221> CDS
   <222> (1)..(1350)
   <223>
<400> 19
   atg gcc gct cgg aat gtg agc gcc gcg cat gag atg cac gat gaa aag        48
   Met Ala Ala Arg Asn Val Ser Ala Ala His Glu Met His Asp Glu Lys
   1               5                   10                  15
   cgc atc gcc gtc gtc ggc atg gcc gtc cag tac gcc gga tgc aaa acc        96
   Arg Ile Ala Val Val Gly Met Ala Val Gln Tyr Ala Gly Cys Lys Thr
               20                  25                  30
   aag gac gag ttc tgg gag gtg ctc atg aac ggc aag gtc gag tcc aag       144
   Lys Asp Glu Phe Trp Glu Val Leu Met Asn Gly Lys Val Glu Ser Lys
           35                  40                  45
   gtg atc agc gac aaa cga ctc ggc tcc aac tac cgc gcc gag cac tac       192
   Val Ile Ser Asp Lys Arg Leu Gly Ser Asn Tyr Arg Ala Glu His Tyr
       50                  55                  60
   aaa gca gag cgc agc aag tat gcc gac acc ttt tgc aac gaa acg tac       240
   Lys Ala Glu Arg Ser Lys Tyr Ala Asp Thr Phe Cys Asn Glu Thr Tyr
   65                  70                  75                  80
   ggc acc ctt gac gag aac gag atc gac aac gag cac gaa ctc ctc ctc      288
   Gly Thr Leu Asp Glu Asn Glu Ile Asp Asn Glu His Glu Leu Leu Leu
                   85                  90                  95
   aac ctc gcc aag cag gca ctc gca gag aca tcc gtc aaa gac tcg aca      336
   Asn Leu Ala Lys Gln Ala Leu Ala Glu Thr Ser Val Lys Asp Ser Thr
               100                 105                 110
   cgc tgc ggc atc gtc agc ggc tgc ctc tcg ttc ccc atg gac aac ctc      384
   Arg Cys Gly Ile Val Ser Gly Cys Leu Ser Phe Pro Met Asp Asn Leu
           115                 120                 125
   cag ggt gaa ctc ctc aac gtg tac caa aac cat gtc gag aaa aag ctc      432
   Gln Gly Glu Leu Leu Asn Val Tyr Gln Asn His Val Glu Lys Lys Leu
       130                 135                 140
   ggg gcc cgc gtc ttc aag gac gcc tcc cat tgg tcc gaa cgc gag cag      480
   Gly Ala Arg Val Phe Lys Asp Ala Ser His Trp Ser Glu Arg Glu Gln
   145                 150                 155                 160
   tcc aac aaa ccc gag gcc ggt gac cgc cgc atc ttc atg gac ccg gcc      528
   Ser Asn Lys Pro Glu Ala Gly Asp Arg Arg Ile Phe Met Asp Pro Ala
                   165                 170                 175
   tcc ttc gtc gcc gaa gaa ctc aac ctc ggc gcc ctt cac tac tcc gtc      576
   Ser Phe Val Ala Glu Glu Leu Asn Leu Gly Ala Leu His Tyr Ser Val
               180                 185                 190
   gac gca gca tgc gcc acg gcg ctc tac gtg ctc cgc ctc gcg cag gat      624
   Asp Ala Ala Cys Ala Thr Ala Leu Tyr Val Leu Arg Leu Ala Gln Asp
           195                 200                 205
   cat ctc gtc tcc ggc gcc gcc gac gtc atg ctc tgc ggt gcc acc tgc      672
   His Leu Val Ser Gly Ala Ala Asp Val Met Leu Cys Gly Ala Thr Cys
       210                 215                 220
   ctg ccg gag ccc ttt ttc atc ctt tcg ggc ttt tcc acc ttc cag gcc      720
   Leu Pro Glu Pro Phe Phe Ile Leu Ser Gly Phe Ser Thr Phe Gln Ala
   225                 230                 235                 240
   atg ccc gtc ggc acg ggc cag aac gtg tcc atg ccg ctg cac aag gac      768
   Met Pro Val Gly Thr Gly Gln Asn Val Ser Met Pro Leu His Lys Asp
                   245                 250                 255
   agc cag ggc ctc acc ccg ggt gag ggc ggc tcc atc atg gtc ctc aag      816
   Ser Gln Gly Leu Thr Pro Gly Glu Gly Gly Ser Ile Met Val Leu Lys
               260                 265                 270
   cgt ctc gat gat gcc atc cgc gac ggc gac cac att tac ggc acc ctt      864
   Arg Leu Asp Asp Ala Ile Arg Asp Gly Asp His Ile Tyr Gly Thr Leu
           275                 280                 285
   ctc ggc gcc aat gtc agc aac tcc ggc aca ggt ctg ccc ctc aag ccc      912
   Leu Gly Ala Asn Val Ser Asn Ser Gly Thr Gly Leu Pro Leu Lys Pro
       290                 295                 300
   ctt ctc ccc agc gag aaa aag tgc ctc atg gac acc tac acg cgc att      960
   Leu Leu Pro Ser Glu Lys Lys Cys Leu Met Asp Thr Tyr Thr Arg Ile
   305                 310                 315                 320
   aac gtg cac ccg cac aag att cag tac gtc gag tgc cac gcc acc ggc     1008
   Asn Val His Pro His Lys Ile Gln Tyr Val Glu Cys His Ala Thr Gly
                   325                 330                 335
   acg ccc cag ggt gat cgt gtg gaa atc gac gcc gtc aag gcc tgc ttt     1056
   Thr Pro Gln Gly Asp Arg Val Glu Ile Asp Ala Val Lys Ala Cys Phe
               340                 345                 350
   gaa ggc aag gtc ccc cgt ttc ggt acc aca aag ggc aac ttt gga cac     1104
   Glu Gly Lys Val Pro Arg Phe Gly Thr Thr Lys Gly Asn Phe Gly His
           355                 360                 365
   acc cts gyc gca gcc ggc ttt gcc ggt atg tgc aag gtc ctc ctc tcc     1152
   Thr Xaa Xaa Ala Ala Gly Phe Ala Gly Met Cys Lys Val Leu Leu Ser
       370                 375                 380
   atg aag cat ggc atc atc ccg ccc acc ccg ggt atc gat gac gag acc     1200
   Met Lys His Gly Ile Ile Pro Pro Thr Pro Gly Ile Asp Asp Glu Thr
   385                 390                 395                 400
   aag atg gac cct ctc gtc gtc tcc ggt gag gcc atc cca tgg cca gag     1248
   Lys Met Asp Pro Leu Val Val Ser Gly Glu Ala Ile Pro Trp Pro Glu
                       405                     410                     415
   acc aac ggc gag ccc aag cgc gcc ggt ctc tcg gcc ttt ggc ttt ggt      1296
   Thr Asn Gly Glu Pro Lys Arg Ala Gly Leu Ser Ala Phe Gly Phe Gly
                   420                     425                     430
   ggc acc aac gcc cat gcc gtc ttt gag gag cat gac ccc tcc aac gcc      1344
   Gly Thr Asn Ala His Ala Val Phe Glu Glu His Asp Pro Ser Asn Ala
               435                     440                     445
   gcc tgc                                                              1350
   Ala Cys
       450
<210> 20
   <211> 450
   <212> PRT
   <213> Schizochytrium sp.
<220>
   <221> misc_feature
   <222> (370)..(370)
   <223> The 'Xaa' at location 370 stands for Leu.
<220>
   <221> misc_feature
   <222> (371)..(371)
   <223> The 'Xaa' at location 371 stands for Ala, or Val.
<400> 20
   Met Ala Ala Arg Asn Val Ser Ala Ala His Glu Met His Asp Glu Lys
   1               5                   10                  15
   Arg Ile Ala Val Val Gly Met Ala Val Gln Tyr Ala Gly Cys Lys Thr
               20                  25                  30
   Lys Asp Glu Phe Trp Glu Val Leu Met Asn Gly Lys Val Glu Ser Lys
           35                  40                  45
   Val Ile Ser Asp Lys Arg Leu Gly Ser Asn Tyr Arg Ala Glu His Tyr
       50                  55                  60
   Lys Ala Glu Arg Ser Lys Tyr Ala Asp Thr Phe Cys Asn Glu Thr Tyr
   65                  70                  75                  80
   Gly Thr Leu Asp Glu Asn Glu Ile Asp Asn Glu His Glu Leu Leu Leu
                   85                  90                  95
   Asn Leu Ala Lys Gln Ala Leu Ala Glu Thr Ser Val Lys Asp Ser Thr
               100                 105                 110
   Arg Cys Gly Ile Val Ser Gly Cys Leu Ser Phe Pro Met Asp Asn Leu
           115                 120                 125
   Gln Gly Glu Leu Leu Asn Val Tyr Gln Asn His Val Glu Lys Lys Leu
       130                 135                 140
   Gly Ala Arg Val Phe Lys Asp Ala Ser His Trp Ser Glu Arg Glu Gln
   145                 150                 155                 160
   Ser Asn Lys Pro Glu Ala Gly Asp Arg Arg Ile Phe Met Asp Pro Ala
                   165                 170                 175
   Ser Phe Val Ala Glu Glu Leu Asn Leu Gly Ala Leu His Tyr Ser Val
               180                 185                 190
   Asp Ala Ala Cys Ala Thr Ala Leu Tyr Val Leu Arg Leu Ala Gln Asp
           195                 200                 205
   His Leu Val Ser Gly Ala Ala Asp Val Met Leu Cys Gly Ala Thr Cys
       210                 215                 220
   Leu Pro Glu Pro Phe Phe Ile Leu Ser Gly Phe Ser Thr Phe Gln Ala
   225                 230                 235                 240
   Met Pro Val Gly Thr Gly Gln Asn Val Ser Met Pro Leu His Lys Asp
                   245                 250                 255
   Ser Gln Gly Leu Thr Pro Gly Glu Gly Gly Ser Ile Met Val Leu Lys
               260                 265                 270
   Arg Leu Asp Asp Ala Ile Arg Asp Gly Asp His Ile Tyr Gly Thr Leu
           275                 280                 285
   Leu Gly Ala Asn Val Ser Asn Ser Gly Thr Gly Leu Pro Leu Lys Pro
       290                 295                 300
   Leu Leu Pro Ser Glu Lys Lys Cys Leu Met Asp Thr Tyr Thr Arg Ile
   305                 310                 315                 320
   Asn Val His Pro His Lys Ile Gln Tyr Val Glu Cys His Ala Thr Gly
                   325                 330                 335
   Thr Pro Gln Gly Asp Arg Val Glu Ile Asp Ala Val Lys Ala Cys Phe
               340                 345                 350
   Glu Gly Lys Val Pro Arg Phe Gly Thr Thr Lys Gly Asn Phe Gly His
           355                 360                 365
   Thr Xaa Xaa Ala Ala Gly Phe Ala Gly Met Cys Lys Val Leu Leu Ser
       370                 375                 380
   Met Lys His Gly Ile Ile Pro Pro Thr Pro Gly Ile Asp Asp Glu Thr
   385                 390                 395                 400
   Lys Met Asp Pro Leu Val Val Ser Gly Glu Ala Ile Pro Trp Pro Glu
                   405                 410                 415
   Thr Asn Gly Glu Pro Lys Arg Ala Gly Leu Ser Ala Phe Gly Phe Gly
               420                 425                 430
   Gly Thr Asn Ala His Ala Val Phe Glu Glu His Asp Pro Ser Asn Ala
           435                 440                 445
   Ala Cys
       450
<210> 21
   <211> 1323
   <212> DNA
   <213> Schizochytrium sp.
<220>
   <221> CDS
   <222> (1)..(1323)
   <223>
<400> 21
   tcg gcc cgc tgc ggc ggt gaa agc aac atg cgc atc gcc atc act ggt        48
   Ser Ala Arg Cys Gly Gly Glu Ser Asn Met Arg Ile Ala Ile Thr Gly
   1               5                   10                  15
   atg gac gcc acc ttt ggc gct ctc aag gga ctc gac gcc ttc gag cgc        96
   Met Asp Ala Thr Phe Gly Ala Leu Lys Gly Leu Asp Ala Phe Glu Arg
   20 25 30
   gcc att tac acc ggc gct cac ggt gcc atc cca ctc cca gaa aag cgc 144
   Ala Ile Tyr Thr Gly Ala His Gly Ala Ile Pro Leu Pro Glu Lys Arg
   35 40 45
   tgg cgc ttt ctc ggc aag gac aag gac ttt ctt gac ctc tgc ggc gtc 192
   Trp Arg Phe Leu Gly Lys Asp Lys Asp Phe Leu Asp Leu Cys Gly Val
   50 55 60
   aag gcc acc ccg cac ggc tgc tac att gaa gat gtt gag gtc gac ttc 240
   Lys Ala Thr Pro His Gly Cys Tyr Ile Glu Asp Val Glu Val Asp Phe
   65 70 75 80
   cag cgc ctc cgc acg ccc atg acc cct gaa gac atg ctc ctc cct cag 288
   Gln Arg Leu Arg Thr Pro Met Thr Pro Glu Asp Met Leu Leu Pro Gln
   85 90 95
   cag ctt ctg gcc gtc acc acc att gac cgc gcc atc ctc gac tcg gga 336
   Gln Leu Leu Ala Val Thr Thr Ile Asp Arg Ala Ile Leu Asp Ser Gly
   100 105 110
   atg aaa aag ggt ggc aat gtc gcc gtc ttt gtc ggc ctc ggc acc gac 384
   Met Lys Lys Gly Gly Asn Val Ala Val Phe Val Gly Leu Gly Thr Asp
   115 120 125
   ctc gag ctc tac cgt cac cgt gct cgc gtc gct ctc aag gag cgc gtc 432
   Leu Glu Leu Tyr Arg His Arg Ala Arg Val Ala Leu Lys Glu Arg Val
   130 135 140
   cgc cct gaa gcc tcc aag aag ctc aat gac atg atg cag tac att aac 480
   Arg Pro Glu Ala Ser Lys Lys Leu Asn Asp Met Met Gln Tyr Ile Asn
   145 150 155 160
   gac tgc ggc aca tcc aca tcg tac acc tcg tac att ggc aac ctc gtc 528
   Asp Cys Gly Thr Ser Thr Ser Tyr Thr Ser Tyr Ile Gly Asn Leu Val
   165 170 175
   gcc acg cgc gtc tcg tcg cag tgg ggc ttc acg ggc ccc tcc ttt acg 576
   Ala Thr Arg Val Ser Ser Gln Trp Gly Phe Thr Gly Pro Ser Phe Thr
   180 185 190
   atc acc gag ggc aac aac tcc gtc tac cgc tgc gcc gag ctc ggc aag 624
   Ile Thr Glu Gly Asn Asn Ser Val Tyr Arg Cys Ala Glu Leu Gly Lys
   195 200 205
   tac ctc ctc gag acc ggc gag gtc gat ggc gtc gtc gtt gcg ggt gtc 672
   Tyr Leu Leu Glu Thr Gly Glu Val Asp Gly Val Val Val Ala Gly Val
   210 215 220
   gat ctc tgc ggc agt gcc gaa aac ctt tac gtc aag tct cgc cgc ttc 720
   Asp Leu Cys Gly Ser Ala Glu Asn Leu Tyr Val Lys Ser Arg Arg Phe
   225 230 235 240
   aag gtg tcc acc tcc gat acc ccg cgc gcc agc ttt gac gcc gcc gcc 768
   Lys Val Ser Thr Ser Asp Thr Pro Arg Ala Ser Phe Asp Ala Ala Ala
   245 250 255
   gat ggc tac ttt gtc ggc gag ggc tgc ggt gcc ttt gtg ctc aag cgt 816
   Asp Gly Tyr Phe Val Gly Glu Gly Cys Gly Ala Phe Val Leu Lys Arg
   260 265 270
   gag act agc tgc acc aag gac gac cgt atc tac gct tgc atg gat gcc 864
   Glu Thr Ser Cys Thr Lys Asp Asp Arg Ile Tyr Ala Cys Met Asp Ala
   275 280 285
   atc gtc cct ggc aac gtc cct agc gcc tgc ttg cgc gag gcc ctc gac 912
   Ile Val Pro Gly Asn Val Pro Ser Ala Cys Leu Arg Glu Ala Leu Asp
   290 295 300
   cag gcg cgc gtc aag ccg ggc gat atc gag atg ctc gag ctc agc gcc 960
   Gln Ala Arg Val Lys Pro Gly Asp Ile Glu Met Leu Glu Leu Ser Ala
   305 310 315 320
   gac tcc gcc cgc cac ctc aag gac ccg tcc gtc ctg ccc aag gag ctc 1008
   Asp Ser Ala Arg His Leu Lys Asp Pro Ser Val Leu Pro Lys Glu Leu
   325 330 335
   act gcc gag gag gaa atc ggc ggc ctt cag acg atc ctt cgt gac gat 1056
   Thr Ala Glu Glu Glu Ile Gly Gly Leu Gln Thr Ile Leu Arg Asp Asp
   340 345 350
   gac aag ctc ccg cgc aac gtc gca acg ggc agt gtc aag gcc acc gtc 1104
   Asp Lys Leu Pro Arg Asn Val Ala Thr Gly Ser Val Lys Ala Thr Val
           355                 360                 365
   ggt gac acc ggt tat gcc tct ggt gct gcc agc ctc atc aag gct gcg      1152
   Gly Asp Thr Gly Tyr Ala Ser Gly Ala Ala Ser Leu Ile Lys Ala Ala
       370                 375                 380
   ctt tgc atc tac aac cgc tac ctg ccc agc aac ggc gac gac tgg gat      1200
   Leu Cys Ile Tyr Asn Arg Tyr Leu Pro Ser Asn Gly Asp Asp Trp Asp
   385                 390                 395                 400
   gaa ccc gcc cct gag gcg ccc tgg gac agc acc ctc ttt gcg tgc cag      1248
   Glu Pro Ala Pro Glu Ala Pro Trp Asp Ser Thr Leu Phe Ala Cys Gln
                   405                 410                 415
   acc tcg cgc gct tgg ctc aag aac cct ggc gag cgt cgc tat gcg gcc      1296
   Thr Ser Arg Ala Trp Leu Lys Asn Pro Gly Glu Arg Arg Tyr Ala Ala
               420                 425                 430
   gtc tcg ggc gtc tcc gag acg cgc tcg                                  1323
   Val Ser Gly Val Ser Glu Thr Arg Ser
           435                 440
<210> 22
   <211> 441
   <212> PRT
   <213> Schizochytrium sp.
<400> 22
   Ser Ala Arg Cys Gly Gly Glu Ser Asn Met Arg Ile Ala Ile Thr Gly
   1               5                   10                  15
   Met Asp Ala Thr Phe Gly Ala Leu Lys Gly Leu Asp Ala Phe Glu Arg
               20                  25                  30
   Ala Ile Tyr Thr Gly Ala His Gly Ala Ile Pro Leu Pro Glu Lys Arg
           35                  40                  45
   Trp Arg Phe Leu Gly Lys Asp Lys Asp Phe Leu Asp Leu Cys Gly Val
       50                  55                  60
   Lys Ala Thr Pro His Gly Cys Tyr Ile Glu Asp Val Glu Val Asp Phe
   65                  70                  75                  80
   Gln Arg Leu Arg Thr Pro Met Thr Pro Glu Asp Met Leu Leu Pro Gln
                   85                  90                  95
   Gln Leu Leu Ala Val Thr Thr Ile Asp Arg Ala Ile Leu Asp Ser Gly
               100                 105                 110
   Met Lys Lys Gly Gly Asn Val Ala Val Phe Val Gly Leu Gly Thr Asp
           115                 120                 125
   Leu Glu Leu Tyr Arg His Arg Ala Arg Val Ala Leu Lys Glu Arg Val
       130                 135                 140
   Arg Pro Glu Ala Ser Lys Lys Leu Asn Asp Met Met Gln Tyr Ile Asn
   145                 150                 155                 160
   Asp Cys Gly Thr Ser Thr Ser Tyr Thr Ser Tyr Ile Gly Asn Leu Val
                   165                 170                 175
   Ala Thr Arg Val Ser Ser Gln Trp Gly Phe Thr Gly Pro Ser Phe Thr
               180                 185                 190
   Ile Thr Glu Gly Asn Asn Ser Val Tyr Arg Cys Ala Glu Leu Gly Lys
           195                 200                 205
   Tyr Leu Leu Glu Thr Gly Glu Val Asp Gly Val Val Val Ala Gly Val
       210                 215                 220
   Asp Leu Cys Gly Ser Ala Glu Asn Leu Tyr Val Lys Ser Arg Arg Phe
   225                 230                 235                 240
   Lys Val Ser Thr Ser Asp Thr Pro Arg Ala Ser Phe Asp Ala Ala Ala
                   245                 250                 255
   Asp Gly Tyr Phe Val Gly Glu Gly Cys Gly Ala Phe Val Leu Lys Arg
               260                 265                 270
   Glu Thr Ser Cys Thr Lys Asp Asp Arg Ile Tyr Ala Cys Met Asp Ala
           275                 280                 285
   Ile Val Pro Gly Asn Val Pro Ser Ala Cys Leu Arg Glu Ala Leu Asp
       290                 295                 300
   Gln Ala Arg Val Lys Pro Gly Asp Ile Glu Met Leu Glu Leu Ser Ala
   305                 310                 315                 320
   Asp Ser Ala Arg His Leu Lys Asp Pro Ser Val Leu Pro Lys Glu Leu
                   325                 330                 335
   Thr Ala Glu Glu Glu Ile Gly Gly Leu Gln Thr Ile Leu Arg Asp Asp
               340                 345                 350
   Asp Lys Leu Pro Arg Asn Val Ala Thr Gly Ser Val Lys Ala Thr Val
           355                 360                 365
   Gly Asp Thr Gly Tyr Ala Ser Gly Ala Ala Ser Leu Ile Lys Ala Ala
       370                 375                 380
   Leu Cys Ile Tyr Asn Arg Tyr Leu Pro Ser Asn Gly Asp Asp Trp Asp
   385                 390                 395                 400
   Glu Pro Ala Pro Glu Ala Pro Trp Asp Ser Thr Leu Phe Ala Cys Gln
                   405                 410                 415
   Thr Ser Arg Ala Trp Leu Lys Asn Pro Gly Glu Arg Arg Tyr Ala Ala
               420                 425                 430
   Val Ser Gly Val Ser Glu Thr Arg Ser
           435                 440
<210> 23
   <211> 1500
   <212> DNA
   <213> Schizochytrium sp.
<220>
   <221> CDS
   <222> (1)..(1500)
   <223>
<400> 23
   tgc tat tcc gtg ctc ctc tcc gaa gcc gag ggc cac tac gag cgc gag       48
   Cys Tyr Ser Val Leu Leu Ser Glu Ala Glu Gly His Tyr Glu Arg Glu
   1               5                   10                  15
   aac cgc atc tcg ctc gac gag gag gcg ccc aag ctc att gtg ctt cgc       96
   Asn Arg Ile Ser Leu Asp Glu Glu Ala Pro Lys Leu Ile Val Leu Arg
               20                  25                  30
   gcc gac tcc cac gag gag atc ctt ggt cgc ctc gac aag atc cgc gag      144
   Ala Asp Ser His Glu Glu Ile Leu Gly Arg Leu Asp Lys Ile Arg Glu
           35                  40                  45
   cgc ttc ttg cag ccc acg ggc gcc gcc ccg cgc gag tcc gag ctc aag      192
   Arg Phe Leu Gln Pro Thr Gly Ala Ala Pro Arg Glu Ser Glu Leu Lys
       50                  55                  60
   gcg cag gcc cgc cgc atc ttc ctc gag ctc ctc ggc gag acc ctt gcc      240
   Ala Gln Ala Arg Arg Ile Phe Leu Glu Leu Leu Gly Glu Thr Leu Ala
   65                  70                  75                  80
   cag gat gcc gct tct tca ggc tcg caa aag ccc ctc gct ctc agc ctc      288
   Gln Asp Ala Ala Ser Ser Gly Ser Gln Lys Pro Leu Ala Leu Ser Leu
                   85                  90                  95
   gtc tcc acg ccc tcc aag ctc cag cgc gag gtc gag ctc gcg gcc aag      336
   Val Ser Thr Pro Ser Lys Leu Gln Arg Glu Val Glu Leu Ala Ala Lys
               100                 105                 110
   ggt atc ccg cgc tgc ctc aag atg cgc cgc gat tgg agc tcc cct gct      384
   Gly Ile Pro Arg Cys Leu Lys Met Arg Arg Asp Trp Ser Ser Pro Ala
           115                 120                 125
   ggc agc cgc tac gcg cct gag ccg ctc gcc agc gac cgc gtc gcc ttc      432
   Gly Ser Arg Tyr Ala Pro Glu Pro Leu Ala Ser Asp Arg Val Ala Phe
       130                 135                 140
   atg tac ggc gaa ggt cgc agc cct tac tac ggc atc acc caa gac att      480
   Met Tyr Gly Glu Gly Arg Ser Pro Tyr Tyr Gly Ile Thr Gln Asp Ile
   145                 150                 155                 160
   cac cgc att tgg ccc gaa ctc cac gag gtc atc aac gaa aag acg aac      528
   His Arg Ile Trp Pro Glu Leu His Glu Val Ile Asn Glu Lys Thr Asn
                   165                 170                 175
   cgt ctc tgg gcc gaa ggc gac cgc tgg gtc atg ccg cgc gcc agc ttc      576
   Arg Leu Trp Ala Glu Gly Asp Arg Trp Val Met Pro Arg Ala Ser Phe
               180                 185                 190
   aag tcg gag ctc gag agc cag cag caa gag ttt gat cgc aac atg att      624
   Lys Ser Glu Leu Glu Ser Gln Gln Gln Glu Phe Asp Arg Asn Met Ile
           195                 200                 205
   gaa atg ttc cgt ctt gga atc ctc acc tca att gcc ttc acc aat ctg      672
   Glu Met Phe Arg Leu Gly Ile Leu Thr Ser Ile Ala Phe Thr Asn Leu
       210                 215                 220
   gcg cgc gac gtt ctc aac atc acg ccc aag gcc gcc ttt ggc ctc agt      720
   Ala Arg Asp Val Leu Asn Ile Thr Pro Lys Ala Ala Phe Gly Leu Ser
   225                 230                 235                 240
   ctt ggc gag att tcc atg att ttt gcc ttt tcc aag aag aac ggt ctc      768
   Leu Gly Glu Ile Ser Met Ile Phe Ala Phe Ser Lys Lys Asn Gly Leu
                   245                 250                 255
   atc tcc gac cag ctc acc aag gat ctt cgc gag tcc gac gtg tgg aac      816
   Ile Ser Asp Gln Leu Thr Lys Asp Leu Arg Glu Ser Asp Val Trp Asn
               260                 265                 270
   aag gct ctg gcc gtt gaa ttt aat gcg ctg cgc gag gcc tgg ggc att      864
   Lys Ala Leu Ala Val Glu Phe Asn Ala Leu Arg Glu Ala Trp Gly Ile
           275                 280                 285
   cca cag agt gtc ccc aag gac gag ttc tgg caa ggc tac att gtg cgc      912
   Pro Gln Ser Val Pro Lys Asp Glu Phe Trp Gln Gly Tyr Ile Val Arg
       290                 295                 300
   ggc acc aag cag gat atc gag gcg gcc atc gcc ccg gac agc aag tac      960
   Gly Thr Lys Gln Asp Ile Glu Ala Ala Ile Ala Pro Asp Ser Lys Tyr
   305                 310                 315                 320
   gtg cgc ctc acc atc atc aat gat gcc aac acc gcc ctc att agc ggc     1008
   Val Arg Leu Thr Ile Ile Asn Asp Ala Asn Thr Ala Leu Ile Ser Gly
                   325                 330                 335
   aag ccc gac gcc tgc aag gct gcg atc gcg cgt ctc ggt ggc aac att     1056
   Lys Pro Asp Ala Cys Lys Ala Ala Ile Ala Arg Leu Gly Gly Asn Ile
               340                 345                 350
   cct gcg ctt ccc gtg acc cag ggc atg tgc ggc cac tgc ccc gag gtg     1104
   Pro Ala Leu Pro Val Thr Gln Gly Met Cys Gly His Cys Pro Glu Val
           355                 360                 365
   gga cct tat acc aag gat atc gcc aag atc cat gcc aac ctt gag ttc     1152
   Gly Pro Tyr Thr Lys Asp Ile Ala Lys Ile His Ala Asn Leu Glu Phe
       370                 375                 380
   ccc gtt gtc gac ggc ctt gac ctc tgg acc aca atc aac cag aag cgc      1200
   Pro Val Val Asp Gly Leu Asp Leu Trp Thr Thr Ile Asn Gln Lys Arg
   385                 390                 395                 400
   ctc gtg cca cgc gcc acg ggc gcc aag gac gaa tgg gcc cct tct tcc      1248
   Leu Val Pro Arg Ala Thr Gly Ala Lys Asp Glu Trp Ala Pro Ser Ser
                   405                 410                 415
   ttt ggc gag tac gcc ggc cag ctc tac gag aag cag gct aac ttc ccc      1296
   Phe Gly Glu Tyr Ala Gly Gln Leu Tyr Glu Lys Gln Ala Asn Phe Pro
               420                 425                 430
   caa atc gtc gag acc att tac aag caa aac tac gac gtc ttt gtc gag      1344
   Gln Ile Val Glu Thr Ile Tyr Lys Gln Asn Tyr Asp Val Phe Val Glu
           435                 440                 445
   gtt ggg ccc aac aac cac cgt agc acc gca gtg cgc acc acg ctt ggt      1392
   Val Gly Pro Asn Asn His Arg Ser Thr Ala Val Arg Thr Thr Leu Gly
       450                 455                 460
   ccc cag cgc aac cac ctt gct ggc gcc atc gac aag cag aac gag gat      1440
   Pro Gln Arg Asn His Leu Ala Gly Ala Ile Asp Lys Gln Asn Glu Asp
   465                 470                 475                 480
   gct tgg acg acc atc gtc aag ctt gtg gct tcg ctc aag gcc cac ctt      1488
   Ala Trp Thr Thr Ile Val Lys Leu Val Ala Ser Leu Lys Ala His Leu
                   485                 490                 495
   gtt cct ggc gtc                                                      1500
   Val Pro Gly Val
               500
<210> 24
   <211> 500
   <212> PRT
   <213> Schizochytrium sp.
<400> 24
   Cys Tyr Ser Val Leu Leu Ser Glu Ala Glu Gly His Tyr Glu Arg Glu
   1               5                   10                  15
   Asn Arg Ile Ser Leu Asp Glu Glu Ala Pro Lys Leu Ile Val Leu Arg
               20                  25                  30
   Ala Asp Ser His Glu Glu Ile Leu Gly Arg Leu Asp Lys Ile Arg Glu
           35                  40                  45
   Arg Phe Leu Gln Pro Thr Gly Ala Ala Pro Arg Glu Ser Glu Leu Lys
       50                  55                  60
   Ala Gln Ala Arg Arg Ile Phe Leu Glu Leu Leu Gly Glu Thr Leu Ala
   65                  70                  75                  80
   Gln Asp Ala Ala Ser Ser Gly Ser Gln Lys Pro Leu Ala Leu Ser Leu
                   85                  90                  95
   Val Ser Thr Pro Ser Lys Leu Gln Arg Glu Val Glu Leu Ala Ala Lys
               100                 105                 110
   Gly Ile Pro Arg Cys Leu Lys Met Arg Arg Asp Trp Ser Ser Pro Ala
           115                 120                 125
   Gly Ser Arg Tyr Ala Pro Glu Pro Leu Ala Ser Asp Arg Val Ala Phe
       130                 135                 140
   Met Tyr Gly Glu Gly Arg Ser Pro Tyr Tyr Gly Ile Thr Gln Asp Ile
   145                 150                 155                 160
   His Arg Ile Trp Pro Glu Leu His Glu Val Ile Asn Glu Lys Thr Asn
                   165                 170                 175
   Arg Leu Trp Ala Glu Gly Asp Arg Trp Val Met Pro Arg Ala Ser Phe
               180                 185                 190
   Lys Ser Glu Leu Glu Ser Gln Gln Gln Glu Phe Asp Arg Asn Met Ile
           195                 200                 205
   Glu Met Phe Arg Leu Gly Ile Leu Thr Ser Ile Ala Phe Thr Asn Leu
       210                 215                 220
   Ala Arg Asp Val Leu Asn Ile Thr Pro Lys Ala Ala Phe Gly Leu Ser
   225                 230                 235                 240
   Leu Gly Glu Ile Ser Met Ile Phe Ala Phe Ser Lys Lys Asn Gly Leu
                   245                 250                 255
   Ile Ser Asp Gln Leu Thr Lys Asp Leu Arg Glu Ser Asp Val Trp Asn
               260                 265                 270
   Lys Ala Leu Ala Val Glu Phe Asn Ala Leu Arg Glu Ala Trp Gly Ile
           275                 280                 285
   Pro Gln Ser Val Pro Lys Asp Glu Phe Trp Gln Gly Tyr Ile Val Arg
       290                 295                 300
   Gly Thr Lys Gln Asp Ile Glu Ala Ala Ile Ala Pro Asp Ser Lys Tyr
   305                 310                 315                 320
   Val Arg Leu Thr Ile Ile Asn Asp Ala Asn Thr Ala Leu Ile Ser Gly
                   325                 330                 335
   Lys Pro Asp Ala Cys Lys Ala Ala Ile Ala Arg Leu Gly Gly Asn Ile
               340                 345                 350
   Pro Ala Leu Pro Val Thr Gln Gly Met Cys Gly His Cys Pro Glu Val
           355                 360                 365
   Gly Pro Tyr Thr Lys Asp Ile Ala Lys Ile His Ala Asn Leu Glu Phe
       370                 375                 380
   Pro Val Val Asp Gly Leu Asp Leu Trp Thr Thr Ile Asn Gln Lys Arg
   385                 390                 395                 400
   Leu Val Pro Arg Ala Thr Gly Ala Lys Asp Glu Trp Ala Pro Ser Ser
                   405                 410                 415
   Phe Gly Glu Tyr Ala Gly Gln Leu Tyr Glu Lys Gln Ala Asn Phe Pro
               420                 425                 430
   Gln Ile Val Glu Thr Ile Tyr Lys Gln Asn Tyr Asp Val Phe Val Glu
           435                 440                 445
   Val Gly Pro Asn Asn His Arg Ser Thr Ala Val Arg Thr Thr Leu Gly
       450                 455                 460
   Pro Gln Arg Asn His Leu Ala Gly Ala Ile Asp Lys Gln Asn Glu Asp
   465                 470                 475                 480
   Ala Trp Thr Thr Ile Val Lys Leu Val Ala Ser Leu Lys Ala His Leu
                   485                 490                 495
   Val Pro Gly Val
   500
<210> 25
   <211> 1530
   <212> DNA
   <213> Schizochytrium sp.
<220>
   <221> CDS
   <222> (1)..(1530)
   <223>
<400> 25
   ctg ctc gat ctc gac agt atg ctt gcg ctg agc tct gcc agt gcc tcc        48
   Leu Leu Asp Leu Asp Ser Met Leu Ala Leu Ser Ser Ala Ser Ala Ser
   1               5                   10                  15
   ggc aac ctt gtt gag act gcg cct agc gac gcc tcg gtc att gtg ccg        96
   Gly Asn Leu Val Glu Thr Ala Pro Ser Asp Ala Ser Val Ile Val Pro
               20                  25                  30
   ccc tgc aac att gcg gat ctc ggc agc cgc gcc ttc atg aaa acg tac       144
   Pro Cys Asn Ile Ala Asp Leu Gly Ser Arg Ala Phe Met Lys Thr Tyr
           35                  40                  45
   ggt gtt tcg gcg cct ctg tac acg ggc gcc atg gcc aag ggc att gcc       192
   Gly Val Ser Ala Pro Leu Tyr Thr Gly Ala Met Ala Lys Gly Ile Ala
       50                  55                  60
   tct gcg gac ctc gtc att gcc gcc ggc cgc cag ggc atc ctt gcg tcc       240
   Ser Ala Asp Leu Val Ile Ala Ala Gly Arg Gln Gly Ile Leu Ala Ser
   65                  70                  75                  80
   ttt ggc gcc ggc gga ctt ccc atg cag gtt gtg cgt gag tcc atc gaa       288
   Phe Gly Ala Gly Gly Leu Pro Met Gln Val Val Arg Glu Ser Ile Glu
                   85                  90                  95
   aag att cag gcc gcc ctg ccc aat ggc ccg tac gct gtc aac ctt atc       336
   Lys Ile Gln Ala Ala Leu Pro Asn Gly Pro Tyr Ala Val Asn Leu Ile
               100                 105                 110
   cat tct ccc ttt gac agc aac ctc gaa aag ggc aat gtc gat ctc ttc       384
   His Ser Pro Phe Asp Ser Asn Leu Glu Lys Gly Asn Val Asp Leu Phe
           115                 120                 125
   ctc gag aag ggt gtc acc ttt gtc gag gcc tcg gcc ttt atg acg ctc       432
   Leu Glu Lys Gly Val Thr Phe Val Glu Ala Ser Ala Phe Met Thr Leu
       130                 135                 140
   acc ccg cag gtc gtg cgg tac cgc gcg gct ggc ctc acg cgc aac gcc       480
   Thr Pro Gln Val Val Arg Tyr Arg Ala Ala Gly Leu Thr Arg Asn Ala
   145                 150                 155                 160
   gac ggc tcg gtc aac atc cgc aac cgt atc att ggc aag gtc tcg cgc       528
   Asp Gly Ser Val Asn Ile Arg Asn Arg Ile Ile Gly Lys Val Ser Arg
                   165                 170                 175
   acc gag ctc gcc gag atg ttc atg cgt cct gcg ccc gag cac ctt ctt       576
   Thr Glu Leu Ala Glu Met Phe Met Arg Pro Ala Pro Glu His Leu Leu
               180                 185                 190
   cag aag ctc att gct tcc ggc gag atc aac cag gag cag gcc gag ctc       624
   Gln Lys Leu Ile Ala Ser Gly Glu Ile Asn Gln Glu Gln Ala Glu Leu
           195                 200                 205
   gcc cgc cgt gtt ccc gtc gct gac gac atc gcg gtc gaa gct gac tcg       672
   Ala Arg Arg Val Pro Val Ala Asp Asp Ile Ala Val Glu Ala Asp Ser
       210                 215                 220
   ggt ggc cac acc gac aac cgc ccc atc cac gtc att ctg ccc ctc atc       720
   Gly Gly His Thr Asp Asn Arg Pro Ile His Val Ile Leu Pro Leu Ile
   225                 230                 235                 240
   atc aac ctt cgc gac cgc ctt cac cgc gag tgc ggc tac ccg gcc aac       768
   Ile Asn Leu Arg Asp Arg Leu His Arg Glu Cys Gly Tyr Pro Ala Asn
                   245                 250                 255
   ctt cgc gtc cgt gtg ggc gcc ggc ggt ggc att ggg tgc ccc cag gcg       816
   Leu Arg Val Arg Val Gly Ala Gly Gly Gly Ile Gly Cys Pro Gln Ala
               260                 265                 270
   gcg ctg gcc acc ttc aac atg ggt gcc tcc ttt att gtc acc ggc acc      864
   Ala Leu Ala Thr Phe Asn Met Gly Ala Ser Phe Ile Val Thr Gly Thr
           275                 280                 285
   gtg aac cag gtc gcc aag cag tcg ggc acg tgc gac aat gtg cgc aag      912
   Val Asn Gln Val Ala Lys Gln Ser Gly Thr Cys Asp Asn Val Arg Lys
       290                 295                 300
   cag ctc gcg aag gcc act tac tcg gac gta tgc atg gcc ccg gct gcc      960
   Gln Leu Ala Lys Ala Thr Tyr Ser Asp Val Cys Met Ala Pro Ala Ala
   305                 310                 315                 320
   gac atg ttc gag gaa ggc gtc aag ctt cag gtc ctc aag aag gga acc     1008
   Asp Met Phe Glu Glu Gly Val Lys Leu Gln Val Leu Lys Lys Gly Thr
                   325                 330                 335
   atg ttt ccc tcg cgc gcc aac aag ctc tac gag ctc ttt tgc aag tac     1056
   Met Phe Pro Ser Arg Ala Asn Lys Leu Tyr Glu Leu Phe Cys Lys Tyr
               340                 345                 350
   gac tcg ttc gag tcc atg ccc ccc gca gag ctt gcg cgc gtc gag aag     1104
   Asp Ser Phe Glu Ser Met Pro Pro Ala Glu Leu Ala Arg Val Glu Lys
           355                 360                 365
   cgc atc ttc agc cgc gcg ctc gaa gag gtc tgg gac gag acc aaa aac     1152
   Arg Ile Phe Ser Arg Ala Leu Glu Glu Val Trp Asp Glu Thr Lys Asn
       370                 375                 380
   ttt tac att aac cgt ctt cac aac ccg gag aag atc cag cgc gcc gag     1200
   Phe Tyr Ile Asn Arg Leu His Asn Pro Glu Lys Ile Gln Arg Ala Glu
   385                 390                 395                 400
   cgc gac ccc aag ctc aag atg tcg ctg tgc ttt cgc tgg tac ctg agc     1248
   Arg Asp Pro Lys Leu Lys Met Ser Leu Cys Phe Arg Trp Tyr Leu Ser
                   405                 410                 415
   ctg gcg agc cgc tgg gcc aac act gga gct tcc gat cgc gtc atg gac     1296
   Leu Ala Ser Arg Trp Ala Asn Thr Gly Ala Ser Asp Arg Val Met Asp
               420                 425                 430
   tac cag gtc tgg tgc ggt cct gcc att ggt tcc ttc aac gat ttc atc     1344
   Tyr Gln Val Trp Cys Gly Pro Ala Ile Gly Ser Phe Asn Asp Phe Ile
           435                 440                 445
   aag gga act tac ctt gat ccg gcc gtc gca aac gag tac ccg tgc gtc     1392
   Lys Gly Thr Tyr Leu Asp Pro Ala Val Ala Asn Glu Tyr Pro Cys Val
       450                 455                 460
   gtt cag att aac aag cag atc ctt cgt gga gcg tgc ttc ttg cgc cgt     1440
   Val Gln Ile Asn Lys Gln Ile Leu Arg Gly Ala Cys Phe Leu Arg Arg
   465                 470                 475                 480
   ctc gaa att ctg cgc aac gca cgc ctt tcc gat ggc gct gcc gct ctt     1488
   Leu Glu Ile Leu Arg Asn Ala Arg Leu Ser Asp Gly Ala Ala Ala Leu
                   485                 490                 495
   gtg gcc agc atc gat gac aca tac gtc ccg gcc gag aag ctg             1530
   Val Ala Ser Ile Asp Asp Thr Tyr Val Pro Ala Glu Lys Leu
               500                 505                 510
<210> 26
   <211> 510
   <212> PRT
   <213> Schizochytrium sp.
<400> 26
   Leu Leu Asp Leu Asp Ser Met Leu Ala Leu Ser Ser Ala Ser Ala Ser
   1               5                   10                  15
   Gly Asn Leu Val Glu Thr Ala Pro Ser Asp Ala Ser Val Ile Val Pro
               20                  25                  30
   Pro Cys Asn Ile Ala Asp Leu Gly Ser Arg Ala Phe Met Lys Thr Tyr
           35                  40                  45
   Gly Val Ser Ala Pro Leu Tyr Thr Gly Ala Met Ala Lys Gly Ile Ala
       50                  55                  60
   Ser Ala Asp Leu Val Ile Ala Ala Gly Arg Gln Gly Ile Leu Ala Ser
   65                  70                  75                  80
   Phe Gly Ala Gly Gly Leu Pro Met Gln Val Val Arg Glu Ser Ile Glu
                   85                  90                  95
   Lys Ile Gln Ala Ala Leu Pro Asn Gly Pro Tyr Ala Val Asn Leu Ile
               100                 105                 110
   His Ser Pro Phe Asp Ser Asn Leu Glu Lys Gly Asn Val Asp Leu Phe
           115                 120                 125
   Leu Glu Lys Gly Val Thr Phe Val Glu Ala Ser Ala Phe Met Thr Leu
       130                 135                 140
   Thr Pro Gln Val Val Arg Tyr Arg Ala Ala Gly Leu Thr Arg Asn Ala
   145                 150                 155                 160
   Asp Gly Ser Val Asn Ile Arg Asn Arg Ile Ile Gly Lys Val Ser Arg
                   165                 170                 175
   Thr Glu Leu Ala Glu Met Phe Met Arg Pro Ala Pro Glu His Leu Leu
               180                 185                 190
   Gln Lys Leu Ile Ala Ser Gly Glu Ile Asn Gln Glu Gln Ala Glu Leu
           195                 200                 205
   Ala Arg Arg Val Pro Val Ala Asp Asp Ile Ala Val Glu Ala Asp Ser
       210                 215                 220
   Gly Gly His Thr Asp Asn Arg Pro Ile His Val Ile Leu Pro Leu Ile
   225                 230                 235                 240
   Ile Asn Leu Arg Asp Arg Leu His Arg Glu Cys Gly Tyr Pro Ala Asn
                   245                 250                 255
   Leu Arg Val Arg Val Gly Ala Gly Gly Gly Ile Gly Cys Pro Gln Ala
               260                 265                 270
   Ala Leu Ala Thr Phe Asn Met Gly Ala Ser Phe Ile Val Thr Gly Thr
           275                 280                 285
   Val Asn Gln Val Ala Lys Gln Ser Gly Thr Cys Asp Asn Val Arg Lys
       290                 295                 300
   Gln Leu Ala Lys Ala Thr Tyr Ser Asp Val Cys Met Ala Pro Ala Ala
   305                 310                 315                 320
   Asp Met Phe Glu Glu Gly Val Lys Leu Gln Val Leu Lys Lys Gly Thr
                   325                 330                 335
   Met Phe Pro Ser Arg Ala Asn Lys Leu Tyr Glu Leu Phe Cys Lys Tyr
               340                 345                 350
   Asp Ser Phe Glu Ser Met Pro Pro Ala Glu Leu Ala Arg Val Glu Lys
           355                 360                 365
   Arg Ile Phe Ser Arg Ala Leu Glu Glu Val Trp Asp Glu Thr Lys Asn
       370                 375                 380
   Phe Tyr Ile Asn Arg Leu His Asn Pro Glu Lys Ile Gln Arg Ala Glu
   385 390 395 400
   Arg Asp Pro Lys Leu Lys Met Ser Leu Cys Phe Arg Trp Tyr Leu Ser
   405 410 415
   Leu Ala Ser Arg Trp Ala Asn Thr Gly Ala Ser Asp Arg Val Met Asp
   420 425 430
   Tyr Gln Val Trp Cys Gly Pro Ala Ile Gly Ser Phe Asn Asp Phe Ile
   435 440 445
   Lys Gly Thr Tyr Leu Asp Pro Ala Val Ala Asn Glu Tyr Pro Cys Val
   450 455 460
   Val Gln Ile Asn Lys Gln Ile Leu Arg Gly Ala Cys Phe Leu Arg Arg
   465 470 475 480
   Leu Glu Ile Leu Arg Asn Ala Arg Leu Ser Asp Gly Ala Ala Ala Leu
   485 490 495
   Val Ala Ser Ile Asp Asp Thr Tyr Val Pro Ala Glu Lys Leu
   500 505 510
<210> 27
   <211> 4512
   <212> DNA
   <213> Schizochytrium sp.
<220>
   <221> CDS
   <222> (1)..(4512)
   <223>
<400> 27
   atg gcg ctc cgt gtc aag acg aac aag aag cca tgc tgg gag atg acc      48
   Met Ala Leu Arg Val Lys Thr Asn Lys Lys Pro Cys Trp Glu Met Thr
   1               5                   10                  15
   aag gag gag ctg acc agc ggc aag acc gag gtg ttc aac tat gag gaa      96
   Lys Glu Glu Leu Thr Ser Gly Lys Thr Glu Val Phe Asn Tyr Glu Glu
               20                  25                  30
   ctc ctc gag ttc gca gag ggc gac atc gcc aag gtc ttc gga ccc gag     144
   Leu Leu Glu Phe Ala Glu Gly Asp Ile Ala Lys Val Phe Gly Pro Glu
           35                  40                  45
   ttc gcc gtc atc gac aag tac ccg cgc cgc gtg cgc ctg ccc gcc cgc     192
   Phe Ala Val Ile Asp Lys Tyr Pro Arg Arg Val Arg Leu Pro Ala Arg
       50                  55                  60
   gag tac ctg ctc gtg acc cgc gtc acc ctc atg gac gcc gag gtc aac     240
   Glu Tyr Leu Leu Val Thr Arg Val Thr Leu Met Asp Ala Glu Val Asn
   65                  70                  75                  80
   aac tac cgc gtc ggc gcc cgc atg gtc acc gag tac gat ctc ccc gtc     288
   Asn Tyr Arg Val Gly Ala Arg Met Val Thr Glu Tyr Asp Leu Pro Val
                   85                  90                  95
   aac gga gag ctc tcc gag ggc gga gac tgc ccc tgg gcc gtc ctg gtc     336
   Asn Gly Glu Leu Ser Glu Gly Gly Asp Cys Pro Trp Ala Val Leu Val
               100                 105                 110
   gag agt ggc cag tgc gat ctc atg ctc atc tcc tac atg ggc att gac     384
   Glu Ser Gly Gln Cys Asp Leu Met Leu Ile Ser Tyr Met Gly Ile Asp
           115                 120                 125
   ttc cag aac cag ggc gac cgc gtc tac cgc ctg ctc aac acc acg ctc     432
   Phe Gln Asn Gln Gly Asp Arg Val Tyr Arg Leu Leu Asn Thr Thr Leu
       130                 135                 140
   acc ttt tac ggc gtg gcc cac gag ggc gag acc ctc gag tac gac att     480
   Thr Phe Tyr Gly Val Ala His Glu Gly Glu Thr Leu Glu Tyr Asp Ile
   145                 150                 155                 160
   cgc gtc acc ggc ttc gcc aag cgt ctc gac ggc ggc atc tcc atg ttc      528
   Arg Val Thr Gly Phe Ala Lys Arg Leu Asp Gly Gly Ile Ser Met Phe
                   165                 170                 175
   ttc ttc gag tac gac tgc tac gtc aac ggc cgc ctc ctc atc gag atg      576
   Phe Phe Glu Tyr Asp Cys Tyr Val Asn Gly Arg Leu Leu Ile Glu Met
               180                 185                 190
   cgc gat ggc tgc gcc ggc ttc ttc acc aac gag gag ctc gac gcc ggc      624
   Arg Asp Gly Cys Ala Gly Phe Phe Thr Asn Glu Glu Leu Asp Ala Gly
           195                 200                 205
   aag ggc gtc gtc ttc acc cgc ggc gac ctc gcc gcc cgc gcc aag atc      672
   Lys Gly Val Val Phe Thr Arg Gly Asp Leu Ala Ala Arg Ala Lys Ile
       210                 215                 220
   cca aag cag gac gtc tcc ccc tac gcc gtc gcc ccc tgc ctc cac aag      720
   Pro Lys Gln Asp Val Ser Pro Tyr Ala Val Ala Pro Cys Leu His Lys
   225                 230                 235                 240
   acc aag ctc aac gaa aag gag atg cag acc ctc gtc gac aag gac tgg      768
   Thr Lys Leu Asn Glu Lys Glu Met Gln Thr Leu Val Asp Lys Asp Trp
                   245                 250                 255
   gca tcc gtc ttt ggc tcc aag aac ggc atg ccg gaa atc aac tac aaa      816
   Ala Ser Val Phe Gly Ser Lys Asn Gly Met Pro Glu Ile Asn Tyr Lys
               260                 265                 270
   ctc tgc gcg cgt aag atg ctc atg att gac cgc gtc acc agc att gac      864
   Leu Cys Ala Arg Lys Met Leu Met Ile Asp Arg Val Thr Ser Ile Asp
           275                 280                 285
   cac aag ggc ggt gtc tac ggc ctc ggt cag ctc gtc ggt gaa aag atc      912
   His Lys Gly Gly Val Tyr Gly Leu Gly Gln Leu Val Gly Glu Lys Ile
       290                 295                 300
   ctc gag cgc gac cac tgg tac ttt ccc tgc cac ttt gtc aag gat cag      960
   Leu Glu Arg Asp His Trp Tyr Phe Pro Cys His Phe Val Lys Asp Gln
   305                 310                 315                 320
   gtc atg gcc gga tcc ctc gtc tcc gac ggc tgc agc cag atg ctc aag     1008
   Val Met Ala Gly Ser Leu Val Ser Asp Gly Cys Ser Gln Met Leu Lys
                   325                 330                 335
   atg tac atg atc tgg ctc ggc ctc cac ctc acc acc gga ccc ttt gac     1056
   Met Tyr Met Ile Trp Leu Gly Leu His Leu Thr Thr Gly Pro Phe Asp
               340                 345                 350
   ttc cgc ccg gtc aac ggc cac ccc aac aag gtc cgc tgc cgc ggc caa     1104
   Phe Arg Pro Val Asn Gly His Pro Asn Lys Val Arg Cys Arg Gly Gln
           355                 360                 365
   atc tcc ccg cac aag ggc aag ctc gtc tac gtc atg gag atc aag gag     1152
   Ile Ser Pro His Lys Gly Lys Leu Val Tyr Val Met Glu Ile Lys Glu
       370                 375                 380
   atg ggc ttc gac gag gac aac gac ccg tac gcc att gcc gac gtc aac     1200
   Met Gly Phe Asp Glu Asp Asn Asp Pro Tyr Ala Ile Ala Asp Val Asn
   385                 390                 395                 400
   atc att gat gtc gac ttc gaa aag ggc cag gac ttt agc ctc gac cgc     1248
   Ile Ile Asp Val Asp Phe Glu Lys Gly Gln Asp Phe Ser Leu Asp Arg
                   405                 410                 415
   atc agc gac tac ggc aag ggc gac ctc aac aag aag atc gtc gtc gac     1296
   Ile Ser Asp Tyr Gly Lys Gly Asp Leu Asn Lys Lys Ile Val Val Asp
               420                 425                 430
   ttt aag ggc atc gct ctc aag atg cag aag cgc tcc acc aac aag aac     1344
   Phe Lys Gly Ile Ala Leu Lys Met Gln Lys Arg Ser Thr Asn Lys Asn
           435                 440                 445
   ccc tcc aag gtt cag ccc gtc ttt gcc aac ggc gcc gcc act gtc ggc     1392
   Pro Ser Lys Val Gln Pro Val Phe Ala Asn Gly Ala Ala Thr Val Gly
       450                 455                 460
   ccc gag gcc tcc aag gct tcc tcc ggc gcc agc gcc agc gcc agc gcc     1440
   Pro Glu Ala Ser Lys Ala Ser Ser Gly Ala Ser Ala Ser Ala Ser Ala
   465                 470                 475                 480
   gcc ccg gcc aag cct gcc ttc agc gcc gat gtt ctt gcg ccc aag ccc     1488
   Ala Pro Ala Lys Pro Ala Phe Ser Ala Asp Val Leu Ala Pro Lys Pro
                   485                 490                 495
   gtt gcc ctt ccc gag cac atc ctc aag ggc gac gcc ctc gcc ccc aag      1536
   Val Ala Leu Pro Glu His Ile Leu Lys Gly Asp Ala Leu Ala Pro Lys
               500                 505                 510
   gag atg tcc tgg cac ccc atg gcc cgc atc ccg ggc aac ccg acg ccc      1584
   Glu Met Ser Trp His Pro Met Ala Arg Ile Pro Gly Asn Pro Thr Pro
           515                 520                 525
   tct ttt gcg ccc tcg gcc tac aag ccg cgc aac atc gcc ttt acg ccc      1632
   Ser Phe Ala Pro Ser Ala Tyr Lys Pro Arg Asn Ile Ala Phe Thr Pro
       530                 535                 540
   ttc ccc ggc aac ccc aac gat aac gac cac acc ccg ggc aag atg ccg      1680
   Phe Pro Gly Asn Pro Asn Asp Asn Asp His Thr Pro Gly Lys Met Pro
   545                 550                 555                 560
   ctc acc tgg ttc aac atg gcc gag ttc atg gcc ggc aag gtc agc atg      1728
   Leu Thr Trp Phe Asn Met Ala Glu Phe Met Ala Gly Lys Val Ser Met
                   565                 570                 575
   tgc ctc ggc ccc gag ttc gcc aag ttc gac gac tcg aac acc agc cgc      1776
   Cys Leu Gly Pro Glu Phe Ala Lys Phe Asp Asp Ser Asn Thr Ser Arg
               580                 585                 590
   agc ccc gct tgg gac ctc gct ctc gtc acc cgc gcc gtg tct gtg tct      1824
   Ser Pro Ala Trp Asp Leu Ala Leu Val Thr Arg Ala Val Ser Val Ser
           595                 600                 605
   gac ctc aag cac gtc aac tac cgc aac atc gac ctc gac ccc tcc aag      1872
   Asp Leu Lys His Val Asn Tyr Arg Asn Ile Asp Leu Asp Pro Ser Lys
       610                 615                 620
   ggt acc atg gtc ggc gag ttc gac tgc ccc gcg gac gcc tgg ttc tac      1920
   Gly Thr Met Val Gly Glu Phe Asp Cys Pro Ala Asp Ala Trp Phe Tyr
   625                 630                 635                 640
   aag ggc gcc tgc aac gat gcc cac atg ccg tac tcg atc ctc atg gag      1968
   Lys Gly Ala Cys Asn Asp Ala His Met Pro Tyr Ser Ile Leu Met Glu
                   645                 650                 655
   atc gcc ctc cag acc tcg ggt gtg ctc acc tcg gtg ctc aag gcg ccc      2016
   Ile Ala Leu Gln Thr Ser Gly Val Leu Thr Ser Val Leu Lys Ala Pro
               660                 665                 670
   ctg acc atg gag aag gac gac atc ctc ttc cgc aac ctc gac gcc aac      2064
   Leu Thr Met Glu Lys Asp Asp Ile Leu Phe Arg Asn Leu Asp Ala Asn
           675                 680                 685
   gcc gag ttc gtg cgc gcc gac ctc gac tac cgc ggc aag act atc cgc      2112
   Ala Glu Phe Val Arg Ala Asp Leu Asp Tyr Arg Gly Lys Thr Ile Arg
       690                 695                 700
   aac gtc acc aag tgc act ggc tac agc atg ctc ggc gag atg ggc gtc      2160
   Asn Val Thr Lys Cys Thr Gly Tyr Ser Met Leu Gly Glu Met Gly Val
   705                 710                 715                 720
   cac cgc ttc acc ttt gag ctc tac gtc gat gat gtg ctc ttt tac aag      2208
   His Arg Phe Thr Phe Glu Leu Tyr Val Asp Asp Val Leu Phe Tyr Lys
                   725                 730                 735
   ggc tcg acc tcg ttc ggc tgg ttc gtg ccc gag gtc ttt gcc gcc cag      2256
   Gly Ser Thr Ser Phe Gly Trp Phe Val Pro Glu Val Phe Ala Ala Gln
               740                 745                 750
   gcc ggc ctc gac aac ggc cgc aag tcg gag ccc tgg ttc att gag aac      2304
   Ala Gly Leu Asp Asn Gly Arg Lys Ser Glu Pro Trp Phe Ile Glu Asn
           755                 760                 765
   aag gtt ccg gcc tcg cag gtc tcc tcc ttt gac gtg cgc ccc aac ggc      2352
   Lys Val Pro Ala Ser Gln Val Ser Ser Phe Asp Val Arg Pro Asn Gly
       770                 775                 780
   agc ggc cgc acc gcc atc ttc gcc aac gcc ccc agc ggc gcc cag ctc      2400
   Ser Gly Arg Thr Ala Ile Phe Ala Asn Ala Pro Ser Gly Ala Gln Leu
   785                 790                 795                 800
   aac cgc cgc acg gac cag ggc cag tac ctc gac gcc gtc gac att gtc      2448
   Asn Arg Arg Thr Asp Gln Gly Gln Tyr Leu Asp Ala Val Asp Ile Val
                   805                 810                 815
   tcc ggc agc ggc aag aag agc ctc ggc tac gcc cac ggt tcc aag acg      2496
   Ser Gly Ser Gly Lys Lys Ser Leu Gly Tyr Ala His Gly Ser Lys Thr
               820                 825                 830
   gtc aac ccg aac gac tgg ttc ttc tcg tgc cac ttt tgg ttt gac tcg      2544
   Val Asn Pro Asn Asp Trp Phe Phe Ser Cys His Phe Trp Phe Asp Ser
           835                 840                 845
   gtc atg ccc gga agt ctc ggt gtc gag tcc atg ttc cag ctc gtc gag      2592
   Val Met Pro Gly Ser Leu Gly Val Glu Ser Met Phe Gln Leu Val Glu
       850                 855                 860
   gcc atc gcc gcc cac gag gat ctc gct ggc aaa gca cgg cat tgc caa      2640
   Ala Ile Ala Ala His Glu Asp Leu Ala Gly Lys Ala Arg His Cys Gln
   865                 870                 875                 880
   ccc cac ctt tgt gca cgc ccc cgg gca aga tca agc tgg aag tac cgc      2688
   Pro His Leu Cys Ala Arg Pro Arg Ala Arg Ser Ser Trp Lys Tyr Arg
                   885                 890                 895
   ggc cag ctc acg ccc aag agc aag aag atg gac tcg gag gtc cac atc      2736
   Gly Gln Leu Thr Pro Lys Ser Lys Lys Met Asp Ser Glu Val His Ile
               900                 905                 910
   gtg tcc gtg gac gcc cac gac ggc gtt gtc gac ctc gtc gcc gac ggc      2784
   Val Ser Val Asp Ala His Asp Gly Val Val Asp Leu Val Ala Asp Gly
           915                 920                 925
   ttc ctc tgg gcc gac agc ctc cgc gtc tac tcg gtg agc aac att cgc      2832
   Phe Leu Trp Ala Asp Ser Leu Arg Val Tyr Ser Val Ser Asn Ile Arg
       930                 935                 940
   gtg cgc atc gcc tcc ggt gag gcc cct gcc gcc gcc tcc tcc gcc gcc      2880
   Val Arg Ile Ala Ser Gly Glu Ala Pro Ala Ala Ala Ser Ser Ala Ala
   945                 950                 955                 960
   tct gtg ggc tcc tcg gct tcg tcc gtc gag cgc acg cgc tcg agc ccc      2928
   Ser Val Gly Ser Ser Ala Ser Ser Val Glu Arg Thr Arg Ser Ser Pro
                   965                 970                 975
   gct gtc gcc tcc ggc ccg gcc cag acc atc gac ctc aag cag ctc aag      2976
   Ala Val Ala Ser Gly Pro Ala Gln Thr Ile Asp Leu Lys Gln Leu Lys
               980                 985                 990
   acc gag ctc ctc gag ctc gat gcc  ccg ctc tac ctc tcg  cag gac ccg    3024
   Thr Glu Leu Leu Glu Leu Asp Ala  Pro Leu Tyr Leu Ser  Gln Asp Pro
           995                 1000                 1005
   acc agc  ggc cag ctc aag aag  cac acc gac gtg gcc  tcc ggc cag       3069
   Thr Ser  Gly Gln Leu Lys Lys  His Thr Asp Val Ala  Ser Gly Gln
       1010                 1015                 1020
   gcc acc  atc gtg cag ccc tgc  acg ctc ggc gac ctc  ggt gac cgc       3114
   Ala Thr  Ile Val Gln Pro Cys  Thr Leu Gly Asp Leu  Gly Asp Arg
       1025                 1030                 1035
   tcc ttc  atg gag acc tac ggc  gtc gtc gcc ccg ctg  tac acg ggc       3159
   Ser Phe  Met Glu Thr Tyr Gly  Val Val Ala Pro Leu  Tyr Thr Gly
       1040                 1045                 1050
   gcc atg  gcc aag ggc att gcc  tcg gcg gac ctc gtc  atc gcc gcc       3204
   Ala Met  Ala Lys Gly Ile Ala  Ser Ala Asp Leu Val  Ile Ala Ala
       1055                 1060                 1065
   ggc aag  cgc aag atc ctc ggc  tcc ttt ggc gcc ggc  ggc ctc ccc       3249
   Gly Lys  Arg Lys Ile Leu Gly  Ser Phe Gly Ala Gly  Gly Leu Pro
       1070                 1075                 1080
   atg cac  cac gtg cgc gcc gcc  ctc gag aag atc cag  gcc gcc ctg       3294
   Met His  His Val Arg Ala Ala  Leu Glu Lys Ile Gln  Ala Ala Leu
       1085                 1090                 1095
   cct cag  ggc ccc tac gcc gtc  aac ctc atc cac tcg  cct ttt gac       3339
   Pro Gln  Gly Pro Tyr Ala Val  Asn Leu Ile His Ser  Pro Phe Asp
       1100                 1105                 1110
   agc aac  ctc gag aag ggc aac  gtc gat ctc ttc ctc  gag aag ggc       3384
   Ser Asn  Leu Glu Lys Gly Asn  Val Asp Leu Phe Leu  Glu Lys Gly
       1115                 1120                 1125
   gtc act  gtg gtg gag gcc tcg  gca ttc atg acc ctc  acc ccg cag       3429
   Val Thr  Val Val Glu Ala Ser  Ala Phe Met Thr Leu  Thr Pro Gln
       1130                 1135                 1140
   gtc gtg  cgc tac cgc gcc gcc  ggc ctc tcg cgc aac  gcc gac ggt       3474
   Val Val  Arg Tyr Arg Ala Ala  Gly Leu Ser Arg Asn  Ala Asp Gly
       1145                 1150                 1155
   tcg gtc  aac atc cgc aac cgc  atc atc ggc aag gtc  tcg cgc acc      3519
   Ser Val  Asn Ile Arg Asn Arg  Ile Ile Gly Lys Val  Ser Arg Thr
       1160                 1165                 1170
   gag ctc  gcc gag atg ttc atc  cgc ccg gcc ccg gag  cac ctc ctc      3564
   Glu Leu  Ala Glu Met Phe Ile  Arg Pro Ala Pro Glu  His Leu Leu
       1175                 1180                 1185
   gag aag  ctc atc gcc tcg ggc  gag atc acc cag gag  cag gcc gag      3609
   Glu Lys  Leu Ile Ala Ser Gly  Glu Ile Thr Gln Glu  Gln Ala Glu
       1190                 1195                 1200
   ctc gcg  cgc cgc gtt ccc gtc  gcc gac gat atc gct  gtc gag gct      3654
   Leu Ala  Arg Arg Val Pro Val  Ala Asp Asp Ile Ala  Val Glu Ala
       1205                 1210                 1215
   gac tcg  ggc ggc cac acc gac  aac cgc ccc atc cac  gtc atc ctc      3699
   Asp Ser  Gly Gly His Thr Asp  Asn Arg Pro Ile His  Val Ile Leu
       1220                 1225                 1230
   ccg ctc  atc atc aac ctc cgc  aac cgc ctg cac cgc  gag tgc ggc      3744
   Pro Leu  Ile Ile Asn Leu Arg  Asn Arg Leu His Arg  Glu Cys Gly
       1235                 1240                 1245
   tac ccc  gcg cac ctc cgc gtc  cgc gtt ggc gcc ggc  ggt ggc gtc      3789
   Tyr Pro  Ala His Leu Arg Val  Arg Val Gly Ala Gly  Gly Gly Val
       1250                 1255                 1260
   ggc tgc  ccg cag gcc gcc gcc  gcc gcg ctc acc atg  ggc gcc gcc      3834
   Gly Cys  Pro Gln Ala Ala Ala  Ala Ala Leu Thr Met  Gly Ala Ala
       1265                 1270                 1275
   ttc atc  gtc acc ggc act gtc  aac cag gtc gcc aag  cag tcc ggc      3879
   Phe Ile  Val Thr Gly Thr Val  Asn Gln Val Ala Lys  Gln Ser Gly
       1280                 1285                 1290
   acc tgc  gac aac gtg cgc aag  cag ctc tcg cag gcc  acc tac tcg      3924
   Thr Cys  Asp Asn Val Arg Lys  Gln Leu Ser Gln Ala  Thr Tyr Ser
       1295                 1300                 1305
   gat atc  tgc atg gcc ccg gcc  gcc gac atg ttc gag  gag ggc gtc      3969
   Asp Ile  Cys Met Ala Pro Ala  Ala Asp Met Phe Glu  Glu Gly Val
       1310                 1315                 1320
   aag ctc  cag gtc ctc aag aag  gga acc atg ttc ccc  tcg cgc gcc      4014
   Lys Leu  Gln Val Leu Lys Lys  Gly Thr Met Phe Pro  Ser Arg Ala
       1325                 1330                 1335
   aac aag  ctc tac gag ctc ttt  tgc aag tac gac tcc  ttc gac tcc      4059
   Asn Lys  Leu Tyr Glu Leu Phe  Cys Lys Tyr Asp Ser  Phe Asp Ser
       1340                 1345                 1350
   atg cct  cct gcc gag ctc gag  cgc atc gag aag cgt  atc ttc aag      4104
   Met Pro  Pro Ala Glu Leu Glu  Arg Ile Glu Lys Arg  Ile Phe Lys
       1355                 1360                 1365
   cgc gca  ctc cag gag gtc tgg  gag gag acc aag gac  ttt tac att      4149
   Arg Ala  Leu Gln Glu Val Trp  Glu Glu Thr Lys Asp  Phe Tyr Ile
       1370                 1375                 1380
   aac ggt  ctc aag aac ccg gag  aag atc cag cgc gcc  gag cac gac      4194
   Asn Gly  Leu Lys Asn Pro Glu  Lys Ile Gln Arg Ala  Glu His Asp
       1385                 1390                 1395
   ccc aag  ctc aag atg tcg ctc  tgc ttc cgc tgg tac  ctt ggt ctt      4239
   Pro Lys  Leu Lys Met Ser Leu  Cys Phe Arg Trp Tyr  Leu Gly Leu
       1400                 1405                 1410
   gcc agc  cgc tgg gcc aac atg  ggc gcc ccg gac cgc  gtc atg gac      4284
   Ala Ser  Arg Trp Ala Asn Met  Gly Ala Pro Asp Arg  Val Met Asp
       1415                 1420                 1425
   tac cag  gtc tgg tgt ggc ccg  gcc att ggc gcc ttc  aac gac ttc      4329
   Tyr Gln  Val Trp Cys Gly Pro  Ala Ile Gly Ala Phe  Asn Asp Phe
       1430                 1435                 1440
   atc aag  ggc acc tac ctc gac  ccc gct gtc tcc aac  gag tac ccc      4374
   Ile Lys  Gly Thr Tyr Leu Asp  Pro Ala Val Ser Asn  Glu Tyr Pro
       1445                 1450                 1455
   tgt gtc  gtc cag atc aac ctg  caa atc ctc cgt ggt  gcc tgc tac      4419
   Cys Val  Val Gln Ile Asn Leu  Gln Ile Leu Arg Gly  Ala Cys Tyr
       1460                 1465                 1470
   ctg cgc  cgt ctc aac gcc ctg  cgc aac gac ccg cgc  att gac ctc      4464
   Leu Arg  Arg Leu Asn Ala Leu  Arg Asn Asp Pro Arg  Ile Asp Leu
       1475                 1480                 1485
   gag acc  gag gat gct gcc ttt  gtc tac gag ccc acc  aac gcg ctc      4509
   Glu Thr  Glu Asp Ala Ala Phe  Val Tyr Glu Pro Thr  Asn Ala Leu
       1490                 1495                 1500
   taa                                                                 4512
<210> 28
   <211> 1503
   <212> PRT
   <213> Schizochytrium sp.
<400> 28
   Met Ala Leu Arg Val Lys Thr Asn Lys Lys Pro Cys Trp Glu Met Thr
   1               5                   10                  15
   Lys Glu Glu Leu Thr Ser Gly Lys Thr Glu Val Phe Asn Tyr Glu Glu
               20                  25                  30
   Leu Leu Glu Phe Ala Glu Gly Asp Ile Ala Lys Val Phe Gly Pro Glu
           35                  40                  45
   Phe Ala Val Ile Asp Lys Tyr Pro Arg Arg Val Arg Leu Pro Ala Arg
       50                  55                  60
   Glu Tyr Leu Leu Val Thr Arg Val Thr Leu Met Asp Ala Glu Val Asn
   65                  70                  75                  80
   Asn Tyr Arg Val Gly Ala Arg Met Val Thr Glu Tyr Asp Leu Pro Val
                   85                  90                  95
   Asn Gly Glu Leu Ser Glu Gly Gly Asp Cys Pro Trp Ala Val Leu Val
               100                 105                 110
   Glu Ser Gly Gln Cys Asp Leu Met Leu Ile Ser Tyr Met Gly Ile Asp
           115                 120                 125
   Phe Gln Asn Gln Gly Asp Arg Val Tyr Arg Leu Leu Asn Thr Thr Leu
       130                 135                 140
   Thr Phe Tyr Gly Val Ala His Glu Gly Glu Thr Leu Glu Tyr Asp Ile
   145                 150                 155                 160
   Arg Val Thr Gly Phe Ala Lys Arg Leu Asp Gly Gly Ile Ser Met Phe
                   165                 170                 175
   Phe Phe Glu Tyr Asp Cys Tyr Val Asn Gly Arg Leu Leu Ile Glu Met
               180                 185                 190
   Arg Asp Gly Cys Ala Gly Phe Phe Thr Asn Glu Glu Leu Asp Ala Gly
           195                 200                 205
   Lys Gly Val Val Phe Thr Arg Gly Asp Leu Ala Ala Arg Ala Lys Ile
       210                 215                 220
   Pro Lys Gln Asp Val Ser Pro Tyr Ala Val Ala Pro Cys Leu His Lys
   225                 230                 235                 240
   Thr Lys Leu Asn Glu Lys Glu Met Gln Thr Leu Val Asp Lys Asp Trp
                   245                 250                 255
   Ala Ser Val Phe Gly Ser Lys Asn Gly Met Pro Glu Ile Asn Tyr Lys
               260                 265                 270
   Leu Cys Ala Arg Lys Met Leu Met Ile Asp Arg Val Thr Ser Ile Asp
           275                 280                 285
   His Lys Gly Gly Val Tyr Gly Leu Gly Gln Leu Val Gly Glu Lys Ile
       290                 295                 300
   Leu Glu Arg Asp His Trp Tyr Phe Pro Cys His Phe Val Lys Asp Gln
   305                 310                 315                 320
   Val Met Ala Gly Ser Leu Val Ser Asp Gly Cys Ser Gln Met Leu Lys
                   325                 330                 335
   Met Tyr Met Ile Trp Leu Gly Leu His Leu Thr Thr Gly Pro Phe Asp
               340                 345                 350
   Phe Arg Pro Val Asn Gly His Pro Asn Lys Val Arg Cys Arg Gly Gln
           355                 360                 365
   Ile Ser Pro His Lys Gly Lys Leu Val Tyr Val Met Glu Ile Lys Glu
       370                 375                 380
   Met Gly Phe Asp Glu Asp Asn Asp Pro Tyr Ala Ile Ala Asp Val Asn
   385                 390                 395                 400
   Ile Ile Asp Val Asp Phe Glu Lys Gly Gln Asp Phe Ser Leu Asp Arg
                   405                 410                 415
   Ile Ser Asp Tyr Gly Lys Gly Asp Leu Asn Lys Lys Ile Val Val Asp
               420                 425                 430
   Phe Lys Gly Ile Ala Leu Lys Met Gln Lys Arg Ser Thr Asn Lys Asn
           435                 440                 445
   Pro Ser Lys Val Gln Pro Val Phe Ala Asn Gly Ala Ala Thr Val Gly
       450                 455                 460
   Pro Glu Ala Ser Lys Ala Ser Ser Gly Ala Ser Ala Ser Ala Ser Ala
   465                 470                 475                 480
   Ala Pro Ala Lys Pro Ala Phe Ser Ala Asp Val Leu Ala Pro Lys Pro
                   485                 490                 495
   Val Ala Leu Pro Glu His Ile Leu Lys Gly Asp Ala Leu Ala Pro Lys
               500                 505                 510
   Glu Met Ser Trp His Pro Met Ala Arg Ile Pro Gly Asn Pro Thr Pro
           515                 520                 525
   Ser Phe Ala Pro Ser Ala Tyr Lys Pro Arg Asn Ile Ala Phe Thr Pro
       530                 535                 540
   Phe Pro Gly Asn Pro Asn Asp Asn Asp His Thr Pro Gly Lys Met Pro
   545                 550                 555                 560
   Leu Thr Trp Phe Asn Met Ala Glu Phe Met Ala Gly Lys Val Ser Met
                   565                 570                 575
   Cys Leu Gly Pro Glu Phe Ala Lys Phe Asp Asp Ser Asn Thr Ser Arg
               580                 585                 590
   Ser Pro Ala Trp Asp Leu Ala Leu Val Thr Arg Ala Val Ser Val Ser
           595                 600                 605
   Asp Leu Lys His Val Asn Tyr Arg Asn Ile Asp Leu Asp Pro Ser Lys
       610                 615                 620
   Gly Thr Met Val Gly Glu Phe Asp Cys Pro Ala Asp Ala Trp Phe Tyr
   625                 630                 635                 640
   Lys Gly Ala Cys Asn Asp Ala His Met Pro Tyr Ser Ile Leu Met Glu
                   645                 650                 655
   Ile Ala Leu Gln Thr Ser Gly Val Leu Thr Ser Val Leu Lys Ala Pro
               660                 665                 670
   Leu Thr Met Glu Lys Asp Asp Ile Leu Phe Arg Asn Leu Asp Ala Asn
           675                 680                 685
   Ala Glu Phe Val Arg Ala Asp Leu Asp Tyr Arg Gly Lys Thr Ile Arg
       690                 695                 700
   Asn Val Thr Lys Cys Thr Gly Tyr Ser Met Leu Gly Glu Met Gly Val
   705                 710                 715                 720
   His Arg Phe Thr Phe Glu Leu Tyr Val Asp Asp Val Leu Phe Tyr Lys
                   725                 730                 735
   Gly Ser Thr Ser Phe Gly Trp Phe Val Pro Glu Val Phe Ala Ala Gln
               740                 745                 750
   Ala Gly Leu Asp Asn Gly Arg Lys Ser Glu Pro Trp Phe Ile Glu Asn
           755                 760                 765
   Lys Val Pro Ala Ser Gln Val Ser Ser Phe Asp Val Arg Pro Asn Gly
       770                 775                 780
   Ser Gly Arg Thr Ala Ile Phe Ala Asn Ala Pro Ser Gly Ala Gln Leu
   785                 790                 795                 800
   Asn Arg Arg Thr Asp Gln Gly Gln Tyr Leu Asp Ala Val Asp Ile Val
                   805                 810                 815
   Ser Gly Ser Gly Lys Lys Ser Leu Gly Tyr Ala His Gly Ser Lys Thr
               820                 825                 830
   Val Asn Pro Asn Asp Trp Phe Phe Ser Cys His Phe Trp Phe Asp Ser
           835                 840                 845
   Val Met Pro Gly Ser Leu Gly Val Glu Ser Met Phe Gln Leu Val Glu
       850                 855                 860
   Ala Ile Ala Ala His Glu Asp Leu Ala Gly Lys Ala Arg His Cys Gln
   865                 870                 875                 880
   Pro His Leu Cys Ala Arg Pro Arg Ala Arg Ser Ser Trp Lys Tyr Arg
                   885                 890                 895
   Gly Gln Leu Thr Pro Lys Ser Lys Lys Met Asp Ser Glu Val His Ile
               900                 905                 910
   Val Ser Val Asp Ala His Asp Gly Val Val Asp Leu Val Ala Asp Gly
           915                 920                 925
   Phe Leu Trp Ala Asp Ser Leu Arg Val Tyr Ser Val Ser Asn Ile Arg
   930 935 940
   Val Arg Ile Ala Ser Gly Glu Ala Pro Ala Ala Ala Ser Ser Ala Ala
   945 950 955 960
   Ser Val Gly Ser Ser Ala Ser Ser Val Glu Arg Thr Arg Ser Ser Pro
   965 970 975
   Ala Val Ala Ser Gly Pro Ala Gln Thr Ile Asp Leu Lys Gln Leu Lys
   980 985 990
   Thr Glu Leu Leu Glu Leu Asp Ala Pro Leu Tyr Leu Ser Gln Asp Pro
   995 1000 1005
   Thr Ser Gly Gln Leu Lys Lys His Thr Asp Val Ala Ser Gly Gln
   1010 1015 1020
   Ala Thr Ile Val Gln Pro Cys Thr Leu Gly Asp Leu Gly Asp Arg
   1025 1030 1035
   Ser Phe Met Glu Thr Tyr Gly Val Val Ala Pro Leu Tyr Thr Gly
   1040 1045 1050
   Ala Met Ala Lys Gly Ile Ala Ser Ala Asp Leu Val Ile Ala Ala
   1055 1060 1065
   Gly Lys Arg Lys Ile Leu Gly Ser Phe Gly Ala Gly Gly Leu Pro
   1070 1075 1080
   Met His His Val Arg Ala Ala Leu Glu Lys Ile Gln Ala Ala Leu
   1085 1090 1095
   Pro Gln Gly Pro Tyr Ala Val Asn Leu Ile His Ser Pro Phe Asp
   1100 1105 1110
   Ser Asn Leu Glu Lys Gly Asn Val Asp Leu Phe Leu Glu Lys Gly
   1115 1120 1125
   Val Thr Val Val Glu Ala Ser Ala Phe Met Thr Leu Thr Pro Gln
   1130 1135 1140
   Val Val Arg Tyr Arg Ala Ala Gly Leu Ser Arg Asn Ala Asp Gly
   1145 1150 1155
   Ser Val Asn Ile Arg Asn Arg Ile Ile Gly Lys Val Ser Arg Thr
   1160 1165 1170
   Glu Leu Ala Glu Met Phe Ile Arg Pro Ala Pro Glu His Leu Leu
   1175 1180 1185
   Glu Lys Leu Ile Ala Ser Gly Glu Ile Thr Gln Glu Gln Ala Glu
   1190 1195 1200
   Leu Ala Arg Arg Val Pro Val Ala Asp Asp Ile Ala Val Glu Ala
   1205 1210 1215
   Asp Ser Gly Gly His Thr Asp Asn Arg Pro Ile His Val Ile Leu
   1220 1225 1230
   Pro Leu Ile Ile Asn Leu Arg Asn Arg Leu His Arg Glu Cys Gly
   1235 1240 1245
   Tyr Pro Ala His Leu Arg Val Arg Val Gly Ala Gly Gly Gly Val
   1250 1255 1260
   Gly Cys Pro Gln Ala Ala Ala Ala Ala Leu Thr Met Gly Ala Ala
   1265 1270 1275
   Phe Ile Val Thr Gly Thr Val Asn Gln Val Ala Lys Gln Ser Gly
   1280 1285 1290
   Thr Cys Asp Asn Val Arg Lys Gln Leu Ser Gln Ala Thr Tyr Ser
   1295 1300 1305
   Asp Ile Cys Met Ala Pro Ala Ala Asp Met Phe Glu Glu Gly Val
   1310 1315 1320
   Lys Leu Gln Val Leu Lys Lys Gly Thr Met Phe Pro Ser Arg Ala
   1325 1330 1335
   Asn Lys Leu Tyr Glu Leu Phe Cys Lys Tyr Asp Ser Phe Asp Ser
   1340 1345 1350
   Met Pro Pro Ala Glu Leu Glu Arg Ile Glu Lys Arg Ile Phe Lys
   1355 1360 1365
   Arg Ala Leu Gln Glu Val Trp Glu Glu Thr Lys Asp Phe Tyr Ile
   1370 1375 1380
   Asn Gly Leu Lys Asn Pro Glu Lys Ile Gln Arg Ala Glu His Asp
   1385 1390 1395
   Pro Lys Leu Lys Met Ser Leu Cys Phe Arg Trp Tyr Leu Gly Leu
   1400 1405 1410
   Ala Ser Arg Trp Ala Asn Met Gly Ala Pro Asp Arg Val Met Asp
   1415 1420 1425
   Tyr Gln Val Trp Cys Gly Pro Ala Ile Gly Ala Phe Asn Asp Phe
   1430 1435 1440
   Ile Lys Gly Thr Tyr Leu Asp Pro Ala Val Ser Asn Glu Tyr Pro
   1445 1450 1455
   Cys Val Val Gln Ile Asn Leu Gln Ile Leu Arg Gly Ala Cys Tyr
   1460 1465 1470
   Leu Arg Arg Leu Asn Ala Leu Arg Asn Asp Pro Arg Ile Asp Leu
   1475 1480 1485
   Glu Thr Glu Asp Ala Ala Phe Val Tyr Glu Pro Thr Asn Ala Leu
   1490 1495 1500
<210> 29
   <211> 1500
   <212> DNA
   <213> Schizochytrium sp.
<220>
   <221> CDS
   <222> (1)..(1500)
   <223>
<400> 29
   aag gtt cag ccc gtc ttt gcc aac ggc gcc gcc act gtc ggc ccc gag 48
   Lys Val Gln Pro Val Phe Ala Asn Gly Ala Ala Thr Val Gly Pro Glu
   1               5                   10                  15
   gcc tcc aag gct tcc tcc ggc gcc agc gcc agc gcc agc gcc gcc ccg      96
   Ala Ser Lys Ala Ser Ser Gly Ala Ser Ala Ser Ala Ser Ala Ala Pro
               20                  25                  30
   gcc aag cct gcc ttc agc gcc gat gtt ctt gcg ccc aag ccc gtt gcc     144
   Ala Lys Pro Ala Phe Ser Ala Asp Val Leu Ala Pro Lys Pro Val Ala
           35                  40                  45
   ctt ccc gag cac atc ctc aag ggc gac gcc ctc gcc ccc aag gag atg     192
   Leu Pro Glu His Ile Leu Lys Gly Asp Ala Leu Ala Pro Lys Glu Met
       50                  55                  60
   tcc tgg cac ccc atg gcc cgc atc ccg ggc aac ccg acg ccc tct ttt     240
   Ser Trp His Pro Met Ala Arg Ile Pro Gly Asn Pro Thr Pro Ser Phe
   65                  70                  75                  80
   gcg ccc tcg gcc tac aag ccg cgc aac atc gcc ttt acg ccc ttc ccc     288
   Ala Pro Ser Ala Tyr Lys Pro Arg Asn Ile Ala Phe Thr Pro Phe Pro
                   85                  90                  95
   ggc aac ccc aac gat aac gac cac acc ccg ggc aag atg ccg ctc acc     336
   Gly Asn Pro Asn Asp Asn Asp His Thr Pro Gly Lys Met Pro Leu Thr
               100                 105                 110
   tgg ttc aac atg gcc gag ttc atg gcc ggc aag gtc agc atg tgc ctc     384
   Trp Phe Asn Met Ala Glu Phe Met Ala Gly Lys Val Ser Met Cys Leu
           115                 120                 125
   ggc ccc gag ttc gcc aag ttc gac gac tcg aac acc agc cgc agc ccc     432
   Gly Pro Glu Phe Ala Lys Phe Asp Asp Ser Asn Thr Ser Arg Ser Pro
       130                 135                 140
   gct tgg gac ctc gct ctc gtc acc cgc gcc gtg tct gtg tct gac ctc     480
   Ala Trp Asp Leu Ala Leu Val Thr Arg Ala Val Ser Val Ser Asp Leu
   145                 150                 155                 160
   aag cac gtc aac tac cgc aac atc gac ctc gac ccc tcc aag ggt acc     528
   Lys His Val Asn Tyr Arg Asn Ile Asp Leu Asp Pro Ser Lys Gly Thr
                   165                 170                 175
   atg gtc ggc gag ttc gac tgc ccc gcg gac gcc tgg ttc tac aag ggc     576
   Met Val Gly Glu Phe Asp Cys Pro Ala Asp Ala Trp Phe Tyr Lys Gly
               180                 185                 190
   gcc tgc aac gat gcc cac atg ccg tac tcg atc ctc atg gag atc gcc     624
   Ala Cys Asn Asp Ala His Met Pro Tyr Ser Ile Leu Met Glu Ile Ala
           195                 200                 205
   ctc cag acc tcg ggt gtg ctc acc tcg gtg ctc aag gcg ccc ctg acc     672
   Leu Gln Thr Ser Gly Val Leu Thr Ser Val Leu Lys Ala Pro Leu Thr
       210                 215                 220
   atg gag aag gac gac atc ctc ttc cgc aac ctc gac gcc aac gcc gag     720
   Met Glu Lys Asp Asp Ile Leu Phe Arg Asn Leu Asp Ala Asn Ala Glu
   225                 230                 235                 240
   ttc gtg cgc gcc gac ctc gac tac cgc ggc aag act atc cgc aac gtc     768
   Phe Val Arg Ala Asp Leu Asp Tyr Arg Gly Lys Thr Ile Arg Asn Val
                   245                 250                 255
   acc aag tgc act ggc tac agc atg ctc ggc gag atg ggc gtc cac cgc     816
   Thr Lys Cys Thr Gly Tyr Ser Met Leu Gly Glu Met Gly Val His Arg
               260                 265                 270
   ttc acc ttt gag ctc tac gtc gat gat gtg ctc ttt tac aag ggc tcg     864
   Phe Thr Phe Glu Leu Tyr Val Asp Asp Val Leu Phe Tyr Lys Gly Ser
           275                 280                 285
   acc tcg ttc ggc tgg ttc gtg ccc gag gtc ttt gcc gcc cag gcc ggc     912
   Thr Ser Phe Gly Trp Phe Val Pro Glu Val Phe Ala Ala Gln Ala Gly
       290                 295                 300
   ctc gac aac ggc cgc aag tcg gag ccc tgg ttc att gag aac aag gtt     960
   Leu Asp Asn Gly Arg Lys Ser Glu Pro Trp Phe Ile Glu Asn Lys Val
   305                 310                 315                 320
   ccg gcc tcg cag gtc tcc tcc ttt gac gtg cgc ccc aac ggc agc ggc    1008
   Pro Ala Ser Gln Val Ser Ser Phe Asp Val Arg Pro Asn Gly Ser Gly
                   325                 330                 335
   cgc acc gcc atc ttc gcc aac gcc ccc agc ggc gcc cag ctc aac cgc    1056
   Arg Thr Ala Ile Phe Ala Asn Ala Pro Ser Gly Ala Gln Leu Asn Arg
               340                 345                 350
   cgc acg gac cag ggc cag tac ctc gac gcc gtc gac att gtc tcc ggc      1104
   Arg Thr Asp Gln Gly Gln Tyr Leu Asp Ala Val Asp Ile Val Ser Gly
           355                 360                 365
   agc ggc aag aag agc ctc ggc tac gcc cac ggt tcc aag acg gtc aac      1152
   Ser Gly Lys Lys Ser Leu Gly Tyr Ala His Gly Ser Lys Thr Val Asn
       370                 375                 380
   ccg aac gac tgg ttc ttc tcg tgc cac ttt tgg ttt gac tcg gtc atg      1200
   Pro Asn Asp Trp Phe Phe Ser Cys His Phe Trp Phe Asp Ser Val Met
   385                 390                 395                 400
   ccc gga agt ctc ggt gtc gag tcc atg ttc cag ctc gtc gag gcc atc      1248
   Pro Gly Ser Leu Gly Val Glu Ser Met Phe Gln Leu Val Glu Ala Ile
                   405                 410                 415
   gcc gcc cac gag gat ctc gct ggc aaa gca cgg cat tgc caa ccc cac      1296
   Ala Ala His Glu Asp Leu Ala Gly Lys Ala Arg His Cys Gln Pro His
               420                 425                 430
   ctt tgt gca cgc ccc cgg gca aga tca agc tgg aag tac cgc ggc cag      1344
   Leu Cys Ala Arg Pro Arg Ala Arg Ser Ser Trp Lys Tyr Arg Gly Gln
           435                 440                 445
   ctc acg ccc aag agc aag aag atg gac tcg gag gtc cac atc gtg tcc      1392
   Leu Thr Pro Lys Ser Lys Lys Met Asp Ser Glu Val His Ile Val Ser
       450                 455                 460
   gtg gac gcc cac gac ggc gtt gtc gac ctc gtc gcc gac ggc ttc ctc      1440
   Val Asp Ala His Asp Gly Val Val Asp Leu Val Ala Asp Gly Phe Leu
   465                 470                 475                 480
   tgg gcc gac agc ctc cgc gtc tac tcg gtg agc aac att cgc gtg cgc      1488
   Trp Ala Asp Ser Leu Arg Val Tyr Ser Val Ser Asn Ile Arg Val Arg
                   485                 490                 495
   atc gcc tcc ggt                                                      1500
   Ile Ala Ser Gly
               500
<210> 30
   <211> 500
   <212> PRT
   <213> Schizochytrium sp.
<400> 30
   Lys Val Gln Pro Val Phe Ala Asn Gly Ala Ala Thr Val Gly Pro Glu
   1               5                   10                  15
   Ala Ser Lys Ala Ser Ser Gly Ala Ser Ala Ser Ala Ser Ala Ala Pro
               20                  25                  30
   Ala Lys Pro Ala Phe Ser Ala Asp Val Leu Ala Pro Lys Pro Val Ala
           35                  40                  45
   Leu Pro Glu His Ile Leu Lys Gly Asp Ala Leu Ala Pro Lys Glu Met
       50                  55                  60
   Ser Trp His Pro Met Ala Arg Ile Pro Gly Asn Pro Thr Pro Ser Phe
   65                  70                  75                  80
   Ala Pro Ser Ala Tyr Lys Pro Arg Asn Ile Ala Phe Thr Pro Phe Pro
                   85                  90                  95
   Gly Asn Pro Asn Asp Asn Asp His Thr Pro Gly Lys Met Pro Leu Thr
               100                 105                 110
   Trp Phe Asn Met Ala Glu Phe Met Ala Gly Lys Val Ser Met Cys Leu
           115                 120                 125
   Gly Pro Glu Phe Ala Lys Phe Asp Asp Ser Asn Thr Ser Arg Ser Pro
       130                 135                 140
   Ala Trp Asp Leu Ala Leu Val Thr Arg Ala Val Ser Val Ser Asp Leu
   145                 150                 155                 160
   Lys His Val Asn Tyr Arg Asn Ile Asp Leu Asp Pro Ser Lys Gly Thr
                   165                 170                 175
   Met Val Gly Glu Phe Asp Cys Pro Ala Asp Ala Trp Phe Tyr Lys Gly
               180                 185                 190
   Ala Cys Asn Asp Ala His Met Pro Tyr Ser Ile Leu Met Glu Ile Ala
           195                 200                 205
   Leu Gln Thr Ser Gly Val Leu Thr Ser Val Leu Lys Ala Pro Leu Thr
       210                 215                 220
   Met Glu Lys Asp Asp Ile Leu Phe Arg Asn Leu Asp Ala Asn Ala Glu
   225                 230                 235                 240
   Phe Val Arg Ala Asp Leu Asp Tyr Arg Gly Lys Thr Ile Arg Asn Val
                   245                 250                 255
   Thr Lys Cys Thr Gly Tyr Ser Met Leu Gly Glu Met Gly Val His Arg
               260                 265                 270
   Phe Thr Phe Glu Leu Tyr Val Asp Asp Val Leu Phe Tyr Lys Gly Ser
           275                 280                 285
   Thr Ser Phe Gly Trp Phe Val Pro Glu Val Phe Ala Ala Gln Ala Gly
       290                 295                 300
   Leu Asp Asn Gly Arg Lys Ser Glu Pro Trp Phe Ile Glu Asn Lys Val
   305                 310                 315                 320
   Pro Ala Ser Gln Val Ser Ser Phe Asp Val Arg Pro Asn Gly Ser Gly
                   325                 330                 335
   Arg Thr Ala Ile Phe Ala Asn Ala Pro Ser Gly Ala Gln Leu Asn Arg
               340                 345                 350
   Arg Thr Asp Gln Gly Gln Tyr Leu Asp Ala Val Asp Ile Val Ser Gly
           355                 360                 365
   Ser Gly Lys Lys Ser Leu Gly Tyr Ala His Gly Ser Lys Thr Val Asn
       370                 375                 380
   Pro Asn Asp Trp Phe Phe Ser Cys His Phe Trp Phe Asp Ser Val Met
   385                 390                 395                 400
   Pro Gly Ser Leu Gly Val Glu Ser Met Phe Gln Leu Val Glu Ala Ile
                   405                 410                 415
   Ala Ala His Glu Asp Leu Ala Gly Lys Ala Arg His Cys Gln Pro His
               420                 425                 430
   Leu Cys Ala Arg Pro Arg Ala Arg Ser Ser Trp Lys Tyr Arg Gly Gln
           435                 440                 445
   Leu Thr Pro Lys Ser Lys Lys Met Asp Ser Glu Val His Ile Val Ser
       450                 455                 460
   Val Asp Ala His Asp Gly Val Val Asp Leu Val Ala Asp Gly Phe Leu
   465 470 475 480
   Trp Ala Asp Ser Leu Arg Val Tyr Ser Val Ser Asn Ile Arg Val Arg
   485 490 495
   Ile Ala Ser Gly
   500
<210> 31
   <211> 1512
   <212> DNA
   <213> Schizochytrium sp.
<220>
   <221> CDS
   <222> (1)..(1512)
   <223>
<400> 31
   gcc ccg ctc tac ctc tcg cag gac ccg acc agc ggc cag ctc aag aag        48
   Ala Pro Leu Tyr Leu Ser Gln Asp Pro Thr Ser Gly Gln Leu Lys Lys
   1               5                   10                  15
   cac acc gac gtg gcc tcc ggc cag gcc acc atc gtg cag ccc tgc acg        96
   His Thr Asp Val Ala Ser Gly Gln Ala Thr Ile Val Gln Pro Cys Thr
               20                  25                  30
   ctc ggc gac ctc ggt gac cgc tcc ttc atg gag acc tac ggc gtc gtc       144
   Leu Gly Asp Leu Gly Asp Arg Ser Phe Met Glu Thr Tyr Gly Val Val
           35                  40                  45
   gcc ccg ctg tac acg ggc gcc atg gcc aag ggc att gcc tcg gcg gac       192
   Ala Pro Leu Tyr Thr Gly Ala Met Ala Lys Gly Ile Ala Ser Ala Asp
       50                  55                  60
   ctc gtc atc gcc gcc ggc aag cgc aag atc ctc ggc tcc ttt ggc gcc       240
   Leu Val Ile Ala Ala Gly Lys Arg Lys Ile Leu Gly Ser Phe Gly Ala
   65                  70                  75                  80
   ggc ggc ctc ccc atg cac cac gtg cgc gcc gcc ctc gag aag atc cag       288
   Gly Gly Leu Pro Met His His Val Arg Ala Ala Leu Glu Lys Ile Gln
                   85                  90                  95
   gcc gcc ctg cct cag ggc ccc tac gcc gtc aac ctc atc cac tcg cct       336
   Ala Ala Leu Pro Gln Gly Pro Tyr Ala Val Asn Leu Ile His Ser Pro
               100                 105                 110
   ttt gac agc aac ctc gag aag ggc aac gtc gat ctc ttc ctc gag aag       384
   Phe Asp Ser Asn Leu Glu Lys Gly Asn Val Asp Leu Phe Leu Glu Lys
           115                 120                 125
   ggc gtc act gtg gtg gag gcc tcg gca ttc atg acc ctc acc ccg cag       432
   Gly Val Thr Val Val Glu Ala Ser Ala Phe Met Thr Leu Thr Pro Gln
       130                 135                 140
   gtc gtg cgc tac cgc gcc gcc ggc ctc tcg cgc aac gcc gac ggt tcg       480
   Val Val Arg Tyr Arg Ala Ala Gly Leu Ser Arg Asn Ala Asp Gly Ser
   145                 150                 155                 160
   gtc aac atc cgc aac cgc atc atc ggc aag gtc tcg cgc acc gag ctc       528
   Val Asn Ile Arg Asn Arg Ile Ile Gly Lys Val Ser Arg Thr Glu Leu
                   165                 170                 175
   gcc gag atg ttc atc cgc ccg gcc ccg gag cac ctc ctc gag aag ctc       576
   Ala Glu Met Phe Ile Arg Pro Ala Pro Glu His Leu Leu Glu Lys Leu
               180                 185                 190
   atc gcc tcg ggc gag atc acc cag gag cag gcc gag ctc gcg cgc cgc       624
   Ile Ala Ser Gly Glu Ile Thr Gln Glu Gln Ala Glu Leu Ala Arg Arg
           195                 200                 205
   gtt ccc gtc gcc gac gat atc gct gtc gag gct gac tcg ggc ggc cac       672
   Val Pro Val Ala Asp Asp Ile Ala Val Glu Ala Asp Ser Gly Gly His
       210                 215                 220
   acc gac aac cgc ccc atc cac gtc atc ctc ccg ctc atc atc aac ctc       720
   Thr Asp Asn Arg Pro Ile His Val Ile Leu Pro Leu Ile Ile Asn Leu
   225                 230                 235                 240
   cgc aac cgc ctg cac cgc gag tgc ggc tac ccc gcg cac ctc cgc gtc      768
   Arg Asn Arg Leu His Arg Glu Cys Gly Tyr Pro Ala His Leu Arg Val
                   245                 250                 255
   cgc gtt ggc gcc ggc ggt ggc gtc ggc tgc ccg cag gcc gcc gcc gcc      816
   Arg Val Gly Ala Gly Gly Gly Val Gly Cys Pro Gln Ala Ala Ala Ala
               260                 265                 270
   gcg ctc acc atg ggc gcc gcc ttc atc gtc acc ggc act gtc aac cag      864
   Ala Leu Thr Met Gly Ala Ala Phe Ile Val Thr Gly Thr Val Asn Gln
           275                 280                 285
   gtc gcc aag cag tcc ggc acc tgc gac aac gtg cgc aag cag ctc tcg      912
   Val Ala Lys Gln Ser Gly Thr Cys Asp Asn Val Arg Lys Gln Leu Ser
       290                 295                 300
   cag gcc acc tac tcg gat atc tgc atg gcc ccg gcc gcc gac atg ttc      960
   Gln Ala Thr Tyr Ser Asp Ile Cys Met Ala Pro Ala Ala Asp Met Phe
   305                 310                 315                 320
   gag gag ggc gtc aag ctc cag gtc ctc aag aag gga acc atg ttc ccc     1008
   Glu Glu Gly Val Lys Leu Gln Val Leu Lys Lys Gly Thr Met Phe Pro
                   325                 330                 335
   tcg cgc gcc aac aag ctc tac gag ctc ttt tgc aag tac gac tcc ttc     1056
   Ser Arg Ala Asn Lys Leu Tyr Glu Leu Phe Cys Lys Tyr Asp Ser Phe
               340                 345                 350
   gac tcc atg cct cct gcc gag ctc gag cgc atc gag aag cgt atc ttc     1104
   Asp Ser Met Pro Pro Ala Glu Leu Glu Arg Ile Glu Lys Arg Ile Phe
           355                 360                 365
   aag cgc gca ctc cag gag gtc tgg gag gag acc aag gac ttt tac att     1152
   Lys Arg Ala Leu Gln Glu Val Trp Glu Glu Thr Lys Asp Phe Tyr Ile
       370                 375                 380
   aac ggt ctc aag aac ccg gag aag atc cag cgc gcc gag cac gac ccc     1200
   Asn Gly Leu Lys Asn Pro Glu Lys Ile Gln Arg Ala Glu His Asp Pro
   385                 390                 395                 400
   aag ctc aag atg tcg ctc tgc ttc cgc tgg tac ctt ggt ctt gcc agc     1248
   Lys Leu Lys Met Ser Leu Cys Phe Arg Trp Tyr Leu Gly Leu Ala Ser
                   405                 410                 415
   cgc tgg gcc aac atg ggc gcc ccg gac cgc gtc atg gac tac cag gtc     1296
   Arg Trp Ala Asn Met Gly Ala Pro Asp Arg Val Met Asp Tyr Gln Val
               420                 425                 430
   tgg tgt ggc ccg gcc att ggc gcc ttc aac gac ttc atc aag ggc acc     1344
   Trp Cys Gly Pro Ala Ile Gly Ala Phe Asn Asp Phe Ile Lys Gly Thr
           435                 440                 445
   tac ctc gac ccc gct gtc tcc aac gag tac ccc tgt gtc gtc cag atc     1392
   Tyr Leu Asp Pro Ala Val Ser Asn Glu Tyr Pro Cys Val Val Gln Ile
       450                 455                 460
   aac ctg caa atc ctc cgt ggt gcc tgc tac ctg cgc cgt ctc aac gcc     1440
   Asn Leu Gln Ile Leu Arg Gly Ala Cys Tyr Leu Arg Arg Leu Asn Ala
   465                 470                 475                 480
   ctg cgc aac gac ccg cgc att gac ctc gag acc gag gat gct gcc ttt     1488
   Leu Arg Asn Asp Pro Arg Ile Asp Leu Glu Thr Glu Asp Ala Ala Phe
                   485                 490                 495
   gtc tac gag ccc acc aac gcg ctc                                     1512
   Val Tyr Glu Pro Thr Asn Ala Leu
               500
<210> 32
   <211> 504
   <212> PRT
   <213> Schizochytrium sp.
<400> 32
   Ala Pro Leu Tyr Leu Ser Gln Asp Pro Thr Ser Gly Gln Leu Lys Lys
   1               5                   10                  15
   His Thr Asp Val Ala Ser Gly Gln Ala Thr Ile Val Gln Pro Cys Thr
   20 25 30
   Leu Gly Asp Leu Gly Asp Arg Ser Phe Met Glu Thr Tyr Gly Val Val
   35 40 45
   Ala Pro Leu Tyr Thr Gly Ala Met Ala Lys Gly Ile Ala Ser Ala Asp
   50 55 60
   Leu Val Ile Ala Ala Gly Lys Arg Lys Ile Leu Gly Ser Phe Gly Ala
   65 70 75 80
   Gly Gly Leu Pro Met His His Val Arg Ala Ala Leu Glu Lys Ile Gln
   85 90 95
   Ala Ala Leu Pro Gln Gly Pro Tyr Ala Val Asn Leu Ile His Ser Pro
   100 105 110
   Phe Asp Ser Asn Leu Glu Lys Gly Asn Val Asp Leu Phe Leu Glu Lys
   115 120 125
   Gly Val Thr Val Val Glu Ala Ser Ala Phe Met Thr Leu Thr Pro Gln
   130 135 140
   Val Val Arg Tyr Arg Ala Ala Gly Leu Ser Arg Asn Ala Asp Gly Ser
   145 150 155 160
   Val Asn Ile Arg Asn Arg Ile Ile Gly Lys Val Ser Arg Thr Glu Leu
   165 170 175
   Ala Glu Met Phe Ile Arg Pro Ala Pro Glu His Leu Leu Glu Lys Leu
   180 185 190
   Ile Ala Ser Gly Glu Ile Thr Gln Glu Gln Ala Glu Leu Ala Arg Arg
   195 200 205
   Val Pro Val Ala Asp Asp Ile Ala Val Glu Ala Asp Ser Gly Gly His
   210 215 220
   Thr Asp Asn Arg Pro Ile His Val Ile Leu Pro Leu Ile Ile Asn Leu
   225 230 235 240
   Arg Asn Arg Leu His Arg Glu Cys Gly Tyr Pro Ala His Leu Arg Val
   245 250 255
   Arg Val Gly Ala Gly Gly Gly Val Gly Cys Pro Gln Ala Ala Ala Ala
   260 265 270
   Ala Leu Thr Met Gly Ala Ala Phe Ile Val Thr Gly Thr Val Asn Gln
   275 280 285
   Val Ala Lys Gln Ser Gly Thr Cys Asp Asn Val Arg Lys Gln Leu Ser
   290 295 300
   Gln Ala Thr Tyr Ser Asp Ile Cys Met Ala Pro Ala Ala Asp Met Phe
   305 310 315 320
   Glu Glu Gly Val Lys Leu Gln Val Leu Lys Lys Gly Thr Met Phe Pro
   325 330 335
   Ser Arg Ala Asn Lys Leu Tyr Glu Leu Phe Cys Lys Tyr Asp Ser Phe
   340 345 350
   Asp Ser Met Pro Pro Ala Glu Leu Glu Arg Ile Glu Lys Arg Ile Phe
           355                 360                 365
   Lys Arg Ala Leu Gln Glu Val Trp Glu Glu Thr Lys Asp Phe Tyr Ile
       370                 375                 380
   Asn Gly Leu Lys Asn Pro Glu Lys Ile Gln Arg Ala Glu His Asp Pro
   385                 390                 395                 400
   Lys Leu Lys Met Ser Leu Cys Phe Arg Trp Tyr Leu Gly Leu Ala Ser
                   405                 410                 415
   Arg Trp Ala Asn Met Gly Ala Pro Asp Arg Val Met Asp Tyr Gln Val
               420                 425                 430
   Trp Cys Gly Pro Ala Ile Gly Ala Phe Asn Asp Phe Ile Lys Gly Thr
           435                 440                 445
   Tyr Leu Asp Pro Ala Val Ser Asn Glu Tyr Pro Cys Val Val Gln Ile
       450                 455                 460
   Asn Leu Gln Ile Leu Arg Gly Ala Cys Tyr Leu Arg Arg Leu Asn Ala
   465                 470                 475                 480
   Leu Arg Asn Asp Pro Arg Ile Asp Leu Glu Thr Glu Asp Ala Ala Phe
                   485                 490                 495
   Val Tyr Glu Pro Thr Asn Ala Leu
               500
<210> 33
   <211> 9
   <212> PRT
   <213> Artificial sequence
<220>
   <223> motif
<220>
   <221> MISC_FEATURE
   <222> (2)..(3)
   <223> x = any amino acid
<220>
   <221> MISC_FEATURE
   <222> (6)..(6)
   <223> x = A or S
<220>
   <221> MISC_FEATURE
   <222> (7)..(8)
   <223> x = any amino acid
<400> 33
   Trp Xaa Xaa Lys Glu Xaa Xaa Xaa Lys
   1               5
<210> 34
   <211> 6
   <212> PRT
   <213> Artificial sequence
<220>
   <223> motif
<220>
   <221> MISC_FEATURE
   <222> (3)..(3)
   <223> x = I or L or V
<400> 34
   Phe Asn Xaa Ser His Ser
   1               5
<210> 35
   <211> 5
   <212> PRT
   <213> Artificial sequence
<220>
   <223> motif
<220>
   <221> MISC_FEATURE
   <222> (1)..(5)
   <223> x = I or L or V
<400> 35
   Xaa Gly Xaa Asp Xaa
   1               5
<210> 36
   <211> 4244
   <212> DNA
   <213> Schizochytrium sp.
<400> 36
   tttctctctc tcgagctgtt gctgctgctg ctgctgctgc tgcttccttg ctggttctca      60
   cgtccgttcg atcaagcgct cgctcgctcg accgatcggt gcgtgcgtgc gtgcgtgagt     120
   cttgttgcca ggcagccgca ggctgtctgt ctgtttgtgt agttttaccc tcggggttcg     180
   gggtctgcct gcctcccgct cccgcccgcc gccgcccgta tccaccccgc tcgcctccgc     240
   ccatcgggcc tcgcctcctc gcgccgcacg catcgcgcgc atcgcatgca tcatgctgcc     300
   acgcacgggg ggacgcgcgc cccgcgtccc ccgccgccgc cgtcgtcgtc tggcgatgcc     360
   gtcgccgccc tccttccttc cctcgcctcc tcttcctccc gagcccccct gtcttccttc     420
   gcccccgcag cggcgcgcag gaagcgagga gagcggggag gagagaagaa aagaaaagaa     480
   aagaaaagaa aataacagcg ccgtctcgcg cagacgcgcg cggccgcgtg cgaggcggcg     540
   tgatggggct tctcgtggcg cggctgcggc ctggcccggc ctcgcctttg aggtgcaggc     600
   tttgggagag aagagtggga cgcggagaag ataagatggt gccatggcgc aggacggaga     660
   ggttgctgaa acttcttcga gcggcacagg cgatggcgag agaccgacag ctgccggcgc     720
   ggaggggatg gatacctccc gaggctggca tggacgagct ggccgcgcgg atctggctgg     780
   ccgcgcggcg gtgggtccgg aggcgcgagg ttggttttct tcatacctga taccatacgg     840
   tattcattct tcctctccag gaaggaagca agtcacatag agtatcacta gcctaatgat     900
   ggactctatg ttttagggca cgtcggagca gaaggcgcga gcgattcgaa tgcgagcgat     960
   agatacagca cagagacctt gccggcgacg cggatgcagg cgagcacgca cgcaccgcac    1020
   gcacggcagc ggtgcacgcg ctcctcggca gatgcacggt tctgcgccgc gcctttacat    1080
   tttttgattt taggtggtgt gcctgccact ttgaacatca tccacaagtc aacgcagcat    1140
   caagaggcaa gcaagtacat acatccattc gaattcaagt tcaagagacg cagcaacagc    1200
   cgccgctccg ctcaagctgc agctagctgg ctgacagggc tcgctggctg tagtggaaaa    1260
   ttccattcac ttttctgcat ccgcggccag caggcccgta cgcacgttct ctcgtttgtt    1320
   tgttcgttcg tgcgtgcgtg cgtgcgtccc agctgcctgt ctaatctgcc gcgcgatcca    1380
   acgaccctcg gtcgtcgccg caagcgaaac ccgacgccga cctggccaat gccgcaagaa    1440
   tgctaagcgc gcagcaatgc tgagagtaat cttcagccca ccaagtcatt atcgctgccc    1500
   aagtctccat cgcagccaca ttcaggcttt ctctctctct ccctccctct ctttctgccg    1560
   ggagagaagg aaagacccgc cgccgccgcc tctgcgcctg tgacgggctg tccgttgtaa    1620
   gccctcttag acagttccta ggtgccgggc gccgccgcgc ctccgtcgca ggcacacgta    1680
   ggcggccacg ggttcccccc gcaccttcca caccttcttc ccccgcagcc ggaccgcgcg    1740
   ccgtctgctt acgcacttcg cgcggccgcc gcccgcgaac ccgagcgcgt gctgtgggcg    1800
   ccgtcttccg gccgcgtcgg aggtcgtccc cgcgccgcgc tactccgggt cctgtgcggt    1860
   acgtacttaa tattaacagt gggacctcgc acaggacctg acggcagcac agacgtcgcc    1920
   gcctcgcatc gctggggacg caggcgaggc atcccggcgc ggccccgcac cggggaggct   1980
   gcggggcggc ctcttccggc cggcggccgc atcaggcgga tgacgcaaga gccctcgcag   2040
   tcgctcgctc gcgggagcgc agcgcggcgc cagcgtggcc aagctcccgc cccttctggc   2100
   tggctgcatg cctgcctgcc tgcctgcctg cgtgcgtgcg tgcgtgcgtg ccttcgtgcg   2160
   tgcctgcctt cgtgcgtgcg tgcgtgagtg cggcggaaga gggatcatgc gaggatcaat   2220
   cacccgccgc acctcgactt ttgaagaagc cgcgatgcga tgcgatgcga tgcgatgcga   2280
   cgcgataccg tgcgaggcta cgaagcgagt ctggccggcc gtcatacaac gcacgttttc   2340
   gagaaggagg gctggcggag gcgtgcatgc cggcgaccat tgcgaacgcg gcgtctcgtg   2400
   gctggcgaag gtgcctggag gatctaacga tcgctgctat gatgctatag ctgtgctgat   2460
   ccccggtcca ttccaccacg tctgtgcctg ccgcctgacc tgcgcttggc tttccttcaa   2520
   gttctcctcc gccgggcctt caggaccgag acgagacctg cagctgcagc tagactcgcg   2580
   ctcgctcgcg gaggattcgc cggccgccgg gccggacggg actcgcgagg tcacacggcc   2640
   gccggcgatc gcgatggctg tgctgacgta ctcgtgcgtg gcagccgtac gtcagcgacg   2700
   ccgcctccgt attgtggatt cgttagttgg ttgttggttg atttgttgat taattttttt   2760
   gttcgtaggc ttggttatag ctaatagttt agtttatact ggtgctcttc ggtgctgatt   2820
   tagctcgact tgggtccaca ccactgcccc tctactgtga atggatcaat ggacgcacga   2880
   cgggccgacg aaagtgcgcg agtgaggtaa cctaagcaac ggcggtcttc agaggggacg   2940
   cacgccctcc gtcgcagtca gtccagacag gcagaaaagc gtcttaggga ccacgcacgc   3000
   acgcacgcac gcacgcacgc ccgcacgcac gctccctccc tcgcgtgcct atttttttag  3060
   gcttccttcc gcacgggcct acctctcgct ccctcgcctc gccgcaccag gcggcagcag   3120
   cgatacctgc cggtgccgcc tccgtcacgc gctcagccgc agctcagccc agccgcgagc   3180
   tagggtttgt tcgtcctgaa ttgtttgatt tgatttgatt tgatttgatc cgatccgatc   3240
   cgatctgatc tgatttgctt tgctttgctt tgtctccctc ccggcgcgga ccaagcgtcc   3300
   gtctgcgcgc cgcagcttcc cttcttctcc cagccctcct tctgctcccg cctctcgcgc   3360
   aagcacgcag cttcgccgcc gcatccggtc ggtcggtcgg tcgatcgacc cgcctgccgc   3420
   tgctgctgtg gccgggcttt tctccatcgg cgactctttc ttctccatac gtcctactac   3480
   gtacatacat actgccggct tcctcctctt ccagcgcggc gacggcggca ggctgcgacg   3540
   tcgtcgccgc cgcgggcgcc gcgcgcgccg ccgccgccgc ccgcgtcgca gggcctcgtc   3600
   gccgccgccg ctccgctccg ctccgaggcc gcgagagggc cgcggcggcg cgatggatgg   3660
   atggatggat ggatggatgg atggattttg ttgatcgatg gcggcgcatg ggcggagatg   3720
   agcgaggacg agcgcgcgag cgcggcagcc ggattcgcag ggcctcgctc gcctcgcgcc   3780
   cgctgccgcg cccgccttgc gagcctgcgc cgcgagcgag cgagcgagcg agcggggctt   3840
   tctttgtctc gcgcgccgct tggcctcgtg tgtcttgtgc ttgcgtagcg ggcgccgcgg   3900
   tggaagatgg ctcattcaat cgacccattc acgcacgcac tccggcgcgc agagaaggcc   3960
   gaggaggagc agcaagcaaa ccaaaagctc tcgcgctcgc ggtctcgggc tcgagcggtc   4020
   tcggagagag agtcttgcgg cgaccaccgg cagcagcagc agcagcagca gcgctgtcga   4080
   gcacgagcac gagcacgagc acgagcacga gcattcgagc aagaggacag acacggttgt   4140
   cagcgcctag ctcgctcgat acagaaagag gcgggttggg cgtaaaaaaa aaggagcacg   4200
   caagccgcca gccagccagc tagctagcca gcctgcctgc caaa                     4244
<210> 37
   <211> 3886
   <212> DNA
   <213> Schizochytrium sp.
<220>
   <221> misc_feature
   <222> (2115)..(2115)
   <223> n = a, c, g, or t
<400> 37
   gatcttgatt gccaagctct ggattgtcga ttccgatgaa tcgagctctt tgttgtcgag     60
   ctctggcttg ccgagctttc agaaatagac aaaattgccg agttcctgat tgcggggctc    120
   tcgattgcca aggtctggtg gattctcgaa ctctcgattg tcaaaatctt ggtcgtctcg    180
   tcggattctt tcctgatttg ttttgtcaag accttgagat tgtgcaaaac cttgatcgtt    240
   gacaaaccct tgatcgacag cagcctttca tcacgctcag ctcttgtcat tgattatatt    300
   ccccctgaca gccaacacct tgatgcaggg tctcaacctt gatttttgga ggccatcatc    360
   agcatcacgc cccggcactc accctcaaca ttcgacagcc aacgcttttt tttcttcgac    420
   taggatctga gaataaaagc aggtcaccac gaccgtaggc caacgcgaca accatggaaa    480
   taaagtgaca acgaacgact tgcaagttta aatgtaaaga gcagcaattg cccgcccaca    540
   gacaaatgaa agcaggcgcc gagtcttatt tgaggaggtg ggcctgtggc aatgggcgaa    600
   agaaaatcaa ggacaaggag agcaggttac gtaccggtat actggtatac gtacatggat    660
   ggttcttggc aagttgacgg gatgtgtgcg agtgaccgtg gtagttaacg aaagagccgc    720
   aagggcaagg aaagcaagag aatgcagact tttccacagg atggatgggt ccgcagcttg    780
   ccgcatgatg aaacgctgta tttcacctgg cacgtggtgg cgcacgcgcc cacatatgat    840
   cgcggcggcg ggtgtattat acattttccc cctcaggtct actgccatcc ctccatgcgt    900
   cgctcgtgcg aacgacgcaa gcctttcgca tcgtgcagcc tctttctggt aaggcaagag    960
   ctaaacccaa acctaaacga aagaacattt ttacctctct ctctctccca ttggtcgcgt   1020
   gcgctccgcc gctcgctcct cctcctgcca gtgtcgcgcc ctaacttccc ccctccctcc   1080
   ctccctccct ccctccctct ctcctgccac cgcccctctc tccgcgctgc gtgcggtgct   1140
   gccctggacc aatggcatgc tgctgcacgc tcggcggatg acgcaagccg cttcgcaatt   1200
   tccggatcag atctcggcgg ggcgtgcgcc gcggggtcac tgcggacctg ccgcggcccc   1260
   tgcttctttc acatccatca tgtcctccaa acctccgcct cctccacgca cgtacgcacg   1320
   cccgctcgca cgcgcgcact gccgctgcga aagcaagcgc ccgcccgccg cccggcgacg   1380
   ggaaggcggc cgcggtctcc ctccgcggtt gcctcgctcc cgcgcggggc tgggcgggca   1440
   gcagaaggcg ggtggcggcg gcggcttccg tcttcgtcag cggcctacgt cggcggcggc   1500
   gcgcgagact acgcatgccc ttgcgtcatg cgctcgcagg tagccgccgc gggcctagcg   1560
   tttccgctgg cgccgcgcct aagcccccgg cgcgcacggt attgccgcga taccgtacgg   1620
   ccaagaccgc cgcagacgtc ggccctctcg cggccagcca gccagcagcg cagcggagga   1680
   agagcgcgca ggcgcggcgg gagggcggcc gcggagcagc gcagagcggg gcggagcagc   1740
   gcggagcaga acgggcagac tcggagcggg cagggcgggc agagctttgg ggtttaagga   1800
   ccgggttacc ggcgaagtga gcggctgcgg ggagcggctg tgggaggggt gagtacgcaa   1860
   gcacgatgcg agcgagagag agacgctgcc gcgaatcaag aaggtaggcg cgctgcgagg   1920
   cgcggcggcg gagcggagcg agggagaggg agagggagag agagggaggg agacgtcgcc   1980
   gcggcggggc ctggcctggc ctggtttggc ttggtcagcg cggccttgtc cgagcgtgca   2040
   gctggagttg ggtggattca tttggatttt cttttgtttt tgtttttctc tctttcccgg   2100
   aaagtgttgg ccggncggtg ttctttgttt tgatttcttc aaaagttttg gtggttggtt   2160
   ctctctcttg gctctctgtc aggcggtccg gtccacgccc cggcctctcc tctcctctcc   2220
   tctcctctcc tctccgtgcg tatacgtacg tacgtttgta tacgtacata catcccgccc   2280
   gccgtgccgg cgagggtttg ctcagcctgg agcaatgcga tgcgatgcga tgcgatgcga   2340
   cgcgacgcga cgcgagtcac tggttcgcgc tgtggctgtg gcttgcttgc ttacttgctt   2400
   tcgagctctc ccgctttctt ctttccttct cacgccacca ccaacgaaag aagatcggcc   2460
   ccggcacgcc gctgagaagg gctggcggcg atgacggcac gcgcgcccgc tgccacgttg   2520
   gcgctcgctg ctgctgctgc tgctgctgct gctgctgctg ctgctgctgc tgctgcttct   2580
   gcgcgcaggc tttgccacga ggccggcgtg ctggccgctg ccgcttccag tccgcgtgga   2640
   gagatcgaat gagagataaa ctggatggat tcatcgaggg atgaatgaac gatggttgga   2700
   tgcctttttc ctttttcagg tccacagcgg gaagcaggag cgcgtgaatc tgccgccatc   2760
   cgcatacgtc tgcatcgcat cgcatcgcat gcacgcatcg ctcgccggga gccacagacg   2820
   ggcgacaggg cggccagcca gccaggcagc cagccaggca ggcaccagag ggccagagag   2880
   cgcgcctcac gcacgcgccg cagtgcgcgc atcgctcgca gtgcagacct tgattccccg   2940
   cgcggatctc cgcgagcccg aaacgaagag cgccgtacgg gcccatccta gcgtcgcctc   3000
   gcaccgcatc gcatcgcatc gcgttcccta gagagtagta ctcgacgaag gcaccatttc   3060
   cgcgctcctc ttcggcgcga tcgaggcccc cggcgccgcg acgatcgcgg cggccgcggc   3120
   gctggcggcg gccctggcgc tcgcgctggc ggccgccgcg ggcgtctggc cctggcgcgc   3180
   gcgggcgccg caggaggagc ggcagcggct gctcgccgcc agagaagagc gcgccgggcc   3240
   cggggaggga cggggaggag aaggagaagg cgcgcaaggc ggccccgaaa gagaagaccc   3300
   tggacttgaa cgcgaagaag aagaagaagg agaagaagtt gaagaagaag aagaagaagg   3360
   agaggaagtt gaagaagacg aggagcaggc gcgttccaag gcgcgttctc ttccggaggc   3420
   gcgttccagc tgcggcggcg gggcgggctg cggggcgggc gcgggcgcgg gtgcgggcag   3480
   aggggacgcg cgcgcggagg cggagggggc cgagcgggag cccctgctgc tgcggggcgc   3540
   ccgggccgca ggtgtggcgc gcgcgacgac ggaggcgacg acgccagcgg ccgcgacgac   3600
   aaggccggcg gcgtcggcgg gcggaaggcc ccgcgcggag caggggcggg agcaggacaa   3660
   ggcgcaggag caggagcagg gccgggagcg ggagcgggag cgggcggcgg agcccgaggc   3720
   agaacccaat cgagatccag agcgagcaga ggccggccgc gagcccgagc ccgcgccgca   3780
   gatcactagt accgctgcgg aatcacagca gcagcagcag cagcagcagc agcagcagca   3840
   gcagcagcag ccacgagagg gagataaaga aaaagcggca gagacg                  3886

## Description

### Field of the Invention

This invention relates to polyunsaturated fatty acid (PUFA) polyketide synthase (PKS) systems from microorganisms, including eukaryotic organisms, such as Thraustochytrid microorganisms. More particularly, this invention relates to nucleic acids encoding non-bacterial PUFA PKS systems, to non-bacterial PUFA PKS systems, to genetically modified organisms comprising non-bacterial PUFA PKS systems, and to methods of making and using the non-bacterial PUFA PKS systems disclosed herein.

### Background of the Invention

Polyketide synthase (PKS) systems are generally known in the art as enzyme complexes derived from fatty acid synthase (FAS) systems, but which are often highly modified to produce specialized products that typically show little resemblance to fatty acids. Researchers have attempted to exploit polyketide synthase (PKS) systems that have been described in the literature as falling into one of three basic types, typically referred to as: Type II, Type I and modular. The Type II system is characterized by separable proteins, each of which carries out a distinct enzymatic reaction. The enzymes work in concert to produce the end product and each individual enzyme of the system typically participates several times in the production of the end product. This type of system operates in a manner analogous to the fatty acid synthase (FAS) systems found in plants and bacteria. Type I PKS systems are similar to the Type II system in that the enzymes are used in an iterative fashion to produce the end product. The Type I differs from Type II in that enzymatic activities, instead of being associated with separable proteins, occur as domains of larger proteins. This system is analogous to the Type I FAS systems found in animals and fungi.

In contrast to the Type I and II systems, in modular PKS systems, each enzyme domain is used only once in the production of the end product. The domains are found in very large proteins and the product of each reaction is passed on to another domain in the PKS protein. Additionally, in all of the PKS systems described above, if a carbon-carbon double bond is incorporated into the end product, it is always in the *trans* configuration.

In the Type I and Type II PKS systems described above, the same set of reactions is carried out in each cycle until the end product is obtained. There is no allowance for the introduction of unique reactions during the biosynthetic procedure. The modular PKS systems require huge proteins that do not utilize the economy of iterative reactions (i.e., a distinct domain is required for each reaction). Additionally, as stated above, carbon-carbon double bonds are introduced in the *trans* configuration in all of the previously described PKS systems.

Polyunsaturated fatty acids (PUFAs) are critical components of membrane lipids in most eukaryotes (Lauritzen et al., Prog. Lipid Res. 40 1 (2001); McConn et al., Plant J. 15, 521 (1998)) and are precursors of certain hormones and signaling molecules (Heller et al., Drugs 55, 487 (1998); Creelman et al., Annu. Rev. Plant Physiol. Plant Mol. Biol. 48, 355 (1997)). Known pathways of PUFA synthesis involve the processing of saturated 16:0 or 18:0 fatty acids (the abbreviation X:Y indicates an acyl group containing X carbon atoms and Y *cis* double bonds; double-bond positions of PUFAs are indicated relative to the methyl carbon of the fatty acid chain (ω3 or ω6) with systematic methylene interruption of the double bonds) derived from fatty acid synthase (FAS) by elongation and aerobic desaturation reactions (Sprecher, Curr. Opin. Clin. Nutr. Metab. Care 2,135 (1999); Parker-Barnes et al., Proc. Natl. Acad. Sci. USA 97, 8284 (2000); Shanklin et al., Annu. Rev. Plant Physiol. Plant Nol. Biol. 49, 611 (1998)). Starting from acetyl-CoA, the synthesis of DHA requires approximately 30 distinct enzyme activities and nearly 70 reactions including the four repetitive steps of the fatty acid synthesis cycle. Polyketide synthases (PKSs) carry out some of the same reactions as FAS (Hopwood et al., Annu. Rev. Genet. 24, 37 (1990); Bentley et al., Annu. Rev. Microbiol. 53, 411 (1999)) and use the same small protein (or domain), acyl carrier protein (ACP), as a covalent attachment site for the growing carbon chain. However, in these enzyme systems, the complete cycle of reduction, dehydration and reduction seen in FAS is often abbreviated so that a highly derivatized carbon chain is produced, typically containing many keto- and hydroxy-groups as well as carbon-carbon double bonds in the *trans* configuration. The linear products of PKSs are often cyclized to form complex biochemicals that include antibiotics and many other secondary products (Hopwood et al., (1990) *supra*; Bentley et al., (1999), *supra*; Keating et al., Curr. Opin. Chem. Biol. 3, 598 (1999)).

Very long chain PUFAs such as docosahexaenoic acid (DHA; 22:6ω3) and eicosapentaenoic acid (EPA; 20:5ω3) have been reported from several species of marine bacteria, including *Shewanella* sp (Nichols et al., Curr. Op. Biotechnol. 10, 240 (1999); Yazawa, Lipids 31, S (1996); DeLong et al., Appl. Environ. Microbiol. 51, 730 (1986)). Analysis of a genomic fragment (cloned as plasmid pEPA) from *Shewanella* sp. strain SCRC2738 led to the identification of five open reading frames (Orfs), totaling 20 Kb, that are necessary and sufficient for EPA production in *E. coli* (Yazawa, (1996), *supra*). Several of the predicted protein domains were homologues of FAS enzymes, while other regions showed no homology to proteins of known function. On the basis of these observations and biochemical studies, it was suggested that PUFA synthesis in *Shewanella* involved the elongation of 16- or 18-carbon fatty acids produced by FAS and the insertion of double bonds by undefined aerobic desaturases (Watanabe et al., J. Biochem. 122, 467 (1997)). The recognition that this hypothesis was incorrect began with a reexamination of the protein sequences encoded by the five *Shewanella* Orfs. At least 11 regions within the five Orfs were identifiable as putative enzyme domains (See Metz et al., Science 293:290-293 (2001)). When compared with sequences in the gene databases, seven of these were more strongly related to PKS proteins than to FAS proteins. Included in this group were domains putatively encoding malonyl-CoA:ACP acyltransferase (MAT), 3-ketoacyl-ACP synthase (KS), 3-ketoacyl-ACP reductase (KR), acyltransferase (AT), phosphopantetheine transferase, chain length (or chain initiation) factor (CLF) and a highly unusual cluster of six ACP domains (i.e., the presence of more than two clustered ACP domains has not previously been reported in PKS or FAS sequences). However, three regions were more highly homologous to bacterial FAS proteins. One of these was similar to the newly-described Triclosan-resistant enoyl reductase (ER) from *Streptococcus pneumoniae* (Heath et al., Nature 406, 145 (2000)); comparison of ORF8 peptide with the *S. pneumoniae* enoyl reductase using the LALIGN program (matrix, BLOSUM50; gap opening penalty, -10; elongation penalty -1) indicated 49% similarity over a 386aa overlap). Two regions were homologues of the *E. coli* FAS protein encoded by *fabA,* which catalyzes the synthesis of *trans*-2-decenoyl-ACP and the reversible isomerization of this product to *cis*-3-decenoyl-ACP (Heath et al., J. Biol. Chem., 271,27795 (1996)). On this basis, it seemed likely that at least some of the double bonds in EPA from *Shewanella* are introduced by a dehydrase-isomerase mechanism catalyzed by the FabA-like domains in Orf7.

Anaerobically-grown *E. coli* cells harboring the pEPA plasmid accumulated EPA to the same levels as aerobic cultures (Metz et al., 2001, *supra*), indicating that an oxygen-dependent desaturase is not involved in EPA synthesis. When pEPA was introduced into a *fabB⁻* mutant of *E. coli,* which is unable to synthesize monounsaturated fatty acids and requires unsaturated fatty acids for growth, the resulting cells lost their fatty acid auxotrophy. They also accumulated much higher levels of EPA than other pEPA-containing strains, suggesting that EPA competes with endogenously produced monounsaturated fatty acids for transfer to glycerolipids. When pEPA-containing *E. coli* cells were grown in the presence of [¹³C]-acetate, the data from ¹³C-NMR analysis of purified EPA from the cells confirmed the identity of EPA and provided evidence that this fatty acid was synthesized from acetyl-CoA and malonyl-CoA (See Metz et al., 2001, *supra*). A cell-free homogenate from pEPA-containing *fabB⁻* cells synthesized both EPA and saturated fatty acids from [¹⁴C]-malonyl-CoA. When the homogenate was separated into a 200,000xg high-speed pellet and a membrane-free supernatant fraction, saturated fatty acid synthesis was confined to the supernatant, consistent with the soluble nature of the Type II FAS enzymes (Magnuson et al., Microbiol. Rev. 57, 522 (1993)). Synthesis of EPA was found only in the high-speed pellet fraction, indicating that EPA synthesis can occur without reliance on enzymes of the E. coli FAS or on soluble intermediates (such as 16:0-ACP) from the cytoplasmic fraction. Since the proteins encoded by the *Shewanella* EPA genes are not particularly hydrophobic, restriction of EPA synthesis activity to this fraction may reflect a requirement for a membrane-associated acyl acceptor molecule. Additionally, in contrast to the *E. coli* FAS, EPA synthesis is specifically NADPH-dependent and does not require NADH. All these results are consistent with the pEPA genes encoding a multifunctional PKS that acts independently of FAS, elongase, and desaturase activities to synthesize EPA directly. It is likely that the PKS pathway for PUFA synthesis that has been identified in *Shewanella* is widespread in marine bacteria. Genes with high homology to the *Shewanella* gene cluster have been identified in *Photobacterium profundum* (Allen et al., Appli. Environ. Microbiol. 65:1710 (1999)) and in *Moritella marina* (*Vibrio marinus*) (Tanaka et al., Biotechnol. Lett. 21:939 (1999)).

The biochemical and molecular-genetic analyses performed with *Shewanella* provide compelling evidence for polyketide synthases that are capable of synthesizing PUFAs from malonyl-CoA. A complete scheme for synthesis of EPA by the *Shewanella* PKS has been proposed. The identification of protein domains homologous to the *E*. *coli* FabA protein, and the observation that bacterial EPA synthesis occurs anaerobically, provide evidence for one mechanism wherein the insertion of *cis* double bonds occurs through the action of a bifunctional dehydratase/2-*trans*, 3*-cis* isomerase (DH/2,3I). In *E. coli,* condensation of the *3-cis* acyl intermediate with malonyl-ACP requires a particular ketoacyl-ACP synthase and this may provide a rationale for the presence of two KS in the *Shewanella* gene cluster (in Orf 5 and Orf 7). However, the PKS cycle extends the chain in two-carbon increments while the double bonds in the EPA product occur at every third carbon. This disjunction can be solved if the double bonds at C-14 and C-8 of EPA are generated by 2-*trans*, 2-*cis* isomerization (DH/2,2I) followed by incorporation of the *cis* double bond into the elongating fatty acid chain. The enzymatic conversion of a *trans* double bond to the *cis* configuration without bond migration is known to occur, for example, in the synthesis of 11-*cis*-retinal in the retinoid cycle (Jang et al., J. Biol. Chem. 275, 28128 (2000)). Although such an enzyme function has not yet been identified in the *Shewanella* PKS, it may reside in one of the unassigned protein domains.

The PKS pathways for PUFA synthesis in *Shewanella* and another marine bacteria, *Vibrio marinus,* are described in detail in U.S. Patent No. 6,140,486 (issued from U.S. Application Serial No. 09/090,793, filed June 4, 1998, entitled "Production of Polyunsaturated Fatty Acids by Expression of Polyketide-like Synthesis Genes in Plants".

Polyunsaturated fatty acids (PUFAs) are considered to be useful for nutritional, pharmaceutical, industrial, and other purposes. An expansive supply of PUFAs from natural sources and from chemical synthesis are not sufficient for commercial needs. Because a number of separate desaturase and elongase enzymes are required for fatty acid synthesis from linoleic acid (LA, 18:2 Δ 9, 12), common in most plant species, to the more saturated and longer chain PUFAs, engineering plant host cells for the expression of PUFAs such as EPA and DHA may require expression of five or six separate enzyme activities to achieve expression, at least for EPA and DHA. Additionally, for production of useable quantities of such PUFAs, additional engineering efforts maybe required, for instance the down regulation of enzymes competing for substrate, engineering of higher enzyme activities such as by mutagenesis or targeting of enzymes to plastid organelles. Therefore it is of interest to obtain genetic material involved in PUFA biosynthesis from species that naturally produce these fatty acids and to express the isolated material alone or in combination in a heterologous system which can be manipulated to allow production of commercial quantities of PUFAs.

The discovery of a PUFA PKS system in marine bacteria such as *Shewanella* and *Vibrio marinus* (see U.S. Patent No. 6,140,486, *ibid.*) provides a resource for new methods of commercial PUFA production. However, these marine bacteria have limitations which will ultimately restrict their usefulness on a commercial level. First, although U.S. Patent No. 6,140,486 discloses that the marine bacteria PUFA PKS systems can be used to genetically modify plants, the marine bacteria naturally live and grow in cold marine environments and the enzyme systems of these bacteria do not function well above 30°C. In contrast, many crop plants, which are attractive targets for genetic manipulation using the PUFA PKS system, have normal growth conditions at temperatures above 30°C andranging to higher than 40°C. Therefore, the marine bacteria PUFA PKS system is not predicted to be readily adaptable to plant expression under normal growth conditions. Moreover, the marine bacteria PUFA PKS genes, being from a bacterial source, may not be compatible with the genomes of eukaryotic host cells, or at least may require significant adaptation to work in eukaryotic hosts. Additionally, the known marine bacteria PUFA PKS systems do not directly produce triglycerides, whereas direct production of triglycerides would be desirable because triglycerides are a lipid storage product in microorganisms and as a result can be accumulated at very high levels (e.g. up to 80-85% of cell weight) in microbial/plant cells (as opposed to a "structural" lipid product (e.g. phospholipids) which can generally only accumulate at low levels (e.g. less than 10-15% of cell weight at maximum)).

Therefore, there is a need in the art for other PUFA PKS systems having greater flexitbility for commercial use.

### Summary of the Invention

One embodiment of the present invention relates to an isolated nucleic acid molecule comprising a nucleic acid sequence selected from the group consisting of: (a) a nucleic acid sequence encoding an amino acid sequence of: SEQ ID NO:2; (b) a nucleic acid sequence encoding an amino acid sequence selected from the group consisting of: SEQ ID NO:8, SEQ ID NO:10, and biologically active fragments thereof, wherein the biologically active fragments exhibit a biological activity selected from the group consisting of β-keto acyl-ACP synthase (KS) activity and malonyl-CoA:ACP acyltransferase (MAT) activity; (c) a nucleic acid sequence encoding an amino acid sequence that is at least 60% identical to said amino acid sequence of (b), wherein said amino acid sequence has a biological activity selected from the group consisting of β-keto acyl-ACP synthase (KS) activity and malonyl-CoA:ACP acyltransferase (MAT) activity; and (d) a nucleic acid sequence that is fully complementary to the nucleic acid sequence of (a), (b), or (c).

In alternate aspects, the nucleic acid sequence encodes an amino acid sequence that is at least 70% identical to an amino acid sequence selected from the group consisting of: SEQ ID NO:8, SEQ ID NO:10. In a preferred embodiment, the nucleic acid sequence encodes an amino acid sequence chosen from: SEQ ID NO:8, SEQ ID NO:10, and/or biologically active fragments thereof. In one aspect the nucleic arid sequence is chosen from: SEQ ID NO:1.

Another embodiment of the present invention relates to a recombinant nucleic acid molecule comprising the nucleic acid molecule as described above, operatively linked to at least one transcription control sequence. In another embodiment, the present invention relates to a recombinant cell transfected with the recombinant nucleic acid molecule described directly above.

Yet another embodiment of the present invention relates to a genetically modified microorganism, wherein the microorganism expresses a PKS system comprising at least one biologically active domain of a polyunsaturated fatty acid (PUFA) polyketide synthase (PKS) system. The at least one domain of the PUFA PKS system is encoded by a nucleic acid sequence chosen from the nucleic acid sequences of the invention.

In this embodiment, the microorganism is genetically modified to affect the activity of the PKS system.

In one aspect, the genetically modified microorganism is a Thraustochytrid, which can include, but is not limited to, a Thraustochytrid from a genus chosen from *Schizochytrium* and *Thraustochytrium.* In another aspect, the microorganism has been further genetically modified to recombinantly express at least one nucleic acid molecule encoding at least one biologically active domain from a bacterial PUFA PKS system, from a Type I PKS system, from a Type II PKS system, and/or from a modular PKS system.

In another aspect of this embodiment, the microorganism endogenously expresses a PUFA PKS system comprising the at least one biologically active domain of a PUFA PKS system, and wherein the genetic modification comprises expression of a recombinant nucleic acid molecule selected from the group consisting of a recombinant nucleic acid molecule encoding at least one biologically active domain from a second PKS system and a recombinant nucleic acid molecule encoding a protein that affects the activity of the PUFA PKS system. Preferably, the recombinant nucleic acid molecule comprises any one of the nucleic acid sequences described above.

In one aspect of this embodiment, the recombinant nucleic acid molecule encodes a phosphopantetheine transferase. In another aspect, the recombinant nucleic acid molecule comprises a nucleic acid sequence encoding at least one biologically active domain from a bacterial PUFA PKS system, from a type I PKS system, from a type II PKS system, and/or from a modular PKS system.

In another aspect of this embodiment, the microorganism is genetically modified by transfection with a recombinant nucleic acid molecule encoding the at least one domain of a polyunsaturated fatty acid (PUFA) polyketide synthase (PKS) system. Such a recombinant nucleic acid molecule can include any recombinant nucleic acid molecule comprising any of the nucleic acid sequences described above. In one aspect, the microorganism has been further genetically modified to recombinantly express at least one nucleic acid molecule encoding at least one biologically active domain from a bacterial PUFA PKS system, from a Type I PKS system, from a Type II PKS system, or from a modular PKS system.

Yet another embodiment of the present invention relates to a genetically modified plant, wherein the plant has been genetically modified to recombinantly express a PKS system comprising at least one biologically active domain of a polyunsaturated fatty acid (PUFA) polyketide synthase (PKS) system. The domain can be encoded by any of the nucleic acid sequences described above. In one aspect, the plant has been further genetically modified to recombinantly express at least one nucleic acid molecule encoding at least one biologically active domain from a bacterial PUFA PKS system, from a Type I PKS system, from a Type II PKS system, and/from a modular PKS system.

Yet another embodiment of the present invention relates to a method to produce a bioactive molecule that is produced by a polyketide synthase system. The method includes the step of culturing under conditions effective to produce the bioactive molecule a genetically modified organism that expresses a PKS system comprising at least one biologically active domain of a polyunsaturated fatty acid (PUFA) polyketide synthase (PKS) system. The domain of the PUFA PKS system is encoded by any of the nucleic acid sequences described above.

In one aspect of this embodiment, the organism endogenously expresses a PKS system comprising the at least one domain of the PUFA PKS system, and the genetic modification is in a nucleic acid sequence encoding the at least one domain of the PUFA PKS system. For example, the genetic modification can change at least one product produced by the endogenous PKS system, as compared to a wild-type organism.

In another aspect of this embodiment, the organism endogenously expresses a PKS system comprising the at least one biologically active domain of the PUFA PKS system, and the genetic modification comprises transfection of the organism with a recombinant nucleic acid molecule selected from the group consisting of: a recombinant nucleic acid molecule encoding at least one biologically active domain from a second PKS system and a recombinant nucleic acid molecule encoding a protein that affects the activity of the PUFA PKS system. For example, the genetic modification can change at least one product produced by the endogenous PKS system, as compared to a wild-type organism.

In yet another aspect of this embodiment, the organism is genetically modified by transfection with a recombinant nucleic acid molecule encoding the at least one domain of the polyunsaturated fatty acid (PUFA) polyketide synthase (PKS) system. In another aspect, the organism produces a polyunsaturated fatty acid (PUFA) profile that differs from the naturally occurring organism without a genetic modification. In another aspect, the organism endogenously expresses a non-bacterial PUFA PKS system, and wherein the genetic modification comprises substitution of a domain from a different PKS system for a nucleic acid sequence encoding at least one domain of the non-bacterial PUFA PKS system.

In yet another aspect, the organism endogenously expresses a non-bacterial PUFA PKS system that has been modified by transfecting the organism with a recombinant nucleic acid molecule encoding a protein that regulates the chain length of fatty acids produced by the PUFA PKS system. For example, the recombinant nucleic acid molecule encoding a protein that regulates the chain length of fatty acids can replace a nucleic acid sequence encoding a chain length factor in the non-bacterial PUFA PKS system. In another aspect, the protein that regulates the chain length of fatty acids produced by the PUFA PKS system is a chain length factor. In another aspect, the protein that regulates the chain length of fatty acids produced by the PUFA PKS system is a chain length factor that directs the synthesis of C20 units.

In one aspect, the organism expresses a non-bacterial PUFA PKS system comprising a genetic modification in a domain chosen from: a domain encoding FabA-like β-hydroxy acyl-ACP dehydrase (DH) domain and a domain encoding β-ketoacyl-ACP synthase (KS), wherein the modification alters the ratio of long chain fatty acids produced by the PUFA PKS system as compared to in the absence of the modification. In one aspect, the modification comprises substituting a DH domain that does not possess isomerization activity for a FabA-like β-hydroxy acyl-ACP dehydrase (DH) in the non-bacterial PUFA PKS system. In another aspect, the modification is selected from the group consisting of a deletion of all or a part of the domain, a substitution of a homologous domain from a different organism for the domain, and a mutation of the domain.

In another aspect, the organism expresses a PKS system and the genetic modification comprises substituting a FabA-like β-hydroxy acyl-ACP dehydrase (DH) domain from a PUFA PKS system for a DH domain that does not posses isomerization activity.

In another aspect, the organism expresses a non-bacterial PUFA PKS system comprising a modification in an enoyl-ACP reductase (ER) domain, wherein the modification results in the production of a different compound as compared to in the absence of the modification. For example, the modification can be selected from the group consisting of a deletion of all or a part of the ER domain, a substitution of an ER domain from a different organism for the ER domain, and a mutation of the ER domain.

In one aspect, the bioactive molecule produced by the present method can include, but is not limited to, an anti-inflammatory formulation, a chemotherapeutic agent, an active excipient, an osteoporosis drug, an anti-depressant, an anti-convulsant, an anti*-Heliobactor pylori* drug, a drug for treatment of neurodegenerative disease, a drug for treatment of degenerative liver disease, an antibiotic, and a cholesterol lowering formulation. In one aspect, the bioactive molecule is a polyunsaturated fatty acid (PUFA). In another aspect, the bioactive molecule is a molecule including carbon-carbon double bonds in the *cis* configuration. In another aspect, the bioactive molecule is a molecule including a double bond at every third carbon.

In one aspect of this embodiment, the organism is a microorganism, and in another aspect, the organism is a plant

Another embodiment of the present invention relates to a method to produce a plant that has a polyunsaturated fatty acid (PUFA) profile that differs from the naturally occurring plant, comprising genetically modifying cells of the plant to express a PKS system comprising at least one recombinant nucleic acid molecule comprising a nucleic acid sequence encoding at least one biologically active domain of a PUFA PKS system. The domain of the PUFA PKS system is encoded by any of the nucleic acid sequences described above.

Yet another embodiment of the present invention relates to a method to produce a humanized animal milk, comprising genetically modifying milk-producing cells of a milk-producing non-human animal with at least one recombinant nucleic acid molecule comprising a nucleic acid sequence encoding at least one biologically active domain of a PUFA PKS system. The domain of the PUFA PKS system is encoded by any of the nucleic acid sequences described above.

Yet another embodiment of the present invention relates to a method produce a recombinant microbe, comprising genetically modifying microbial cells to express at least one recombinant nucleic acid molecule comprising a comprising a nucleic acid sequence encoding at least one biologically active domain of a PUFA PKS system. The domain of the PUFA PKS system is encoded by any of the nucleic acid sequences described above.

Yet another embodiment of the present invention relates to a recombinant host cell which has been modified to express a polyunsaturated fatty acid (PUFA) polyketide synthase (PKS) system, wherein the PKS catalyzes both iterative and non-iterative enzymatic reactions. The PUFA PKS system comprises: (a) at least two enoyl ACP-reductase (ER) domains; (b) at least six acyl carrier protein (ACP) domains; (c) at least two β-keto acyl-ACP synthase (KS) domains; (d) at least one acyl transferase (AT) domain; (e) at least one ketoreductase (KR) domain; (f) at least two FabA-like β-hydroxy acyl-ACP dehydrase (DH) domains; (g) at least one chain length factor (CLF) domain; and (h) at least one malonyl-CoA:ACP acyltransferase (MAT) domain. In one aspect, the PUFA PKS system is a eukaryotic PUFA PKS system. In another aspect, the PUFA PKS system is an algal PUFA PKS system, and preferably a Thraustochytriales PUFA PKS system, which can include, but is not limited to, a *Schizochytrium* PUFA PKS system or a *Thraustochytrium* PUFA PKS system.

In this embodiment, the PUFA PKS system can be expressed in a prokaryotic host cell or in a eukaryotic host cell. In one aspect, the host cell is a plant cell. Accordingly, one embodiment of the invention is a method to produce a product containing at least one PUFA, comprising growing a plant comprising such a plant cell under conditions effective to produce the product. The host cell is a microbial cell and in this case, one embodiment of the present invention is a method to produce a product containing at least one PUFA, comprising culturing a culture containing such a microbial cell under conditions effective to produce the product. In one aspect, the PKS system catalyzes the direct production of triglycerides.

Yet another embodiment of the present invention relates to a genetically modified microorganism comprising a polyunsaturated fatty acid (PUFA) polyketide synthase (PKS) system, wherein the PKS catalyzes both iterative and non-iterative enzymatic reactions. The PUFA PKS system comprises: (a) at least two enoyl ACP-reductase (ER) domains; (b) at least six acyl carrier protein (ACP) domains; (c) at least two β-keto acyl-ACP synthase (KS) domains; (d) at least one acyltransferase (AT) domain; (e) at least one ketoreductase (KR) domain; (f) at least two FabA-like β-hydroxy acyl-ACP dehydrase (DH) domains; (g) at least one chain length factor (CLF) domain; and (h) at least one malonyl-CoA:ACP acyltransferase (MAT) domain. The genetic modification affects the activity of the PUFA PKS system. In one aspect of this embodiment, the microorganism is a eukaryotic microorganism.

Yet another embodiment of the present invention relates to a recombinant host cell which has been modified to express a non-bacterial polyunsaturated fatty acid (PUFA) polyketide synthase (PKS) system, wherein the non-bacterial PUFA PKS catalyzes both iterative and non-iterative enzymatic reactions. The non-bacterial PUFA PKS system comprises: (a) at least one enoyl ACP-reductase (ER) domain; (b) multiple acyl carrier protein (ACP) domains; (c) at least two β-keto acyl-ACP synthase (KS) domains; (d) at least one acyltransferase (AT) domain; (e) at least one ketoreductase (KR) domain; (f) at least two FabA-like β-hydroxy acyl-ACP dehydrase (DH) domains; (g) at least one chain length factor (CLF) domain; and (h) at least one malonyl-CoA:ACP acyltransferase (MAT) domain.

### Brief Description of the Figures

Fig. 1 is a graphical representation of the domain structure of the *Schizochytrium* PUFA PKS system.
Fig. 2 shows a comparison of PKS domains from *Schizochytrium* and *Shewanella.*
Fig. 3 shows a comparison of PKS domains from *Schizochytrium* and a related PKS system from *Nostoc* whose product is a long chain fatty acid that does not contain any double bonds.

### Detailed Description of the Invention

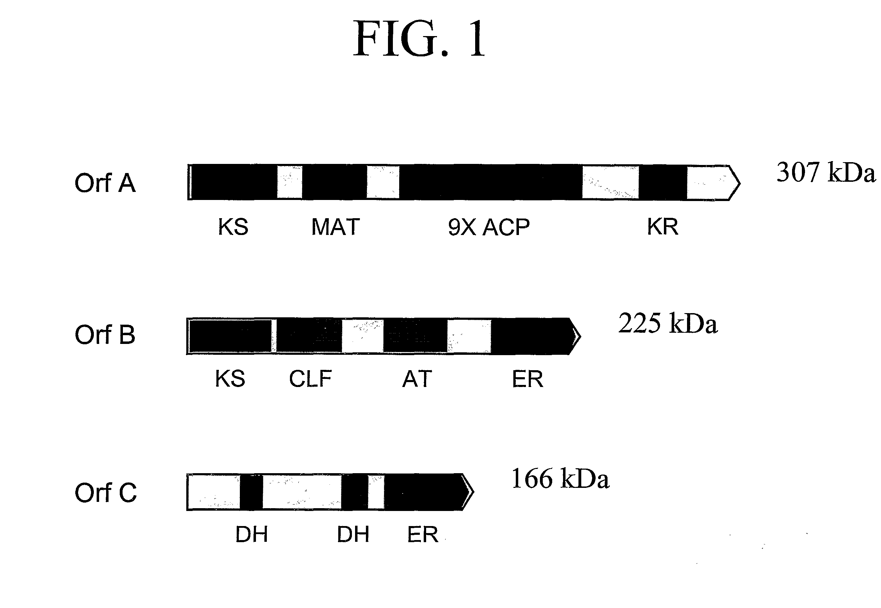

The present invention generally relates to non-bacterial derived polyunsaturated fatty acid (PUFA) polyketide synthase (PKS) systems, to genetically modified organisms comprising non-bacterial PUFA PKS systems, to methods of making and using such systems for the production of products of interest, including bioactive molecules, and to novel methods for identifying new eukaryotic microorganisms having such a PUFA PKS system. As used herein, a PUFA PKS system generally has the following identifying features: (1) it produces PUFAs as a natural product of the system; and (2) it comprises several multifunctional proteins assembled into a complex that conducts both *iterative* processing of the fatty acid chain as well *non-iterative* processing, including *trans-cis* isomerization and enoyl reduction reactions in selected cycles (See Fig. 1, for example).

More specifically, first, a PUFA PKS system that forms the basis of this invention produces polyunsaturated fatty acids (PUFAs) as products (i.e., an organism that endogenously (naturally) contains such a PKS system makes PUFAs using this system). The PUFAs referred to herein are preferably polyunsaturated fatty acids with a carbon chain length of at least 16 carbons, and more preferably at least 18 carbons, and more preferably at least 20 carbons, and more preferably 22 or more carbons, with at least 3 or more double bonds, and preferably 4 or more, and more preferably 5 or more, and even more preferably 6 or more double bonds, wherein all double bonds are in the *cis* configuration. It is an object of the present invention to find or create via genetic manipulation or manipulation of the endproduct, PKS systems which produce polyunsaturated fatty acids of desired chain length and with desired numbers of double bonds. Examples of PUFAs include, but are not limited to, DHA (docosahexaenoic acid (C22:6, ω-3)), DPA (docosapentaenoic acid (C22:5, ω-6)), and EPA (eicosapentaenoic acid (C20:5, ω-3)).

Second, the PUFA PKS system described herein incorporates both iterative and non-iterative reactions, which distinguish the system from previously described PKS systems (e.g., type I, type II or modular). More particularly, the PUFA PKS system described herein contains domains that appear to function during each cycle as well as those which appear to function during only some of the cycles. A key aspect of this may be related to the domains showing homology to the bacterial Fab A enzymes. For example, the Fab A enzyme *of E. coli* has been shown to possess two enzymatic activities. It possesses a dehydration activity in which a water molecule (H₂O) is abstracted from a carbon chain containing a hydroxy group, leaving a *trans* double bond in that carbon chain. In addition, it has an isomerase activity in which the *trans* double bond is converted to the *cis* configuration. This isomerization is accomplished in conjunction with a migration of the double bond position to adjacent carbons. In PKS (and FAS) systems, the main carbon chain is extended in 2 carbon increments. One can therefore predict the number of extension reactions required to produce the PUFA products of these PKS systems. For example, to produce DHA (C22:6, all *cis)* requires 10 extension reactions. Since there are only 6 double bonds in the end product, it means that during some of the reaction cycles, a double bond is retained (as a *cis* isomer), and in others, the double bond is reduced prior to the next extension.

Before the discovery of a PUFA PKS system in marine bacteria (see U.S. Patent No. 6,140,486), PKS systems were not known to possess this combination of iterative and selective enzymatic reactions, and they were not thought of as being able to produce carbon-carbon double bonds in the *cis* configuration. However, the PUFA PKS system described by the present invention has the capacity to introduce *cis* double bonds and the capacity to vary the reaction sequence in the cycle.

Therefore, the present inventors propose to use these features of the PUFA PKS system to produce a range of bioactive molecules that could not be produced by the previously described (Type II, Type I and modular) PKS systems. These bioactive molecules include, but are limited to, polyunsaturated fatty acids (PUFAs), antibiotics or other bioactive compounds, many of which will be discussed below. For example, using the knowledge of the PUFA PKS gene structures described herein, any of a number of methods can be used to alter the PUFA PKS genes, or combine portions of these genes with other synthesis systems, including other PKS systems, such that new products are produced. The inherent ability of this particular type of system to do both iterative and selective reactions will enable this system to yield products that would not be found if similar methods were applied to other types of PKS systems.

In one embodiment, a PUFA PKS system according to the present invention comprises at least the following biologically active domains: (a) at least two enoyl ACP-reductase (ER) domains; (b) at least six acyl carrier protein (ACP) domains; (c) at least two β-veto acyl-ACP synthase (KS) domains; (d) at least one acyltransferase (AT) domain; (e) at least one ketoreductase (KR) domain; (f) at least two FabA-like β-hydroxy acyl-ACP dehydrase (DH) domains; (g) at least one chain length factor (CLF) domain; and (h) at least one malonyl-CoA:ACP acyltransferase (MAT) domain. The functions of these domains are generally individually known in the art and will be described in detail below with regard to the PUFA PKS system of the present invention.

In another embodiment, the PUFA PKS system comprises at least the following biologically active domains: (a) at least one enoyl ACP-reductase (ER) domain; (b) multiple acyl carrier protein (ACP) domains (at least four, and preferably at least five, and more preferably at least six, and even more preferably seven, eight, nine, or more than nine); (c) at least two β-keto acyl-ACP synthase (KS) domains; (d) at least one acyltransferase (AT) domain; (e) at least one ketoreductase (KR) domain; (f) at least two FabA-like β-hydroxy acyl-ACP dehydrase (DH) domains; (g) at least one chain length factor (CLF) domain; and (h) at least one malonyl-CoA:ACP acyltransferase (MAT) domain. Preferably, such a PUFA PKS system is a non-bacterial PUFA-PKS system.

In one embodiment, a PUFA PKS system of the present invention is a non-bacterial PUFA PKS system. In other words, in one embodiment, the PUFA PKS system of the present invention is isolated from an organism that is not a bacteria, or is a homologue of or derived from a PUFA PKS system from an organism that is not a bacteria, such as a eukaryote or an archaebacterium. Eukaryotes are separated from prokaryotes based on the degree of differentiation of the cells. The higher group with more differentiation is called eukaryotic. The lower group with less differentiated cells is called prokaryotic. In general, prokaryotes do no possess a nuclear membrane, do not exhibit mitosis during cell division, have only one chromosome, their cytoplasm contains 70S ribosomes, they do not possess any mitochondria, endoplasmic reticulum, chloroplasts, lysosomes or golgi apparatus, their flagella (if present) consists of a single fibril. In contrast eukaryotes have a nuclear membrane, they do exhibit mitosis during cell division, they have many chromosomes, their cytoplasm contains 80S ribosomes, they do possess mitochondria, endoplasmic reticulum, chloroplasts (in algae), lysosomes and golgi apparatus, and their flagella (if present) consists of many fibrils. In general, bacteria are prokaryotes, while algae, fungi, protist, protozoa and higher plants are eukaryotes. The PUFA PKS systems of the marine bacteria (e.g., *Shewanella* and *Vibrio marinus)* are not the basis of the present invention, although the present invention does contemplate the use of domains from these bacterial PUFA PKS systems *in conjunction* with domains from the non-bacterial PUFA PKS systems of the present invention. For example, according to the present invention, genetically modified organisms can be produced which incorporate non-bacterial PUFA PKS functional domains with bacteria PUFA PKS functional domains, as well as PKS functional domains or proteins from other PKS systems (type I, type II, modular) or FAS systems.

*Schizochytrium* is a Thraustochytrid marine microorganism that accumulates large quantities of triacylglycerols rich in DHA and docosapentaenoic acid (DPA; 22:5 ω-6); e.g., 30% DHA + DPA by dry weight (Barclay et al., J. Appl. Phycol. 6, 123 (1994)). In eukaryotes that synthesize 20- and 22-carbon PUFAs by an elongation/desaturation pathway, the pools of 18-, 20- and 22-carbon intermediates are relatively large so that *in vivo* labeling experiments using [¹⁴C]-acetate reveal clear precursor-product kinetics for the predicted intermediates (Gellerman et al., Biochim. Biophys. Acta 573:23 (1979)). Furthermore, radiolabeled intermediates provided exogenously to such organisms are converted to the final PUFA products. The present inventors have shown that [-¹⁴C]-acetate was rapidly taken up by *Schizochytrium* cells and incorporated into fatty acids, but at the shortest labeling time (1 min), DHA contained 31% of the label recovered in fatty acids, and this percentage remained essentially unchanged duringthe 10-15 min of [¹⁴C]-acetate incorporation and the subsequent 24 hours of culture growth (See Example 3). Similarly, DPA represented 10% of the label throughout the experiment. There is no evidence for a precursor-product relationship between 16- or 18-carbon fatty acids and the 22-carbon polyunsaturated fatty acids. These results are consistent with rapid synthesis of DHA from **[¹⁴C]-acetate** involving very small (possibly enzyme-bound) pools of intermediates. A cell-free homogenate derived from *Schizochytrium* cultures incorporated [1-¹⁴C]-malonyl-CoA into DHA, DPA, and saturated fatty acids. The same biosynthetic activities were retained by a 100,000xg supernatant fraction but were not present in the membrane pellet Thus, DHA and DPA synthesis in *Schizochytrium* does not involve membrane-bound desaturases or fatty acid elongation enzymes like those described for other eukaryotes (Parker-Barnes et al., 2000, *supra*; Shanklin et al., 1998, *supra*). These fractionation data contrast with those obtained from the *Shewanella* enzymes (See Metz et al., 2001, *supra*) and may indicate use of a different (soluble) acyl acceptor molecule, such as CoA, by the *Schizochytrium* enzyme.

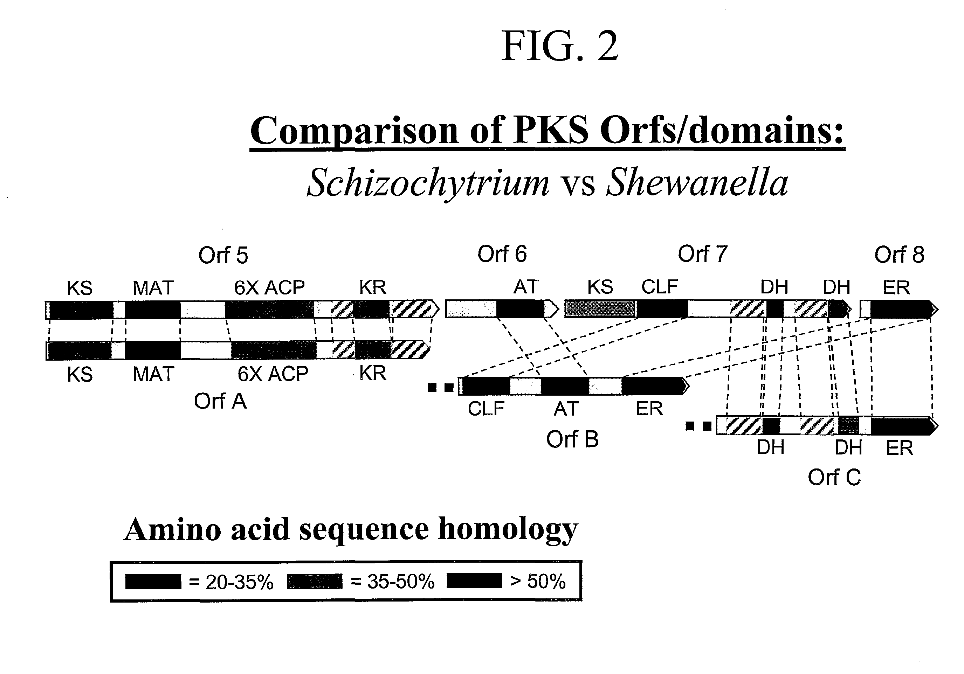

In copending US Application Serial No. 09/231,899 (US Patent No. 6,566, 583), a cDNA library from *Schizochytrium* was constructed and approximately 8,000 random clones (ESTs) were sequenced. Within this dataset, only one moderately expressed gene (0.3% of all sequences) was identified as a fatty acid desaturase, although a second putative desaturase was represented by a single clone (0.01%). By contrast, sequences that exhibited homology to 8 of the 11 domains of the *Shewanella* PKS genes shown in Fig. 2 were all identified at frequencies of 0.2-0.5%. In U.S. Application Serial No. 09/231,899, several cDNA clones showing homology to the *Shewanella* PKS genes were sequenced, and various clones were assembled into nucleic acid sequences representing two partial open reading frames and one complete open reading frame. Nucleotides 390-4443 of the cDNA sequence containing the first partial open reading frame described in U.S. Application SerialNo. 09/231,899 (denoted therein as SEQ ID NO:69) match nucleotides 4677-8730 (plus the stop codon) of the sequence denoted herein as OrfA (SEQ ID NO:1). Nucleotides 1-4876 ofthe cDNA sequence containing the second partial open reading frame described in U.S. Application Serial No. 09/231,899 (denoted therein as SEQ ID NO:71) matches nucleotides 1311-6177 (plus the stop codon) of the sequence denoted herein as OrfB (SEQ ID NO:3). Nucleotides 145-4653 of the cDNA sequence containing the complete open reading frame described in U.S. Application Serial No. 09/231,899 (denoted therein as SEQ ID NO:76 and incorrectly designated as a partial open reading frame) match the entire sequence (plus the stop codon) of the sequence denoted herein as OrfC (SEQ ID NO:5).

Further sequencing of cDNA and genomic clones by the present inventors allowed the identification of the full-length genomic sequence of each of OrfA, OrfB and OrfC and the complete identification of the domains with homology to those in *Shewanella* (see Fig. 2). It is noted that in *Schizochytrium,* the genomic DNA and cDNA are identical, due to the lack of introns in the organism genome, to the best of the present inventors' knowledge. Therefore, reference to a nucleotide sequence from *Schizochytrium* can refer to genomic DNA or cDNA. Based on the comparison of the *Schizochytrium* PKS domains to *Shewanella,* clearly, the *Schizochytrium* genome encodes proteins that are highly similar to the proteins in *Shewanella* that are capable of catalyzing EPA synthesis. The proteins in *Schizochytrium* constitute a PUFA PKS system that catalyzes DHA and DPA synthesis. As discussed in detail herein, simple modification of the reaction scheme identified for *Shewanella* will allow for DHA synthesis in *Schizochytrium.* The homology between the prokaryotic *Shewanella* and eukaryotic *Schizochytrium* genes suggests that the PUFA PKS has undergone lateral gene transfer.

Fig. 1 is a graphical representation of the three open reading frames from the *Schizochytrium* PUFA PKS system, and includes the domain structure of this PUFA PKS system. As described in Example 1 below, the domain structure of each open reading frame is as follows:

### Open Reading Frame A (OrfA):

The complete nucleotide sequence for OrfA is represented herein as SEQ ID NO:1. Nucleotides 4677-8730 of SEQ ID NO:1 correspond to nucleotides 390-4443 of the sequence denoted as SEQ ID NO:69 in U.S. Application Serial No. 09/231,899. Therefore, nucleotides 1-4676 of SEQ ID NO:1 represent additional sequence that was not disclosed in U.S. Application Serial No. 09/231,899. This novel region of SEQ ID NO:1 encodes the following domains in OrfA: (1) the ORFA-KS domain; (2) the ORFA-MAT domain; and (3) at least a portion of the ACP domain region (e.g., at least ACP domains 1-4). It is noted that nucleotides 1-389 of SEQ ID NO:69 in U.S. Application Serial No. 09/231,899 do not match with the 389 nucleotides that are upstream of position 4677 in SEQ ID NO:1 disclosed herein. Therefore, positions 1-389 of SEQ ID NO:69 in U.S. Application Serial No. 09/231,899 appear to be incorrectly placed next to nucleotides 390-4443 of that sequence. Most of these first 389 nucleotides (about positions 60-389) are a match with an upstream portion of OrfA (SEQ ID NO:1) of the present invention and therefore, it is believed that an error occurred in the effort to prepare the contig of the cDNA constructs in U.S. Application Serial No. 09/231,899. The region in which the alignment error occurred in U.S. Application Serial No. 09/231,899 is within the region of highly repetitive sequence (i.e., the ACP region, discussed below), which probably created some confusion in the assembly of that sequence from various cDNA clones.

OrfA is a 8730 nucleotide sequence (not including the stop codon) which encodes a 2910 amino acid sequence, represented herein as SEQ ID NO:2. Within OrfA are twelve domains: (a) one β-keto acyl-ACP synthase (KS) domain; (b) one malonyl-CoA:ACP acyltransferase (MAT) domain; (c) nine acyl carrier protein (ACP) domains; and (d) one ketoreductase (KR) domain.

The nucleotide sequence for OrfA has been deposited with GenBank as Accession No. AF378327 (amino acid sequence Accession No. AAK728879). OrfA was compared with known sequences in a standard BLAST search (BLAST 2.0 Basic BLAST homology search using blastp for amino acid searches, blastn for nucleic acid searches, and blastX for nucleic acid searches and searches of the translated amino acid sequence in all 6 open reading frames with standard default parameters, wherein the query sequence is filtered for low complexity regions by default (described in Altschul, S.F., Madden, T.L., Schääffer, A.A., Zhang, J., Zhang, Z., Miller, W. & Lipman, D.J. (1997) "Gapped BLAST and PSI-BLAST: a new generation of protein database search programs." Nucleic Acids Res. 25:3389-3402).

At the nucleic acid level, OrfA has no significant homology to any known nucleotide sequence. At the amino acid level, the sequences with the greatest degree of homology to ORFA were: *Nostoc sp.* 7120 heterocyst glycolipid synthase (Accession No. NC_003272), which was 42% identical to ORFA over 1001 amino acid residues; and *Moritella marinus (Vibrio marinus*) ORF8 (Accession No. AB025342), which was 40% identical to ORFA over 993 amino acid residues.

The first domain in OrfA is a KS domain, also referred to herein as ORFA-KS. This domain is contained within the nucleotide sequence spanning from a starting point of between about positions 1 and 40 of SEQ ID NO:1 (OrfA) to an ending point of between about positions 1428 and 1500 of SEQ ID NO:1. The nucleotide sequence containing the sequence encoding the ORFA-KS domain is represented herein as SEQ ID NO:7 (positions 1-1500 of SEQ ID NO:1). The amino acid sequence containing the KS domain spans from a starting point of between about positions 1 and 14 of SEQ ID NO:2 (ORFA) to an ending point of between about positions 476 and 500 of SEQ ID NO:2. The amino acid sequence containing the ORFA-KS domain is represented herein as SEQ ID NO:8 (positions 1-500 of SEQ ID NO:2). It is noted that the ORFA-KS domain contains an active site motif: DXAC* (*acyl binding site C₂₁₅).

According to the present invention, a domain or protein having 3-keto acyl-ACP synthase (KS) biological activity (function) is characterized as the enzyme that carries out the initial step of the FAS (and PKS) elongation reaction cycle. The acyl group destined for elongation is linked to a cysteine residue at the active site of the enzyme by a thioester bond. In the multi-step reaction, the acyl-enzyme undergoes condensation with malonyl-ACP to form -keto acyl-ACP, CO₂ and free enzyme. The KS plays a key role in the elongation cycle and in many systems has been shown to possess greater substrate specificity than other enzymes of the reaction cycle. For example, *E. coli* has three distinct KS enzymes - each with its own particular role in the physiology of the organism (Magnuson et al., Microbiol. Rev. 57, 522 (1993)). The two KS domains of the PUFA-PKS systems could have distinct roles in the PUFA biosynthetic reaction sequence.

As a class of enzymes, KS's have been well characterized. The sequences of many verified KS genes are know, the active site motifs have been identified and the crystal structures of several have been determined. Proteins (or domains of proteins) can be readily identified as belonging to the KS family of enzymes by homology to known KS sequences.

The second domain in OrfA is a MAT domain, also referred to herein as ORFA-MAT. This domain is contained within the nucleotide sequence spanning from a starting point of between about positions 1723 and 1798 of SEQ ID NO: 1 (OrfA) to an ending point of between about positions 2805 and 3000 of SEQ ID NO:1. The nucleotide sequence containing the sequence encoding the ORFA-MAT domain is represented herein as SEQ ID NO:9 (positions 1723-3000 of SEQ ID NO:1). The amino acid sequence containing the MAT domain spans from a starting point of between about positions 575 and 600 of SEQ ID NO:2 (ORFA) to an ending point of between about positions 935 and 1000 of SEQ ID NO:2. The amino acid sequence containing the ORFA-MAT domain is represented herein as SEQ ID NO:10 (positions 575-1000 of SEQ ID NO:2). It is noted that the ORFA-MAT domain contains an active site motif: GHS*XG (*acyl binding site S₇₀₆), represented herein as SEQ ID NO:11.

According to the present invention, a domain or protein having malonyl-CoA:ACP acyltransferase (MAT) biological activity (function) is characterized as one that transfers the malonyl moiety from malonyl-CoA to ACP. In addition to the active site motif (GxSxG), these enzymes possess an extended motif ® and Q amino acids in key positions) that identifies them as MAT enzymes (in contrast to the AT domain of Schizochytrium Orf B). In some PKS systems (but not the PUFA PKS domain) MAT domains will preferentially load methyl- or ethyl- malonate on to the ACP group (from the corresponding CoA ester), thereby introducing branches into the linear carbon chain. MAT domains can be recognized by their homology to known MAT sequences and by their extended motif structure.

Domains 3-11 of OrfA are nine tandem ACP domains, also referred to herein as ORFA-ACP (the first domain in the sequence is ORFA-ACP 1, the second domain is ORFA-ACP2, the third domain is ORFA-ACP3, etc.). The first ACP domain, ORFA-ACP1, is contained within the nucleotide sequence spanning from about position 3343 to about position 3600 of SEQ ID NO:1 (OrfA). The nucleotide sequence containing the sequence encoding the ORFA-ACP1 domain is represented herein as SEQ ID NO:12 (positions 3343-3600 of SEQ ID NO:1). The amino acid sequence containing the first ACP domain spans from about position 1115 to about position 1200 of SEQ ID NO:2. The amino acid sequence containing the ORFA-ACP1 domain is represented herein as SEQ ID NO:13 (positions 1115-1200 of SEQ ID NO:2). It is noted that the ORFA-ACP1 domain contains an active site motif: LGIDS* (*pantetheine binding motif S₁₁₅₇), represented herein by SEQ ID NO:14.

The nucleotide and amino acid sequences of all nine ACP domains are highly conserved and therefore, the sequence for each domain is not represented herein by an individual sequence identifier. However, based on the information disclosed herein, one of skill in the art can readily determine the sequence containing each of the other eight ACP domains (see discussion below).

All nine ACP domains together span a region of OrfA of from about position 3283 to about position 6288 of SEQ ID NO:1, which corresponds to amino acid positions of from about 1095 to about 2096 of SEQ ID NO:2. The nucleotide sequence for the entire ACP region containing all nine domains is represented herein as SEQ ID NO:16. The region represented by SEQ ID NO:16 includes the linker segments between individual ACP domains. The repeat interval for the nine domains is approximately every 330 nucleotides of SEQ ID NO:16 (the actual number of amino acids measured between adjacent active site serines ranges from 104 to 116 amino acids). Each of the nine ACP domains contains a pantetheine binding motif LGIDS* (represented herein by SEQ ID NO:14), wherein S* is the pantetheine binding site serine (S). The pantetheine binding site serine (S) is located near the center of each ACP domain sequence. At each end of the ACP domain region and between each ACP domain is a region that is highly enriched for proline (P) and alanine (A), which is believed to be a linker region. For example, between ACP domains 1 and 2 is the sequence: APAPVKAAAPAAPVASAPAPA, represented herein as SEQ ID NO:15. The locations of the active site serine residues (i.e., the pantetheine binding site) for each of the nine ACP domains, with respect to the amino acid sequence of SEQ ID NO:2, are as follows: ACP1 = S₁₁₅₇; ACP2 = S₁₂₆₆; ACP3 = S₁₃₇₇; ACP4 = S₁₄₈₈; ACP5 = S₁₆₀₄; ACP6 = S₁₇₁₅; ACP7 = S₁₈₁₉; ACP8 = S₁₉₃₀; and ACP9 = S₂₀₃₄. Given that the average size of an ACP domain is about 85 amino acids, excluding the linker, and about 110 amino acids including the linker, with the active site serine being approximately in the center of the domain, one of skill in the art can readily determine the positions of each of the nine ACP domains in OrfA.

According to the present invention, a domain or protein having acyl carrier protein (ACP) biological activity (function) is characterized as being small polypeptides (typically, 80 to 100 amino acids long), that function as carriers for growing fatty acyl chains via a thioester linkage to a covalently bound co-factor of the protein. They occur as separate units or as domains within larger proteins. ACPs are converted from inactive apo-forms to functional holo-forms by transfer of the phosphopantetheinyl moeity of CoA to a highly conserved serine residue of the ACP. Acyl groups are attached to ACP by a thioester linkage at the free terminus of the phosphopantetheinyl moiety. ACPs can be identified by labeling with radioactive pantetheine and by sequence homology to known ACPs. The presence of variations of the above mentioned motif (LGIDS*) is also a signature of an ACP.

Domain 12 in OrfA is a KR domain, also referred to herein as ORFA-KR. This domain is contained within the nucleotide sequence spanning from a starting point of about position 6598 of SEQ ID NO:1 to an ending point of about position 8730 of SEQ ID NO:1. The nucleotide sequence containing the sequence encoding the ORFA-KR domain is represented herein as SEQ ID NO:17 (positions 6598-8730 of SEQ ID NO:1). The amino acid sequence containing the KR domain spans from a starting point of about position 2200 of SEQ ID NO:2 (ORFA) to an ending point of about position 2910 of SEQ ID NO:2. The amino acid sequence containing the ORFA-KR domain is represented herein as SEQ ID NO:18 (positions 2200-2910 of SEQ ID NO:2). Within the KR domain is a core region with homology to short chain aldehyde-dehydrogenases (KR is a member of this family). This core region spans from about position 7198 to about position 7500 of SEQ ID NO:1, which corresponds to amino acid positions 2400-2500 of SEQ ID NO:2.

According to the present invention, a domain or protein having ketoreductase activity, also referred to as 3-ketoacyl-ACP reductase (KR) biological activity (function), is characterized as one that catalyzes the pyridine-nucleotide-dependent reduction of 3-keto acyl forms of ACP. It is the first reductive step in the *de novo* fatty acid biosynthesis elongation cycle and a reaction often performed in polyketide biosynthesis. Significant sequence similarity is observed with one family of enoyl ACP reductases (ER), the other reductase of FAS (but not the ER family present in the PUFA PKS system), and the short-chain alcohol dehydrogenase family. Pfam analysis of the PUFA PKS region indicated above reveals the homology to the short-chain alcohol dehydrogenase family in the core region. Blast analysis of the same region reveals matches in the core area to known KR enzymes as well as an extended region of homology to domains from the other characterized PUFA PKS systems.

### Open Reading Frame B (OrfB):

The complete nucleotide sequence for OrfB is represented herein as SEQ ID NO:3. Nucleotides 1311-6177 of SEQ ID NO:3 correspond to nucleotides 1-4867 of the sequence denoted as SEQ ID NO:71 in U.S. Application Serial No. 09/231,899 (The cDNA sequence in U.S. Application Serial No. 09/231,899 contains about 345 additional nucleotides beyond the stop codon, including a polyA tail). Therefore, nucleotides 1-1310 of SEQ ID NO:1 represent additional sequence that was not disclosed in U.S. Application Serial No. 09/231,899. This novel region of SEQ ID NO:3 contains most of the KS domain encoded by OrfB.

OrfB is a 6177 nucleotide sequence (not including the stop codon) which encodes a 2059 amino acid sequence, represented herein as SEQ ID NO:4. Within OrfB are four domains: (a) one β-keto acyl-ACP synthase (KS) domain; (b) one chain length factor (CLF) domain; (c) one acyl transferase (AT) domain; and, (d) one enoyl ACP-reductase (ER) domain.

The nucleotide sequence for OrfB has been deposited with GenBank as Accession No. AF378328 (amino acid sequence Accession No. AAK728880). OrfB was compared with known sequences in a standard BLAST search as described above. At the nucleic acid level, OrfB has no significant homology to any known nucleotide sequence. At the amino acid level, the sequences with the greatest degree of homology to ORFB were: *Shewanella sp.* hypothetical protein (Accession No. U73935), which was 53% identical to ORFB over 458 amino acid residues; *Moritella marinus* (*Vibrio marinus*) ORF11 (Accession No. AB025342), which was 53% identical to ORFB over 460 amino acid residues; *Photobacterium profundum* omega-3 polyunsaturated fatty acid synthase PfaD (Accession No. AF409100), which was 52% identical to ORFB over 457 amino acid residues; and *Nostoc sp.* 7120 hypothetical protein (Accession No. NC_003272), which was 53% identical to ORFB over 430 amino acid residues.

The first domain in OrfB is a KS domain, also referred to herein as ORFB-KS. This domain is contained within the nucleotide sequence spanning from a starting point of between about positions 1 and 43 of SEQ ID NO:3 (OrfB) to an ending point of between about positions 1332 and 1350 of SEQ ID NO:3. The nucleotide sequence containing the sequence encoding the ORFB-KS domain is represented herein as SEQ ID NO:19 (positions 1-1350 of SEQ ID NO:3). The amino acid sequence containing the KS domain spans from a starting point of between about positions 1 and 15 of SEQ ID NO:4 (ORFB) to an ending point of between about positions 444 and 450 of SEQ ID NO:4. The amino acid sequence containing the ORFB-KS domain is represented herein as SEQ ID NO:20 (positions 1-450 of SEQ ID NO:4). It is noted that the ORFB-KS domain contains an active site motif: DXAC* (*acyl binding site C₁₉₆). KS biological activity and methods of identifying proteins or domains having such activity is described above.

The second domain in OrfB is a CLF domain, also referred to herein as ORFB-CLF. This domain is contained within the nucleotide sequence spanning from a starting point of between about positions 1378 and 1402 of SEQ ID NO:3 (OrfB) to an ending point of between about positions 2682 and 2700 of SEQ ID NO:3. The nucleotide sequence containing the sequence encoding the ORFB-CLF domain is represented herein as SEQ ID NO:21 (positions 1378-2700 of SEQ ID NO:3). The amino acid sequence containing the CLF domain spans from a starting point of between about positions 460 and 468 of SEQ ID NO:4 (ORFB) to an ending point of between about positions 894 and 900 of SEQ ID NO:4. The amino acid sequence containing the ORFB-CLF domain is represented herein as SEQ ID NO:22 (positions 460-900 of SEQ ID NO:4). It is noted that the ORFB-CLF domain contains a KS active site motif without the acyl-binding cysteine.

According to the present invention, a domain or protein is referred to as a chain length factor (CLF) based on the following rationale. The CLF was originally described as characteristic of Type II (dissociated enzymes) PKS systems and was hypothesized to play a role in determining the number of elongation cycles, and hence the chain length, of the end product. CLF amino acid sequences show homology to KS domains (and are thought to form heterodimers with a KS protein), but they lack the active site cysteine. CLF's role in PKS systems is currently controversial. New evidence (C. Bisang et al., *Nature* 401,502 (1999)) suggests a role in priming (providing the initial acyl group to be elongated) the PKS systems. In this role the CLF domain is thought to decarboxylate malonate (as malonyl-ACP), thus forming an acetate group that can be transferred to the KS active site. This acetate therefore acts as the 'priming' molecule that can undergo the initial elongation (condensation) reaction. Homologues of the Type II CLF have been identified as 'loading' domains in some modular PKS systems. A domain with the sequence features of the CLF is found in all currently identified PUFA PKS systems and in each case is found as part of a multidomain protein.

The third domain in OrfB is an AT domain, also referred to herein as ORFB-AT. This domain is contained within the nucleotide sequence spanning from a starting point of between about positions 2701 and 3598 of SEQ ID NO:3 (OrfB) to an ending point of between about positions 3975 and 4200 of SEQ ID NO:3. The nucleotide sequence containing the sequence encoding the ORFB-AT domain is represented herein as SEQ ID NO:23 (positions 2701-4200 of SEQ ID NO:3). The amino acid sequence containing the AT domain spans from a starting point of between about positions 901 and 1200 of SEQ ID NO:4 (ORFB) to an ending point of between about positions 1325 and 1400 of SEQ ID NO:4. The amino acid sequence containing the ORFB-AT domain is represented herein as SEQ ID NO:24 (positions 901-1400 of SEQ ID NO:4). It is noted that the ORFB-AT domain contains an active site motif of GxS*xG (*acyl binding site S₁₁₄₀) that is characteristic of acyltransferse (AT) proteins.

An "acyltransferase" or "AT" refers to a general class of enzymes that can carry out a number of distinct acyl transfer reactions. The *Schizochytrium* domain shows good homology to a domain present in all of the other PUFA PKS systems currently examined and very weak homology to some acyltransferases whose specific functions have been identified (e.g. to malonyl-CoA:ACP acyltransferase, MAT). In spite of the weak homology to MAT, this AT domain is not believed to function as a MAT because it does not possess an extended motif structure characteristic of such enzymes (see MAT domain description, above). For the purposes of this disclosure, the functions of the AT domain in a PUFA PKS system include, but are not limited to: transfer of the fatty acyl group from the ORFA ACP domain(s) to water (i.e. a thioesterase - releasing the fatty acyl group as a free fatty acid), transfer of a fatty acyl group to an acceptor such as CoA, transfer of the acyl group among the various ACP domains, or transfer of the fatty acyl group to a lipophilic acceptor molecule (e.g. to lysophosphadic acid).

The fourth domain in OrfB is an ER domain, also referred to herein as ORFB-ER. This domain is contained within the nucleotide sequence spanning from a starting point of about position 4648 of SEQ ID NO:3 (OrfB) to an ending point of about position 6177 of SEQ ID NO:3. The nucleotide sequence containing the sequence encoding the ORFB-ER domain is represented herein as SEQ ID NO:25 (positions 4648-6177 of SEQ ID NO:3). The amino acid sequence containing the ER domain spans from a starting point of about position 1550 of SEQ ID NO:4 (ORFB) to an ending point of about position 2059 of SEQ ID NO:4. The amino acid sequence containing the ORFB-ER domain is represented herein as SEQ ID NO:26 (positions 1550-2059 of SEQ ID NO:4).

We describe that this domain has enoyl reductase (ER) biological activity. The ER enzyme reduces the *trans*-double bond (introduced by the DH activity) in the fatty acyl-ACP, resulting in fully saturating those carbons. The ER domain in the PUFA-PKS shows homology to a newly characterized family of ER enzymes (Heath et al., *Nature* **406**, 145 (2000)). Heath and Rock identified this new class of ER enzymes by cloning a gene of interest from *Streptococcus pneumoniae,* purifying a protein expressed from that gene, and showing that it had ER activity in an *in vitro* assay. The sequence of the Schizochytrium ER domain of OrfB shows homology to the *S. pneumoniae* ER protein. All of the PUFA PKS systems currently examined contain at least one domain with very high sequence homology to the *Schizochytrium* ER domain. The *Schizochytrium* PUFA PKS system contains two ER domains (one on OrfB and one on OrfC).

### Open Reading Frame C (OrfC):

The complete nucleotide sequence for OrfC is represented herein as SEQ ID NO:5. Nucleotides 1-4509 of SEQ ID NO:5 (i.e., the entire open reading frame sequence, not including the stop codon) correspond to nucleotides 145-4653 of the sequence denoted as SEQ ID NO:76 in U.S. Application Serial No. 09/231,899 (The cDNA sequence in U.S. Application Serial No. 09/231,899 contains about 144 nucleotides upstream of the start codon for OrfC and about 110 nucleotides beyond the stop codon, including a polyA tail). OrfC is a 4509 nucleotide sequence (not including the stop codon) which encodes a 1503 amino acid sequence, represented herein as SEQ ID NO:6. Within OrfC are three domains: (a) two FabA-like β-hydroxy acyl-ACP dehydrase (DH) domains; and (b) one enoyl ACP-reductase (ER) domain.

The nucleotide sequence for OrfC has been deposited with GenBank as Accession No. AF378329 (amino acid sequence Accession No. AAK728881). OrfC was compared with known sequences in a standard BLAST search as described above. At the nucleic acid level, OrfC has no significant homology to any known nucleotide sequence. At the amino acid level (Blastp), the sequences with the greatest degree of homology to ORFC were: *Moritella marinus* (*Vibrio marinus*) ORF11 (Accession No. ABO25342), which is 45% identical to ORFC over 514 amino acid residues, *Shewanella sp.* hypothetical protein 8 (Accession No. U73935), which is 49% identical to ORFC over 447 amino acid residues, *Nostoc sp.* hypothetical protein (Accession No. NC_003272), which is 49% identical to ORFC over 430 amino acid residues, and *Shewanella sp.* hypothetical protein 7 (Accession No. U73935), which is 37% identical to ORFC over 930 amino acid residues.

The first domain in OrfC is a DH domain, also referred to herein as ORFC-DH1. This is one of two DH domains in OrfC, and therefore is designated DH1. This domain is contained within the nucleotide sequence spanning from a starting point of between about positions 1 and 778 of SEQ ID NO:5 (OrfC) to an ending point of between about positions 1233 and 1350 of SEQ ID NO:5. The nucleotide sequence containing the sequence encoding the ORFC-DH1 domain is represented herein as SEQ ID NO:27 (positions 1-1350 of SEQ ID NO:5). The amino acid sequence containing the DH1 domain spans from a starting point of between about positions 1 and 260 of SEQ ID NO:6 (ORFC) to an ending point of between about positions 411 and 450 of SEQ ID NO:6. The amino acid sequence containing the ORFC-DH1 domain is represented herein as SEQ ID NO:28 (positions 1-450 of SEQ ID NO:6).

The characteristics of both the DH domains (see below for DH 2) in the PUFA PKS systems have been described in the preceding sections. This class of enzyme removes HOH from a β-keto acyl-ACP and leaves a *trans* double bond in the carbon chain. The DH domains of the PUFA PKS systems show homology to bacterial DH enzymes associated with their FAS systems (rather than to the DH domains of other PKS systems). A subset of bacterial DH's, the FabA-like DH's, possesses *cis-trans* isomerase activity (Heath et al., J. Biol. Chem., 271, 27795 (1996)). It is the homologies to the FabA-like DH's that indicate that one or both of the DH domains is responsible for insertion of the cis double bonds in the PUFA PKS products.

The second domain in OrfC is a DH domain, also referred to herein as ORFC-DH2. This is the second of two DH domains in OrfC, and therefore is designated DH2. This domain is contained within the nucleotide sequence spanning from a starting point of between about positions 1351 and 2437 of SEQ ID NO:5 (OrfC) to an ending point of between about positions 2607 and 2850 of SEQ ID NO:5. The nucleotide sequence containing the sequence encoding the ORFC-DH2 domain is represented herein as SEQ ID NO:29 (positions 1351-2850 of SEQ ID NO:5). The amino acid sequence containing the DH2 domain spans from a starting point of between about positions 451 and 813 of SEQ ID NO:6 (ORFC) to an ending point of between about positions 869 and 950 of SEQIDNO:6. The amino acid sequence containing the ORFC-DH2 domain is represented herein as SEQ ID NO:30 (positions 451-950 of SEQ ID NO:6). DHbiological activity has been described above.

The third domain in OrfC is an ER domain, also referred to herein as ORFC-ER. This domain is contained within the nucleotide sequence spanning from a starting point of about position 2998 of SEQ ID NO:5 (OrfC) to an ending point of about position 4509 of SEQ ID NO:5. The nucleotide sequence containing the sequence encoding the ORFC-ER domain is represented herein as SEQ ID NO:31 (positions 2998-4509 of SEQ ID NO:5). The amino acid sequence containing the ER domain spans from a starting point of about position 1000 of SEQ ID NO:6 (ORFC) to an ending point of about position 1502 of SEQ ID NO:6. The amino acid sequence containing the ORFC-ER domain is represented herein as SEQ ID NO:32 (positions 1000-1502 of SEQ ID NO:6). ER biological activity has been described above.

One embodiment of the present invention relates to an isolated nucleic acid molecule comprising a nucleic acid sequence from a non-bacterial PUFA PKS system, a homologue thereof, a fragment thereof, and/or a nucleic acid sequence that is complementary to any of such nucleic acid sequences. In one aspect, the present invention relates to an isolated nucleic acid molecule as set out in claim 1.

According to the present invention, an amino acid sequence that has a biological activity of at least one domain of a PUFA PKS system is an amino acid sequence that has the biological activityof at least one domain of the PUFA PKS system described in detail herein, as exemplified by the *Schizochytrium* PUFA PKS system. The biological activities of the various domains within the *Schizochytrium* PUFA PKS system have been described in detail above. Therefore, an isolated nucleic acid molecule described herein can encode the translation product of any PUFA PKS open reading frame, PUFA PKS domain, biologically active fragment thereof, or any homologue of a naturally occurring PUFA PKS open reading frame or domain which has biological activity. A homologue of given protein or domain is a protein or polypeptide that has an amino acid sequence which differs from the naturally occurring reference amino acid sequence (i.e., of the reference protein or domain) in that at least one or a few, but not limited to one or a few, amino acids have been deleted (e.g., a truncated version of the protein, such as a peptide or fragment), inserted, inverted, substituted and/or derivatized (e.g., by glycosylation, phosphorylation, acetylation, myristoylation, prenylation, palmitation, amidation and/or addition of glycosylphosphatidyl inositol). Preferred homologues of a PUFA PKS protein or domain are described in detail below. It is noted thathomologues can include synthetically produced homologues, naturally occurring allelic variants of a given protein or domain, or homologous sequences from organisms other than the organism from which the reference sequence was derived.

In general, the biological activity or biological action of a protein or domain refers to any function(s) exhibited or performed by the protein or domain that is ascribed to the naturally occurring form of the protein or domain as measured or observed *in vivo* (i.e., in the natural physiological environment of the protein) or *in vitro* (i.e., under laboratory conditions). Biological activities of PUFA PKS systems and the individual proteins/domains that make up a PUFA PKS system have been described in detail elsewhere herein. Modifications of a protein or domain, such as in a homologue or mimetic (discussed below), may result in proteins or domains having the same biological activity as the naturally occurring protein or domain, or in proteins or domains having decreased or increased biological activity as compared to the naturally occurring protein or domain. Modifications which result in a decrease in expression or a decrease in the activity of the protein or domain, can be referred to as inactivation (complete or partial), down-regulation, or decreased action of a protein or domain. Similarly, modifications which result in an increase in expression or an increase in the activity of the protein or domain, can be referred to as amplification, overproduction, activation, enhancement, up-regulation or increased action of a protein or domain. A functional domain of a PUFA PKS system is a domain (i.e., a domain can be a portion of a protein) that is capable of performing a biological function (i.e., has biological activity).

In accordance with the present invention, an isolated nucleic acid molecule is a nucleic acid molecule that has been removed from its natural milieu (i.e., that has been subject to human manipulation), its natural milieu being the genome or chromosome in which the nucleic acid molecule is found in nature. As such, "isolated" does not necessarily reflect the extent to which the nucleic acid molecule has been purified, but indicates that the molecule does not include an entire genome or an entire chromosome in which the nucleic acid molecule is found in nature. An isolated nucleic acid molecule can include a gene. An isolated nucleic acid molecule that includes a gene is not a fragment of a chromosome that includes such gene, but rather includes the coding region and regulatory regions associated with the gene, but no additional genes naturally found on the same chromosome. An isolated nucleic acid molecule can also include a specified nucleic acid sequence flanked by (i.e., at the 5' and/or the 3' end of the sequence) additional nucleic acids that do not normally flank the specified nucleic acid sequence in nature (i.e., heterologous sequences). Isolated nucleic acid molecule can include DNA, RNA (e.g., mRNA), or derivatives of either DNA or RNA (e.g., cDNA). Although the phrase "nucleic acid molecule" primarily refers to the physical nucleic acid molecule and the phrase "nucleic acid sequence" primarily refers to the sequence of nucleotides on the nucleic acid molecule, the two phrases can be used interchangeably, especially with respect to a nucleic acid molecule, or a nucleic acid sequence, being capable of encoding a protein or domain of a protein.

Preferably, an isolated nucleic acid molecule of the present invention is produced using recombinant DNA technology (e.g., polymerase chain reaction (PCR) amplification, cloning) or chemical synthesis. Isolated nucleic acid molecules include natural nucleic acid molecules and homologues thereof, including, but not limited to, natural allelic variants and modified nucleic acid molecules in which nucleotides have been inserted, deleted, substituted, and/or inverted in such a manner that such modifications provide the desired effect on PUFA PKS system biological activity as described herein. Protein homologues (e.g., proteins encoded by nucleic acid homologues) have been discussed in detail above.

A nucleic acid molecule homologue can be produced using a number of methods known to those skilled in the art (see, for example, Sambrook et al., Molecular Cloning: A Laboratory Manual, Cold Spring Harbor Labs Press, 1989). For example, nucleic acid molecules can be modified using a variety of techniques including, but not limited to, classic mutagenesis techniques and recombinant DNA techniques, such as site-directed mutagenesis, chemical treatment of a nucleic acid molecule to induce mutations, restriction enzyme cleavage of a nucleic acid fragment, ligation of nucleic acid fragments, PCR amplification and/or mutagenesis of selected regions of a nucleic acid sequence, synthesis of oligonucleotide mixtures and ligation of mixture groups to "build" a mixture of nucleic acid molecules and combinations thereof. Nucleic acid molecule homologues can be selected from a mixture of modified nucleic acids by screening for the function of the protein encoded by the nucleic acid and/or by hybridization with a wild-type gene.

The minimum size of a nucleic acid molecule of the present invention is a size sufficient to form a probe or oligonucleotide primer that is capable of forming a stable hybrid (e.g., under moderate, high or very high stringency conditions) with the complementary sequence of a nucleic acid molecule useful in the present invention, or of a size sufficient to encode an amino acid sequence having a biological activity of at least one domain of a PUFA PKS system according to the present invention. As such, the size of the nucleic acid molecule encoding such a protein can be dependent on nucleic acid composition and percent homology or identity between the nucleic acid molecule and complementary sequence as well as upon hybridization conditions *per se* (e.g., temperature, salt concentration, and formamide concentration). The minimal size of a nucleic acid molecule that is used as an oligonucleotide primer or as a probe is typically at least about 12 to about 15 nucleotides in length if the nucleic acid molecules are GC-rich and at least about 15 to about 18 bases in length if they are AT-rich. There is no limit, other than a practical limit, on the maximal size of a nucleic acid molecule of the present invention, in that the nucleic acid molecule can include a sequence sufficient to encode a biologically active fragment of a domain of a PUFA PKS system, an entire domain of a PUFA PKS system, several domains within an open reading frame (Orf) of a PUFA PKS system, an entire Orf of a PUFA PKS system, or more than one Orf of a PUFA PKS system.

In one embodiment of the present invention, an isolated nucleic acid molecule comprises or consists essentially of a nucleic acid sequence selected from the group of: SEQ ID NO:2, SEQ ID NO:8, SEQ ID NO:10, optionally SEQ ID NO:18, or biologically active fragments thereof. In one aspect, the nucleic acid sequence is selected from the group of SEQ ID NO:1.

In one embodiment of the present invention, any of the above-described PUFA PKS amino acid sequences, as well as homologues of such sequences, can be produced with from at least one, and up to about 20, additional heterologous amino acids flanking each of the C- and/or N-terminal end of the given amino acid sequence. The resulting protein or polypeptide can be referred to as "consisting essentially of" a given amino acid sequence. According to the present invention, the heterologous amino acids are a sequence of amino acids that are not naturally found (i.e., not found in nature, *in vivo*) flanking the given amino acid sequence or which would not be encoded by the nucleotides that flank the naturally occurring nucleic acid sequence encoding the given amino acid sequence as it occurs in the gene, if such nucleotides in the naturally occurring sequence were translated using standard codon usage for the organism from which the given amino acid sequence is derived. Similarly, the phrase "consisting essentially of", when used with reference to a nucleic acid sequence herein, refers to a nucleic acid sequence encoding a given amino acid sequence that can be flanked by from at least one, and up to as many as about 60, additional heterologous nucleotides at each of the 5' and/or the 3' end of the nucleic acid sequence encoding the given amino acid sequence. The heterologous nucleotides are not naturally found (i.e., not found in nature, *in vivo*) flanking the nucleic acid sequence encoding the given amino acid sequence as it occurs in the natural gene.

The present invention also includes an isolated nucleic acid molecule comprising a nucleic acid sequence encoding an amino acid sequence having a biological activity of at least one domain of a PUFA PKS system. In one aspect, such a nucleic acid sequence encodes a homologue of any of the *Schizochytrium* PUFA PKS ORFs or domains, including: SEQ ID NO:2, SEQ ID NO:8 or SEQ ID NO:10, optionally SEQ ID NO: 18, wherein the homologue has a biological activity of at least one domain of a PUFA PKS system as described previously herein.

In one aspect of the invention, a homologue of a *Schizochytrium* PUFA PKS protein or domain encompassed by the present invention comprises an amino acid sequence that is at least 60% identical to at least 500 consecutive amino acids of an amino acid sequence chosen from: SEQ ID NO:2, wherein said amino acid sequence has a biological activity of at least one domain of a PUFA PKS system. In a further aspect, the amino acid sequence of the homologue is at least 60% identical to at least 600 consecutive amino acids, and more preferably to at least 700 consecutive amino acids, and more preferably to at least 800 consecutive amino acids, and more preferably to at least 900 consecutive amino acids, and more preferably to at least 1000 consecutive amino acids, and more preferably to at least 1100 consecutive amino acids, andmorepreferablyto at least 1200 consecutive amino acids, and more preferably to at least 1300 consecutive amino acids, and more preferably to at least 1400 consecutive amino acids, and more preferably to at least 1500 consecutive amino acids of SEQ ID NO:2. In a further aspect, the amino acid sequence of the homologue is at least 60% identical to at least 1600 consecutive amino acids, and more preferably to at least 1700 consecutive amino acids, and more preferably to at least 1800 consecutive amino acids, and more preferably to at least 1900 consecutive amino acids, and more preferably to at least 2000 consecutive amino acids of any of SEQ ID NO:2.In a further aspect, the amino acid sequence of the homologue is at least 60% identical to at least 2100 consecutive amino acids, andmorepreferablyto at least 2200 consecutive amino acids, and more preferably to at least 2300 consecutive amino acids, and more preferably to at least 2400 consecutive amino acids, and more preferably to at least 2500 consecutive amino acids, and more preferably to at least 2600 consecutive amino acids, and more preferably to at least 2700 consecutive amino acids, and more preferably to at least 2800 consecutive amino acids, and even more preferably, to the full length of SEQ ID NO:2.

In another aspect, a homologue of a *Schizochytrium* PUFA PKS protein or domain encompassed by the present invention comprises an amino acid sequence that is atleast 65% identical, and more preferably at least 70% identical, and more preferably at least 75% identical, and more preferably at least 80% identical, and more preferably at least 85% identical, and more preferably at least 90% identical, and more preferably at least 95% identical, and more preferably at least 96% identical, and more preferably at least 97% identical, and more preferably at least 98% identical, and more preferably at least 99% identical to an amino acid sequence chosen from: SEQ ID NO:2, over any of the consecutive amino acid lengths described in the paragraph above, wherein the amino acid sequence has a biological activity of at least one domain of a PUFA PKS system.

In one aspect of the invention, a homologue of a *Schizochytrium* PUFA PKS protein or domain encompassed by the present invention comprises an amino acid sequence that is at least 60% identical to an amino acid sequence chosen from: SEO ID NO:8 or SEQ ID NO:10, and optionally SEQ ID NO:18, wherein said amino acid sequence has abiological activity of at least one domain of a PUFA PKS system. In a further aspect, the amino acid sequence of the homologue is at least 65% identical, and more preferably at least 70% identical, and more preferably at least 75% identical, and more preferably at least 80% identical, and more preferably at least 85% identical, and more preferably at least 90% identical, and more preferably at least 95% identical, andmorepreferably at least 96% identical, and more preferably at least 97% identical, and more preferably at least 98% identical, and more preferably at least 99% identical to an amino acid sequence chosen from: SEQ ID NO:8 or SEQ ID NO: 10, and optionally SEQ ID NO: 18, wherein the amino acid sequence has a biological activity of at least one domain of a PUFA PKS system.

According to the present invention, the term "contiguous" or "consecutive", with regard to nucleic acid or amino acid sequences described herein, means to be connected in an unbroken sequence. For example, for a first sequence to comprise 30 contiguous (or consecutive) amino acids of a second sequence, means that the first sequence includes an unbroken sequence of 30 amino acid residues that is 100% identical to an unbroken sequence of 30 amino acid residues in the second sequence. Similarly, for a first sequence to have "100% identity" with a second sequence means that the first sequence exactly matches the second sequence with no gaps between nucleotides or amino acids.

As used herein, unless otherwise specified, reference to a percent (%) identity refers to an evaluation of homology which is performed using: (1) a BLAST 2.0 Basic BLAST homology search using blastp for amino acid searches, blastn for nucleic acid searches, and blastX for nucleic acid searches and searches of translated amino acids in all 6 open reading frames, all with standard default parameters, wherein the query sequence is filtered for low complexity regions by default (described in Altschul, S.F., Madden, T.L., Schääffer, A.A., Zhang, J., Zhang, Z., Miller, W. & Lipman, D.J. (1997) "Gapped BLAST and PSI-BLAST: a new generation of protein database search programs." Nucleic Acids Res. 25:3389-3402 ); (2) a BLAST 2 alignment (using the parameters described below); (3) and/or PSI-BLAST with the standard default parameters (Position-Specific Iterated BLAST). It is noted that due to some differences in the standard parameters between BLAST 2.0 Basic BLAST and BLAST 2, two specific sequences might be recognized as having significant homology using the BLAST 2 program, whereas a search performed in BLAST 2.0 Basic BLAST using one of the sequences as the query sequence may not identify the second sequence in the top matches. In addition, PSI-BLAST provides an automated, easy-to-use version of a "profile" search, which is a sensitive way to look for sequence homologues. The program first performs a gapped BLAST database search. The PSI-BLAST program uses the information from any significant alignments returned to construct a position-specific score matrix, which replaces the query sequence for the next round of database searching. Therefore, it is to be understood that percent identity can be determined by using any one of these programs.

Two specific sequences can be aligned to one another using BLAST 2 sequence as described in Tatusova and Madden, (1999), "Blast 2 sequences - a new tool for comparing protein and nucleotide sequences", FEMS Microbiol Lett. 174:247-250. BLAST 2 sequence alignment is performed in blastp or blastn using the BLAST 2.0 algorithm to perform a Gapped BLAST search (BLAST 2.0) between the two sequences allowing for the introduction of gaps (deletions and insertions) in the resulting alignment. For purposes of clarity herein, a BLAST 2 sequence alignment is performed using the standard default parameters as follows.

For blastn, using 0 BLOSUM62 matrix:
Reward for match = 1
Penalty for mismatch = -2
Open gap (5) and extension gap (2) penalties
gap x_dropoff (50) expect (10) word size (11) filter (on)

For blastp, using 0 BLOSUM62 matrix:
Open gap (11) and extension gap (1) penalties
gap x_dropoff (50) expect (10) word size (3) filter (on).

In another embodiment of the invention, an amino acid sequence having the biological activity of at least one domain of a PUFA PKS system of the present invention includes an amino acid sequence that is sufficiently similar to a naturally occurring PUFA PKS protein or polypeptide that a nucleic acid sequence encoding the amino acid sequence is capable of hybridizing under moderate, high, or very high stringency conditions (described below) to (i.e., with) a nucleic acid molecule encoding the naturally occurring PUFA PKS protein or polypeptide (i.e., to the complement of the nucleic acid strand encoding the naturally occurring PUFA PKS protein orpolypeptide). An amino acid sequence having the biological activity of at least one domain of a PUFA PKS system of the present invention is encoded by a nucleic acid sequence that hybridizes under moderate, high or very high stringency conditions to the complement of a nucleic acid sequence that encodes a protein comprising an amino acid sequence represented by SEQ ID NO:2.

Methods to deduce a complementary sequence are known to those skilled in the art. It should be noted that since amino acid sequencing and nucleic acid sequencing technologies are not entirely error-free, the sequences presented herein, at best, represent apparent sequences of PUFA PKS domains and proteins of the present invention.

As used herein, hybridization conditions refer to standard hybridization conditions under which nucleic acid molecules are used to identify similar nucleic acid molecules. Such standard conditions are disclosed, for example, in Sambrook et al., Molecular Cloning: A Laboratory Manual, Cold Spring Harbor Labs Press, 1989. Sambrook et al., *ibid*., is incorporated by reference herein in its entirety (see specifically, pages 9.31-9.62). In addition, formulae to calculate the appropriate hybridization and wash conditions to achieve hybridization permitting varying degrees of mismatch of nucleotides are disclosed, for example, in Meinkoth et al., 1984, Anal. Biochem. 138, 267-284.

More particularly, moderate stringency hybridization and washing conditions, as referred to herein, refer to conditions which permit isolation of nucleic acid molecules having at least about 70% nucleic acid sequence identity with the nucleic acid molecule being used to probe in the hybridization reaction (i.e., conditions permitting about 30% or less mismatch of nucleotides). High stringency hybridization and washing conditions, as referred to herein, refer to conditions which permit isolation of nucleic acid molecules having at least about 80% nucleic acid sequence identity with the nucleic acid molecule being used to probe in the hybridization reaction (i.e., conditions permitting about 20% or less mismatch of nucleotides). Very high stringency hybridization and washing conditions, as referred to herein, refer to conditions which permit isolation of nucleic acid molecules having at least about 90% nucleic acid sequence identity with the nucleic acid molecule being used to probe in the hybridization reaction (i.e., conditions permitting about 10% or less mismatch of nucleotides). As discussed above, one of skill in the art can use the formulae in Meinkoth et al., *ibid.* to calculate the appropriate hybridization and wash conditions to achieve these particular levels of nucleotide mismatch. Such conditions will vary, depending on whether DNA:RNA or DNA:DNA hybrids are being formed. Calculated melting temperatures for DNA:DNA hybrids are 10°C less than for DNA:RNA hybrids. In particular embodiments, stringent hybridization conditions for DNA:DNA hybrids include hybridization at an ionic strength of 6X SSC (0.9 M Na⁺) at a temperature of between about 20°C and about 35 °C (lower stringency), more preferably, between about 28 °C and about 40 °C (more stringent), and even more preferably, between about 35°C and about 45 °C (even more stringent), with appropriate wash conditions. In particular embodiments, stringent hybridization conditions for DNA:RNA hybrids include hybridization at an ionic strength of 6X SSC (0.9 M Na⁺) at a temperature of between about 30°C and about 45°C, more preferably, between about 38°C and about 50°C, and even more preferably, between about 45°C and about 55°C, with similarly stringent wash conditions. These values are based on calculations of a melting temperature for molecules larger than about 100 nucleotides, 0% formamide and a G + C content of about 40%. Alternatively, Tₘ can be calculated empirically as set forth in Sambrook et al., *supra*, pages 9.31 to 9.62. In general, the wash conditions should be as stringent as possible, and should be appropriate for the chosen hybridization conditions. For example, hybridization conditions can include a combination of salt and temperature conditions that are approximately 20-25°C below the calculated Tₘ of a particular hybrid, and wash conditions typically include a combination of salt and temperature conditions that are approximately 12-20 °C below the calculated Tₘ of the particular hybrid. One example of hybridization conditions suitable for use with DNA:DNA hybrids includes a 2-24 hour hybridization in 6X SSC (50% formamide) at about 42°C, followed by washing steps that include one or more washes at room temperature in about 2X SSC, followed by additional washes at higher temperatures and lower ionic strength (e.g., at least one wash as about 37°C in about 0.1X-0.5X SSC, followed by at least one wash at about 68°C in about 0.1X-0.5X SSC).

Another embodiment of the present invention includes a recombinant nucleic acid molecule comprising a recombinant vector and a nucleic acid molecule comprising a nucleic acid sequence encoding an amino acid sequence having a biological activity of at least one domain of a PUFA PKS system in accordance with the invention. Such nucleic acid sequences are described in detail above. According to the present invention, a recombinant vector is an engineered (i.e., artificially produced) nucleic acid molecule that is used as a tool for manipulating a nucleic acid sequence of choice and for introducing such a nucleic acid sequence into a host cell. The recombinant vector is therefore suitable for use in cloning, sequencing, and/or otherwise manipulating the nucleic acid sequence of choice, such as by expressing and/or delivering the nucleic acid sequence of choice into a host cell to form a recombinant cell. Such a vector typically contains heterologous nucleic acid sequences, that is nucleic acid sequences that are not naturally found adjacent to nucleic acid sequence to be cloned or delivered, although the vector can also contain regulatory nucleic acid sequences (e.g., promoters, untranslated regions) which are naturally found adjacent to nucleic acid molecules of the present invention or which are useful for expression of the nucleic acid molecules of the present invention (discussed in detail below). The vector can be either RNA or DNA, either prokaryotic or eukaryotic, and typically is a plasmid. The vector can be maintained as an extrachromosomal element (e.g., a plasmid) or it can be integrated into the chromosome of a recombinant organism (e.g., a microbe or a plant). The entire vector can remain in place within a host cell, or under certain conditions, the plasmid DNA can be deleted, leaving behind the nucleic acid molecule of the present invention. The integrated nucleic acid molecule can be under chromosomal promoter control, under native or plasmid promoter control, or under a combination of several promoter controls. Single or multiple copies of the nucleic acid molecule can be integrated into the chromosome. A recombinant vector of the present invention can contain at least one selectable marker.

In one embodiment, a recombinant vector used in a recombinant nucleic acid molecule of the present invention is an expression vector. As used herein, the phrase "expression vector" is used to refer to a vector that is suitable for production of an encoded product (e.g., a protein of interest). In this embodiment, a nucleic acid sequence encoding the product to be produced (e.g., a PUFA PKS domain) is inserted into the recombinant vector to produce a recombinant nucleic acid molecule. The nucleic acid sequence encoding the protein to be produced is inserted into the vector in a manner that operatively links the nucleic acid sequence to regulatory sequences in the vector which enable the transcription and translation of the nucleic acid sequence within the recombinant host cell.

In another embodiment, a recombinant vector used in a recombinant nucleic acid molecule of the present invention is a targeting vector. As used herein, the phrase "targeting vector" is used to refer to a vector that is used to deliver a particular nucleic acid molecule into a recombinant host cell, wherein the nucleic acid molecule is used to delete or inactivate an endogenous gene within the host cell or microorganism (i.e., used for targeted gene disruption or knock-out technology). Such a vector may also be known in the art as a "knock-out" vector. In one aspect of this embodiment, a portion of the vector, but more typically, the nucleic acid molecule inserted into the vector (i.e., the insert), has a nucleic acid sequence that is homologous to a nucleic acid sequence of a target gene in the host cell (i.e., a gene which is targeted to be deleted or inactivated). The nucleic acid sequence of the vector insert is designed to bind to the target gene such that the target gene and the insert undergo homologous recombination, whereby the endogenous target gene is deleted, inactivated or attenuated (i.e., by at least a portion of the endogenous target gene being mutated or deleted).

Typically, a recombinant nucleic acid molecule includes at least one nucleic acid molecule of the present invention operatively linked to one or more transcription control sequences. As used herein, the phrase "recombinant molecule" or "recombinant nucleic acid molecule" primarily refers to a nucleic acid molecule or nucleic acid sequence operatively linked to a transcription control sequence, but can be used interchangeably with the phrase "nucleic acid molecule", when such nucleic acid molecule is a recombinant molecule as discussed herein. According to the present invention, the phrase "operatively linked" refers to linking a nucleic acid molecule to a transcription control sequence in a manner such that the molecule is able to be expressed when transfected (i.e., transformed, transduced, transfected, conjugated or conduced) into a host cell. Transcription control sequences are sequences which control the initiation, elongation, or termination of transcription. Particularly important transcription control sequences are those which control transcription initiation, such as promoter, enhancer, operator and repressor sequences. Suitable transcription control sequences include any transcription control sequence that can function in a host cell or organism into which the recombinant nucleic acid molecule is to be introduced.

Recombinant nucleic acid molecules of the present invention can also contain additional regulatory sequences, such as translation regulatory sequences, origins of replication, and other regulatory sequences that are compatible with the recombinant cell. In one embodiment, a recombinant molecule of the present invention, including those which are integrated into the host cell chromosome, also contains secretory signals (i.e., signal segment nucleic acid sequences) to enable an expressed protein to be secreted from the cell that produces the protein. Suitable signal segments include a signal segment that is naturally associated with the protein to be expressed or any heterologous signal segment capable of directing the secretion of the protein according to the present invention. In another embodiment, a recombinant molecule of the present invention comprises a leader sequence to enable an expressed protein to be delivered to and inserted into the membrane of a host cell. Suitable leader sequences include a leader sequence that is naturally associated with the protein, or any heterologous leader sequence capable of directing the delivery and insertion of the protein to the membrane of a cell.

The present inventors have found that the *Schizochytrium* PUFA PKS Orfs A and B are closely linked in the genome and region between the Orfs has been sequenced. The Orfs are oriented in opposite directions and 4244 base pairs separate the start (ATG) codons (i.e. they are arranged as follows: 3'OrfA5' - 4244 bp - 5'OrfB3'). Examination of the 4244 bp intergenic region did not reveal any obvious Orfs (no significant matches were found on a BlastX search). Both Orfs A and B are highly expressed in *Schizochytrium,* at least during the time of oil production, implying that active promoter elements are embedded in this intergenic region. These genetic elements are believed to have utility as a bi-directional promoter sequence for transgenic applications. For example, in a preferred embodiment, one could clone this region, place any genes of interest at each end and introduce the construct into *Schizochytrium* (or some other host in which the promoters can be shown to function). It is predicted that the regulatory elements, under the appropriate conditions, would provide for coordinated, high level expression of the two introduced genes. The complete nucleotide sequence for the regulatory region containing *Schizochytrium* PUFA PKS regulatory elements (e.g., a promoter) is represented herein as SEQ ID NO:36.

In a similar manner, OrfC is highly expressed in *Schizochytrium* during the time of oil production and regulatory elements are expected to reside in the region upstream of its start codon. A region of genomic DNA upstream of OrfC has been cloned and sequenced and is represented herein as (SEQ ID NO:37). This sequence contains the 3886 nt immediately upstream of the OrfC start codon. Examination of this region did not reveal any obvious Orfs (i.e., no significant matches were found on a BlastX search). It is believed that regulatory elements contained in this region, under the appropriate conditions, will provide for high-level expression of a gene placed behind them. Additionally, under the appropriate conditions, the level of expression may be coordinated with genes under control of the A-B intergenic region (SEQ ID NO:36).

Therefore, in one embodiment, a recombinant nucleic acid molecule useful in the present invention, as disclosed herein, can include a PUFA PKS regulatory region contained within SEQ ID NO:36 and/or SEQ ID NO:37. Such a regulatory region can include any portion (fragment) of SEQ ID NO:36 and/or SEQ ID NO:37 that has at least basal PUFA PKS transcriptional activity.

One or more recombinant molecules of the present invention can be used to produce an encoded product (e.g., a PUFA PKS domain, protein, or system) of the present invention. In one embodiment, an encoded product is produced by expressing a nucleic acid molecule as described herein under conditions effective to produce the protein. A preferred method to produce an encoded protein is by transfecting a host cell with one or more recombinant molecules to form a recombinant cell. Suitable host cells to transfect include, but are not limited to, any bacterial, fungal (e.g., yeast), insect, plant or animal cell that can be transfected. Host cells can be either untransfected cells or cells that are already transfected with at least one other recombinant nucleic acid molecule.

According to the present invention, the term "transfection" is used to refer to any method by which an exogenous nucleic acid molecule (i.e., a recombinant nucleic acid molecule) can be inserted into a cell. The term "transformation" can be used interchangeably with the term "transfection" when such term is used to refer to the introduction of nucleic acid molecules into microbial cells, such as algae, bacteria and yeast. In microbial systems, the term "transformation" is used to describe an inherited change due to the acquisition of exogenous nucleic acids by the microorganism and is essentially synonymous with the term "transfection". However, in animal cells, transformation has acquired a second meaning which can refer to changes in the growth properties of cells in culture after they become cancerous, for example. Therefore, to avoid confusion, the term "transfection" is preferably used with regard to the introduction of exogenous nucleic acids into animal cells, and the term "transfection" will be used herein to generally encompass transfection of animal cells, plant cells and transformation of microbial cells, to the extent that the terms pertain to the introduction of exogenous nucleic acids into a cell. Therefore, transfection techniques include, but are not limited to, transformation, particle bombardment, electroporation, microinjection, lipofection, adsorption, infection and protoplast fusion.

It will be appreciated by one skilled in the art that use of recombinant DNA technologies can improve control of expression of transfected nucleic acid molecules by manipulating, for example, the number of copies of the nucleic acid molecules within the host cell, the efficiency with which those nucleic acid molecules are transcribed, the efficiency with which the resultant transcripts are translated, and the efficiency of post-translational modifications. Additionally, the promoter sequence might be genetically engineered to improve the level of expression as compared to the native promoter. Recombinant techniques useful for controlling the expression of nucleic acid molecules include, but are not limited to, integration of the nucleic acid molecules into one or more host cell chromosomes, addition of vector stability sequences to plasmids, substitutions or modifications of transcription control signals (e.g., promoters, operators, enhancers), substitutions or modifications of translational control signals (e.g., ribosome binding sites, Shine-Dalgarno sequences), modification of nucleic acid molecules to correspond to the codon usage of the host cell, and deletion of sequences that destabilize transcripts.

General discussion above with regard to recombinant nucleic acid molecules and transfection of host cells is intended to be applied to any recombinant nucleic acid molecule discussed herein, including those encoding any amino acid sequence having a biological activity of at least one domain from a PUFA PKS, those encoding amino acid sequences from other PKS systems, and those encoding other proteins or domains.

We describe the use of a novel method to identify a microorganism that has a PUFA PKS system that is homologous in structure, domain organization and/or function to a *Schizochytrium* PUFA PKS system. The microorganism may be a non-bacterial microorganism, and the microorganism identified by this method may be a eukaryotic microorganism. In addition, we describe the microorganisms identified by such method and the invention relates to the use of these microorganisms and the PUFA PKS systems from these microorganisms in the various applications for a PUFA PKS system (e.g., genetically modified organisms and methods of producing bioactive molecules). The unique screening method described and demonstrated herein enables the rapid identification of new microbial strains containing a PUFA PKS system homologous to the *Schizochytrium* PUFA PKS system of the present invention. Applicants have used this method to discover and disclose herein that a *Thraustochytrium* microorganism contains a PUFA PKS system that is homologous to that found in *Schizochytrium.* This discovery is described in detail in Example 2 below.

Microbial organisms with a PUFA PKS system similar to that found in *Schizochytrium,* such as the *Thraustochytrium* microorganism discovered by the present inventors and described in Example 2, can be readily identified/isolated/screened by the following methods used separately or in any combination of these methods.

In general, the method to identify a non-bacterial microorganism that has a polyunsaturated fatty acid (PUFA) polyketide synthase (PKS) system includes a first step of (a) selecting a microorganism that produces at least one PUFA; and a second step of (b) identifying a microorganism from (a) that has an ability to produce increased PUFAs under dissolved oxygen conditions of less than about 5% of saturation in the fermentation medium, as compared to production of PUFAs by said microorganism under dissolved oxygen conditions of greater than 5% of saturation, more preferably 10% of saturation, more preferably greater than 15% of saturation and more preferably greater than 20% of saturation in the fermentation medium. A microorganism that produces at least one PUFA and has an ability to produce increased PUFAs under dissolved oxygen conditions of less than about 5% of saturation is identified as a candidate for containing a PUFA PKS system. Subsequent to identifying a microorganism that is a strong candidate for containing a PUFA PKS system, the method can include an additional step (c) of detecting whether the organism identified in step (b) comprises a PUFA PKS system.

Step (b) may be performed by culturing the microorganism selected for the screening process in low oxygen/anoxic conditions and aerobic conditions, and, in addition to measuring PUFA content in the organism, the fatty acid profile is determined, as well as fat content. By comparing the results under low oxygen/anoxic conditions with the results under aerobic conditions, the method provides a strong indication of whether the test microorganism contains a PUFA PKS system of the present invention. This embodiment is described in detail below.

Initially, microbial strains to be examined for the presence of a PUFA PKS system are cultured under aerobic conditions to induce production of a large number of cells (microbial biomass). As one element of the identification process, these cells are then placed under low oxygen or anoxic culture conditions (e.g., dissolved oxygen less than about 5% of saturation, more preferably less than about 2%, even more preferably less than about 1%, and most preferably dissolved oxygen of about 0% of saturation in the culture medium) and allowed to grow for approximately another 24-72 hours. In this process, the microorganisms should be cultured at a temperature greater than about 15°C, and more preferably greater than about 20°C, and even more preferably greater than about 25°C, and even more preferably greater than 30°C. The low or anoxic culture environment can be easily maintained in culture chambers capable of inducing this type of atmospheric environment in the chamber (and thus in the cultures) or by culturing the cells in a manner that induces the low oxygen environment directly in the culture flask/vessel itself.

In a culturing method, the microbes can be cultured in shake flasks which, instead of normally containing a small amount of culture medium -- less than about 50% of total capacity and usually less than about 25% of total capacity - to keep the medium aerated as it is shaken on a shaker table, are instead filled to greater than about 50% of their capacity, and more preferably greater than about 60%, and most preferably greater than about 75% of their capacity with culture medium. High loading of the shake flask with culture medium prevents it from mixing very well in the flask when it is placed on a shaker table, preventing oxygen diffusion into the culture. Therefore as the microbes grow, they use up the existing oxygen in the medium and naturally create a low or no oxygen environment in the shake flask.

After the culture period, the cells are harvested and analyzed for content of bioactive compounds of interest (e.g., lipids), but most particularly, for compounds containing two or more unsaturated bonds, and more preferably three or more double bonds, and even more preferably four or more double bonds. For lipids, those strains possessing such compounds at greater than about 5%, and more preferably greater than about 10%, and more preferably greater than about 15%, and even more preferably greater than about 20% of the dry weight of the microorganism are identified as predictably containing a novel PKS system of the type described above. For other bioactive compounds, such as antibiotics or compounds that are synthesized in smaller amounts, those strains possessing such compounds at greater than about 0.5%, and more preferably greater than about 0.1%, and more preferably greater than about 0.25%, and more preferably greater than about 0.5%, and more preferably greater than about 0.75%, and more preferably greater than about 1%, and more preferably greater than about 2.5%, and more preferably greater than about 5% of the dry weight of the microorganism are identified as predictably containing a novel PKS system of the type described above.

Alternatively, or in conjunction with this method, prospective microbial strains containing novel PUFA PKS systems as described herein can be identified by examining the fatty acid profile of the strain (obtained by culturing the organism or through published or other readily available sources). If the microbe contains greater than about 30%, and more preferably greater than about 40%, and more preferably greater than about 45%, and even more preferably greater than about 50% of its total fatty acids as C14:0, C16:0 and/or C16:1, while also producing at least one long chain fatty acid with three or more unsaturated bonds, and more preferably 4 or more double bonds, and more preferably 5 or more double bonds, and even more preferably 6 or more double bonds, then this microbial strain is identified as a likely candidate to possess a novel PUFA PKS system of the type described in this invention. Screening this organism under the low oxygen conditions described above, and confirming production of bioactive molecules containing two or more unsaturated bonds would suggest the existence of a novel PUFA PKS system in the organism, which could be further confirmed by analysis of the microbes' genome.

The success of this method can also be enhanced by screening eukaryotic strains that are known to contain C17:0 and or C17:1 fatty acids (in conjunction with the large percentages of C14:0, C16:0 and C16:1 fatty acids described above)-because the C17:0 and C17:1 fatty acids are potential markers for a bacterial (prokaryotic) based or influenced fatty acidproduction system. Another marker for identifying strains containing novel PUFA PKS systems is the production of simple fatty acid profiles by the organism. According to the present invention, a "simple fatty acid profile" is defined as 8 or fewer fatty acids being produced by the strain at levels greater than 10% of total fatty acids.

Use of any of these methods or markers (singly or preferably in combination) would enable one of skill in the art to readily identify microbial strains that are highly predicted to contain a novel PUFA PKS system of the type described in this invention.

Combining many of the methods and markers described above, a novel biorational screen (using shake flask cultures) has been developed for detectingmicroorganisms containing PUFA producing PKS systems. This screening system is conducted as follows:

A portion of a culture of the strain/microorganism to be tested is placed in 250 mL baffled shake flask with 50 mL culture media (aerobic treatment), and another portion of culture of the same strain is placed in a 250 mL non-baffled shake flask with 200 mL culture medium (anoxic/low oxygen treatment). Various culture media can be employed depending on the type and strain of microorganism being evaluated. Both flasks are placed on a shaker table at 200 rpm. After 48-72 hr of culture time, the cultures are harvested by centrifugation and the cells are analyzed for fatty acid methyl ester content via gas chromatography to determine the following data for each culture: (1) fatty acid profile; (2) PUFA content; and (3) fat content (approximated as amount total fatty acids/cell dry weight).

These data are then analyzed asking the following five questions (Yes/No):
Comparing the data from the low O₂/anoxic flask with the data from the aerobic flask:
   (1) Did the DHA (or other PUFA content) (as % FAME (fatty acid methyl esters)) stay about the same or preferably increased in the low oxygen culture compared to the aerobic culture?
   (2) Is C14:0 + C16:0 + C16:1 greater than about 40% TFA in the anoxic culture?
   (3) Are there very little (<1% as FAME) or no precursors (C18:3n-3 + C18:2n-6+C18:3n-6) to the conventional oxygen dependent elongase/desaturase pathway in the anoxic culture?
   (4) Did fat content (as amount total fatty acids/cell dry weight) increase in the low oxygen culture compared to the aerobic culture?
   (5) Did DHA (or other PUFA content) increase as % cell dry weight in the low oxygen culture compared to the aerobic culture?

If the first three questions are answered yes, this is a good indication that the strain contains a PKS genetic system for making long chain PUFAs. The more questions that are answered yes (preferably the first three questions must be answered yes), the stronger the indication that the strain contains such a PKS genetic system. If all five questions are answered yes, then there is a very strong indication that the strain contains a PKS genetic system for making long chain PUFAs. The lack of 18:3n-3/18:2n-6/18:3n-6 would indicate that the low oxygen conditions would have turned off or inhibited the conventional pathway for PUFA synthesis. A high 14:0/16:0/16:1 fatty is an preliminary indicator of a bacterially influenced fatty acid synthesis profile (the presence of C17:0 and 17:1 is also and indicator of this) and of a simple fatty acid profile. The increased PUFA synthesis and PUFA containing fat synthesis under the low oxygen conditions is directly indicative of a PUFA PKS system, since this system does not require oxygen to make highly unsaturated fatty acids.

Finally, in the identification method, once a strong candidate is identified, the microbe is preferably screened to detect whether or not the microbe contains a PUFA PKS system. For example, the genome of the microbe can be screened to detect the presence of one or more nucleic acid sequences that encode a domain of a PUFA PKS system as described herein. Preferably, this step of detection includes a suitable nucleic acid detection method, such as hybridization, amplification and or sequencing of one or more nucleic acid sequences in the microbe of interest. The probes and/or primers used in the detection methods can be derived from any known PUFA PKS system, including the marine bacteria PUFA PKS systems described in U.S. Patent No. 6,140,486, or the Thraustochytrid PUFA PKS systems described in U.S. Application Serial No. 09/231,899 and herein. Once novel PUFA PKS systems are identified, the genetic material from these systems can also be used to detect additional novel PUFA PKS systems. Methods ofhybridization, amplification and sequencing of nucleic acids for the purpose of identification and detection of a sequence are well known in the art. Using these detection methods, sequence homology and domain structure (e.g., the presence, number and/or arrangement of various PUFA PKS functional domains) can be evaluated and compared to the knownPUFAPKS systems described herein.

A PUFA PKS system can be identified using biological assays. For example, in U.S. Application Serial No. 09/231,899, Example 7, the results of a key experiment using a well-known inhibitor of some types of fatty acid synthesis systems, i.e., thiolactomycin, is described. The inventors showed that the synthesis of PUFAs in whole cells of *Schizochytrium* could be specifically blocked without blocking the synthesis of short chain saturated fatty acids. The significance of this result is as follows: the inventors knew from analysis of cDNA sequences from *Schizochytrium* that a Type I fatty acid synthase system is present in *Schizochytrium.* It was known that thiolactomycin does not inhibit Type I FAS systems, and this is consistent with the inventors' data - i.e., production of the saturated fatty acids (primarily C 14:0 and C16:0 in *Schizochytrium*) was not inhibited by the thiolactomycin treatment. There are no indications in the literature or in the inventors' own data that thiolactomycin has any inhibitory effect on the *elongation* of C14:0 or C16:0 fatty acids or their desaturation (i.e. the conversion of short chain saturated fatty acids to PUFAs by the classical pathway). Therefore, the fact that the PUFA production in *Schizochytrium* was blocked by thiolactomycin strongly indicates that the classical PUFA synthesis pathway does not produce the PUFAs in *+Schizochytrium*, but rather that a different pathway of synthesis is involved. Further, it had previously been determined that the *Shewanella* PUFA PKS system is inhibited by thiolactomycin (note that the PUFA PKS system of the present invention has elements of both Type I and Type II systems), and it was known that thiolactomycin is an inhibitor of Type II FAS systems (such as that found in *E. coli*). Therefore, this experiment indicated that *Schizochytrium* produced PUFAs as a result of a pathway not involving the Type I FAS. A similar rationale and detection step could be used to detect a PUFA PKS system in a microbe identified using the novel screening method disclosed herein.

In addition, Example 3 shows additional biochemical data which provides evidence that PUFAs in *Schizochytrium* are not produced by the classical pathway (i.e., precursor product kinetics between C16:0 andDHA are not observed in whole cells and, *in vitro* PUFA synthesis can be separated from the membrane fraction - all of the fatty acid desaturases of the classical PUFA synthesis pathway, with the exception of the delta 9 desaturase which inserts the first double bond of the series, are associated with cellular membranes). This type of biochemical data could be used to detect PUFA PKS activity in microbe identified by the novel screening method described above.

Microbial strains to screen using the screening/identification method of the present invention are chosen from the group consisting of: bacteria, algae, fungi, protozoa or protists, but most preferably from the eukaryotic microbes consisting of algae, fungi, protozoa and protists. These microbes are preferably capable of growth and production of the bioactive compounds containing two or more unsaturated bonds at temperatures greater than about 15°C, more preferably greater than about 20°C, even more preferably greater than about 25 ° C and most preferably greater than about 30°C.

Novel bacterial PUFA PKS systems can be identified in bacteria that produce PUFAs at temperatures exceeding about 20°C, preferably exceeding about 25°C and even more preferably exceeding about 30 °C. As described previously herein, the marine bacteria, *Shewanella* and *Vibrio marinus*, described in U.S. PatentNo. 6,140,486, do notproduce PUFAs at higher temperatures, which limits the usefulness ofPUFAPKS systems derived from these bacteria, particularly in plant applications under field conditions. Therefore, the screening method can be used to identify bacteria that have a PUFA PKS system which are capable of growth and PUFA production at higher temperatures (e.g., above about 20,25, or 30°C). Inhibitors of eukaryotic growth such as nystatin (antifungal) or cycloheximide (inhibitor of eukaryotic protein synthesis) can be added to agar plates used to culture/select initial strains from water samples/soil samples collected from the types of habitats/niches described below. This process would help select for enrichment of bacterial strains without (or minimal) contamination of eukaryotic strains. This selection process, in combination with culturing the plates at elevated temperatures (e.g. 30°C), and then selecting strains thatproduce at least one PUFA would initially identify candidate bacterial strains with a PUFA PKS system that is operative at elevated temperatures (as opposed to those bacterial strains in the prior art which only exhibit PUFA production at temperatures less than about 20 °C and more preferably below about 5 °C).

Locations for collection of the preferred types of microbes for screening for a PUFA PKS system according to the present invention include any of the following: low oxygen environments (or locations near these types of low oxygen environments including in the guts of animals including invertebrates that consume microbes or microbe-containing foods (including types of filter feeding organisms), low or non-oxygen containing aquatic habitats (including freshwater, saline and marine), and especially at-or near-low oxygen environments (regions) in the oceans. The microbial strains would preferably not be obligate anaerobes but be adapted to live in both aerobic and low or anoxic environments. Soil environments containing both aerobic and low oxygen or anoxic environments would also excellent environments to find these organisms in and especially in these types of soil in aquatic habitats or temporary aquatic habitats.

A particularly preferred microbial strain would be a strain (selected from the group consisting of algae, fungi (including yeast), protozoa or protists) that, during a portion of its life cycle, is capable of consuming whole bacterial cells (bacterivory) by mechanisms such as phagocytosis, phagotrophic or endocytic capability and/or has a stage of its life cycle in which it exists as an amoeboid stage or naked protoplast. This method of nutrition would greatly increase the potential for transfer of a bacterial PKS system into a eukaryotic cell if a mistake occurred and the bacterial cell (or its DNA) did not get digested and instead are functionally incorporated into the eukaryotic cell.

Strains of microbes (other than the members of the Thraustochytrids) capable of bacterivory (especially by phagocytosis or endocytosis) can be found in the following microbial classes (including but not limited to example genera):

In the algae and algae-like microbes (including stramenopiles): of the class Euglenophyceae (for example genera *Euglena*, and *Peranema*), the class Chrysophyceae (for example the genus *Ochromonas*), the class Dinobryaceae (for example the genera *Dinobryon*, *Platychrysis*, and *Chrysochromulina*), the Dinophyceae (including the genera *Crypthecodinium*, *Gymnodinium*, *Peridinium*, *Ceratium*, *Gyrodinium,* and *Oxyrrhis*), the class Cryptophyceae (for example the genera *Cryptomonas*, and *Rhodomonas*), the class Xanthophyceae (for example the genus *Olisthodiscus)* (and including forms of algae in which an amoeboid stage occurs as in the flagellates Rhizochloridaceae, and zoospores/gametes of *Aphanochaete pascheri*, *Bumilleria stigeoclonium* and *Vaucheria geminata*), the class Eustigmatophyceae, and the class Prymnesiopyceae (including the genera Prymnesium and Diacronema).

In the Stramenopiles including the: Proteromonads, Opalines, Developayella, Diplophorys, Larbrinthulids, Thraustochytrids, Bicosecids, Oomycetes, Hypochytridiomycetes, Commation, Reticulosphaera, Pelagomonas, Pelapococcus, Ollicola, Aureococcus, Parmales, Raphidiophytes, Synurids, Rhizochromulinaales, Pedinellales, Dictyochales, Chrysomeridales, Sarcinochrysidales, Hydrurales, Hibberdiales, and Chromulinales.

In the Fungi: Class Myxomycetes (form myxamoebae) -- slime molds, class Acrasieae including the orders Acrasiceae (for example the genus Sappinia), class Guttulinaceae (for example the genera *Guttulinopsis, and Guttulina),* class Dictysteliaceae (for example the genera *Acrasis, Dictyostelium*, *Polysphondylium, and Coenonia*), and class Phycomyceae including the orders Chytridiales, Ancylistales, Blastocladiales, Monoblepharidales, Saprolegniales, Peronosporales, Mucorales, and Entomophthorales.

In the Protozoa: Protozoa strains with life stages capable of bacterivory (including byphageocytosis) can be selected from the types classified as ciliates, flagellates or amoebae. Protozoan ciliates include the groups: Chonotrichs, Colpodids, Cyrtophores, Haptorids, Karyorelicts, Oligohymenophora, Polyhymenophora (spirotrichs), Prostomes and Suctoria. Protozoan flagellates include the Biosoecids, Bodonids, Cercomonads, Chrysophytes (for example the genera Anthophysa, Chrysamoemba, Chrysosphaerella, Dendromonas, Dinobryon, Mallomonas, Ochromonas, Paraphysomonas, Poterioochromonas, Spumella, Syncrypta, Synura, and Uroglena), Collar flagellates, Cryptophytes (for example the genera Chilomonas, Cryptomonas, Cyanomonas, and Goniomonas), Dinoflagellates, Diplomonads, Euglenoids, Heterolobosea, Pedinellids, Pelobionts, Phalansteriids, Pseudodendromonads, Spongomonads and Volvocales (and other flagellates including the unassigned flagellate genera ofArtodiscus, Clautriavia, Helkesimastix, Kathablepharis and Multicilia). Amoeboid protozoans include the groups: Actinophryids, Centrohelids, Desmothoricids, Diplophryids, Eumamoebae, Heterolobosea, Leptomyxids, Nucleariid filose amoebae, Pelebionts, Testate amoebae and Vampyrellids (and including the unassigned amoebid genera Gymnophrys, Biomyxa, Microcometes, Reticulomyxa, Belonocystis, Elaeorhanis, Allelogromia, Gromia or Lieberkuhnia). The protozoan orders include the following: Percolomonadeae, Heterolobosea, Lyromonadea, Pseudociliata, Trichomonadea, Hypermastigea, Heteromiteae, Telonemea, Cyathobodonea, Ebridea, Pyytomyxea, Opalinea, Kinetomonadea, Hemimastigea, ,Protostelea, Myxagastrea, Dictyostelea, Choanomonadea, Apicomonadea, Eogregarinea, Neogregarinea, Coelotrolphea, Eucoccidea, Haemosporea, Piroplasmea, Spirotrichea, Prostomatea, Litostomatea, Phyllopharyngea, Nassophorea, Oligohymenophorea, Colpodea, Karyorelicta, Nucleohelea, Centrohelea, Acantharea, Sticholonchea, Polycystinea, Phaeodarea, Lobosea, Filosea, Athalamea, Monothalamea, Polythalamea, Xenophyophorea, Schizocladea, Holosea, Entamoebea, Myxosporea, Actinomyxea, Halosporea, Paramyxea, Rhombozoa and Orthonectea.

We describe strains of the microorganisms listed above that have been collected from one of the preferred habitats listed above.

We describe microorganisms identified using the novel PUFA PKS screening method described above, to the PUFA PKS genes and proteins encoded thereby, and to the use of such microorganisms and/or PUFA PKS genes and proteins (including homologues and fragments thereof) in any of the methods described herein. In particular, organisms identified by the screening method of the present invention which are then genetically modified to regulate the production of bioactive molecules by said PUFA PKS system.

The present invention relates to an isolated nucleic acid molecule comprising a nucleic acid sequence encoding at least one biologically active domain or biologically active fragment thereof of a polyunsaturated fatty acid (PUFA) polyketide synthase (PKS) system from a Thraustochytrid microorganism. As discussed above, the present inventors have successfully used the method to identify a non-bacterial microorganism that has a PUFA PKS system to identify additional members of the order Thraustochytriales which contain a PUFA PKS system. The identification of three such microorganisms is described in Example 2. Specifically, the present inventors have used the screening method of the present invention to identify *Thraustochytrium* sp. 23B (ATCC 20892) as being highly predicted to contain a PUFA PKS system, followed by detection of sequences in the *Thraustochytrium* sp. 23B genome that hybridize to the *Schizochytrium* PUFAPKS genes disclosed herein. *Schizochytrium limacium* (IFO 32693) and *Ulkenia* (BP-5601) have also been identified as good candidates for containing PUFA PKS systems. Based on these data and on the similarities among members of the order Thraustochytriales, it is believed that many other Thraustochytriales PUFA PKS systems can now be readily identified using the methods and tools provided by the present invention. Therefore, Thraustochytriales PUFA PKS systems and portions and/or homologues thereof (e.g., proteins, domains and fragments thereof), genetically modified organisms comprising such systems and portions and/or homologues thereof, and methods of using such microorganisms and PUFA PKS systems, are encompassed by the present invention.

Developments have resulted in revision of the taxonomy of the Thraustochytrids. Taxonomic theorists place Thraustochytrids with the algae or algae-like protists. However, because of taxonomic uncertainty, it would be best for the purposes of the present invention to consider the strains described in the present invention as Thraustochytrids (Order: Thraustochytriales; Family: Thraustochytriaceae; Genus: *Thraustochytrium*, *Schizochytrium*, *Labyrinthuloides*, or *Japonochytrium*). For the present invention, members of the labrinthulids are considered to be included in the Thraustochytrids. Taxonomic changes are summarized below. Strains of certain unicellular microorganisms disclosed herein are members of the order Thraustochytriales. Thraustochytrids are marine eukaryotes with a evolving taxonomic history. Problems with the taxonomic placement of the Thraustochytrids have been reviewed by Moss (1986), Bahnweb and Jackle (1986) and Chamberlain and Moss (1988). According to the present invention, the phrases "Thraustochytrid", "Thraustochytriales microorganism" and "microorganism of the order Thraustochytriales" can be used interchangeably.

For convenience purposes, the Thraustochytrids were first placed by taxonomists with other colorless zoosporic eukaryotes in the Phycomycetes (algae-like fungi). The name Phycomycetes, however, was eventually dropped from taxonomic status, and the Thraustochytrids were retained in the Oomycetes (the biflagellate zoosporic fungi). It was initially assumed that the Oomycetes were related to the heterokont algae, and eventually a wide range of ultrastructural and biochemical studies, summarized by Barr (Barr, 1981, Biosystems 14:359-370) supported this assumption. The Oomycetes were in fact accepted by Leedale (Leedale, 1974, Taxon 23:261-270) and other phycologists as part of the heterokont algae. However, as a matter of convenience resulting from their heterotrophic nature, the Oomycetes and Thraustochytrids have been largely studied by mycologists (scientists who study fungi) rather than phycologists (scientists who study algae).

From another taxonomic perspective, evolutionary biologists have developed two general schools of thought as to how eukaryotes evolved. One theory proposes an exogenous origin of membrane-bound organelles through a series of endosymbioses (Margulis, 1970, Origin of Eukaryotic Cells. Yale University Press, New Haven); e.g., mitochondria were derived from bacterial endosymbionts, chloroplasts from cyanophytes, and flagella from spirochaetes. The other theory suggests a gradual evolution of the membrane-bound organelles from the non-membrane-bounded systems of the prokaryote ancestor via an autogenous process (Cavalier-Smith, 1975, Nature (Lond.) 256:462-468). Both groups of evolutionary biologists however, have removed the Oomycetes and Thraustochytrids from the fungi and place them either with the chromophyte algae in the kingdom Chromophyta (Cavalier-Smith, 1981, BioSystems 14:461-481) (this kingdom has been more recently expanded to include other protists and members of this kingdom are now called Stramenopiles) or with all algae in the kingdom Protoctista (Margulis and Sagen, 1985, Biosystems 18:141-147).

With the development of electron microscopy, studies on the ultrastructure of the zoospores of two genera of Thraustochytrids, *Thraustochytrium* and *Schizochytrium*, (Perkins, 1976, pp. 279-312 in "Recent Advances in Aquatic Mycology" (ed. E.B.G. Jones), John Wiley & Sons, New York; Kazama, 1980, Can. J. Bot. 58:2434-2446; Barr, 1981, Biosystems 14:359-370) have provided good evidence that the Thraustochytriaceae are only distantly related to the Oomycetes. Additionally, genetic data representing a correspondence analysis (a form of multivariate statistics) of 5 S ribosomal RNA sequences indicate that Thraustochytriales are clearly a unique group of eukaryotes, completely separate from the fungi, and most closely related to the red and brown algae, and to members of the Oomycetes (Mannella, et al., 1987, Mol. Evol. 24:228-235). Most taxonomists have agreed to remove the Thraustochytrids from the Oomycetes (Bartnicki-Garcia, 1987, pp. 389-403 in "Evolutionary Biology of the Fungi" (eds. Rayner, A.D.M., Brasier, C.M. & Moore, D.), Cambridge University Press, Cambridge).

In summary, employing the taxonomic system of Cavalier-Smith (Cavalier-Smith, 1981, BioSystems 14:461-481, 1983; Cavalier-Smith, 1993, Microbiol Rev. 57:953-994), the Thraustochytrids are classified with the chromophyte algae in the kingdom Chromophyta (Stramenopiles). This taxonomic placement has been more recently reaffirmed by Cavalier-Smith et al. using the 18s rRNA signatures of the Heterokonta to demonstrate that Thraustochytrids are chromists not Fungi (Cavalier-Smith et al., 1994, Phil. Tran. Roy. Soc. London Series BioSciences 346:387-397). This places them in a completely different kingdom from the fungi, which are all placed in the kingdom Eufungi. The taxonomic placement of the Thraustochytrids is therefore summarized below:
Kingdom: Chromophyta (Stramenopiles)
Phylum: Heterokonta
Order: Thraustochytriales
Family: Thraustochytriaceae
Genus: *Thraustochytrium*, *Schizochytrium*, *Labyrinthuloides*, or *Japonochytrium*

Some early taxonomists separated a few original members of the genus *Thraustochytrium* (those with an amoeboid life stage) into a separate genus called *Ulkenia.* However it is now known that most, if not all, Thraustochytrids (including *Thraustochytrium* and *Schizochytrium*), exhibit amoeboid stages and as such, *Ulkenia* is not considered by some to be a valid genus. As used herein, the genus *Thraustochytrium* will include *Ulkenia*.

Despite the uncertainty of taxonomic placement within higher classifications of Phylum and Kingdom, the Thraustochytrids remain a distinctive and characteristic grouping whose members remain classifiable within the order Thraustochytriales.

Polyunsaturated fatty acids (PUFAs) are essential membrane components in higher eukaryotes and the precursors of many lipid-derived signaling molecules. The PUFA PKS system of the present invention uses pathways for PUFA synthesis that do not require desaturation and elongation of saturated fatty acids. The pathways catalyzed by PUFA PKSs that are distinct from previously recognized PKSs in both structure and mechanism. Generation of *cis* double bonds is suggested to involve position-specific isomerases; these enzymes are believed to be useful in the production of new families of antibiotics.

To produce significantly high yields of various bioactive molecules using the PUFA PKS system of the present invention, an organism, preferably a microorganism or a plant, can be genetically modified to affect the activity of a PUFA PKS system. In one aspect, such an organism can endogenously contain and express a PUFA PKS system, and the genetic modification can be a genetic modification of one or more of the functional domains of the endogenous PUFA PKS system, whereby the modification has some effect on the activity of the PUFA PKS system. In another aspect, such an organism can endogenously contain and express a PUFA PKS system, and the genetic modification can be an introduction of at least one exogenous nucleic acid sequence (e.g., a recombinant nucleic acid molecule), wherein the exogenous nucleic acid sequence encodes at least one biologically active domain or protein from a second PKS system and/or a protein that affects the activity of said PUFA PKS system (e.g., a phosphopantetheinyl transferases (PPTase), discussed below). In yet another aspect, the organism does not necessarily endogenously (naturally) contain a PUFA PKS system, but is genetically modified to introduce at least one recombinant nucleic acid molecule encoding an amino acid sequence having the biological activity of at least one domain of a PUFA PKS system. In this aspect, PUFAPKS activityis affected by introducing or increasing PUFA PKS activity in the organism. Various embodiments associated with each of these aspects will be discussed in greater detail below.

Therefore, according to the present invention, one embodiment relates to a genetically modified microorganism, wherein the microorganisms expresses a PKS system comprising at least one biologically active domain according to the present invention of a polyunsaturated fatty acid (PUFA) polyketide synthase (PKS) system.

The genetic modification affects the activity of the PKS system in the organism. The screening process referenced in part (b) has been described in detail above and includes the steps of: (a) selecting a microorganism that produces at least one PUFA; and, (b) identifying a microorganism from (a) that has an ability to produce increased PUFAs under dissolved oxygen conditions of less than about 5% of saturation in the fermentation medium, as compared to production ofPUFAs by the microorganism under dissolved oxygen conditions of greater than about 5% of saturation, and preferably about 10%, and more preferably about 15%, and more preferably about 20%-of saturation in the fermentation medium. The genetically modified microorganism described herein can include any one or more of the above-identified nucleic acid sequences, and/or any of the other homologues of any of the *Schizochytrium* PUFA PKS ORFs or domains as described in detail above.

As used herein, a genetically modified microorganism can include a genetically modified bacterium, protist, microalgae, fungus, or other microbe, and particularly, any of the genera of the order Thraustochytriales (e.g., a Thraustochytrid) described herein (e.g., *Schizochytrium*, *Thraustochytrium*, *Japonochytrium*, *Labyrinthuloides*). Such a genetically modified microorganism has a genome which is modified (i.e., mutated or changed) from its normal (i.e., wild-type or naturally occurring) form such that the desired result is achieved (i.e., increased or modified PUFA PKS activity and/or production of a desired product using the PKS system). Genetic modification of a microorganism can be accomplished using classical strain development and/or molecular genetic techniques. Such techniques known in the art and are generally disclosed for microorganisms, for example, in Sambrook et al., 1989, Molecular Cloning: A Laboratory Manual, Cold Spring Harbor Labs Press. The reference Sambrook et al., *ibid.,* is incorporated by reference herein in its entirety. A genetically modified microorganism can include a microorganism in which nucleic acid molecules have been inserted, deleted or modified (i.e., mutated; e.g., by insertion, deletion, substitution, and/or inversion of nucleotides), in such a manner that such modifications provide the desired effect within the microorganism.

Preferred microorganism host cells to modify according to the present invention include, but are not limited to, any bacteria, protist, microalga, fungus, or protozoa. In one aspect, preferred microorganisms to genetically modify include, but are not limited to, any microorganism of the order Thraustochytriales. Particularly preferred host cells for use in the present invention could include microorganisms from a genus including, but not limited to: *Thraustochytrium, Labyrinthuloides*, *Japonochytrium*, and *Schizochytrium.* Preferred species within these genera include, but are not limited to: any *Schizochytrium* species, including *Schizochytrium aggregatum*, *Schizochytrium limacinum*, *Schizochytrium minutum;* any *Thraustochytrium* species (including former *Ulkenia* species such as *U. visurgensis*, *U. amoeboida*, *U. sarkariana*, *U. profunda, U. radiata, U. minuta* and *Ulkenia sp.* BP-5601), and including *Thraustochytrium striatum*, *Thraustochytrium aureum*, *Thraustochytrium roseum*; and any *Japonochytrium* species. Particularly preferred strains of Thraustochytriales include, but are not limited to: *Schizochytrium sp.* (S31)(ATCC 20888); *Schizochytrium sp.* (S8)(ATCC 20889); *Schizochytrium sp.* (LC-RM)(ATCC 18915); *Schizochytrium sp.* (SR21); *Schizochytrium aggregatum* (Goldstein et Belsky)(ATCC 28209); *Schizochytrium limacinum* (Honda et Yokochi)(IFO 32693); *Thraustochytrium sp.* (23B)(ATCC 20891); *Thraustochytrium striatum* (Schneider)(ATCC 24473); *Thraustochytrium aureum* (Goldstein)(ATCC 34304); *Thraustochytrium roseum* (Goldstein)(ATCC 28210); and *Japonochytrium sp.* (L1)(ATCC 28207). Other examples of suitable host microorganisms for genetic modification include, but are not limited to, yeast including *Saccharomyces cerevisiae, Saccharomyces carlsbergensis*, or other yeast such as *Candida*, *Kluyveromyces*, or other fungi, for example, filamentous fungi such as *Aspergillus*, *Neurospora, Penicillium*, etc. Bacterial cells also may be used as hosts. This includes *Escherichia coli*, which can be useful in fermentation processes. Alternatively, a host such as a *Lactobacillus* species or *Bacillus* species can be used as a host.

Another embodiment of the present invention relates to a genetically modified plant, wherein the plant has been genetically modified to recombinantly express a PKS system comprising at least one biologically active domain according to the invention of a polyunsaturated fatty acid (PUFA) polyketide synthase (PKS) system.

The genetically modified plant can include any one or more of the above-identified nucleic acid sequences, and/or any of the other homologues of any of the *Schizochytrium* PUFA PKS ORFs or domains as described in detail above.

As used herein, a genetically modified plant can include any genetically modified plant including higher plants and particularly, any consumable plants or plants useful for producing a desired bioactive molecule of the presentinvention. Such a geneticallymodified plant has a genome which is modified (i.e., mutated or changed) from its normal (i.e., wild-type or naturally occurring) form such that the desired result is achieved (i.e., increased or modified PUFA PKS activity and/or production of a desired product using the PKS system). Genetic modification of a plant can be accomplished using classical strain development and/or molecular genetic techniques. Methods for producing a transgenic plant, wherein a recombinant nucleic acid molecule encoding a desired amino acid sequence is incorporated into the genome of the plant, are known in the art. A preferred plant to genetically modify according to the present invention is preferably a plant suitable for consumption by animals, including humans.

Preferred plants to genetically modify according to the present invention (i.e., plant host cells) include, but are not limited to any higher plants, and particularly consumable plants, including crop plants and especially plants used for their oils. Such plants can include, for example: canola, soybeans, rapeseed, linseed, corn, safflowers, sunflowers and tobacco. Other preferred plants include those plants that are known to produce compounds used as pharmaceutical agents, flavoring agents, neutraceutical agents, functional food ingredients or cosmetically active agents or plants that are genetically engineered to produce these compounds/agents.

According to the present invention, a genetically modified microorganism or plant includes a microorganism or plant that has been modified using recombinant technology. As used herein, genetic modifications which result in a decrease in gene expression, in the function of the gene, or in the function of the gene product (i.e., the protein encoded by the gene) can be referred to as inactivation (complete or partial), deletion, interruption, blockage or down-regulation of a gene. For example, a genetic modification in a gene which results in a decrease in the function of the protein encoded by such gene, can be the result of a complete deletion of the gene (i.e., the gene does not exist, and therefore the protein does not exist), a mutation in the gene which results in incomplete or no translation of the protein (e.g., the protein is not expressed), or a mutation in the gene which decreases or abolishes the natural function of the protein (e.g., a protein is expressed which has decreased or no enzymatic activity or action). Genetic modifications that result in an increase in gene expression or function can be referred to as amplification, overproduction, overexpression, activation, enhancement, addition, or up-regulation of a gene.

The genetic modification of a microorganism or plant according to the present invention preferably affects the activity of the PKS system expressed by the plant, whether the PKS system is endogenous and genetically modified, endogenous with the introduction of recombinant nucleic acid molecules into the organism, or provided completely by recombinant technology. According to the present invention, to "affect the activity of a PKS system" includes any genetic modification that causes any detectable or measurable change or modification in the PKS system expressed by the organism as compared to in the absence of the genetic modification. A detectable change or modification in the PKS system can include, but is not limited to: the introduction of PKS system activity into an organism such that the organism now has measurable/detectable PKS system activity (i.e., the organism did not contain a PKS system prior to the genetic modification), the introduction into the organism of a functional domain from a different PKS system than a PKS system endogenously expressed by the organism such that the PKS system activity is modified (e.g., a bacterial PUFA PKS domain or a type I PKS domain is introduced into an organism that endogenously expresses a non-bacterial PUFA PKS system), a change in the amount of a bioactive molecule produced by the PKS system (e.g., the system produces more (increased amount) or less (decreased amount) of a given product as compared to in the absence of the genetic modification), a change in the type of a bioactive molecule produced by the PKS system (e.g., the system produces a new or different product, or a variant of a product that is naturally produced by the system), and/or a change in the ratio of multiple bioactive molecules produced by the PKS system (e.g., the system produces a different ratio of one PUFA to another PUFA, produces a completely different lipid profile as compared to in the absence of the genetic modification, or places various PUFAs in different positions in a triacylglycerol as compared to the natural configuration). Such a genetic modification includes any type of genetic modification and specifically includes modifications made by recombinant technology and by classical mutagenesis.

It should be noted that reference to increasing the activity of a functional domain or protein in a PUFA PKS system refers to any genetic modification in the organism containing the domain or protein (or into which the domain or protein is to be introduced) which results in increased functionality of the domain or protein system and can include higher activity of the domain or protein (e.g., specific activity or *in vivo* enzymatic activity), reduced inhibition or degradation of the domain or protein system, and overexpression of the domain or protein. For example, gene copy number can be increased, expression levels can be increased by use of a promoter that gives higher levels of expression than that of the native promoter, or a gene can be altered by genetic engineering or classical mutagenesis to increase the activity of the domain or protein encoded by the gene.

Similarly, reference to decreasing the activity of a functional domain or protein in a PUFA PKS system refers to any genetic modification in the organism containing such domain or protein (or into which the domain or protein is to be introduced) which results in decreased functionality of the domain or protein and includes decreased activity of the domain or protein, increased inhibition or degradation of the domain or protein and a reduction or elimination of expression of the domain or protein. For example, the action of domain or protein of the present invention can be decreased by blocking or reducing the production of the domain or protein, "knocking out" the gene or portion thereof encoding the domain or protein, reducing domain or protein activity, or inhibiting the activity of the domain or protein. Blocking or reducing the production of an domain or protein can include placing the gene encoding the domain or protein under the control of a promoter that requires the presence of an inducing compound in the growth medium. By establishing conditions such that the inducer becomes depleted from the medium, the expression of the gene encoding the domain or protein (and therefore, of protein synthesis) could be turned off. Blocking or reducing the activity of domain or protein could also include using an excision technology approach similar to that described in U.S. Patent No. 4,743,546.

To use this approach, the gene encoding the protein of interest is cloned between specific genetic sequences that allow specific, controlled excision of the gene from the genome. Excision could be prompted by, for example, a shift in the cultivation temperature of the culture, as in U.S. Patent No. 4,743,546, or by some other physical or nutritional signal.

In one embodiment of the present invention, a genetic modification includes a modification of a nucleic acid sequence encoding an amino acid sequence that has a biological activity of at least one domain of a non-bacterial PUFA PKS system as described herein. Such a modification can be to an amino acid sequence within an endogenously (naturally) expressed non-bacterial PUFA PKS system, whereby a microorganism that naturally contains such a system is genetically modified by, for example, classical mutagenesis and selection techniques and/or molecular genetic techniques, include genetic engineering techniques. Genetic engineering techniques can include, for example, using a targeting recombinant vector to delete a portion of an endogenous gene, or to replace a portion of an endogenous gene with a heterologous sequence. Examples of heterologous sequences that could be introduced into a host genome include sequences encoding at least one functional domain from another PKS system, such as a different non-bacterial PUFA PKS system, a bacterial PUFA PKS system, a type I PKS system, a type II PKS system, or a modular PKS system. Other heterologous sequences to introduce into the genome of a host includes a sequence encoding a protein or functional domain that is not a domain of a PKS system, but which will affect the activity of the endogenous PKS system. For example, one could introduce into the host genome a nucleic acid molecule encoding a phosphopantetheinyl transferase (discussed below). Specific modifications that could be made to an endogenous PUFA PKS system are discussed in detail below.

In another aspect of this embodiment of the invention, the genetic modification can include: (1) the introduction of a recombinant nucleic acid molecule encoding an amino acid sequence having a biological activity of at least one domain of a non-bacterial PUFA PKS system; and/or (2) the introduction of a recombinant nucleic acid molecule encoding a protein or functional domain that affects the activity of a PUFA PKS system, into a host. The host can include: (1) a host cell that does not express any PKS system, wherein all functional domains of a PKS system are introduced into the host cell, and wherein at least one functional domain is from a non-bacterial PUFA PKS system; (2) a host cell that expresses a PKS system (endogenous or recombinant) having at least one functional domain of a non-bacterial PUFA PKS system, wherein the introduced recombinant nucleic acid molecule can encode at least one additional non-bacterial PUFA PKS domain function or another protein or domain that affects the activity of the host PKS system; and (3) a host cell that expresses a PKS system (endogenous or recombinant) which does not necessarily include a domain function from a non-bacterial PUFA PKS, and wherein the introduced recombinant nucleic acid molecule includes a nucleic acid sequence encoding at least one functional domain of a non-bacterial PUFA PKS system. In other words, the present invention intends to encompass any genetically modified organism (e.g., microorganism or plant), wherein the organism comprises at least one non-bacterial PUFA PKS domain function (either endogenously or by recombinant modification), and wherein the genetic modification has a measurable effect on the non-bacterial PUFA PKS domain function or on the PKS system when the organism comprises a functional PKS system.

Therefore, using the non-bacterial PUFA PKS systems of the present invention, which, for example, makes use of genes from Thraustochytrid PUFA PKS systems, gene mixing can be used to extend the range of PUFA products to include EPA, DHA, ARA, GLA, SDA and others, as well as to produce a wide variety ofbioactive molecules, including antibiotics, other pharmaceutical compounds, and other desirable products. The method to obtain these bioactive molecules includes not only the mixing of genes from various organisms but also various methods of genetically modifying the non-bacterial PUFA PKS genes disclosed herein. Knowledge of the genetic basis and domain structure of the non-bacterial PUFA PKS system of the present invention provides a basis for designing novel genetically modified organisms which produce a variety of bioactive molecules. Although mixing and modification of any PKS domains and related genes are contemplated by the present inventors, by way of example, various possible manipulations of the PUFA-PKS system are discussed below with regard to genetic modification and bioactive molecule production.

For example, non-bacterial PUFA-PKS system products, such as those produced by Thraustochytrids, are altered by modifying the CLF (chain length factor) domain. This domain is characteristic of Type II (dissociated enzymes) PKS systems. Its amino acid sequence shows homology to KS (keto synthase pairs) domains, but it lacks the active site cysteine. CLF may function to determine the number of elongation cycles, and hence the chain length, of the end product. Using the current state of knowledge of FAS and PKS synthesis, a rational strategy for production of ARA by directed modification of the non-bacterial PUFA-PKS system is provided. There is controversy in the literature concerning the function of the CLF in PKS systems (C. Bisang et al., Nature 401, 502 (1999)) and it is realized that other domains maybe involved in determination of the chain length of the end product, However, it is significant that *Schizochytrium* produces both DHA (C22:6, ω-3) and DPA (C22:5, ω-6). In the PUFA-PKS system the *cis* double bonds are introduced during synthesis of the growing carbon chain. Since placement of the ω-3 and ω-6 double bonds occurs early in the synthesis of the molecules, one would not expect that they would affect subsequent end-product chain length determination. Thus, without being bound by theory, the present inventors believe that introduction of a factor (e.g. CLF) that directs synthesis of C20 units (instead of C22 units) into the *Schizochytrium* PUFA-PKS system will result in the production of EPA (C20:5, ω-3) and ARA (C20:4, ω-6). For example, in heterologous systems, one could exploit the CLF by directly substituting a CLF from an EPA producing system (such as one from *Photobacterium*) into the *Schizochytrium* gene set. The fatty acids of the resulting transformants can then be analyzed for alterations in profiles to identify the transformants producing EPA and/or ARA.

In addition to dependence on development of a heterologous system (recombinant system, such as could be introduced into plants), the CLF concept can be exploited in *Schizochytrium* (i.e., by modification of a *Schizochytrium* genome). Transformation and homologous recombination has been demonstrated in *Schizochytrium.* One can exploit this by constructing a clone with the CLF of OrfB replaced with a CLF from a C20 PUFA-PKS system. A marker gene will be inserted downstream of the coding region. One can then transform the wild type cells, select for the marker phenotype and then screen for those that had incorporated the new CLF. Again, one would analyze these for any effects on fatty acid profiles to identify transformants producing EPA and/or ARA. If some factor other than those associated with the CLF are found to influence the chain length of the end product, a similar strategy could be employed to alter those factors.

We describe alteration of the PUFA-PKS products involves modification or substitution of the β-hydroxy acyl-ACP dehydrase/keto synthase pairs. During *cis*-vaccenic acid (C8:1, Δ11) synthesis in *E*. *coli,* creation of the *cis* double bond is believed to depend on a specific DH enzyme, β-hydroxy acyl-ACP dehydrase, the product of the *FabA* gene. This enzyme removes HOH from a β-keto acyl-ACP and leaves a *trans* double bond in the carbon chain. A subset of DH's, FabA-like, possess *cis-trans* isomerase activity (Heath et al., 1996, *supra*). A novel aspect of bacterial and non-bacterial PUFA-PKS systems is the presence of two FabA-like DH domains. Without being bound by theory, the present inventors believe that one or both of these DH domains will possess *cis-trans* isomerase activity (manipulation of the DH domains is discussed in greater detail below).

Another aspect of the unsaturated fatty acid synthesis in *E. coli* is the requirement for a particular KS enzyme, β-ketoacyl-ACP synthase, the product of the *FabB* gene. This is the enzyme that carries out condensation of a fatty acid, linked to a cysteine residue at the active site (by a thio-ester bond), with a malonyl-ACP. In the multi-step reaction, CO₂ is released and the linear chain is extended by two carbons. It is believed that only this KS can extend a carbon chain that contains a double bond. This extension occurs only when the double bond is in the *cis* configuration; if it is in the *trans* configuration, the double bond is reduced by enoyl-ACP reductase (ER) prior to elongation (Heath et al., 1996, *supra*). All of the PUFA-PKS systems characterized so far have two KS domains, one of which shows greater homology to the FabB-like KS of *E*. *coli* than the other. Again, without being bound by theory, the present inventors believe that in PUFA-PKS systems, the specificities and interactions of the DH (FabA-like) and KS (FabB-like) enzymatic domains determine the number and placement of *cis* double bonds in the end products. Because the number of 2-carbon elongationreactions is greater than the number of double bonds present in the PUFA-PKS end products, it can be determined that in some extension cycles complete reduction occurs. Thus the DH and KS domains can be used as targets for alteration of the DHA/DPA ratio or ratios of other long chain fatty acids. These can be modified and/or evaluated by introduction of homologous domains from other systems or by mutagenesis of these gene fragments.

The ER (enoyl-ACP reductase - an enzyme which reduces the trans-double bond in the fatty acyl-ACP resulting in fully saturated carbons) domains can be modified or substituted to change the type of product made by the PKS system. For example, the present inventors know that *Schizochytrium* PUFA-PKS system differs from the previously described bacterial systems in that it has two (rather than one) ER domains. Without being bound by theory, the present inventors believe these ER domains can strongly influence the resulting PKS production product. The resulting PKS product could be changed by separately knocking out the individual domains or by modifying their nucleotide sequence or by substitution of ER domains from other organisms.

In another embodiment, nucleic acid molecules encoding proteins or domains that are not part of a PKS system, but which affect a PKS system, can be introduced into an organism. For example, all of the PUFA PKS systems described above contain multiple, tandem, ACP domains. ACP (as a separate protein or as a domain of a larger protein) requires attachment of a phosphopantetheine cofactor to produce the active, holo-ACP. Attachment of phosphopantetheine to the apo-ACP is carried out by members of the superfamily of enzymes - the phosphopantetheinyl transferases (PPTase) (Lambalot R.H., et al., Chemistry and Biology, 3, 923 (1996)).

By analogy to other PKS and FAS systems, the present inventors presume that activation of the multiple ACP domains present in the *Schizochytrium* ORFA protein is carried out by a specific, endogenous, PPTase. The gene encoding this presumed PPTase has not yet been identified in *Schizochytrium.* If such a gene is present in *Schizochytrium*, one can envision several approaches that could be used in an attempt to identify and clone it. These could include (but would not be limited to): generation and partial sequencing of a cDNA library prepared from actively growing *Schizochytrium* cells (note, one sequence was identified in the currently available *Schizochytrium* cDNA library set which showed homology to PPTase's; however, it appears to be part of a multidomain FAS protein, and as such may not encode the desired OrfA specific PPTase); use of degenerate oligonucleotide primers designed using amino acid motifs present in many PPTase's in PCR reactions (to obtain a nucleic acid probe molecule to screen genomic or cDNA libraries); genetic approaches based on protein-protein interactions (e.g. a yeast two-hybrid system) in which the ORFA-ACP domains would be used as a "bait" to find a "target" (i.e. the PPTase); and purification and partial sequencing of the enzyme itself as a means to generate a nucleic acid probe for screening of genomic or cDNA libraries.

It is also conceivable that a heterologous PPTase may be capable of activating the *Schizochytrium* ORFA ACP domains. It has been shown that some PPTases, for example the sfp enzyme of *Bacillus subtilis* (Lambalot et al., *supra*) and the svp enzyme of *Streptomyces verticillus* (Sanchez et al., 2001, Chemistry & Biology 8:725-738 ), have a broad substrate tolerance. These enzymes can be tested to see if they will activate the *Schizochytrium* ACP domains. Also, a recent publication described the expression of a fungal PKS protein in tobacco (Yalpani et al., 2001, The Plant Cell 13:1401-1409). Products of the introduced PKS system (encoded by the 6-methylsalicyclic acid synthase gene of *Penicillium patulum*) were detected in the transgenic plant, even though the corresponding fungal PPTase was not present in those plants. This suggested that an endogenous plant PPTase(s) recognized and activated the fungal PKS ACP domain. Of relevance to this observation, the present inventors have identified two sequences (genes) in the *Arabidopsis* whole genome database that are likely to encode PPTases. These sequences (GenBank Accession numbers; AAG51443 and AAC05345) are currently listed as encoding "Unknown Proteins". They can be identified as putative PPTases based on the presence in the translated protein sequences of several signature motifs including; G(I/V)D and WxxKE(A/S)xxK (SEQ ID NO:33), (listed in Lambalot et al., 1996 as characteristic of all PPTases). In addition, these two putative proteins contain two additional motifs typically found in PPTases typically associated with PKS and non-ribosomal peptide synthesis systems; i.e., FN(I/L/V)SHS (SEQ ID NO:34) and (I/V/L)G(I/L/V)D(I/L/V) (SEQ ID NO:35). Furthermore, these motifs occur in the expected relative positions in the protein sequences. It is likely that homologues of the *Arabidopsis* genes are present in other plants, such as tobacco. Again, these genes can be cloned and expressed to see if the enzymes they encode can activate the *Schizochytrium* ORFA ACP domains, or alternatively, OrfA could be expressed directly in the transgenic plant (either targeted to the plastid or the cytoplasm).

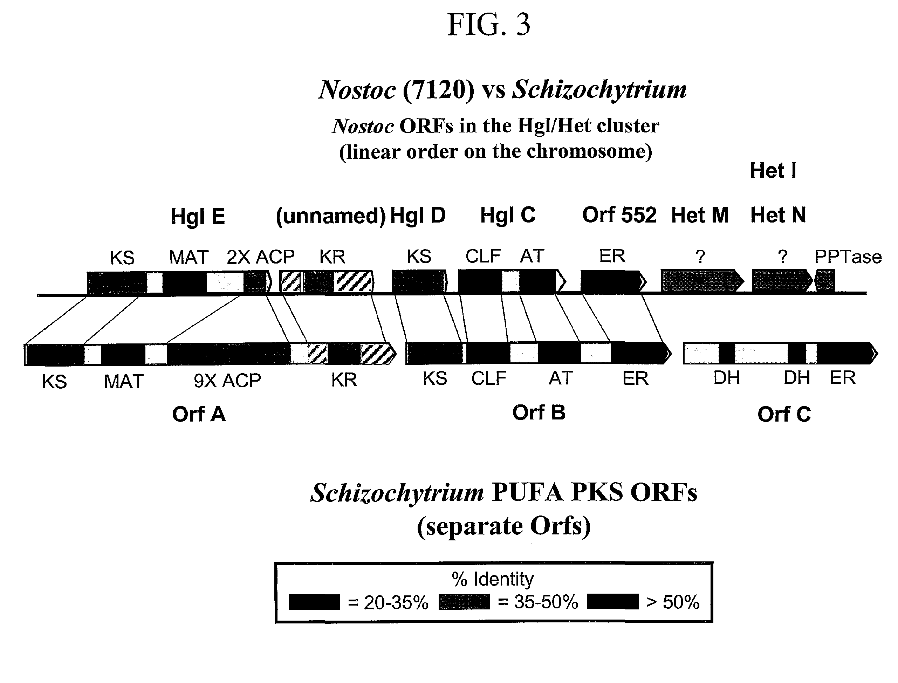

Another heterologous PPTase which may recognize the ORFA ACP domains as substrates is the Het I protein *of Nostoc* sp. PCC 7120 (formerly called *Anabaena* sp. PCC 7120). As noted in U.S. Patent No. 6,140,486, several of the PUFA-PKS genes of *Shewanella* showed a high degree of homology to protein domains present in a PKS cluster found in *Nostoc* (Figure 2 of that patent). This *Nostoc* PKS system is associated with the synthesis of long chain (C26 or C28) hydroxy fatty acids that become esterified to sugar moieties and form a part of the heterocyst cell wall. These *Nostoc* PKS domains are also highly homologous to the domains found in Orfs B and C of the *Schizochytrium* PKS proteins (i.e. the same ones that correspond to those found in the *Shewanella* PKS proteins). Until very recently, none of the *Nostoc* PKS domains present in the GenBank databases showed high homology to any of the domains of *Schizochytrium* OrfA (or the homologous *Shewanella* Orf 5 protein). However, the complete genome of *Nostoc* has recently been sequenced and as a result, the sequence of the region just upstream of the PKS gene cluster is now available. In this region are three Orfs that show homology to the domains (KS, MAT, ACP and KR) of OrfA (see Fig. 3). Included in this set are two ACP domains, both of which show high homology to the ORFA ACP domains. At the end of the *Nostoc* PKS cluster is the gene that encodes the Het I PPTase. Previously, it was not obvious what the substrate of the Het I enzyme could be, however the presence of tandem ACP domains in the newly identified Orf (Hgl E) of the cluster strongly suggests to the present inventors that it is those ACPs. The homology of the ACP domains of *Schizochytrium* and *Nostoc,* as well as the tandem arrangement of the domains in both proteins, makes Het I a likely candidate for heterologous activation of the *Schizochytrium* ORFA ACPs. The present inventors are believed to be the first to recognize and contemplate this use for *Nostoc* Het I PPTase.

As indicated in Metz et al., 2001, *supra,* one novel feature of the PUFA PKS systems is the presence of two dehydratase domains, both of which show homology to the FabA proteins *of E. coli.* With the availability of the new *Nostoc* PKS gene sequences mentioned above, one can now compare the two systems and their products. The sequence of domains in the *Nostoc* cluster (from HglE to Het I) as the present inventors have defined them is (see Fig. 3):
*KS-MAT-2xACP, KR, KS, CLF-AT, ER* (HetM, HetN) HetI
In the Schizochytrium PUFA-PKS Orfs A,B&C the sequence (OrfA - B - C) is:
*KS-MAT-9xACP-KR KS-CLF-AT-ER* DH-DH-ER
One can see the correspondence of the domains sequence (there is also a high amino acid sequence homology). The product of the *Nostoc* PKS system is a long chain hydroxy fatty acid (C26 or C28 with one or two hydroxy groups) that contains no double bonds *(cis* or *trans*). The product of the *Schizochytrium* PKS system is a long chain polyunsaturated fatty acid (C22, with 5 or 6 double bonds - all *cis).* An obvious difference between the two domain sets is the presence of the two DH domains in the *Schizochytrium* proteins - just the domains implicated in the formation of the *cis* double bonds of DHA and DPA (presumably HetM and HetN in the *Nostoc* system are involved in inclusion of the hydroxyl groups and also contain a DH domain whose origin differs from the those found in the PUFA). Also, the role of the duplicated ER domain in the *Schizochytrium* Orfs B and C is not known (the second ER domain in is not present other characterized PUFA PKS systems). The amino acid sequence homology between the two sets of domains implies an evolutionary relationship. One can conceive of the PUFA PKS gene set being derived from (in an evolutionary sense) an ancestral Nostoc-like PKS gene set by incorporation of the DH (FabA-like) domains. The addition of the DH domains would result in the introduction of *cis* double bonds in the new PKS end product structure.

The comparisons of the *Schizochytrium* and *Nostoc* PKS domain structures as well as the comparison of the domain organization between the *Schizochytrium* and *Shewanella* PUFA-PKS proteins demonstrate nature's ability to alter domain order as well as incorporate new domains to create novel end products. In addition, the genes can now be manipulated in the laboratory to create new products. The implication from these observations is that it should be possible to continue to manipulate the systems in either a directed or random way to influence the end products. For example, in a preferred embodiment, one could envision substituting one of the DH (FabA-like) domains of the PUFA-PKS system for a DH domain that did not posses isomerization activity, potentially creating a molecule with a mix of *cis-*and *trans-* double bonds. The current products of the *Schizochytrium* PUFA PKS system are DHA and DPA (C22:5 ω6). If one manipulated the system to produce C20 fatty acids, one would expect the products to be EPA and ARA (C20:4 ω6). This could provide a new source for ARA. One could also substitute domains from related PUFA-PKS systems that produced a different DHA to DPA ratio - for example by using genes from *Tharaustochytrium* 23B (the PUFA PKS system of which is identified for the first time herein).

Additionally, one could envision specifically altering one of the ER domains (e.g. removing, or inactivating) in the *Schizochytrium* PUFA PKS system (other PUFA PKS systems described so far do not have two ER domains) to determine its effect on the end product profile. Similar strategies could be attempted in a directed manner for each of the distinct domains of the PUFA-PKS proteins using more or less sophisticated approaches. Of course one would not be limited to the manipulation of single domains. Finally, one could extend the approach by mixing domains from the PUFA-PKS system and other PKS or FAS systems (e.g., type I, type II, modular) to create an entire range of new end products. For example, one could introduce the PUFA-PKS DH domains into systems that do not normally incorporate *cis* double bonds into their end products.

Accordingly, encompassed by the present invention are methods to geneticallymodify microbial or plant cells by: genetically modifying at least one nucleic acid sequence in the organism that encodes an amino acid sequence having the biological activity of at least one functional domain of a non-bacterial PUFA PKS system according to the present invention, and/or expressing at least one recombinant nucleic acid molecule comprising a nucleic acid sequence encoding such amino acid sequence. Various embodiments of such sequences, methods to genetically modify an organism, and specific modifications have been described in detail above. Typically, the method is used to produce a particular genetically modified organism that produces a particular bioactive molecule or molecules.

One embodiment of the present invention relates to a recombinant host cell which has been modified to express a polyunsaturated fatty acid (PUFA) polyketide synthase (PKS) system, wherein the PKS catalyzes both iterative and non-iterative enzymatic reactions, and wherein the PUFA PKS system comprises: (a) at least two enoyl ACP-reductase (ER) domains; (b) at least six acyl carrier protein (ACP) domains; (c) at least two β-keto acyl-ACP synthase (KS) domains; (d) at least one acyltransferase (AT) domain; (e) at least one ketoreductase (KR) domain; (f) at least two FabA-like β-hydroxy acyl-ACP dehydrase (DH) domains; (g) at least one chain length factor (CLF) domain; and (h) at least one malonyl-CoA:ACP acyltransferase (MAT) domain. In one embodiment, the PUFA PKS system is a eukaryotic PUFA PKS system. In a preferred embodiment, the PUFA PKS system is an algal PUFA PKS system. In a more preferred embodiment, the PUFA PKS system is a Thraustochytriales PUFA PKS system. Such PUFA PKS systems can include, but are not limited to, a *Schizochytrium* PUFA PKS system, and a *Thraustochytrium* PUFA PKS system. In one embodiment, the PUFA PKS system can be expressed in a prokaryotic host cell. In another embodiment, the PUFA PKS system can be expressed in a eukaryotic host cell.

Another embodiment of the present invention relates to a recombinant host cell which has been modified to express a non-bacterial PUFA PKS system, wherein the PKS system catalyzes both iterative and non-iterative enzymatic reactions, and wherein the non-bacterial PUFA PKS system comprises at least the following biologically active domains: (a) at least one enoyl ACP-reductase (ER) domain; (b) multiple acyl carrier protein (ACP) domains (at least four); (c) at least two β-keto acyl-ACP synthase (KS) domains; (d) at least one acyltransferase (AT) domain; (e) at least one ketoreductase (KR) domain; (f) at least two FabA-like β-hydroxy acyl-ACP dehydrase (DH) domains; (g) at least one chain length factor (CLF) domain; and (h) at least one malonyl-CoA:ACP acyltransferase (MAT) domain.

One aspect of this embodiment of the invention relates to a method to produce a product containing at least one PUFA, comprising growing a plant comprising any of the recombinant host cells described above, wherein the recombinant host cell is a plant cell, under conditions effective to produce the product. Another aspect of this embodiment of the invention relates to a method to produce a product containing at least one PUFA, comprising culturing a culture containing any of the recombinant host cells described above, wherein the host cell is a microbial cell, under conditions effective to produce the product. In a preferred embodiment, the PKS system in the host cell catalyzes the direct production of triglycerides.

Another embodiment of the present invention relates to a microorganism comprising a non-bacterial, polyunsaturated fatty acid (PUFA) polyketide synthase (PKS) system, wherein the PKS catalyzes both iterative and non-iterative enzymatic reactions, and wherein the PUFA PKS system comprises: (a) at least two enoyl ACP-reductase (ER) domains; (b) at least six acyl carrier protein (ACP) domains; (c) at least two β-keto acyl-ACP synthase (KS) domains; (d) at least one acyltransferase (AT) domain; (e) at least one ketoreductase (KR) domain; (f) at least two FabA-like β-hydroxy acyl-ACP dehydrase (DH) domains; (g) at least one chain length factor (CLF) domain; and (h) at least one malonyl-CoA:ACP acyltransferase (MAT) domain. Preferably, the microorganism is a non-bacterial microorganism and more preferably, a eukaryotic microorganism.

Yet another embodiment of the present invention relates to a microorganism comprising a non-bacterial, polyunsaturated fatty acid (PUFA) polyketide synthase (PKS) system, wherein the PKS catalyzes both iterative and non-iterative enzymatic reactions, and wherein the PUFA PKS system comprises: (a) at least one enoyl ACP-reductase (ER) domain; (b) multiple acyl carrier protein (ACP) domains (at least four); (c) at least two β-keto acyl-ACP synthase (KS) domains; (d) at least one acyltransferase (AT) domain; (e) at least one ketoreductase (KR) domain; (f) at least two FabA-like β-hydroxy acyl-ACP dehydrase (DH) domains; (g) at least one chain length factor (CLF) domain; and (h) at least one malonyl-CoA:ACP acyltransferase (MAT) domain.

In one embodiment of the present invention, it is contemplated that a mutagenesis program could be combined with a selective screening process to obtain bioactive molecules of interest. This would include methods to search for a range of bioactive compounds. This search would not be restricted to production of those molecules with *cis* double bonds. The mutagenesis methods could include, but are not limited to: chemical mutagenesis, gene shuffling, switching regions of the genes encoding specific enzymatic domains, or mutagenesis restricted to specific regions of those genes, as well as other methods.

For example, high throughput mutagenesis methods could be used to influence or optimize production of the desired bioactive molecule. Once an effective model system has been developed, one could modify these genes in a high throughput manner. Utilization of these technologies can be envisioned on two levels. First, if a sufficiently selective screen for production of a product of interest (e.g., ARA) can be devised, it could be used to attempt to alter the system to produce this product (e.g., in lieu of, or in concert with, other strategies such as those discussed above). Additionally, if the strategies outlined above resulted in a set of genes that did produce the product of interest, the high throughput technologies could then be used to optimize the system. For example, if the introduced domain only functioned at relatively low temperatures, selection methods could be devised to permit removing that limitation. In one embodiment of the invention, screening methods are used to identify additional non-bacterial organisms having novel PKS systems similar to the PUFA PKS system of *Schizochytrium,* as described herein (see above). Homologous PKS systems identified in such organisms can be used in methods similar to those described herein for the *Schizochytrium,* as well as for an additional source of genetic material from which to create, further modify and/or mutate a PKS system for expression in that microorganism, in another microorganism, or in a higher plant, to produce a variety of compounds.

It is recognized that many genetic alterations, either random or directed, which one may introduce into a native (endogenous, natural) PKS system, will result in an inactivation of enzymatic functions. We describe a system to select for only those modifications that do not block the ability of the PKS system to produce a product. For example, the FabB- strain of *E. coli* is incapable of synthesizing unsaturated fatty acids and requires supplementation of the medium with fatty acids that can substitute for its normal unsaturated fatty acids in order to grow (see Metz et al., 2001, *supra*). However, this requirement (for supplementation of the medium) can be removed when the strain is transformed with a functional PUFA-PKS system (i.e. one that produces a PUFA product in the *E. coli* host - see (Metz et al., 2001, *supra*, Figure 2A). The transformed FabB- strain now requires a functional PUFA-PKS system (to produce the unsaturated fatty acids) for growth without supplementation. The key element in this example is that production of a wide range of unsaturated fatty acid will suffice (even unsaturated fatty acid substitutes such as branched chain fatty acids). Therefore, one could create a large number of mutations in one or more of the PUFA PKS genes disclosedherein, and then transform the appropriatelymodifiedFabB- strain (e.g. create mutations in an expression construct containing an ER domain and transform aFabB-strain having the other essential domains on a separate plasmid - or integrated into the chromosome) and select only for those transformants that grow without supplementation of the medium (i.e., that still possessed an ability to produce amolecule that could complement the FabB- defect). Additional screens could be developed to look for particular compounds (e.g. use of GC for fatty acids) being produced in this selective subset of an active PKS system. One could envision a number of similar selective screens for bioactive molecules of interest.

As described above, in one embodiment of the present invention, a genetically modified microorganism or plant includes a microorganism or plant which has an enhanced abilityto synthesize desired bioactive molecules (products) or which has a newly introduced ability to synthesize specific products (e.g., to synthesize a specific antibiotic). According to the present invention, "an enhanced ability to synthesize" a product refers to any enhancement, or up-regulation, in a pathway related to the synthesis of the product such that the microorganism or plant produces an increased amount of the product (including any production of a product where there was none before) as compared to the wild-type microorganism or plant, cultured or grown, under the same conditions. Methods to produce such genetically modified organisms have been described in detail above.

One embodiment of the present invention is a method to produce desired bioactive molecules (also referred to as products or compounds) by growing or culturing a genetically modified microorganism or plant of the present invention (described in detail above). Such a method includes the step of culturing in a fermentation medium or growing in a suitable environment, such as soil, a microorganism or plant, respectively, that has a genetic modification as described previously herein and in accordance with the present invention. In a preferred embodiment, method to produce bioactive molecules of the present invention includes the step of culturing under conditions effective to produce the bioactive molecule a genetically modified organism that expresses a PKS system comprising at least one biologically active domain of apolyunsaturated fatty acid (PUFA) polyketide synthase (PKS) system in accordance with the invention. In this preferred aspect of the method, the organism is genetically modified to affect the activity of the PKS system (described in detail above). Preferred host cells for genetic modification related to the PUFA PKS system of the invention are described above.

In the method of production of desired bioactive compounds of the present invention, a genetically modified microorganism is cultured or grown in a suitable medium, under conditions effective to produce the bioactive compound. An appropriate, or effective, medium refers to any medium in which a genetically modified microorganism of the present invention, when cultured, is capable of producing the desired product Such a medium is typically an aqueous medium comprising assimilable carbon, nitrogen and phosphate sources. Such a medium can also include appropriate salts, minerals, metals and other nutrients. Microorganisms of the present invention can be cultured in conventional fermentation bioreactors. The microorganisms can be cultured by any fermentation process which includes, but is not limited to, batch, fed-batch, cell recycle, and continuous fermentation. Preferred growth conditions for potential host microorganisms according to the present invention are well known in the art. The desired bioactive molecules produced by the genetically modified microorganism can be recovered from the fermentation medium using conventional separation and purification techniques. For example, the fermentation medium can be filtered or centrifuged to remove microorganisms, cell debris and other particulate matter, and the product can be recovered from the cell-free supernatant by conventional methods, such as, for example, ion exchange, chromatography, extraction, solvent extraction, membrane separation, electrodialysis, reverse osmosis, distillation, chemical derivatization and crystallization. Alternatively, microorganisms producing the desired compound, or extracts and various fractions thereof, can be used without removal of the microorganism components from the product.

In the method for production of desired bioactive compounds of the present invention, a genetically modified plant is cultured in a fermentation medium or grown in a suitable medium such as soil. An appropriate, or effective, fermentation medium has been discussed in detail above. A suitable growth medium for higher plants includes any growth medium for plants, including, but not limited to, soil, sand, any other particulate media that support root growth (e.g. vermiculite, perlite, etc.) or Hydroponic culture, as well as suitable light, water and nutritional supplements which optimize the growth of the higher plant. The genetically modified plants of the present invention are engineered to produce significant quantities of the desired product through the activity of the PKS system that is genetically modified according to the present invention. The compounds can be recovered through purification processes which extract the compounds from the plant. In a preferred embodiment, the compound is recovered by harvesting the plant. In this embodiment, the plant can be consumed in its natural state or further processed into consumable products.

As described above, a genetically modified microorganism useful in the present invention can, in one aspect, endogenously contain and express a PUFA PKS system, and the genetic modification can be a genetic modification of one or more of the functional domains of the endogenous PUFA PKS system, whereby the modification has some effect on the activity of the PUFA PKS system. In another aspect, such an organism can endogenously contain and express a PUFA PKS system, and the genetic modification can be an introduction of at least one exogenous nucleic acid sequence (e.g., a recombinant nucleic acid molecule), wherein the exogenous nucleic acid sequence encodes at least one biologically active domain or protein from a second PKS system and/or a protein that affects the activity of said PUFA PKS system (e.g., a phosphopantetheinyl transferases (PPTase), discussed below). In yet another aspect, the organism does not necessarily endogenously (naturally) contain a PUFA PKS system, but is genetically modified to introduce at least one recombinant nucleic acid molecule encoding an amino acid sequence having the biological activity of at least one domain of a PUFA PKS system. In this aspect, PUFA PKS activity is affected by introducing or increasing PUFA PKS activity in the organism. Various embodiments associated with each of these aspects have been discussed in detail above.

In one embodiment of the method to produce bioactive compounds, the genetic modification changes at least one product produced by the endogenous PKS system, as compared to a wild-type organism.

In another embodiment, the organism endogenously expresses a PKS system comprising the at least one biologically active domain of the PUFA PKS system, and the genetic modification comprises transfection of the organism with a recombinant nucleic acid molecule selected from the group consisting of: a recombinant nucleic acid molecule encoding at least one biologically active domain from a second PKS system and a recombinant nucleic acid molecule encoding a protein that affects the activity of the PUFA PKS system. In this embodiment, the genetic modification preferably changes at least one product produced by the endogenous PKS system, as compared to a wild-type organism. A second PKS system can include another PUFA PKS system (bacterial or non-bacterial), a type I PKS system, a type II PKS system, and/or a modular PKS system. Examples of proteins that affect the activity of a PKS system have been described above (e.g., PPTase).

In another embodiment, the organism is genetically modified by transfection with a recombinant nucleic acid molecule encoding the at least one domain of the polyunsaturated fatty acid (PUFA) polyketide synthase (PKS) system. Such recombinant nucleic acid molecules have been described in detail previously herein.

In another embodiment, the organism endogenously expresses a non-bacterial PUFA PKS system, and the genetic modification comprises substitution of a domain from a different PKS system for a nucleic acid sequence encoding at least one domain of the non-bacterial PUFA PKS system. In another embodiment, the organism endogenously expresses a non-bacterial PUFA PKS system that has been modified by transfecting the organism with a recombinant nucleic acid molecule encoding a protein that regulates the chain length of fatty acids produced by the PUFA PKS system. In one aspect, the recombinant nucleic acid molecule encoding a protein that regulates the chain length of fatty acids replaces a nucleic acid sequence encoding a chain length factor in the non-bacterial PUFA PKS system. In another aspect, the protein that regulates the chain length of fatty acids produced by the PUFA PKS system is a chain length factor. In another aspect, the protein that regulates the chain length of fatty acids produced by the PUFA PKS system is a chain length factor that directs the synthesis of C20 units.

In another embodiment, the organism expresses a non-bacterial PUFA PKS system comprising a genetic modification in a domain encoding β-ketoacyl-ACP synthase (KS), wherein the modification alters the ratio of long chain fatty acids produced by the PUFA PKS system as compared to in the absence of the modification. In one aspect of this embodiment, the modification is selected from the group consisting of a deletion of all or a part of the domain, a substitution of a homologous domain from a different organism for the domain, and a mutation of the domain.

In one embodiment of the method to produce a bioactive molecule, the organism produces a polyunsaturated fatty acid (PUFA) profile that differs from the naturally occurring organism without a genetic modification.

Many other genetic modifications useful for producing bioactive molecules will be apparent to those of skill in the art, given the present disclosure, and various other modifications have been discussed previously herein. The present invention contemplates any genetic modification related to a PUFA PKS system as described herein which results in the production of a desired bioactive molecule.

Bioactive molecules, according to the present invention, include any molecules (compounds, products, etc.) that have a biological activity, and that can be produced by a PKS system that comprises at least one amino acid sequence having a biological activity of at least one functional domain of a non-bacterial PUFA PKS system as described herein. Such bioactive molecules can include, but are not limited to: a polyunsaturated fatty acid (PUFA), an anti-inflammatory formulation, a chemotherapeutic agent, an active excipient, an osteoporosis drug, an anti-depressant, an anti-convulsant, an anti-*Heliobactor pylori* drug, a drug for treatment of neurodegenerative disease, a drug for treatment of degenerative liver disease, an antibiotic, and a cholesterol lowering formulation. One advantage of the non-bacterial PUFA PKS system of the present invention is the ability of such a system to introduce carbon-carbon double bonds in the *cis* configuration, and molecules including a double bond at every third carbon. This ability can be utilized to produce a variety of compounds.

Preferably, bioactive compounds of interest are produced by the genetically modified microorganism in an amount that is greater than about 0.05%, and preferably greater than about 0.1%, and more preferably greater than about 0.25%, andmorepreferablygreaterthan about 0.5%, and more preferably greater than about 0.75%, and more preferably greater than about 1%, and more preferably greater than about 2.5%, and more preferably greater than about 5%, and more preferably greater than about 10%, and more preferably greater than about 15%, and even more preferably greater than about 20% of the dry weight of the microorganism. For lipid compounds, preferably, such compounds are produced in an amount that is greater than about 5% of the dry weight of the microorganism. For other bioactive compounds, such as antibiotics or compounds that are synthesized in smaller amounts, those strains possessing such compounds at of the dryweight of the microorganism are identified as predictably containing a novel PKS system of the type described above. In some embodiments, particular bioactive molecules (compounds) are secreted by the microorganism, rather than accumulating. Therefore, suchbioactive molecules are generally recovered from the culture medium and the concentration of molecule produced will vary depending on the microorganism and the size of the culture.

Yet another embodiment of the present invention relates to a method to produce a humanized animal milk. This method includes the steps of genetically modifying milk-producing cells of a milk-producing non-human animal with at least one recombinant nucleic acid molecule comprising a nucleic acid sequence encoding at least one biologically active domain of a PUFA PKS system according to the invention. The PUFA PKS system is a non-bacterial PUFA PKS system.

Methods to genetically modify a host cell and to produce a genetically modified non-human, milk-producing animal, are known in the art. Examples of host animals to modify include cattle, sheep, pigs, goats, yaks, etc., which are amenable to genetic manipulation and cloning for rapid expansion of a transgene expressing population. For animals, PKS-like transgenes can be adapted for expression in target organelles, tissues and body fluids through modification of the gene regulatory regions. Of particular interest is the production of PUFAs in the breast milk of the host animal.

The following examples are provided for the purpose of illustration and are not intended to limit the scope of the present invention.

### Examples

### Example 1

The following example describes the further analysis of PKS related sequences from *Schizochytrium.*

The present inventors have sequenced the genomic DNA including the entire length of all three open reading frames (Orfs) in the *Schizochytrium* PUFA PKS system using the general methods outlined in Examples 8 and 9 from PCT Publication No. WO 0042195 and U.S. Application Serial No. 09/231,899. The biologically active domains in the *Schizochytrium* PKS proteins are depicted graphically in Fig. 1. The domain structure of the *Schizochytrium* PUFA PKS system is described more particularly as follows.

### Open Reading Frame A (OrfA):

The complete nucleotide sequence for OrfA is represented herein as SEQ ID NO:1. OrfA is a 8730 nucleotide sequence (not including the stop codon) which encodes a 2910 amino acid sequence, represented herein as SEQ ID NO:2. Within OrfA are twelve domains:
(a) one β-keto acyl-ACP synthase (KS) domain;
(b) one malonyl-CoA:ACP acyltransferase (MAT) domain;
(c) nine acyl carrier protein (ACP) domains;
(d) one ketoreductase (KR) domain.

The domains contained within OrfA have been determined based on:
(1) results of an analysis with Pfam program (Pfam is a database of multiple alignments of protein domains or conserved protein regions. The alignments represent some evolutionary conserved structure that has implications for the protein's function. Profile hidden Markov models (profile HMMs) built from the Pfam alignments can be very useful for automatically recognizing that a new protein belongs to an existing protein family, even if the homology is weak. Unlike standard pairwise alignment methods (e.g. BLAST, FASTA), Pfam HMMs deal sensibly with multidomain proteins. The reference provided for the Pfam version used is: Bateman A, Birney E, Cerruti L, Durbin R, Etwiller L, Eddy SR, Griffiths-Jones S, Howe KL, Marshall M, Sonnhammer EL (2002) Nucleic Acids Research 30(1):276-280); and/or
(2) homology comparison to bacterial PUFA-PKS systems (e.g., *Shewanella*) using a BLAST 2.0 Basic BLAST homology search using blastp for amino acid searches with standard default parameters, wherein the query sequence is filtered for low complexity regions by default (described in Altschul, S.F., Madden, T.L., Schääffer, A.A., Zhang, J., Zhang, Z., Miller, W. & Lipman, D.J. (1997) "Gapped BLAST and PSI-BLAST: a new generation of protein database search programs." Nucleic Acids Res. 25:3389-3402 ).

Sequences provided for individual domains are believed to contain the full length of the sequence encoding a functional domain, and may contain additional flanking sequence within the Orf.

### ORFA-KS

The first domain in OrfA is a KS domain, also referred to herein as ORFA-KS. This domain is contained within the nucleotide sequence spanning from a starting point of between about positions 1 and 40 of SEQ ID NO: 1 (OrfA) to an ending point of between about positions 1428 and 1500 of SEQ ID NO:1. The nucleotide sequence containing the sequence encoding the ORFA-KS domain is represented herein as SEQ ID NO:7 (positions 1-1500 of SEQ ID NO:1). The amino acid sequence containing the KS domain spans from a starting point of between about positions 1 and 14 of SEQ ID NO:2 (ORFA) to an ending point of between about positions 476 and 500 of SEQ ID NO:2. The amino acid sequence containing the ORFA-KS domain is represented herein as SEQ ID NO:8 (positions 1-500 of SEQ ID NO:2). It is noted that the ORFA-KS domain contains an active site motif: DXAC* (*acyl binding site C₂₁₅).

### ORFA-MAT

The second domain in OrfA is a MAT domain, also referred to herein as ORFA-MAT. This domain is contained within the nucleotide sequence spanning from a starting point of between about positions 1723 and 1798 of SEQ ID NO: 1 (OrfA) to an ending point of between about positions 2805 and 3000 of SEQ ID NO:1. The nucleotide sequence containing the sequence encoding the ORFA-MAT domain is represented herein as SEQ ID NO:9 (positions 1723-3000 of SEQ ID NO:1). The amino acid sequence containing the MAT domain spans from a starting point of between about positions 575 and 600 of SEQ ID NO:2 (ORFA) to an ending point of between about positions 935 and 1000 of SEQ ID NO:2. The amino acid sequence containing the ORFA-MAT domain is represented herein as SEQ ID NO:10 (positions 575-1000 of SEQ ID NO:2). It is noted that the ORFA-MAT domain contains an active site motif: GHS*XG (*acyl binding site S₇₀₆), represented herein as SEQ ID NO:11.

### ORFA-ACP#1-9

Domains 3-11 of OrfA are nine tandem ACP domains, also referred to herein as ORFA-ACP (the first domain in the sequence is ORFA-ACP1, the second domain is ORFA-ACP2, the third domain is ORFA-ACP3, etc.). The first ACP domain, ORFA-ACP1, is contained within the nucleotide sequence spanning from about position 3343 to about position 3600 of SEQ ID NO: 1 (OrfA). The nucleotide sequence containing the sequence encoding the ORFA-ACP1 domain is represented herein as SEQ ID NO:12 (positions 3343-3600 of SEQ ID NO:1). The amino acid sequence containing the first ACP domain spans from about position 1115 to about position 1200 of SEQ ID NO:2. The amino acid sequence containing the ORFA-ACP 1 domain is represented herein as SEQ ID NO:13 (positions 1115-1200 of SEQ ID NO:2). It is noted that the ORFA-ACP1 domain contains an active site motif: LGIDS* (*pantetheine binding motif S₁₁₅₇), represented herein by SEQ ID NO:14. The nucleotide and amino acid sequences of all nine ACP domains are highly conserved and therefore, the sequence for each domain is not represented herein by an individual sequence identifier. However, based on this information, one of skill in the art can readily determine the sequence for each of the other eight ACP domains. The repeat interval for the nine domains is approximately about 110 to about 330 nucleotides of SEQ ID NO:1.

All nine ACP domains together span a region of OrfA of from about position 3283 to about position 6288 of SEQ ID NO:1, which corresponds to amino acid positions of from about 1095 to about 2096 of SEQ ID NO:2. This region includes the linker segments between individual ACP domains. Each of the nine ACP domains contains a pantetheine binding motif LGIDS* (represented herein by SEQ ID NO:14), wherein * is the pantetheine binding site S. At each end of the ACP domain region and between each ACP domain is a region that is highly enriched for proline (P) and alanine (A), which is believed to be a linker region. For example, between ACP domains 1 and 2 is the sequence: APAPVKAAAPAAPVASAPAPA, represented herein as SEQ ID NO:15.

### ORFA-KR

Domain 12 in OrfA is a KR domain, also referred to herein as ORFA-KR. This domain is contained within the nucleotide sequence spanning from a starting point of about position 6598 of SEQ ID NO:1 to an ending point of about position 8730 of SEQ ID NO:1. The nucleotide sequence containing the sequence encoding the ORFA-KR domain is represented herein as SEQ ID NO:17 (positions 6598-8730 of SEQ ID NO:1). The amino acid sequence containing the KR domain spans from a starting point of about position 2200 of SEQ ID NO:2 (ORFA) to an ending point of about position 2910 of SEQ ID NO:2. The amino acid sequence containing the ORFA-KR domain is represented herein as SEQ ID NO:18 (positions 2200-2910 of SEQ ID NO:2). Within the KR domain is a core region with homology to short chain aldehyde-dehydrogenases (KR is a member of this family). This core region spans from about position 7198 to about position 7500 of SEQ ID NO:1, which corresponds to amino acid positions 2400-2500 of SEQ ID NO:2.

### Open Reading Frame B (OrfB):

The complete nucleotide sequence for OrfB is represented herein as SEQ ID NO:3. OrfB is a 6177 nucleotide sequence (not including the stop codon) which encodes a 2059 amino acid sequence, represented herein as SEQ ID NO:4. Within OrfB are four domains:
(a) β-keto acyl-ACP synthase (KS) domain;
(b) one chain length factor (CLF) domain;
(c) one acyl transferase (AT) domain;
(d) one enoyl ACP-reductase (ER) domain.

The domains contained within ORFB have been determined based on: (1) results of an analysis with Pfam program, described above; and/or (2) homology comparison to bacterial PUFA-PKS systems (e.g., *Shewanella*) using a BLAST 2.0 Basic BLAST homology search, also described above. Sequences provided for individual domains are believed to contain the full length of the sequence encoding a functional domain, and may contain additional flanking sequence within the Orf.

### ORFB-KS

The first domain in OrfB is a KS domain, also referred to herein as ORFB-KS. This domain is contained within the nucleotide sequence spanning from a starting point of between about positions 1 and 43 of SEQ ID NO:3 (OrfB) to an ending point of between about positions 1332 and 1350 of SEQ ID NO:3. The nucleotide sequence containing the sequence encoding the ORFB-KS domain is represented herein as SEQ ID NO:19 (positions 1-1350 of SEQ ID NO:3). The amino acid sequence containing the KS domain spans from a starting point of between about positions 1 and 15 of SEQ ID NO:4 (ORFB) to an ending point of between about positions 444 and 450 of SEQ ID NO:4. The amino acid sequence containing the ORFB-KS domain is represented herein as SEQ ID NO:20 (positions 1-450 of SEQ ID NO:4). It is noted that the ORFB-KS domain contains an active site motif: DXAC* (*acyl binding site C₁₉₆).

### ORFB-CLF

The second domain in OrfB is a CLF domain, also referred to herein as ORFB-CLF. This domain is contained within the nucleotide sequence spanning from a starting point of between about positions 1378 and 1402 of SEQ ID NO:3 (OrfB) to an ending point of between about positions 2682 and 2700 of SEQ ID NO:3. The nucleotide sequence containing the sequence encoding the ORFB-CLF domain is represented herein as SEQ ID NO:21 (positions 1378-2700 of SEQ ID NO:3). The amino acid sequence containing the CLF domain spans from a starting point of between about positions 460 and 468 of SEQ ID NO:4 (ORFB) to an ending point of between about positions 894 and 900 of SEQ ID NO:4. The amino acid sequence containing the ORFB-CLF domain is represented herein as SEQ ID NO:22 (positions 460-900 of SEQ ID NO:4). It is noted that the ORFB-CLF domain contains a KS active site motif without the acyl-binding cysteine.

### ORFB-AT

The third domain in OrfB is an AT domain, also referred to herein as ORFB-AT. This domain is contained within the nucleotide sequence spanning from a starting point of between about positions 2701 and 3598 of SEQ ID NO:3 (OrfB) to an ending point of between about positions 3975 and 4200 of SEQ ID NO:3. The nucleotide sequence containing the sequence encoding the ORFB-AT domain is represented herein as SEQ ID NO:23 (positions 2701-4200 of SEQ ID NO:3). The amino acid sequence containing the AT domain spans from a starting point of between about positions 901 and 1200 of SEQ ID NO:4 (ORFB) to an ending point of between about positions 1325 and 1400 of SEQ ID NO:4. The amino acid sequence containing the ORFB-AT domain is represented herein as SEQ ID NO:24 (positions 901-1400 of SEQ ID NO:4). It is noted that the ORFB-AT domain contains an AT active site motif of GxS*xG (*acyl binding site S₁₁₄₀).

### ORFB-ER

The fourth domain in OrfB is an ER domain, also referred to herein as ORFB-ER. This domain is contained within the nucleotide sequence spanning from a starting point of about position 4648 of SEQ ID NO:3 (OrfB) to an ending point of about position 6177 of SEQ ID NO:3. The nucleotide sequence containing the sequence encoding the ORFB-ER domain is represented herein as SEQ ID NO:25 (positions 4648-6177 of SEQ ID NO:3). The amino acid sequence containing the ER domain spans from a starting point of about position 1550 of SEQ ID NO:4 (ORFB) to an ending point of about position 2059 of SEQ ID NO:4. The amino acid sequence containing the ORFB-ER domain is represented herein as SEQ ID NO:26 (positions 1550-2059 of SEQ ID NO:4).

### Open Reading Frame C (OrfC):

The complete nucleotide sequence for OrfC is represented herein as SEQ ID NO:5. OrfC is a 4509 nucleotide sequence (not including the stop codon) which encodes a 1503 amino acid sequence, represented herein as SEQ ID NO:6. Within OrfC are three domains:
(a) two FabA-like β-hydroxy acyl-ACP dehydrase (DH) domains;
(b) one enoyl ACP-reductase (ER) domain.

The domains contained within ORFC have been determined based on: (1) results of an analysis with Pfam program, described above; and/or (2) homology comparison to bacterial PUFA-PKS systems (e.g., *Shewanella*) using a BLAST 2.0 Basic BLAST homology search, also described above. Sequences provided for individual domains are believed to contain the full length of the sequence encoding a functional domain, and may contain additional flanking sequence within the Orf.

### ORFC-DH1

The first domain in OrfC is a DH domain, also referred to herein as ORFC-DH1. This is one of two DH domains in OrfC, and therefore is designated DH1. This domain is contained within the nucleotide sequence spanning from a starting point of between about positions 1 and 778 of SEQ ID NO:5 (OrfC) to an ending point of between about positions 1233 and 1350 of SEQ ID NO:5. The nucleotide sequence containing the sequence encoding the ORFC-DH1 domain is represented herein as SEQ ID NO:27 (positions 1-1350 of SEQ ID NO:5). The amino acid sequence containing the DH1 domain spans from a starting point of between about positions 1 and 260 of SEQ ID NO:6 (ORFC) to an ending point of between about positions 411 and 450 of SEQ ID NO:6. The amino acid sequence containing the ORFC-DH1 domain is represented herein as SEQ ID NO:28 (positions 1-450 of SEQ ID NO:6).

### ORFC-DH2

The second domain in OrfC is a DH domain, also referred to herein as ORFC-DH2. This is the second of two DH domains in OrfC, and therefore is designated DH2. This domain is contained within the nucleotide sequence spanning from a starting point of between about positions 1351 and 2437 of SEQ ID NO:5 (OrfC) to an ending point of between about positions 2607 and 2850 of SEQ ID NO:5. The nucleotide sequence containing the sequence encoding the ORFC-DH2 domain is represented herein as SEQ ID NO:29 (positions 1351-2850 of SEQ ID NO:5). The amino acid sequence containing the DH2 domain spans from a starting point of between about positions 451 and 813 of SEQ ID NO:6 (ORFC) to an ending point of between about positions 869 and 950 of SEQ ID NO:6. The amino acid sequence containing the ORFC-DH2 domain is represented herein as SEQ ID NO:30 (positions 451-950 of SEQ ID NO:6).

### ORFC-ER

The third domain in OrfC is an ER domain, also referred to herein as ORFC-ER. This domain is contained within the nucleotide sequence spanning from a starting point of about position 2998 of SEQ ID NO:5 (OrfC) to an ending point of about position 4509 of SEQ ID NO:5. The nucleotide sequence containing the sequence encoding the ORFC-ER domain is represented herein as SEQ ID NO:31 (positions 2998-4509 of SEQ ID NO:5). The amino acid sequence containing the ER domain spans from a starting point of about position 1000 of SEQ ID NO:6 (ORFC) to an ending point of about position 1502 of SEQ ID NO:6. The amino acid sequence containing the ORFC-ER domain is represented herein as SEQ ID NO:32 (positions 1000-1502 of SEQ ID NO:6).

### Example 2

The following example describes the use of the screening process of the present invention to identify three other non-bacterial organisms comprising a PUFA PKS system according to the present invention.

*Thraustochytrium* sp. 23B (ATCC 20892) was cultured according to the screening method described in U.S. Provisional Application Serial No. 60/298,796 and as described in detail herein.

The biorational screen (using shake flask cultures) developed for detecting microorganisms containing PUFA producing PKS systems is as follows:
Two mL of a culture of the strain/microorganism to be tested is placed in 250 mL baffled shake flask with 50 mL culture media (aerobic treatment) and another 2 mL of culture of the same strain is placed in a 250 mL non-baffled shake flask with 200 mL culture medium (anoxic treatment). Both flasks are placed on a shaker table at 200 rpm. After 48-72 hr of culture time, the cultures are harvested by centrifugation and the cells analyzed for fatty acid methyl esters via gas chromatography to determine the following data for each culture: (1) fatty acid profile; (2) PUFA content; (3) fat content (estimated as amount total fatty acids (TFA)).
These data are then analyzed asking the following five questions:

### Selection Criteria: Low O₂/Anoxic Flask vs. Aerobic Flask (Yes/No)

(1) Did the DHA (or other PUFA content) (as % FAME) stay about the same or preferably increase in the low oxygen culture compared to the aerobic culture?
(2) Is C14:0 + C16:0 + C16:1 greater than about 40% TFA in the anoxic culture?
(3) Is there very little (>1% as FAME) or no precursors (C18:3n-3 +C18:2n-6+C18:3n-6) to the conventional oxygen dependent elongase/desaturase pathway in the anoxic culture?
(4) Did fat content (as amount total fatty acids/cell dry weight) increase in the low oxygen culture compared to the aerobic culture?
(5) Did DHA (or other PUFA content) increase as % cell dry weight in the low oxygen culture compared to the aerobic culture?

If first three questions are answered yes, there is a good indication that the strain contains a PKS genetic system for making long chain PUFAs. The more questions that are answered yes (preferably the first three questions must be answered yes), the stronger the indication that the strain contains such a PKS genetic system. If all five questions are answered yes, then there is a very strong indication that the strain contains a PKS genetic system for making long chain PUFAs.

Following the method outlined above, a frozen vial of *Thraustochytrium* sp. 23B (ATCC 20892) was used to inoculate a 250 mL shake flask containing 50 mL of RCA medium. The culture was shaken on a shaker table (200 rpm) for 72 hr at 25°C. RCA medium contains the following:

**RCA Medium**

| | |
|---|---|
| Deionized water | 1000 mL |
| Reef Crystals® sea salts | 40g/L |
| Glucose | 20 g/L |
| Monosodium glutamate (MSG) | 20 g/L |
| Yeast extract | 1 g/L |
| PII metals* | 5 mL/L |
| Vitamin mix* | 1 mL/L |
| pH | 7.0 |

| | |
|---|---|
| *PII metal mix and vitamin mix are same as those outlined in U.S. Patent No. 5,130,742. | |

25 mL of the 72 hr old culture was then used to inoculate another 250 mL shake flask containing 50 mL of low nitrogen RCA medium (10 g/L MSG instead of 20 g/L) and the other 25 mL of culture was used to inoculate a 250 mL shake flask containing 175 mL of low-nitrogen RCA medium. The two flasks were then placed on a shaker table (200 rpm) for 72 hr at 25°C. The cells were then harvested via centrifugation and dried by lyophilization. The dried cells were analyzed for fat content and fatty acid profile and content using standard gas chromatograph procedures (such as those outlined in US 5,130,742).

The screening results for *Thraustochytrium* 23B were as follows:

| | |
|---|---|
| Did DHA as % FAME increase? | Yes (38->44%) |
| C14:0 + C16:0 + C16:1 greater than about 40% TFA? | Yes (44%) |
| No C18:3(n-3) or C18:3(n-6)? | Yes (0%) |
| Did fat content increase? | Yes (2-fold increase) |
| Did DHA (or other HUFA content increase)? | Yes (2.3-fold increase) |

The results, especially the significant increase in DHA content (as % FAME) under low oxygen conditions, conditions, strongly indicates the presence of a PUFA producing PKS system in this strain of *Thraustochytrium.*

In order to provide additional data confirming the presence of a PUFA PKS system, southern blot of *Thraustochytrium* 23B was conducted using PKS probes from *Schizochytrium* strain 20888, a strain which has already been determined to contain a PUFA producing PKS system (i.e., SEQ ID Nos:1-32 described above). Fragments of *Thraustochytrium* 23B genomic DNA which are homologous to hybridization probes from PKS PUFA synthesis genes were detected using the Southern blot technique. *Thraustochytrium* 23B genomic DNA was digested with either *Cla*I or *Kpn*I restriction endonucleases, separated by agarose gel electrophoresis (0.7% agarose, in standard Tris-Acetate-EDTA buffer), and blotted to a Schleicher &Schuell Nytran Supercharge membrane by capillary transfer. Two digoxigenin labeled hybridization probes were used - one specific for the Enoyl Reductase (ER) region of *Schizochytrium* PKS Orf B (nucleotides 5012-5511 of Orf B; SEQ ID NO:3), and the other specific for a conserved region at the beginning of *Schizochytrium* PKS Orf C (nucleotides 76-549 of OrfC; SEQ ID NO:5).

The OrfB-ER probe detected an approximately 13kb ClaI fragment and an approximately 3.6 kb KpnI fragment in the *Thraustochytrium* 23B genomic DNA. The OrfC probe detected an approximately 7.5 kb ClaI fragment and an approximately 4.6 kb KpnI fragment in the *Thraustochytrium* 23B genomic DNA.

Finally, a recombinant genomic library, consisting of DNA fragments from *Thraustochytrium* 23B genomic DNA inserted into vector lambda FIX II (Stratagene), was screened using digoxigenin labeled probes corresponding to the following segments of *Schizochytrium* 20888 PUFA-PKS genes: nucleotides 7385-7879 of Orf A (SEQ ID NO:1), nucleotides 5012-5511 of Orf B (SEQ ID NO:3), and nucleotides 76-549 of Orf C (SEQ ID NO:5). Each of these probes detected positive plaques from the *Thraustochytrium* 23B library, indicating extensive homology between the *Schizochytrium* PUFA-PKS genes and the genes of *Thraustochytrium* 23B.

In summary, these results demonstrate that *Thraustochytrium* 23B genomic DNA contains sequences that are homologous to PKS genes from *Schizochytrium* 20888.

This Thraustochytrid microorganism is encompassed herein as an additional sources of these genes for use in the embodiments above.

*Thraustochytrium* 23B (ATCC 20892) is significantly different from *Schizochytrium* sp. (ATCC 20888) in its fatty acid profile. *Thraustochytrium* 23B can have DHA:DPA(n-6) ratios as high as 14:1 compared to only 2-3:1 in *Schizochytrium* (ATCC 20888). *Thraustochytrium* 23B can also have higher levels of C20:5(n-3). Analysis of the domains in the PUFA PKS system of *Thraustochytrium* 23B in comparison to the known *Schizochytrium* PUFA PKS system should provide us with key information on how to modify these domains to influence the ratio and types of PUFA produced using these systems.

The screening method described above has been utilized the identify other potential candidate strains containing a PUFA PKS system. Two additional strains that have been identified by the present inventors to have PUFA PKS systems are *Schizochytrium limacium* (SR21) Honda & Yokochi (IF032693)and Ulkenia (BP-5601). Both were screened as above but in N2 media (glucose: 60g/L; KH₂PO₄: 4.0 g/l; yeast extract: 1.0 g/L; corn steep liquor: 1 mL/L; NH₄NO₃: 1.0 g/L; artificial sea salts (Reef Crystals): 20 g/L; all above concentrations mixed in deionized water). For both the *Schizochytrium* and *Ulkenia* strains, the answers to the first three screen questions discussed above for *Thraustochytrium* 23B was yes (*Schizochytrium* - DHA % FAME 32->41% aerobic vs anoxic, 58% 14:0/16:0/16:1, 0% precursors) and (*Ulkenia* - DHA% FAME 28->44% aerobic vs anoxic, 63% 14:0/16:0/16:1, 0% precursors), indicating that these strains are good candidates for containing a PUFA PKS system. Negative answers were obtained for the final two questions for each strain: fat decreased from 61% dry wt to 22% dry weight, and DHA from 21-9% dry weight in S. *limacium* and fat decreased from 59 to 21% dry weight in *Ulkenia* and DHA from 16% to 9% dry weight. These Thraustochytrid microorganisms are also claimed herein as additional sources of the genes for use in the embodiments above.

### Example 3

The following example demonstrates that DHA and DPA synthesis in *Schizochytrium* does not involve membrane-bound desaturases or fatty acid elongation enzymes like those described for other eukaryotes (Parker-Barnes et al., 2000, *supra*; Shanklin et al., 1998, *supra*).

*Schizochytrium* accumulates large quantities of triacylglycerols rich in DHA and docosapentaenoic acid (DPA; 22:5ω6); e.g., 30% DHA + DPA by dry weight. In eukaryotes that synthesize 20- and 22-carbon PUFAs by an elongation/desaturation pathway, the pools of 18-, 20- and 22-carbon intermediates are relatively large so that *in vivo* labeling experiments using [¹⁴C]-acetate reveal clear precursor-product kinetics for the predicted intermediates. Furthermore, radiolabeled intermediates provided exogenously to such organisms are converted to the final PUFA products.

[1-¹⁴C]acetate was supplied to a 2-day-old culture as a single pulse at zero time. Samples of cells were then harvested by centrifugation and the lipids were extracted. In addition, [1-¹⁴C]acetate uptake by the cells was estimated by measuring the radioactivity of the sample before and after centrifugation. Fatty acid methyl esters derived from the total cell lipids were separated by AgNO₃-TLC (solvent, hexane:diethyl ether:acetic acid, 70:30:2 by volume). The identity of the fatty acid bands was verified by gas chromatography, and the radioactivity in them was measured by scintillation counting. Results showed that [1-¹⁴C]-acetate was rapidly taken up by *Schizochytrium* cells and incorporated into fatty acids, but at the shortest labeling time (1 min) DHA contained 31% of the label recovered in fatty acids and this percentage remained essentially unchanged during the 10-15 min of [¹⁴C]-acetate incorporation and the subsequent 24 hours of culture growth (data not shown). Similarly, DPA represented 10% of the label throughout the experiment. There is no evidence for a precursor-product relationship between 16- or 18-carbon fatty acids and the 22-carbon polyunsaturated fatty acids. These results are consistent with rapid synthesis of DHA from [¹⁴C]-acetate involving very small (possibly enzyme-bound) pools of intermediates.

Next, cells were disrupted in 100 mM phosphate buffer (pH 7.2), containing 2 mM DTT, 2 mM EDTA, and 10% glycerol, by vortexing with glass beads. The cell-free homogenate was centrifuged at 100,000g for 1 hour. Equivalent aliquots of total homogenate, pellet (H-S pellet), and supernatant (H-S super) fractions were incubated in homogenization buffer supplemented with 20 µM acetyl-CoA, 100 µM[1-¹⁴C]malonyl-CoA (0.9 Gbq/mol), 2mM NADH, and 2 mM NADPH for 60 min at 25°C. Assays were extracted and fatty acid methyl esters were prepared and separated as described above before detection of radioactivity with an Instantimager (Packard Instruments, Meriden, CT). Results showed that a cell-free homogenate derived from *Schizochytrium* cultures incorporated [1-¹⁴C]-malonyl-CoA into DHA, DPA, and saturated fatty acids (data not shown). The same biosynthetic activities were retained by a 100,000xg supernatant fraction but were not present in the membrane pellet. These data contrast with those obtained during assays of the bacterial enzymes (see Metz et al., 2001, *supra*) and may indicate use of a different (soluble) acyl acceptor molecule. Thus, DHA and DPA synthesis in *Schizochytrium* does not involve membrane-bound desaturases or fatty acid elongation enzymes like those described for other eukaryotes.

While various embodiments of the present invention as defined in the claims have been described in detail, it is apparent that modifications and adaptations of those embodiments will occur to those skilled in the art. It is to be expressly understood, however, that such modifications and adaptations are within the scope of the following claims.

## Claims

1. An isolated nucleic acid molecule comprising a nucleic acid sequence selected from the group consisting of:
a. a nucleic acid sequence encoding an amino acid sequence of: SEQ ID NO:2;
b. a nucleic acid sequence encoding an amino acid sequence selected from the group consisting of: SEQ ID NO:8, SEQ ID NO:10, and biologically active fragments thereof, wherein the biologically active fragments exhibit a biological activity selected from the group consisting of β-keto acyl-ACP synthase (KS) activity and malonyl-CoA:ACP acyltransferase (MAT) activity;
c. a nucleic acid sequence encoding an amino acid sequence that is at least 60% identical to said amino acid sequence of (b), wherein said amino acid sequence has a biological activity selected from the group consisting of β-keto acyl-ACP synthase (KS) activity and malonyl-CoA:ACP acyltransferase (MAT) activity; and
d. a nucleic acid sequence that is fully complementary to the nucleic acid sequence of (a), (b), or (c).

2. The isolated nucleic acid molecule of Claim 1, wherein said nucleic sequence encodes an amino acid sequence that is at least 70% identical to an amino acid sequence selected from the group consisting of: SEQ ID NO:8 and SEQ ID NO:10; wherein said amino acid sequence has a biological activity selected from the group consisting of β-keto acyl-ACP synthase (KS) activity and malonyl-CoA:ACP acyltransferase (MAT) activity.

3. The isolated nucleic acid molecule of Claim 1, wherein said nucleic sequence encodes an amino acid sequence that is at least 80% identical to an amino acid sequence selected from the group consisting of: SEQ ID NO:8 and SEQ ID NO:10; wherein said amino acid sequence has a biological activity selected from the group consisting of β-keto acyl-ACP synthase (KS) activity and malonyl-CoA:ACP acyltransferase (MAT) activity.

4. The isolated nucleic acid molecule of Claim 1, wherein said nucleic sequence encodes an amino acid sequence that is at least 90% identical to an amino acid sequence selected from the group consisting of: SEQ ID NO:8 and SEQ ID NO:10; wherein said amino acid sequence has a biological activity selected from the group consisting of β-keto acyl-ACP synthase (KS) activity and malonyl-CoA:ACP acyltransferase (MAT) activity.

5. The isolated nucleic acid molecule of claim 1, further comprising a nucleic sequence encoding an amino acid sequence that is at least 60% identical to SEQ ID NO:18 or a biologically active fragment thereof, wherein the biologically active fragment exhibits ketoreductase (KR) biological activity.

6. The isolated nucleic acid molecule of Claim 1, wherein said nucleic acid sequence encodes the amino acid sequence of: SEQ ID NO:2.

7. The isolated nucleic acid molecule of Claim 1, wherein said nucleic acid molecule comprises the nucleic acid sequence of: SEQ ID NO:1.

8. A recombinant nucleic acid molecule comprising the nucleic acid molecule of Claim 1, operatively linked to at least one transcription control sequence.

9. A recombinant cell transfected with the recombinant nucleic acid molecule of Claim 8.

10. A genetically modified microorganism, wherein said microorganism expresses a PKS system comprising at least one biologically active domain of a polyunsaturated fatty acid (PUFA) polyketide synthase (PKS) system, wherein said microorganism has been genetically modified by transfection with a recombinant nucleic acid molecule encoding said at least one domain of said PUFA PKS system, and wherein said recombinant nucleic acid molecule comprises a nucleic acid sequence according to anyone of claims 1 to 7, and wherein said recombinant nucleic acid molecule is not endogenous to said genetically modified microorganism.

11. The genetically modified microorganism of Claim 10, wherein said microorganism is a Thraustochytrid.

12. The genetically modified microorganism of Claim 11, wherein said Thraustochytrid is from a genus selected from the group consisting of *Schizochytrium* and *Thraustochytrium.*

13. The genetically modified microorganism of Claim 10, wherein said microorganism has been further genetically modified to recombinantly express at least one nucleic acid molecule encoding at least one biologically active domain from a bacterial PUFA PKS system.

14. The genetically modified microorganism of Claim 10, wherein said microorganism has been further genetically modified to recombinantly express at least one biologically active domain of a PKS system from a Type I PKS system.

15. The genetically modified microorganism of Claim 10, wherein said microorganism has been further genetically modified to recombinantly express at least one biologically active domain of a PKS system from a Type II PKS system.

16. The genetically modified microorganism of Claim 10, wherein said microorganism has been further genetically modified to recombinantly express at least one biologically active domain of a PKS system from a modular PKS system.

17. A genetically modified plant, wherein said plant has been genetically modified to recombinantly express a PKS system comprising at least one biologically active domain of a polyunsaturated fatty acid (PUFA) polyketide synthase (PKS) system, wherein said domain is encoded by a nucleic acid sequence according to any of claims 1 to 7.

18. The genetically modified plant of Claim 17, wherein said plant has been further genetically modified to recombinantly express at least one nucleic acid molecule encoding at least one biologically active domain from a bacterial PUFA PKS system.

19. The genetically modified plant of Claim 17, wherein said plant has been further genetically modified to recombinantly express at least one biologically active domain of a PKS system from a Type I PKS system.

20. The genetically modified plant of Claim 17, wherein said plant has been further genetically modified to recombinantly express at least one biologically active domain of a PKS system from a Type II PKS system.

21. The genetically modified plant of Claim 17, wherein said plant has been further genetically modified to recombinantly express at least one biologically active domain of a PKS system from a modular PKS system.

22. A method to produce a bioactive molecule that is produced by a polyketide synthase system, comprising growing or culturing under conditions effective to produce said bioactive molecule a genetically modified organism, wherein said genetically modified organism is the genetically modified microorganism according to anyone of Claims 10 to 16 or the genetically modified plant according to anyone of Claims 17 to 21.

23. The method of Claim 22, wherein said genetically modified organism is a Thraustochytrid microorganism and wherein said Thraustochytrid is from a genus selected from the group consisting of *Thraustochytrium* and *Schizochytrium.*

24. The method of Claim 22, wherein said genetically modified organism is a Thraustochytrid microorganism and wherein said Thraustochytrid is *Thraustochytrium* sp. 23B (A TCC 20892).

25. The method of Claim 22, wherein said genetically modified organism is a Thraustochytrid microorganism and wherein said Thraustochytrid is *Schizochytrium* sp. SR21.

26. The method of Claim 22, wherein said genetically modified organism is a Thraustochytrid microorganism and wherein said Thraustochytrid is *Schizochytrium* strain ATCC 20888.

27. The method of Claim 22, wherein said genetically modified organism is a Thraustochytrid microorganism and wherein said Thraustochytrid is *Schizochytrium limacinum* (IFO 32693).

28. The method of Claim 22, wherein said genetically modified organism is a Thraustochytrid microorganism and wherein said Thraustochytrid is *Ulkenia* (BP5601).

29. The method of Claim 22, wherein said genetically modified organism produces a polyunsaturated fatty acid (PUFA) profile that differs from the naturally occurring organism without a genetic modification.

30. The method of Claim 22, wherein said bioactive molecule is selected from the group consisting of: an anti-inflammatory formulation, a chemotherapeutic agent, an active excipient, an osteoporosis drug, an anti-depressant, an anti-convulsant, an anti-Heliobactor pylori drug, a drug for treatment of neurodegenerative disease, a drug for treatment of degenerative liver disease, an antibiotic, and a cholesterol lowering formulation.

31. The method of Claim 22, wherein said bioactive molecule is an antibiotic.

32. The method of Claim 22, wherein said bioactive molecule is a polyunsaturated fatty acid (PUFA).

33. The method of Claim 22, wherein said bioactive molecule is a molecule including carbon-carbon double bonds in the cis configuration.

34. The method of Claim 22, wherein said bioactive molecule is a molecule including a double bond at every third carbon.

35. A method to produce a plant that has a polyunsaturated fatty acid (PUFA) profile that differs from the naturally occurring plant, comprising genetically modifying cells of said plant to express a PKS system comprising at least one recombinant nucleic acid molecule comprising a nucleic acid sequence encoding at least one biologically active domain of a PUFA PKS system, wherein said at least one domain of said PUFA PKS system is encoded by a nucleic acid sequence according to anyone of claims 1 to 7.

36. A method to produce a humanized animal milk, comprising genetically modifying milk producing cells of a milk-producing non-human animal with at least one recombinant nucleic acid molecule comprising a nucleic acid sequence encoding at least one biologically active domain of a PUFA PKS system, wherein said at least one domain of said PUFA PKS system is encoded by a nucleic acid sequence according to anyone of claims 1 to 7.

37. A method to produce a recombinant microbe, comprising genetically modifying microbial cells to express at least one recombinant nucleic acid molecule comprising a nucleic acid sequence encoding at least one biologically active domain of a PUFA PKS system, wherein said at least one domain of said PUFA PKS system is encoded by a nucleic acid sequence according to anyone of claims 1 to 7.

## Patentansprüche

1. Isoliertes Nukleinsäuremolekül, umfassend eine aus der aus:
a. einer für eine Aminosäuresequenz unter SEQ ID NO:2 codierenden Nukleinsäuresequenz;
b. einer für eine aus der aus SEQ ID NO:8, SEQ ID NO:10 und biologisch aktiven Fragmenten davon bestehenden Gruppe ausgewählte Aminosäuresequenz codierenden Nukleinsäuresequenz, wobei die biologisch aktiven Fragmente eine aus der aus β-Ketoacyl-ACP-Synthase(KS)-Aktivität und Malonyl-CoA:ACP-Acyltransferase(MAT)-Aktivität bestehenden Gruppe ausgewählte biologische Aktivität zeigen;
c. einer für eine Aminosäuresequenz, die zu wenigstens 60% mit der Aminosäuresequenz unter (b) identisch ist, codierenden Nukleinsäuresequenz, wobei die Aminosäuresequenz eine aus der aus β-Ketoacyl-ACP-Synthase(KS)-Aktivität und Malonyl-CoA:ACP-Acyltransferase(MAT)-Aktivität bestehenden Gruppe ausgewählte biologische Aktivität aufweist; und
d. einer Nukleinsäuresequenz, die zu der Nukleinsäuresequenz unter (a), (b) oder (c) vollständig komplementär ist, bestehenden Gruppe ausgewählte Nukleinsäuresequenz.

2. Isoliertes Nukleinsäuremolekül nach Anspruch 1, wobei die Nukleinsäuresequenz für eine Aminosäuresequenz codiert, die zu wenigstens 70% mit einer aus der aus SEQ ID NO:8 und SEQ ID NO:10 bestehenden Gruppe ausgewählten Aminosäuresequenz identisch ist, wobei die Aminosäuresequenz eine aus der aus β-Ketoacyl-ACP-Synthase(KS)-Aktivität und Malonyl-CoA:ACP-Acyltransferase(MAT)-Aktivität bestehenden Gruppe ausgewählte biologische Aktivität aufweist.

3. Isoliertes Nukleinsäuremolekül nach Anspruch 1, wobei die Nukleinsäuresequenz für eine Aminosäuresequenz codiert, die zu wenigstens 80% mit einer aus der aus SEQ ID NO:8 und SEQ ID NO:10 bestehenden Gruppe ausgewählten Aminosäuresequenz identisch ist, wobei die Aminosäuresequenz eine aus der aus β-Ketoacyl-ACP-Synthase(KS)-Aktivität und Malonyl-CoA:ACP-Acyltransferase(MAT)-Aktivität bestehenden Gruppe ausgewählte biologische Aktivität aufweist.

4. Isoliertes Nukleinsäuremolekül nach Anspruch 1, wobei die Nukleinsäuresequenz für eine Aminosäuresequenz codiert, die zu wenigstens 90% mit einer aus der aus SEQ ID NO:8 und SEQ ID NO:10 bestehenden Gruppe ausgewählten Aminosäuresequenz identisch ist, wobei die Aminosäuresequenz eine aus der aus β-Ketoacyl-ACP-Synthase(KS)-Aktivität und Malonyl-CoA:ACP-Acyltransferase(MAT)-Aktivität bestehenden Gruppe ausgewählte biologische Aktivität aufweist.

5. Isoliertes Nukleinsäuremolekül nach Anspruch 1, ferner umfassend eine für eine Aminosäuresequenz, die zu wenigstens 60% mit SEQ ID NO:18 identisch ist, oder ein biologisch aktives Fragment davon codierende Nukleinsäuresequenz, wobei das biologisch aktive Fragment eine biologische Ketoreduktase(KR)-Aktivität zeigt.

6. Isoliertes Nukleinsäuremolekül nach Anspruch 1, wobei die Nukleinsäuresequenz für die Aminosäuresequenz unter SEQ ID NO:2 codiert.

7. Isoliertes Nukleinsäuremolekül nach Anspruch 1, wobei das Nukleinsäuremolekül die Nukleinsäuresequenz unter SEQ ID NO:1 umfasst.

8. Rekombinantes Nukleinsäuremolekül, umfassend das Nukleinsäuremolekül nach Anspruch 1 in operativer Verknüpfung mit wenigstens einer Transkriptionskontrollsequenz.

9. Rekombinante Zelle, transfiziert mit dem rekombinanten Nukleinsäuremolekül nach Anspruch 8.

10. Gentechnisch veränderter Mikroorganismus, wobei der Mikroorganismus ein wenigstens eine biologisch aktive Domäne eines PUFA(Polyunsaturated Fatty Acid)-Polyketidsynthase(PKS)-Systems umfassendes PKS-System exprimiert, wobei der Mikroorganismus durch Transfektion mit einem für die wenigstens eine Domäne des PUFA-PKS-Systems codierenden rekombinanten Nukleinsäuremolekül gentechnisch verändert wurde und wobei das rekombinante Nukleinsäuremolekül eine Nukleinsäuresequenz gemäß einem der Ansprüche 1 bis 7 umfasst und wobei das rekombinante Nukleinsäuremolekül nicht endogen für den gentechnisch veränderten Mikroorganismus ist.

11. Gentechnisch veränderter Mikroorganismus nach Anspruch 10, wobei der Mikroorganismus zu den *Thraustochytriacae* gehört.

12. Gentechnisch veränderter Mikroorganismus nach Anspruch 11, wobei der *Thraustochytriacae*-Mikroorganismus aus einer aus der aus *Schizochytrium* und *Thraustochytrium* bestehenden Gruppe ausgewählten Gattung stammt.

13. Gentechnisch veränderter Mikroorganismus nach Anspruch 10, wobei der Mikroorganismus weiter gentechnisch verändert wurde, so dass wenigstens ein für wenigstens eine biologisch aktive Domäne aus einem bakteriellen PUFA-PKS-System codierendes Nukleinsäuremolekül rekombinant exprimiert wird.

14. Gentechnisch veränderter Mikroorganismus nach Anspruch 10, wobei der Mikroorganismus weiter gentechnisch verändert wurde, so dass wenigstens eine biologisch aktive Domäne eines PKS-Systems aus einem Typ-I-PKS-System rekombinant exprimiert wird.

15. Gentechnisch veränderter Mikroorganismus nach Anspruch 10, wobei der Mikroorganismus weiter gentechnisch verändert wurde, so dass wenigstens eine biologisch aktive Domäne eines PKS-Systems aus einem Typ-II-PKS-System rekombinant exprimiert wird.

16. Gentechnisch veränderter Mikroorganismus nach Anspruch 10, wobei der Mikroorganismus weiter gentechnisch verändert wurde, so dass wenigstens eine biologisch aktive Domäne eines PKS-Systems aus einem modularen PKS-System rekombinant exprimiert wird.

17. Gentechnisch veränderte Pflanze, wobei die Pflanze gentechnisch verändert wurde, so dass ein wenigstens eine biologisch aktive Domäne eines PUFA(Polyunsaturated Fatty Acid)-Polyketidsynthase(PKS)-Systems umfassendes PKS-System rekombinant exprimiert wird, wobei die Domäne von einer Nukleinsäuresequenz gemäß einem der Ansprüche 1 bis 7 codiert ist.

18. Gentechnisch veränderte Pflanze nach Anspruch 17, wobei die Pflanze weiter gentechnisch verändert wurde, so dass wenigstens ein für wenigstens eine biologisch aktive Domäne aus einem bakteriellen PUFA-PKS-System codierendes Nukleinsäuremolekül rekombinant exprimiert wird.

19. Gentechnisch veränderte Pflanze nach Anspruch 17, wobei die Pflanze weiter gentechnisch verändert wurde, so dass wenigstens eine biologisch aktive Domäne eines PKS-Systems aus einem Typ-I-PKS-System rekombinant exprimiert wird.

20. Gentechnisch veränderte Pflanze nach Anspruch 17, wobei die Pflanze weiter gentechnisch verändert wurde, so dass wenigstens eine biologisch aktive Domäne eines PKS-Systems aus einem Typ-II-PKS-System rekombinant exprimiert wird.

21. Gentechnisch veränderte Pflanze nach Anspruch 17, wobei die Pflanze weiter gentechnisch verändert wurde, so dass wenigstens eine biologisch aktive Domäne eines PKS-Systems aus einem modularen PKS-System rekombinant exprimiert wird.

22. Verfahren zur Herstellung eines bioaktiven Moleküls, das von einem Polyketidsynthase-System produziert wird, umfassend das Anziehen oder Kultivieren eines gentechnisch veränderten Organismus unter für die Produktion des bioaktiven Moleküls wirksamen Bedingungen, wobei es sich bei dem gentechnisch veränderten Organismus um den gentechnisch veränderten Mikroorganismus gemäß einem der Ansprüche 10 bis 16 oder die gentechnisch veränderte Pflanze gemäß einem der Ansprüche 17 bis 21 handelt.

23. Verfahren nach Anspruch 22, wobei es sich bei dem gentechnisch veränderten Organismus um einen *Thraustochytriacae*-Mikroorganismus handelt und wobei der *Thraustochytriacae*-Mikroorganismus aus einer aus der aus *Schizochytrium* und *Thraustochytrium* bestehenden Gruppe ausgewählten Gattung stammt.

24. Verfahren nach Anspruch 22, wobei es sich bei dem gentechnisch veränderten Organismus um einen *Thraustochytriacae*-Mikroorganismus handelt und wobei es sich bei dem *Thraustochytriacae-*Mikroorganismus um *Thraustochytrium* sp. 23B (ATCC 20892) handelt.

25. Verfahren nach Anspruch 22, wobei es sich bei dem gentechnisch veränderten Organismus um einen *Thraustochytriacae*-Mikroorganismus handelt und wobei es sich bei dem *Thraustochytriacae-*Mikroorganismus um *Schizochytrium* sp. SR21 handelt.

26. Verfahren nach Anspruch 22, wobei es sich bei dem gentechnisch veränderten Organismus um einen *Thraustochytriacae*-Mikroorganismus handelt und wobei es sich bei dem *Thraustochytriacae-*Mikroorganismus um *Schizochytrium*-Stamm ATCC 20888 handelt.

27. Verfahren nach Anspruch 22, wobei es sich bei dem gentechnisch veränderten Organismus um einen *Thraustochytriacae*-Mikroorganismus handelt und wobei es sich bei dem *Thraustochytriacae*-Mikroorganismus um *Schizochytrium limacinum* (IFO 32693) handelt.

28. Verfahren nach Anspruch 22, wobei es sich bei dem gentechnisch veränderten Organismus um einen *Thraustochytriacae*-Mikroorganismus handelt und wobei es sich bei dem *Thraustochytriacae-*Mikroorganismus um *Ulkenia* (BP5601) handelt.

29. Verfahren nach Anspruch 22, wobei der gentechnisch veränderte Organismus ein PUFA(Polyunsaturated Fatty Acid)-Profil produziert, das sich von dem natürlich vorkommenden Organismus ohne eine gentechnische Veränderung unterscheidet.

30. Verfahren nach Anspruch 22, wobei das bioaktive Molekül aus der aus einer entzündungshemmenden Formulierung, einem Chemotherapeutikum, einem aktiven Hilfsstoff, einem Osteoporose-Arzneistoff, einem Antidepressivum, einem Antikonvulsivum, einem Arzneistoff gegen Heliobacter pylori, einem Arzneistoff zur Behandlung einer neurodegenerativen Krankheit, einem Arzneistoff zur Behandlung einer degenerativen Leberkrankheit, einem Antibiotikum und einer cholesterinsenkenden Formulierung bestehenden Gruppe ausgewählt ist.

31. Verfahren nach Anspruch 22, wobei es sich bei dem bioaktiven Molekül um ein Antibiotikum handelt.

32. Verfahren nach Anspruch 22, wobei es sich bei dem bioaktiven Molekül um eine PUFA (Polyunsaturated Fatty Acid) handelt.

33. Verfahren nach Anspruch 22, wobei es sich bei dem bioaktiven Molekül um ein Kohlenstoff-KohlenstoffDoppelbindungen in der cis-Konfiguration enthaltendes Molekül handelt.

34. Verfahren nach Anspruch 22, wobei es sich bei dem bioaktiven Molekül um ein eine Doppelbindung an jedem dritten Kohlenstoffatom enthaltendes Molekül handelt.

35. Verfahren zur Herstellung einer Pflanze, die ein PUFA(Polyunsaturated Fatty Acid)-Profil aufweist, das sich von der natürlich vorkommenden Pflanze unterscheidet, wobei man bei dem Verfahren Zellen der Pflanze gentechnisch verändert, so dass ein wenigstens ein eine für wenigstens eine biologisch aktive Domäne eines PUFA-PKS-Systems codierende Nukleinsäuresequenz umfassendes rekombinantes Nukleinsäuremolekül umfassendes PKS-System exprimiert wird, wobei die wenigstens eine Domäne des PUFA-PKS-Systems von einer Nukleinsäuresequenz gemäß einem der Ansprüche 1 bis 7 codiert ist.

36. Verfahren zur Herstellung einer humanisierten Tiermilch, wobei man bei dem Verfahren milchproduzierende Zellen eines milchproduzierenden nichtmenschlichen Tiers mit wenigstens einem eine für wenigstens eine biologisch aktive Domäne eines PUFA-PKS-Systems codierende Nukleinsäuresequenz umfassenden rekombinanten Nukleinsäuremolekül gentechnisch verändert, wobei die wenigstens eine Domäne des PUFA-PKS-Systems von einer Nukleinsäuresequenz gemäß einem der Ansprüche 1 bis 7 codiert ist.

37. Verfahren zur Herstellung einer rekombinanten Mikrobe, bei dem man Mikrobenzellen gentechnisch verändert, so dass wenigstens ein eine für wenigstens eine biologisch aktive Domäne eines PUFA-PKS-Systems codierende Nukleinsäuresequenz umfassendes rekombinantes Nukleinsäuremolekül exprimiert wird, wobei die wenigstens eine Domäne des PUFA-PKS-Systems von einer Nukleinsäuresequenz gemäß einem der Ansprüche 1 bis 7 codiert ist.

## Revendications

1. Molécule d'acide nucléique isolée comprenant une séquence d'acide nucléique choisie dans le groupe constitué de :
a. une séquence d'acide nucléique codant pour une séquence d'acides aminés de : SEQ ID NO: 2 ;
b. une séquence d'acide nucléique codant pour une séquence d'acides aminés choisie dans le groupe constitué de : SEQ ID NO: 8, SEQ ID NO: 10, et des fragments biologiquement actifs de celle-ci, où les fragments biologiquement actifs présentent une activité biologique choisie dans le groupe constitué d'une activité β-cétoacyl-ACP synthase (KS) et d'une activité malonyl-CoA:ACP acyltransférase (MAT) ;
c. une séquence d'acide nucléique codant pour une séquence d'acides aminés qui est au moins 60 % identique à ladite séquence d'acides aminés de (b), où ladite séquence d'acides aminés a une activité biologique choisie dans le groupe constitué d'une activité β-cétoacyl-ACP synthase (KS) et d'une activité malonyl-CoA:ACP acyltransférase (MAT) ; et
d. une séquence d'acide nucléique qui est totalement complémentaire de la séquence d'acide nucléique de (a), (b), ou (c).

2. Molécule d'acide nucléique isolée de la revendication 1, où ladite séquence nucléique code pour une séquence d'acides aminés qui est au moins 70 % identique à une séquence d'acides aminés choisie dans le groupe constitué de : SEQ ID NO: 8 et SEQ ID NO: 10 ; où ladite séquence d'acides aminés a une activité biologique choisie dans le groupe constitué d'une activité β-cétoacyl-ACP synthase (KS) et d'une activité malonyl-CoA:ACP acyltransférase (MAT).

3. Molécule d'acide nucléique isolée de la revendication 1, où ladite séquence nucléique code pour une séquence d'acides aminés qui est au moins 80 % identique à une séquence d'acides aminés choisie dans le groupe constitué de : SEQ ID NO: 8 et SEQ ID NO: 10 ; où ladite séquence d'acides aminés a une activité biologique choisie dans le groupe constitué d'une activité β-cétoacyl-ACP synthase (KS) et d'une activité malonyl-CoA:ACP acyltransférase (MAT).

4. Molécule d'acide nucléique isolée de la revendication 1, où ladite séquence nucléique code pour une séquence d'acides aminés qui est au moins 90 % identique à une séquence d'acides aminés choisie dans le groupe constitué de : SEQ ID NO: 8 et SEQ ID NO: 10 ; où ladite séquence d'acides aminés a une activité biologique choisie dans le groupe constitué d'une activité β-cétoacyl-ACP synthase (KS) et d'une activité malonyl-CoA:ACP acyltransférase (MAT).

5. Molécule d'acide nucléique isolée de la revendication 1, comprenant en outre une séquence nucléique codant pour une séquence d'acides aminés qui est au moins 60 % identique à SEQ ID NO: 18 ou un fragment biologiquement actif de celle-ci, où le fragment biologiquement actif présente une activité biologique cétoréductase (KR).

6. Molécule d'acide nucléique isolée de la revendication 1, où ladite séquence d'acide nucléique code pour la séquence d'acides aminés de : SEQ ID NO: 2.

7. Molécule d'acide nucléique isolée de la revendication 1, où ladite molécule d'acide nucléique comprend la séquence d'acide nucléique de : SEQ ID NO: 1.

8. Molécule d'acide nucléique recombinante comprenant la molécule d'acide nucléique de la revendication 1, fonctionnellement liée à au moins une séquence de contrôle de transcription.

9. Cellule recombinante transfectée avec la molécule d'acide nucléique recombinante de la revendication 8.

10. Micro-organisme génétiquement modifié, où ledit micro-organisme exprime un système PKS comprenant au moins un domaine biologiquement actif d'un système acide gras polyinsaturé (PUFA) polycétide synthase (PKS), où ledit micro-organisme a été génétiquement modifié par transfection avec une molécule d'acide nucléique recombinante codant pour ledit au moins un domaine dudit système PUFA PKS, et où ladite molécule d'acide nucléique recombinante comprend une séquence d'acide nucléique selon l'une quelconque des revendications 1 à 7, et où ladite molécule d'acide nucléique recombinante n'est pas endogène pour ledit micro-organisme génétiquement modifié.

11. Micro-organisme génétiquement modifié de la revendication 10, où ledit micro-organisme est un thraustochytride.

12. Micro-organisme génétiquement modifié de la revendication 11, où ledit thraustochytride est d'un genre choisi dans le groupe constitué de *Schizochytrium* et *Thraustochytrium.*

13. Micro-organisme génétiquement modifié de la revendication 10, où ledit micro-organisme a été en outre génétiquement modifié pour exprimer de façon recombinante au moins une molécule d'acide nucléique codant pour au moins un domaine biologiquement actif d'un système PUFA PKS bactérien.

14. Micro-organisme génétiquement modifié de la revendication 10, où ledit micro-organisme a été en outre génétiquement modifié pour exprimer de façon recombinante au moins un domaine biologiquement actif d'un système PKS d'un système PKS de type I.

15. Micro-organisme génétiquement modifié de la revendication 10, où ledit micro-organisme a été en outre génétiquement modifié pour exprimer de façon recombinante au moins un domaine biologiquement actif d'un système PKS d'un système PKS de type II.

16. Micro-organisme génétiquement modifié de la revendication 10, où ledit micro-organisme a été en outre génétiquement modifié pour exprimer de façon recombinante au moins un domaine biologiquement actif d'un système PKS d'un système PKS modulaire.

17. Plante génétiquement modifiée, où ladite plante a été génétiquement modifiée pour exprimer de façon recombinante un système PKS comprenant au moins un domaine biologiquement actif d'un système acide gras polyinsaturé (PUFA) polycétide synthase (PKS), où ledit domaine est codé par une séquence d'acide nucléique selon l'une quelconque des revendications 1 à 7.

18. Plante génétiquement modifiée de la revendication 17, où ladite plante a été en outre génétiquement modifiée pour exprimer de façon recombinante au moins une molécule d'acide nucléique codant pour au moins un domaine biologiquement actif d'un système PUFA PKS bactérien.

19. Plante génétiquement modifiée de la revendication 17, où ladite plante a été en outre génétiquement modifiée pour exprimer de façon recombinante au moins un domaine biologiquement actif d'un système PKS d'un système PKS de type I.

20. Plante génétiquement modifiée de la revendication 17, où ladite plante a été en outre génétiquement modifiée pour exprimer de façon recombinante au moins un domaine biologiquement actif d'un système PKS d'un système PKS de type II.

21. Plante génétiquement modifiée de la revendication 17, où ladite plante a été en outre génétiquement modifiée pour exprimer de façon recombinante au moins un domaine biologiquement actif d'un système PKS d'un système PKS modulaire.

22. Procédé de production d'une molécule bioactive qui est produite par un système polycétide synthase, comprenant la croissance ou la culture dans des conditions efficaces pour produire ladite molécule bioactive d'un organisme génétiquement modifié, où ledit organisme génétiquement modifié est le micro-organisme génétiquement modifié selon l'une quelconque des revendications 10 à 16 ou la plante génétiquement modifiée selon l'une quelconque des revendications 17 à 21.

23. Procédé de la revendication 22, dans lequel ledit organisme génétiquement modifié est un micro-organisme thraustochytride et dans lequel ledit thraustochytride est d'un genre choisi dans le groupe constitué de *Thraustochytrium* et *Schizochytrium.*

24. Procédé de la revendication 22, dans lequel ledit organisme génétiquement modifié est un micro-organisme thraustochytride et dans lequel ledit thraustochytride est *Thraustochytrium* sp. 23B (ATCC 20892).

25. Procédé de la revendication 22, dans lequel ledit organisme génétiquement modifié est un micro-organisme thraustochytride et dans lequel ledit thraustochytride est *Schizochytrium* sp. SR21.

26. Procédé de la revendication 22, dans lequel ledit organisme génétiquement modifié est un micro-organisme thraustochytride et dans lequel ledit thraustochytride est *Schizochytrium* souche ATCC 20888.

27. Procédé de la revendication 22, dans lequel ledit organisme génétiquement modifié est un micro-organisme thraustochytride et dans lequel ledit thraustochytride est *Schizochytrium limacinum* (IFO 32693).

28. Procédé de la revendication 22, dans lequel ledit organisme génétiquement modifié est un micro-organisme thraustochytride et dans lequel ledit thraustochytride est *Ulkenia* (BP5601).

29. Procédé de la revendication 22, dans lequel ledit organisme génétiquement modifié produit un profil d'acides gras polyinsaturés (PUFA) qui diffère de l'organisme d'origine naturelle sans modification génétique.

30. Procédé de la revendication 22, dans lequel ladite molécule bioactive est choisie dans le groupe constitué de :
une formulation anti-inflammatoire, un agent chimiothérapeutique, un excipient actif, un médicament contre l'ostéoporose, un antidépresseur, un anticonvulsivant, un médicament anti-Heliobactor pylori, un médicament pour le traitement d'une maladie neurodégénérative, un médicament pour le traitement d'une maladie dégénérative du foie, un antibiotique est une formulation hypocholestérolémiante.

31. Procédé de la revendication 22, dans lequel ladite molécule bioactive est un antibiotique.

32. Procédé de la revendication 22, dans lequel ladite molécule bioactive est un acide gras polyinsaturé (PUFA).

33. Procédé de la revendication 22, dans lequel ladite molécule bioactive est une molécule comprenant des doubles liaisons carbone-carbone dans la configuration cis.

34. Procédé de la revendication 22, dans lequel ladite molécule bioactive est une molécule comprenant une double liaison tous les trois carbones.

35. Procédé de production d'une plante qui a un profil d'acides gras polyinsaturés (PUFA) qui diffère de la plante d'origine naturelle, comprenant la modification génétique de cellules de ladite plante pour exprimer un système PKS comprenant au moins une molécule d'acide nucléique recombinante comprenant une séquence d'acide nucléique codant pour au moins un domaine biologiquement actif d'un système PUFA PKS, dans lequel ledit au moins un domaine dudit système PUFA PKS est codé par une séquence d'acide nucléique selon l'une quelconque des revendications 1 à 7.

36. Procédé de production d'un lait animal humanisé, comprenant la modification génétique de cellules productrices de lait d'un animal non humain producteur de lait avec au moins une molécule d'acide nucléique recombinante comprenant une séquence d'acide nucléique codant pour au moins un domaine biologiquement actif d'un système PUFA PKS, dans lequel ledit au moins un domaine dudit système PUFA PKS est codé par une séquence d'acide nucléique selon l'une quelconque des revendications 1 à 7.

37. Procédé de production d'un microbe recombinant, comprenant la modification génétique de cellules microbiennes pour exprimer au moins une molécule d'acide nucléique recombinante comprenant une séquence d'acide nucléique codant pour au moins un domaine biologiquement actif d'un système PUFA PKS, dans lequel ledit au moins un domaine dudit système PUFA PKS est codé par une séquence d'acide nucléique selon l'une quelconque des revendications 1 à 7.
